# EUROPEAN PATENT APPLICATION

(11) **EP 3 730 619 A1**
(43) Date of publication of application: **28.10.2020**
(21) Application number: 20173519.8
(22) Date of filing: 23.06.2014
(51) Int. Cl.: C12N 15/113, A61K 31/7125, A61K 47/54, A61P 3/06

(54) **COMPOSITIONS AND METHODS FOR MODULATION OF TARGET NUCLEIC ACIDS**

(30) Priority: 21.06.2013 US 201361838190 P; 29.08.2013 US 201361871683 P
(62) Divisional of application: 19179457.7
(71) Applicant: Ionis Pharmaceuticals, Inc., Carlsbad, CA 92010 (US)
(72) Inventor: PRAKASH, Thazha, P., Carlsbad, CA 92010 (US); LIMA, Walter, F., Carlsbad, CA 92010 (US); KINBERGER, Garth, A., Carlsbad, CA 92010 (US); MURRAY, Heather, Carlsbad, CA 92010 (US); SWAYZE, Eric, E., Carlsbad, CA 92010 (US); CROOKE, Stanley, T., Carlsbad, CA 92010 (US)
(74) Representative: Holland, David Christopher

(57) **Abstract**

The present disclosure pertains generally to chemically-modified oligonucleotides for use in research, diagnostics, and/or therapeutics. In certain embodiments, the present disclosure describes compounds and methods for the modulation of a target nucleic acid. In certain embodiments, the present disclosure describes compounds and methods for the modulation of Apoliprotein C-III expression.

## Description

### SEQUENCE LISTING

The present application is being filed along with a Sequence Listing in electronic format. The Sequence Listing is provided as a file entitled CORE0116WOSEQ_ST25.txt, created on June 23, 2014, which is 48 Kb in size. The information in the electronic format of the sequence listing is incorporated herein by reference in its entirety.

### BACKGROUND OF THE INVENTION

### FIELD

The present disclosure pertains generally to chemically-modified oligonucleotides for use in research, diagnostics, and/or therapeutics. In certain embodiments, the present disclosure describes compounds and methods for the modulation of Apoliprotein C-III expression.

### BACKGROUND

The principle behind antisense technology is that an antisense compound hybridizes to a target nucleic acid and modulates the amount, activity, and/or function of the target nucleic acid. For example in certain instances, antisense compounds result in altered transcription or translation of a target. Such modulation of expression can be achieved by, for example, target mRNA degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi refers to antisense-mediated gene silencing through a mechanism that utilizes the RNA-induced siliencing complex (RISC). An additional example of modulation of RNA target function is by an occupancy-based mechanism such as is employed naturally by microRNA. MicroRNAs are small non-coding RNAs that regulate the expression of protein-coding RNAs. The binding of an antisense compound to a microRNA prevents that microRNA from binding to its messenger RNA targets, and thus interferes with the function of the microRNA. MicroRNA mimics can enhance native microRNA function. Certain antisense compounds alter splicing of pre-mRNA. Regardless of the specific mechanism, sequence-specificity makes antisense compounds attractive as tools for target validation and gene functionalization, as well as therapeutics to selectively modulate the expression of genes involved in the pathogenesis of diseases.

Antisense technology is an effective means for modulating the expression of one or more specific gene products and can therefore prove to be uniquely useful in a number of therapeutic, diagnostic, and research applications. Chemically modified nucleosides may be incorporated into antisense compounds to enhance one or more properties, such as nuclease resistance, pharmacokinetics or affinity for a target nucleic acid. In 1998, the antisense compound, Vitravene® (fomivirsen; developed by Isis Pharmaceuticals Inc., Carlsbad, CA) was the first antisense drug to achieve marketing clearance from the U.S. Food and Drug Administration (FDA), and is currently a treatment of cytomegalovirus (CMV)-induced retinitis in AIDS patients.

New chemical modifications have improved the potency and efficacy of antisense compounds, uncovering the potential for oral delivery as well as enhancing subcutaneous administration, decreasing potential for side effects, and leading to improvements in patient convenience. Chemical modifications increasing potency of antisense compounds allow administration of lower doses, which reduces the potential for toxicity, as well as decreasing overall cost of therapy. Modifications increasing the resistance to degradation result in slower clearance from the body, allowing for less frequent dosing. Different types of chemical modifications can be combined in one compound to further optimize the compound's efficacy.

### SUMMARY OF THE INVENTION

The present disclosure pertains generally to chemically-modified oligonucleotides for use in research, diagnostics, and/or therapeutics. In certain embodiments, the present disclosure describes compounds and methods for the modulation of Apoliprotein C-III expression. In certain embodiments, the present invention provides compounds and methods for the modulation of Apoliprotein C-III nucleic acids. The present invention includes, but is not limited to the following numbered embodiments:
Embodiment 1. A compound comprising a single-stranded oligonucleotide consisting of 13 to 30 linked nucleosides and having a nucleobase sequence comprising at least 8 contiguous nucleobases complementary to an equal-length portion within a target region of an Apolipoprotein C-III transcript, wherein the 5'-terminal nucleoside of the single-stranded oligonucleotide comprises a stabilized phosphate moiety and an internucleoside linking group linking the 5'-terminal nucleoside to the remainder of the oligonucleotide.
Embodiment 2. The compound of embodiment 1, wherein the compound comprises a conjugate group.
Embodiment 3. The compound of embodiment 1 or 2, wherein the conjugate group is attached to the oligonucleotide.
Embodiment 4. The compound of any of embodiments 1 to 3, wherein the conjugate group is attached to the oligonucleotide at a nucleoside at position 1, 2, 3, 4, 6, 7, 8, 9, 18, 19, 20, or 21 from the 5'-end of the oligonucleotide or at position 1, 2, 3, 12, 13, 14, 15, 17, 18, 19, 20, or 21 from the 3'-end of the oligonucleotide.
Embodiment 5. The compound of any of embodiments 1 to 4, wherein the conjugate group is attached to the oligonucleotide at a nucleoside at position 1 from the 5'-end of the oligonucleotide.
Embodiment 6. The compound of any of embodiments 1 to 4, wherein the conjugate group is attached to the oligonucleotide at a nucleoside at position 8 from the 5'-end of the oligonucleotide.
Embodiment 7. The compound of any of embodiments 1 to 6, wherein the Apolipoprotein C-III transcript comprises the nucleobase sequence as set forth in SEQ ID NO: 1.
Embodiment 8. The compound of any of embodiments 1 to 6, wherein the Apolipoprotein C-III transcript comprises the nucleobase sequence as set forth in SEQ ID NO: 2.
Embodiment 9. The compound of any of embodiments 1 to 8, wherein the complementary region comprises at least 10 contiguous nucleobases complementary to an equal-length portion within a target region of an Apolipoprotein C-III transcript.
Embodiment 10. The compound of any of embodiments 1 to 8, wherein the complementary region comprises at least 12 contiguous nucleobases complementary to an equal-length portion within a target region of an Apolipoprotein C-III transcript.
Embodiment 11. The compound of any of embodiments 1 to 8, wherein the complementary region comprises at least 14 contiguous nucleobases complementary to an equal-length portion within a target region of an Apolipoprotein C-III transcript.
Embodiment 12. The compound of any of embodiments 1 to 8, wherein the complementary region comprises at least 16 contiguous nucleobases complementary to an equal-length portion within a target region of an Apolipoprotein C-III transcript.
Embodiment 13. The compound of any of embodiments 1 to 8, wherein the complementary region comprises at least 18 contiguous nucleobases complementary to an equal-length portion within a target region of an Apolipoprotein C-III transcript.
Embodiment 14. The compound of any of embodiments 1 to 13, wherein the 5'-terminal nucleoside of the single-stranded oligonucleotide has Formula I: 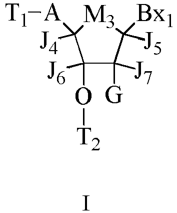 wherein:
   T₁ is a phosphorus moiety;
   T₂ is an internucleoside linking group linking the 5'-terminal nucleoside of Formula I to the remainder of the oligonucleotide;
   A has a formula selected from among: 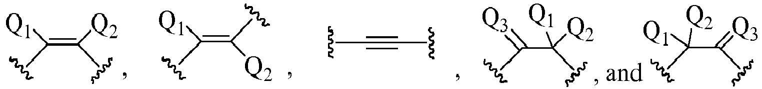
   Q₁ and Q₂ are each independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, substituted C₂-C₆ alkynyl, and N(R₃)(R₄);
   Q₃ is selected from among: O, S, N(R₅), and C(R₆)(R₇);
   each R₃, R₄ R₅, R₆ and R₇ is independently selected from among: H, C₁-C₆ alkyl, substituted C₁-C₆ alkyl, and C₁-C₆ alkoxy;
   M₃ is selected from among: O, S, NR₁₄, C(R₁₅)(R₁₆), C(R₁₅)(R₁₆)C(R₁₇)(R₁₈), C(R₁₅)=C(R₁₇), OC(R₁₅)(R₁₆), and OC(R₁₅)(Bx₂);
   R₁₄ is selected from among: H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   R₁₅, R₁₆, R₁₇ and R₁₈ are each independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   if Bx₂ is present, then Bx₂ is a nucleobase and Bx₁ is selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   if Bx₂ is not present, then Bx₁ is a nucleobase;
   either each of J₄, J₅, J₆ and J₇ is independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   or J₄ forms a bridge with one of J₅ or J₇ wherein the bridge comprises from 1 to 3 linked biradical groups selected from O, S, NR₁₉, C(R₂₀)(R₂₁), C(R₂₀)=C(R₂₁), C[=C(R₂₀)(R₂₁)] and C(=O) and the other two of J₅, J₆ and J₇ are independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   each R₁₉, R₂₀ and R₂₁ is independently selected from among: H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
   G is selected from among: H, OH, halogen, O-[C(R₈)(R₉)]ₙ-[(C=O)ₘ-Xₗ]ⱼ-Z, and a conjugate group;
   each R₈ and R₉ is independently selected from among: H, halogen, C₁-C₆ alkyl, and substituted C₁-C₆ alkyl;
   X₁ is O, S or N(E₁);
   Z is selected from among: H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl, and N(E₂)(E₃);
   E₁, E₂ and E₃ are each independently selected from among: H, C₁-C₆ alkyl, and substituted C₁-C₆ alkyl;
   n is from 1 to 6;
   m is 0 or 1;
   j is 0 or 1;
   provided that, if j is 1, then Z is other than halogen or N(E₂)(E₃);
   each substituted group comprises one or more optionally protected substituent groups independently selected from among: a halogen, OJ₁, N(J₁)(J₂), =NJ₁, SJ₁, N₃, CN, OC(=X₂)J₁, OC(=X₂)N(J₁)(J₂), and C(=X₂)N(J₁)(J₂);
   X₂ is O, S or NJ₃; and
   each J₁, J₂ and J₃ is independently selected from among: H and C₁-C₆ alkyl.
Embodiment 15. The compound of embodiment 14, wherein M₃ is selected from among: O, CH=CH, OCH₂, and OC(H)(Bx₂).
Embodiment 16. The compound of embodiment 14, wherein M₃ is O.
Embodiment 17. The compound of any of embodiments 14-16, wherein each of J₄, J₅, J₆ and J₇ is H.
Embodiment 18. The compound of any of embodiments 14-17, wherein J₄ forms a bridge with either J₅ or J₇.
Embodiment 19. The compound of any of embodiments 14-18, wherein A has the formula: 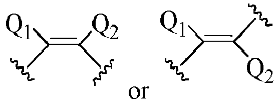 wherein:
   Q₁ and Q₂ are each independently selected from among: H, halogen, C₁-C₆ alkyl, substituted C₁-C₆ alkyl, C₁-C₆ alkoxy, and substituted C₁-C₆ alkoxy.
Embodiment 20. The compound of embodiment 19, wherein each of Q₁ and Q₂ is H.
Embodiment 21. The compound of embodiment 19, wherein Q₁ and Q₂ are each independently selected from among: H and a halogen.
Embodiment 22. The compound of embodiment 19, wherein one of Q₁ and Q₂ is H and the other of Q₁ and Q₂ is F, CH₃ or OCH₃.
Embodiment 23. The compound of any of embodiments 14 to 22, wherein T₁ has the formula: 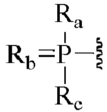 wherein:
   Rₐ and R_{c} are each independently selected from among: protected hydroxyl, protected thiol, C₁-C₆ alkyl, substituted C₁-C₆ alkyl, C₁-C₆ alkoxy, substituted C₁-C₆ alkoxy, protected amino or substituted amino; and
   R_{b} is O or S.
Embodiment 24. The compound of embodiment 23, wherein R_{b} is O and Rₐ and R_{c} are each, independently selected from among: OCH₃, OCH₂CH₃, OCH(CH₃)₂.
Embodiment 25. The compound of any of embodiments 14 to 24, wherein G is selected from among: a halogen, OCH₃, OCH₂F, OCHF₂, OCF₃, OCH₂CH₃, O(CH₂)₂F, OCH₂CHF₂, OCH₂CF₃, OCH₂-CH=CH₂, O(CH₂)₂-OCH₃, O(CH₂)₂-SCH₃, O(CH₂)₂-OCF₃, O(CH₂)₃N(R₁₀)(R₁₁), O(CH₂)₂-ON(R₁₀)(R₁₁), O(CH₂)₂-O(CH₂)₂-N(R₁₀)(R₁₁), OCH₂C(=O)-N(R₁₀)(R₁₁), OCH₂C(=O)-N(R₁₂)-(CH₂)₂-N(R₁₀)(R₁₁), and O(CH₂)₂-N(R₁₂)-C(=NR₁₃)[N(R₁₀)(R₁₁)]; wherein R₁₀, R₁₁, R₁₂ and R₁₃ are each, independently, H or C₁-C₆ alkyl.
Embodiment 26. The compound of any of embodiments 14-25, wherein G is selected from among: a halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂, and OCH₂-N(H)-C(=NH)NH₂.
Embodiment 27. The compound of any of embodiments 14-26, wherein G is selected from among: F, OCH₃, and O(CH₂)₂-OCH₃.
Embodiment 28. The compound of embodiment 27, wherein G is O(CH₂)₂-OCH₃.
Embodiment 29. The compound of any of embodiments 14-24, wherein G is a conjugate group.
Embodiment 30. The compound of embodiment 29, wherein the conjugate of the conjugate group is selected from among: cholesterol, palmityl, stearoyl, lithocholic-oleyl, C₂₂ alkyl, C₂₀ alkyl, C₁₆ alkyl, C₁₈ alkyl, and C₁₀ alkyl.
Embodiment 31. The compound of embodiment 30, wherein the conjugate group comprises C₁₆ alkyl.
Embodiment 32. The compound of any of embodiments 29 to 31, wherein the conjugate group comprises a linker.
Embodiment 33. The compound of embodiment 32, wherein the linker is selected from among: hexanamide, 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C₁-C₁₀ alkyl, substituted or unsubstituted C₂-C₁₀ alkenyl, and substituted or unsubstituted C₂-C₁₀ alkynyl.
Embodiment 34. The compound of any of embodiments 14-33, wherein the nucleobase is a modified nucleobase.
Embodiment 35. The compound of any of embodiments 14-34, wherein the nucleobase is a pyrimidine, substituted pyrimidine, purine or substituted purine.
Embodiment 36. The compound of any of embodiments 14-35, wherein the nucleobase is uracil, thymine, cytosine, 5-methylcytosine, adenine or guanine.
Embodiment 37. The compound of any of embodiments 14-36, wherein the 5'-terminal nucleoside of the single-stranded oligonucleotide has Formula III: 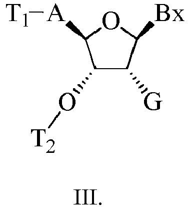
Embodiment 38. The compound of embodiment 37, wherein A has the formula: 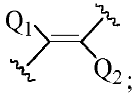 wherein Q₁ and Q₂ are each independently selected from among: H, a halogen, C₁-C₆ alkyl, substituted C₁-C₆ alkyl, C₁-C₆ alkoxy, and substituted C₁-C₆ alkoxy.
Embodiment 39. The compound of embodiment 38, wherein Q₁ and Q₂ are each independently selected from among: H, F, CH₃, and OCH₃.
Embodiment 40. The compound of any of embodiments 14-39, wherein the 5'-terminal nucleoside has Formula V: 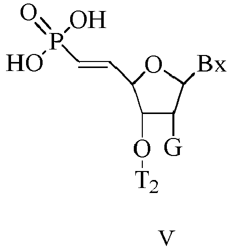 wherein:
   Bx is selected from among: uracil, thymine, cytosine, 5-methyl cytosine, adenine, and guanine;
   T₂ is a phosphorothioate internucleoside linking group linking the compound of Formula V to the remainder of the oligonucleotide; and
   G is selected from among: a halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂, OCH₂-N(H)-C(=NH)NH₂, and a conjugate group.
Embodiment 41. The compound of any of embodiments 1-40, wherein the remainder of the oligonucleotide comprises at least one RNA-like nucleoside.
Embodiment 42. The compound of embodiment 41, wherein essentially each nucleoside of the remainder of the oligonucleotide is an RNA-like nucleoside.
Embodiment 43. The compound of embodiment 42, wherein each nucleoside of the remainder of the oligonucleotide is an RNA-like nucleoside.
Embodiment 44. The compound of any of embodiments 41-43, wherein each RNA-like nucleoside is independently selected from among: a 2'-endo furanosyl nucleoside and an RNA-surrogate nucleoside.
Embodiment 45. The compound of embodiment 44, wherein each RNA-like nucleoside is a 2'-endo furanosyl nucleoside.
Embodiment 46. The compound of embodiment 45, wherein each RNA-like nucleoside is selected from among: 2'-F, 2'-MOE, 2'-OMe, LNA, F-HNA, and cEt.
Embodiment 47. The compound of any of embodiments 1-46, wherein the remainder of the oligonucleotide comprises at least one region having sugar motif:

   -[(A)ₓ-(B)_{y}-(A)_{z}]_{q}-

   wherein
   A is a modified nucleoside of a first type,
   B is a modified nucleoside of a second type;
   each x and each y is independently 1 or 2;
   z is 0 or 1;
   q is 1-15;
Embodiment 48. The compound of embodiment 47, wherein the modifications of the first type and the modifications of the second type are selected from among: 2'-F, 2'-OMe, and F-HNA.
Embodiment 49. The compound of embodiment 47, wherein the modifications of the first type are 2'-F and the modifications of the second type are 2'-OMe.
Embodiment 50. The compound of embodiment 47, wherein the modifications of the first type are 2'-OMe and the modifications of the second type are 2'-F.
Embodiment 51. The compound of any of embodiments 47 to 50, wherein each x and each y is 1.
Embodiment 52. The compound of any of embodiments 1-51, wherein the remainder of the oligonucleotide comprises 1-4 3'terminal nucleosides, each comprising the same sugar modification, wherein the sugar modification of the 1-4 3'terminal nucleosides is different from the sugar modification of the immediately adjacent nucleoside.
Embodiment 53. The compound of embodiment 52, wherein the 3'-terminal nucleosides are each 2'-MOE nucleosides.
Embodiment 54. The compound of embodiment 52 or 53 comprising two 3'-terminal nucleosides.
Embodiment 55. The compound of any of embodiments 1-54, comprising at least one modified internucleoside linkage.
Embodiment 56. The compound of embodiment 55, wherein each internucleoside linkage is selected from phosphorothioate and phosphodiester.
Embodiment 57. The compound of embodiment 55 or 56, wherein each of the 6-10 3'-most internucleoside linkages is phosphorothioate linkage.
Embodiment 58. The compound of any of embodiments 55 to 57, wherein the 5'-most internucleoside linkage is a phosphorothioate linkage.
Embodiment 59. The compound of any of embodiments 55 to 58, comprising a region of alternating linkages.
Embodiment 60. The compound of any of embodiments 1-59, comprising a 5'region having the motif:

   (Nucleoside of Formula I, III, or V) -s-(A-s-B-o-A)ₓ(-s-B)_{Y}

   wherein:
   A is a nucleoside of a first type;
   B is a nucleoside of a second type;
   s is a phosphorothioate linkage;
   o is a phosphodiester linkage;
   X is 1-8; and
   Y is 1 or 0.
Embodiment 61. The compound of any of embodiments 1-60, comprising a 3'region having the motif:

   -(A-s-B-s-A)_{z}(-s-B)_{q}-s-(D)-(s-D)ᵣ

   wherein:
   s is a phosphorothioate linkage;
   A is a nucleoside of a first type;
   B is a nucleoside of a second type;
   D is a nucleoside of a third type;
   Z is 1-5;
   q is 1 or 0; and
   and r is 0-3.
Embodiment 62. The compound embodiment 60 or 61, wherein A is a 2'-F nucleoside.
Embodiment 63. The compound of any of embodiments 60 to 62, wherein B is a 2'-OMe nucleoside.
Embodiment 64. The compound of any of embodiments 61 to 63, wherein D is a 2'-MOE nucleoside.
Embodiment 65. The compound of any of embodiments 61 to 64, wherein the oligonucleotide comprises a hybridizing region and a 3'-terminal region, wherein the hybridizing region comprisies nucleosides A and B and the terminal region comprising nucleosides D, wherein the hybridizing region is complementary to a target region of an Apoliprotein CIII transcript.
Embodiment 66. The compound of any of embodiments 1-60, comprising the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s

   wherein:
   s is a phosphorothioate linkage;
   A is a nucleoside of a first type;
   B is a nucleoside of a second type; and
   D is a nucleoside of a third type.
Embodiment 67. The compound of any of embodiments 1-60, consisting of the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s

   wherein:
   s is a phosphorothioate linkage;
   A is a nucleoside of a first type;
   B is a nucleoside of a second type; and
   D is a nucleoside of a third type.
Embodiment 68. The compound of embodiment 66 or 67, wherein A is a 2'-F nucleoside.
Embodiment 69. The compound of any of embodiments 66 to 68, wherein B is a 2'-OMe nucleoside.
Embodiment 70. The compound of any of embodiments 66 to 69, wherein D is a 2'-MOE nucleoside.
Embodiment 71. The compound of any of embodiments 1-70, wherein the remainder of the oligonucleotide comprises at least one conjugate group.
Embodiment 72. The compound of embodiment 71, wherein the conjugate of the conjugate group is selected from among: cholesterol, palmityl, stearoyl, lithocholic-oleyl, C₂₂ alkyl, C₂₀ alkyl, C₁₆ alkyl, C₁₈ alkyl, and C₁₀ alkyl.
Embodiment 73. The compound of embodiment 71, wherein the conjugate of the conjugate group is C₁₆ alkyl.
Embodiment 74. The compound of any of embodiments 71 to 73, wherein the conjugate group comprises a linker.
Embodiment 75. The compound of embodiment 74, wherein the linker is selected from among: hexanamide, 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C₁-C₁₀ alkyl, substituted or unsubstituted C₂-C₁₀ alkenyl, and substituted or unsubstituted C₂-C₁₀ alkynyl.
Embodiment 76. The compound of embodiment 74, wherein the linker is hexanamide.
Embodiment 77. The compound of any of embodiments 1-63, wherein the oligonucleotide has two mismatches relative to a target region of the Apolipoprotein C-III transcript.
Embodiment 78. The compound of any of embodiments 1-63, wherein the oligonucleotide has three mismatches relative to a target region of the Apolipoprotein C-III transcript.
Embodiment 79. The compound of any of embodiments 1-63, wherein the oligonucleotide has four mismatches relative to a target region of the Apolipoprotein C-III transcript.
Embodiment 80. The compound of any of embodiments 1-79, wherein the oligonucleotide comprises a hybridizing region and 0-4 3'-terminal nucleosides.
Embodiment 81. The compound of any of embodiments 1-79, wherein the oligonucleotide comprises a hybridizing region and 1-4 3'-terminal nucleosides.
Embodiment 82. The compound of embodiment 80 or 81, wherein the hybridizing region is 100% complementary to a target region of the Apolipoprotein C-III transcript.
Embodiment 83. The compound of embodiment 80 or 81, wherein the hybridizing region has one mismatch relative to a target region of the Apolipoprotein C-III transcript.
Embodiment 84. The compound of embodiment 80 or 81, wherein the hybridizing region has two mismatches relative a target region of the Apolipoprotein C-III transcript.
Embodiment 85. The compound of embodiment 80 or 81 wherein the hybridizing region has three mismatches relative to a target region of the Apolipoprotein C-III transcript.
Embodiment 86. The compound of embodiment 80 or 81 wherein the hybridizing region has four mismatches relative to a target region of the Apolipoprotein C-III transcript.
Embodiment 87. The compound of any of embodiments 81-86, wherein one or more of the 3'-terminal nucleosides is not complementary to the target RNA.
Embodiment 88. The compound of any of embodiments 81-87, wherein the nucleobase of each 3'-terminal nucleoside is a purine.
Embodiment 89. The compound of embodiment 88, wherein the nucleobase of each 3'-terminal nucleoside is an adenine.
Embodiment 90. The compound of any of embodiments 1-89, wherein the oligonucleotide comprises at least one modified nucleobase.
Embodiment 91. The compound of any of embodiments 1-90, wherein each cytosine residue comprises a 5-methylcytosine.
Embodiment 92. The compound of any of embodiments 1-90, wherein the nucleobase sequence of the oligonucleotide comprises a nucleobase sequence selected from among: SEQ ID NO: 3, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, or 86.
Embodiment 93. The compound of any of embodiments 1-90, wherein the nucleobase sequence of the oligonucleotide comprises the nucleobase sequence of SEQ ID NO: 3.
Embodiment 94. The compound of any of embodiments 1-90, wherein the nucleobase sequence of the oligonucleotide consists of a nucleobase sequence selected from among: SEQ ID NO: 3, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, or 86.
Embodiment 95. The compound of any of embodiments 1-90, wherein the nucleobase sequence of the oligonucleotide consists of the nucleobase sequence of SEQ ID NO: 3.
Embodiment 96. The compound of embodiment 1, wherein the compound comprises ISIS No. 594290.
Embodiment 97. The compound of embodiment 1, wherein the compound comprises ISIS No. 594231.
Embodiment 98. A method of reducing the activity or amount of an Apolipoprotein C-III transcript in a cell, comprising contacting a cell with at least one compound of any of embodiments 1 to 97; and thereby reducing the activity or amount of the Apolipoprotein C-III transcript in the cell.
Embodiment 99. The method of embodiment 98, wherein the Apolipoprotein C-III transcript is Apolipoprotein C-III pre-mRNA.
Embodiment 100. The method of embodiment 98, wherein the Apolipoprotein C-III transcript is Apolipoprotein C-III mRNA.
Embodiment 101. The method of any of embodiments 98 to 100, wherein the cell is in vitro.
Embodiment 102. The method of any of embodiments 98 to 100, wherein the cell is in an animal.
Embodiment 103. The method of embodiment 102, wherein the animal is a human.
Embodiment 104. A method of reducing the activity or amount of an Apolipoprotein C-III protein in a cell, comprising contacting a cell with at least one compound of any of embodiments 1 to 97; and thereby reducing the activity or amount of the Apolipoprotein C-III protein in the cell.
Embodiment 105. The method of embodiment 104, wherein the cell is in vitro.
Embodiment 106. The method of embodiment 104, wherein the cell is in an animal.
Embodiment 107. The method of embodiment 106, wherein the animal is a human.
Embodiment 108. A method of decreasing total cholesterol, comprising contacting a cell with at least one compound of any of embodiments 1 to 97; and thereby decreasing total cholesterol.
Embodiment 109. The method of embodiment 108, wherein the cell is in vitro.
Embodiment 110. The method of embodiment 108, wherein the cell is in an animal.
Embodiment 111. The method of embodiment 110, wherein the animal is a human.
Embodiment 112. A method of decreasing triglycerides, comprising contacting a cell with at least one compound of any of embodiments 1 to 97; and thereby decreasing triglycerides.
Embodiment 113. The method of embodiment 112, wherein the cell is in vitro.
Embodiment 114. The method of embodiment 112, wherein the cell is in an animal.
Embodiment 115. The method of embodiment 112, wherein the animal is a human.
Embodiment 116. A method of lowering LDL, comprising contacting a cell with at least one compound of any of embodiments 1 to 97; and thereby lowering LDL.
Embodiment 117. The method of embodiment 116, wherein the cell is in vitro.
Embodiment 118. The method of embodiment 116, wherein the cell is in an animal.
Embodiment 119. The method of embodiment 118, wherein the animal is a human.
Embodiment 120. A method of increasing HDL, comprising contacting a cell with at least one compound of any of embodiments 1 to 97; and thereby increasing HDL.
Embodiment 121. The method of embodiment 120, wherein the cell is in vitro.
Embodiment 122. The method of embodiment 120, wherein the cell is in an animal.
Embodiment 123. The method of embodiment 122, wherein the animal is a human.
Embodiment 124. A pharmaceutical composition comprising at least one compound of any of embodiments 1-97 and a pharmaceutically acceptable carrier or diluent.
Embodiment 125. Use of a compound of any of embodiments 1 to 97 or the pharmaceutical composition of embodiment 124 for the manufacture of a medicament for use in treatment of a disease.

In certain embodiments, compounds and methods disclosed herein are useful for treating diseases or conditions associated with Apolipoprotein C-III. In certain such diesaes or conditions, the expression, amount, or concentration of Apolipoprotein C-III protien in a patient is mis-regulated, for example is abnormally high. In certain embodiments, the expression, amount, or concentration of Apolipoprotein C-III protein in a patient is not abnormal. In such embodiments, it may nevertheless be therapeutically beneficial to reduce Apolipoprotein C-III protein. In certain embodiments Apolipoprotein C-III protein is reduced to a level below what is ordinarily considered a normal level.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated by reference in their entirety for any purpose.

### A. Definitions

Unless specific definitions are provided, the nomenclature used in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society, Washington D.C., 1994; "Remington's Pharmaceutical Sciences," Mack Publishing Co., Easton, Pa., 21st edition, 2005; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989, which are hereby incorporated by reference for any purpose. Where permitted, all patents, applications, published applications and other publications and other data referred to throughout in the disclosure are incorporated by reference herein in their entirety.

Unless otherwise indicated, the following terms have the following meanings:
As used herein, "nucleoside" means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.
As used herein, "chemical modification" means a chemical difference in a compound when compared to a naturally occurring counterpart. Chemical modifications of oligonucleotides include nucleoside modifications (including sugar moiety modifications and nucleobase modifications) and internucleoside linkage modifications. In reference to an oligonucleotide, chemical modification does not include differences only in nucleobase sequence.
As used herein, "furanosyl" means a structure comprising a 5-membered ring comprising four carbon atoms and one oxygen atom.
As used herein, "naturally occurring sugar moiety" means a ribofuranosyl as found in naturally occurring RNA or a deoxyribofuranosyl as found in naturally occurring DNA.
As used herein, "sugar moiety" means a naturally occurring sugar moiety or a modified sugar moiety of a nucleoside.
As used herein, "modified sugar moiety" means a substituted sugar moiety or a sugar surrogate.
As used herein, "substituted sugar moiety" means a furanosyl that is not a naturally occurring sugar moiety. Substituted sugar moieties include, but are not limited to furanosyls comprising substituents at the 2'-position, the 3'-position, the 5'-position and/or the 4'-position. Certain substituted sugar moieties are bicyclic sugar moieties.
As used herein, "2'-substituted sugar moiety" means a furanosyl comprising a substituent at the 2'-position other than H or OH. Unless otherwise indicated, a 2'-substituted sugar moiety is not a bicyclic sugar moiety (i.e., the 2'-substituent of a 2'-substituted sugar moiety does not form a bridge to another atom of the furanosyl ring.
As used herein, "MOE" means -OCH₂CH₂OCH₃.
As used herein, "2'-F nucleoside" refers to a nucleoside comprising a sugar comprising fluoroine at the 2' position. Unless otherwise indicated, the fluorine in a 2'-F nucleoside is in the ribo position (replacing the OH of a natural ribose).
As used herein, "2'-F ANA" refers to a 2'-F substituted nucleoside, wherein the fluoro group is in the arabino position. 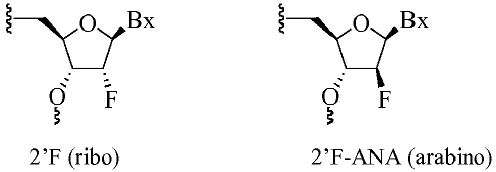
As used herein the term "sugar surrogate" means a structure that does not comprise a furanosyl and that is capable of replacing the naturally occurring sugar moiety of a nucleoside, such that the resulting nucleoside sub-units are capable of linking together and/or linking to other nucleosides to form an oligomeric compound which is capable of hybridizing to a complementary oligomeric compound. Such structures include rings comprising a different number of atoms than furanosyl (e.g., 4, 6, or 7-membered rings); replacement of the oxygen of a furanosyl with a non-oxygen atom (e.g., carbon, sulfur, or nitrogen); or both a change in the number of atoms and a replacement of the oxygen. Such structures may also comprise substitutions corresponding to those described for substituted sugar moieties (e.g., 6-membered carbocyclic bicyclic sugar surrogates optionally comprising additional substituents). Sugar surrogates also include more complex sugar replacements (e.g., the non-ring systems of peptide nucleic acid). Sugar surrogates include without limitation morpholinos, cyclohexenyls and cyclohexitols.
As used herein, "bicyclic sugar moiety" means a modified sugar moiety comprising a 4 to 7 membered ring (including but not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments the 4 to 7 membered ring is a furanosyl. In certain such embodiments, the bridge connects the 2'-carbon and the 4'-carbon of the furanosyl.
As used herein, "nucleotide" means a nucleoside further comprising a phosphate linking group. As used herein, "linked nucleosides" may or may not be linked by phosphate linkages and thus includes, but is not limited to "linked nucleotides." As used herein, "linked nucleosides" are nucleosides that are connected in a continuous sequence (i.e. no additional nucleosides are present between those that are linked).
As used herein, "nucleobase" means a group of atoms that can be linked to a sugar moiety to create a nucleoside that is capable of incorporation into an oligonucleotide, and wherein the group of atoms is capable of bonding with a complementary naturally occurring nucleobase of another oligonucleotide or nucleic acid. Nucleobases may be naturally occurring or may be modified.
As used herein the terms, "unmodified nucleobase" or "naturally occurring nucleobase" means the naturally occurring heterocyclic nucleobases of RNA or DNA: the purine bases adenine (A) and guanine (G), and the pyrimidine bases thymine (T), cytosine (C) (including 5-methyl C), and uracil (U).
As used herein, "modified nucleobase" means any nucleobase that is not a naturally occurring nucleobase.
As used herein, "modified nucleoside" means a nucleoside comprising at least one chemical modification compared to naturally occurring RNA or DNA nucleosides. Modified nucleosides comprise a modified sugar moiety and/or a modified nucleobase.
As used herein, "bicyclic nucleoside" or "BNA" means a nucleoside comprising a bicyclic sugar moiety.
As used herein, "constrained ethyl nucleoside" or "cEt" means a nucleoside comprising a bicyclic sugar moiety comprising a 4'-CH(CH₃)-O-2'bridge.
As used herein, "locked nucleic acid nucleoside" or "LNA" means a nucleoside comprising a bicyclic sugar moiety comprising a 4'-CH₂-O-2'bridge.
As used herein, "2'-substituted nucleoside" means a nucleoside comprising a substituent at the 2'-position other than H or OH. Unless otherwise indicated, a 2'-substituted nucleoside is not a bicyclic nucleoside.
As used herein, "2'-deoxynucleoside" means a nucleoside comprising 2'-H furanosyl sugar moiety, as found in naturally occurring deoxyribonucleosides (DNA). In certain embodiments, a 2'-deoxynucleoside may comprise a modified nucleobase or may comprise an RNA nucleobase (e.g., uracil).
As used herein, "RNA-like nucleoside" means a modified nucleoside that adopts a northern configuration and functions like RNA when incorporated into an oligonucleotide. RNA-like nucleosides include, but are not limited to 2'-endo furanosyl nucleosides and RNA surrogates.
As used herein, "2'-endo-furanosyl nucleoside" means an RNA-like nucleoside that comprises a substituted sugar moiety that has a 2'-endo conformation. 2'-endo-furanosyl nucleosides include, but are not limitied to: 2'-MOE, 2'-F, 2'-OMe, LNA, ENA, and cEt nucleosides.
As used herein, "RNA-surrogate nucleoside" means an RNA-like nucleoside that does not comprise a furanosyl. RNA-surrogate nucleosides include, but are not limited to hexitols and cyclopentanes.
As used herein, "phosphorous moiety" refers to a to monovalent P^{V} phosphorus radical group. In certain embodiments, a phosphorus moiety is selected from: a phosphate, phosphonate, alkylphosphonate, aminoalkyl phosphonate, phosphorothioate, phosphoramidite, alkylphosphonothioate, phosphorodithioate, thiophosphoramidate, phosphotriester and the like. In certain embodiments, modified phosphorous moieties have the following structural formula: 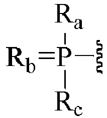 wherein:
   Rₐ and R_{c} are each, independently, OH, SH, C₁-C₆ alkyl, substituted C₁-C₆ alkyl, C₁-C₆ alkoxy, substituted C₁-C₆ alkoxy, amino or substituted amino; and
   R_{b} is O or S.
The term "phosphate moiety" as used herein, refers to a terminal phosphate group that includes unmodified phosphates (-O-P(=O)(OH)OH) as well as modified phosphates. Modified phosphates include but are not limited to phosphates in which one or more of the O and OH groups is replaced with H, O, S, N(R) or alkyl where R is H, an amino protecting group or unsubstituted or substituted alkyl.
As used herein, "phosphate stabilizing modification" refers to a modification that results in stabilization of a 5'-phosphate moiety of the 5'-terminal nucleoside of an oligonucleotide, relative to the stability of an unmodified 5'-phosphate of an unmodified nucleoside under biologic conditions. Such stabilization of a 5'-phophate group includes but is not limited to resistance to removal by phosphatases. Phosphate stabilizing modifications include, but are not limited to, modification of one or more of the atoms that binds directly to the phosphorus atom, modification of one or more atoms that link the phosphorus to the 5'-carbon of the nucleoside, and modifications at one or more other positions of the nucleoside that result in stabilization of the phosphate. In certain embodiments, a phosphate stabilizing modification comprises a carbon linking the phosphorous atom to the 5'-carbon of the sugar. Phosphate moieties that are stabilized by one or more phosphate stabilizing modification are refered to herein as "stabilized phosphate moieties."
As used herein, "oligonucleotide" means a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.
As used herein "oligonucleoside" means an oligonucleotide in which none of the internucleoside linkages contains a phosphorus atom. As used herein, oligonucleotides include oligonucleosides.
As used herein, "modified oligonucleotide" means an oligonucleotide comprising at least one modified nucleoside and/or at least one modified internucleoside linkage.
As used herein "internucleoside linkage" means a covalent linkage between adjacent nucleosides in an oligonucleotide.
As used herein "naturally occurring internucleoside linkage" means a 3' to 5' phosphodiester linkage.
As used herein, "modified internucleoside linkage" means any internucleoside linkage other than a naturally occurring internucleoside linkage.
As used herein, "oligomeric compound" means a polymeric structure comprising two or more substructures. In certain embodiments, an oligomeric compound comprises an oligonucleotide. In certain embodiments, an oligomeric compound comprises one or more conjugate groups and/or terminal groups. In certain embodiments, an oligomeric compound consists of an oligonucleotide. Oligomeric compounds also include naturally occurring nucleic acids.
As used herein, "terminal group" means one or more atom attached to either, or both, the 3' end or the 5' end of an oligonucleotide. In certain embodiments a terminal group is a conjugate group. In certain embodiments, a terminal group comprises one or more terminal group nucleosides.
As used herein, "conjugate" means an atom or group of atoms bound to an oligonucleotide or oligomeric compound. In general, conjugate groups modify one or more properties of the compound to which they are attached, including, but not limited to pharmacodynamic, pharmacokinetic, binding, absorption, cellular distribution, cellular uptake, charge and/or clearance properties.
As used herein, "conjugate linking group" means any atom or group of atoms used to attach a conjugate to an oligonucleotide or oligomeric compound.
As used herein, "single-stranded" means an oligomeric compound that is not hybridized to its complement and which lacks sufficient self-complementarity to form a stable self-duplex.
As used herein, "antisense compound" means a compound comprising or consisting of an oligonucleotide at least a portion of which is complementary to a target nucleic acid to which it is capable of hybridizing, resulting in at least one antisense activity.
As used herein, "antisense activity" means any detectable and/or measurable change attributable to the hybridization of an antisense compound to its target nucleic acid.
As used herein, "detecting" or "measuring" means that a test or assay for detecting or measuring is performed. Such detection and/or measuring may result in a value of zero. Thus, if a test for detection or measuring results in a finding of no activity (activity of zero), the step of detecting or measuring the activity has nevertheless been performed.
As used herein, "detectable and/or measureable activity" means a statistically significant activity that is not zero.
As used herein, "essentially unchanged" means little or no change in a particular parameter, particularly relative to another parameter which changes much more. In certain embodiments, a parameter is essentially unchanged when it changes less than 5%. In certain embodiments, a parameter is essentially unchanged if it changes less than two-fold while another parameter changes at least ten-fold. For example, in certain embodiments, an antisense activity is a change in the amount of a target nucleic acid. In certain such embodiments, the amount of a non-target nucleic acid is essentially unchanged if it changes much less than the target nucleic acid does, but the change need not be zero.
As used herein, "expression" means the process by which a gene ultimately results in a protein. Expression includes, but is not limited to, transcription, post-transcriptional modification (e.g., splicing, polyadenlyation, addition of 5'-cap), and translation.
As used herein, "target nucleic acid" means a nucleic acid molecule to which an antisense compound hybridizes.
As used herein, "targeting" or "targeted to" means the association of an antisense compound to a particular target nucleic acid molecule or a particular region of a target nucleic acid molecule. An antisense compound targets a target nucleic acid if it is sufficiently complementary to the target nucleic acid to allow hybridization under physiological conditions.
As used herein, "selectivity" refers to the ability of an antisense compound to exert an antisense activity on a target nucleic acid to a greater extent than on a non-target nucleic acid.
As used herein, "nucleobase complementarity" or "complementarity" when in reference to nucleobases means a nucleobase that is capable of base pairing with another nucleobase. For example, in DNA, adenine (A) is complementary to thymine (T). For example, in RNA, adenine (A) is complementary to uracil (U). In certain embodiments, complementary nucleobase means a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair. Nucleobases comprising certain modifications may maintain the ability to pair with a counterpart nucleobase and thus, are still capable of nucleobase comp lementarity.
As used herein, "non-complementary" in reference to nucleobases means a pair of nucleobases that do not form hydrogen bonds with one another.
As used herein, "complementary" in reference to oligomeric compounds (e.g., linked nucleosides, oligonucleotides, or nucleic acids) means the capacity of such oligomeric compounds or regions thereof to hybridize to another oligomeric compound or region thereof through nucleobase complementarity. Complementary oligomeric compounds need not have nucleobase complementarity at each nucleoside. Rather, some mismatches are tolerated. In certain embodiments, complementary oligomeric compounds or regions are complementary at 70% of the nucleobases (70% complementary). In certain embodiments, complementary oligomeric compounds or regions are 80% complementary. In certain embodiments, complementary oligomeric compounds or regions are 90% complementary. In certain embodiments, complementary oligomeric compounds or regions are 95% complementary. In certain embodiments, complementary oligomeric compounds or regions are 100% complementary.
As used herein, "mismatch" means a nucleobase of a first oligomeric compound that is not capable of pairing with a nucleobase at a corresponding position of a second oligomeric compound, when the first and second oligomeric compound are aligned. Either or both of the first and second oligomeric compounds may be oligonucleotides.
As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleobases.
As used herein, "specifically hybridizes" means the ability of an oligomeric compound to hybridize to one nucleic acid site with greater affinity than it hybridizes to another nucleic acid site.
As used herein, "fully complementary" in reference to an oligonucleotide or portion thereof means that each nucleobase of the oligonucleotide or portion thereof is capable of pairing with a nucleobase of a complementary nucleic acid or contiguous portion thereof. Thus, a fully complementary region comprises no mismatches or unhybridized nucleobases in either strand.
As used herein, "percent complementarity" means the percentage of nucleobases of an oligomeric compound that are complementary to an equal-length portion of a target nucleic acid. Percent complementarity is calculated by dividing the number of nucleobases of the oligomeric compound that are complementary to nucleobases at corresponding positions in the target nucleic acid by the total length of the oligomeric compound.
As used herein, "percent identity" means the number of nucleobases in a first nucleic acid that are the same type (independent of chemical modification) as nucleobases at corresponding positions in a second nucleic acid, divided by the total number of nucleobases in the first nucleic acid.
As used herein, "modulation" means a change of amount or quality of a molecule, function, or activity when compared to the amount or quality of a molecule, function, or activity prior to modulation. For example, modulation includes the change, either an increase (stimulation or induction) or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.
As used herein, "motif" means a pattern of chemical modifications in an oligonucleotide or a region thereof. Motifs may be defined by modifications at certain nucleosides and/or at certain linking groups of an oligonucleotide.
As used herein, "nucleoside motif" means a pattern of nucleoside modifications in an oligonucleotide or a region thereof. The linkages of such an oligonucleotide may be modified or unmodified. Unless otherwise indicated, motifs herein describing only nucleosides are intended to be nucleoside motifs. Thus, in such instances, the linkages are not limited.
As used herein, "sugar motif" means a pattern of sugar modifications in an oligonucleotide or a region thereof.
As used herein, "linkage motif" means a pattern of linkage modifications in an oligonucleotide or region thereof. The nucleosides of such an oligonucleotide may be modified or unmodified. Unless otherwise indicated, motifs herein describing only linkages are intended to be linkage motifs. Thus, in such instances, the nucleosides are not limited.
As used herein, "nucleobase modification motif" means a pattern of modifications to nucleobases along an oligonucleotide. Unless otherwise indicated, a nucleobase modification motif is independent of the nucleobase sequence.
As used herein, "sequence motif" means a pattern of nucleobases arranged along an oligonucleotide or portion thereof. Unless otherwise indicated, a sequence motif is independent of chemical modifications and thus may have any combination of chemical modifications, including no chemical modifications.
As used herein, "type of modification" in reference to a nucleoside or a nucleoside of a "type" means the chemical modification of a nucleoside and includes modified and unmodified nucleosides. Accordingly, unless otherwise indicated, a "nucleoside having a modification of a first type" may be an unmodified nucleoside.
As used herein, "differently modified" mean chemical modifications or chemical substituents that are different from one another, including absence of modifications. Thus, for example, a MOE nucleoside and an unmodified DNA nucleoside are "differently modified," even though the DNA nucleoside is unmodified. Likewise, DNA and RNA are "differently modified," even though both are naturally-occurring unmodified nucleosides. Nucleosides that are the same but for comprising different nucleobases are not differently modified. For example, a nucleoside comprising a 2'-OMe modified sugar and an unmodified adenine nucleobase and a nucleoside comprising a 2'-OMe modified sugar and an unmodified thymine nucleobase are not differently modified.
As used herein, "the same type of modifications" refers to modifications that are the same as one another, including absence of modifications. Thus, for example, two unmodified DNA nucleosides have "the same type of modification," even though the DNA nucleoside is unmodified. Such nucleosides having the same type modification may comprise different nucleobases.
As used herein, "separate regions" means portions of an oligonucleotide wherein the chemical modifications or the motif of chemical modifications of any neighboring portions include at least one difference to allow the separate regions to be distinguished from one another.
As used herein, "pharmaceutically acceptable carrier or diluent" means any substance suitable for use in administering to an animal. In certain embodiments, a pharmaceutically acceptable carrier or diluent is sterile saline. In certain embodiments, such sterile saline is pharmaceutical grade saline.
As used herein, "substituent" and "substituent group," means an atom or group that replaces the atom or group of a named parent compound. For example a substituent of a modified nucleoside is any atom or group that differs from the atom or group found in a naturally occurring nucleoside (e.g., a modified 2'-substuent is any atom or group at the 2'-position of a nucleoside other than H or OH). Substituent groups can be protected or unprotected. In certain embodiments, compounds of the present invention have substituents at one or at more than one position of the parent compound. Substituents may also be further substituted with other substituent groups and may be attached directly or via a linking group such as an alkyl or hydrocarbyl group to a parent compound.
Likewise, as used herein, "substituent" in reference to a chemical functional group means an atom or group of atoms that differs from the atom or a group of atoms normally present in the named functional group. In certain embodiments, a substituent replaces a hydrogen atom of the functional group (e.g., in certain embodiments, the substituent of a substituted methyl group is an atom or group other than hydrogen which replaces one of the hydrogen atoms of an unsubstituted methyl group). Unless otherwise indicated, groups amenable for use as substituents include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxy (-O-Rₐₐ), aryl, aralkyl, heterocyclic radical, heteroaryl, heteroarylalkyl, amino (-N(R_{bb})(R_{cc})), imino(=NR_{bb}), amido (-C(O)N(R_{bb})(R_{cc}) or -N(R_{bb})C(O)Rₐₐ), azido (-N₃), nitro (-NO₂), cyano (-CN), carbamido (-OC(O)N(R_{bb})(R_{cc}) or -N(R_{bb})C(O)ORₐₐ), ureido (-N(R_{bb})C(O)N(R_{bb})(R_{cc})), thioureido (-N(R_{bb})C(S)N(R_{bb})-(R_{cc})), guanidinyl (-N(R_{bb})C(=NR_{bb})N(R_{bb})(R_{cc})), amidinyl (-C(=NR_{bb})N(R_{bb})(R_{cc}) or -N(R_{bb})C(=NR_{bb})(Rₐₐ)), thiol (-SR_{bb}), sulfinyl (-S(O)R_{bb}), sulfonyl (-S(O)₂R_{bb}) and sulfonamidyl (-S(O)₂N(R_{bb})(R_{cc}) or -N(R_{bb})S-(O)₂R_{bb}). Wherein each Rₐₐ, R_{bb} and R_{cc} is, independently, H, an optionally linked chemical functional group or a further substituent group with a preferred list including without limitation, alkyl, alkenyl, alkynyl, aliphatic, alkoxy, acyl, aryl, aralkyl, heteroaryl, alicyclic, heterocyclic and heteroarylalkyl. Selected substituents within the compounds described herein are present to a recursive degree.
As used herein, "alkyl," as used herein, means a saturated straight or branched hydrocarbon radical containing up to twenty four carbon atoms. Examples of alkyl groups include without limitation, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred.
As used herein, "alkenyl," means a straight or branched hydrocarbon chain radical containing up to twenty four carbon atoms and having at least one carbon-carbon double bond. Examples of alkenyl groups include without limitation, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.
As used herein, "alkynyl," means a straight or branched hydrocarbon radical containing up to twenty four carbon atoms and having at least one carbon-carbon triple bond. Examples of alkynyl groups include, without limitation, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substituent groups.
As used herein, "acyl," means a radical formed by removal of a hydroxyl group from an organic acid and has the general Formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substituent groups.
As used herein, "alicyclic" means a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substituent groups.
As used herein, "aliphatic" means a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation, polyalkoxys, such as polyalkylene glycols, polyamines, and polyimines. Aliphatic groups as used herein may optionally include further substituent groups.
As used herein, "alkoxy" means a radical formed between an alkyl group and an oxygen atom wherein the oxygen atom is used to attach the alkoxy group to a parent molecule. Examples of alkoxy groups include without limitation, methoxy, ethoxy, propoxy, isopropoxy, n-butoxy, sec-butoxy, tert-butoxy, n-pentoxy, neopentoxy, n-hexoxy and the like. Alkoxy groups as used herein may optionally include further substituent groups.
As used herein, "aminoalkyl" means an amino substituted C₁-C₁₂ alkyl radical. The alkyl portion of the radical forms a covalent bond with a parent molecule. The amino group can be located at any position and the aminoalkyl group can be substituted with a further substituent group at the alkyl and/or amino portions.
As used herein, "aralkyl" and "arylalkyl" mean an aromatic group that is covalently linked to a C₁-C₁₂ alkyl radical. The alkyl radical portion of the resulting aralkyl (or arylalkyl) group forms a covalent bond with a parent molecule. Examples include without limitation, benzyl, phenethyl and the like. Aralkyl groups as used herein may optionally include further substituent groups attached to the alkyl, the aryl or both groups that form the radical group.
As used herein, "aryl" and "aromatic" mean a mono- or polycyclic carbocyclic ring system radicals having one or more aromatic rings. Examples of aryl groups include without limitation, phenyl, naphthyl, tetrahydronaphthyl, indanyl, idenyl and the like. Preferred aryl ring systems have from about 5 to about 20 carbon atoms in one or more rings. Aryl groups as used herein may optionally include further substituent groups.
As used herein, "halo" and "halogen," mean an atom selected from fluorine, chlorine, bromine and iodine.
As used herein, "heteroaryl," and "heteroaromatic," mean a radical comprising a mono- or polycyclic aromatic ring, ring system or fused ring system wherein at least one of the rings is aromatic and includes one or more heteroatoms. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include without limitation, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl and the like. Heteroaryl radicals can be attached to a parent molecule directly or through a linking moiety such as an aliphatic group or hetero atom. Heteroaryl groups as used herein may optionally include further substituent groups.
As used herein, "parenteral administration," means administration through injection or infusion. Parenteral administration includes, but is not limited to, subcutaneous administration, intravenous administration, or intramuscular administration.
As used herein, "systemic administration" means administration to an area other than the intended locus of activity. Examples or systemic administration are subcutaneous administration and intravenous administration, and intraperitoneal administration.
As used herein, "subcutaneous administration" means administration just below the skin.
As used herein, "intravenous administration" means administration into a vein.
As used herein, "cerebrospinal fluid" or "CSF" means the fluid filling the space around the brain and spinal cord.
As used herein, "administration into the cerebrospinal fluid" means any administration that delivers a substance directly into the CSF.
As used herein, "intracerebroventricular" or "ICV" mean administration into the ventricular system of the brain.
As used herein, "intrathecal" or "IT" means administration into the CSF under the arachnoid membrane which covers the brain and spinal cord. IT injection is performed through the theca of the spinal cord into the subarachnoid space, where a pharmaceutical agent is injected into the sheath surrounding the spinal cord.
As used herein, "Apo CIII transcript" means a transcript transcribed from an Apo CIII gene. In certain embodiments, an Apo CIII transcript comprises SEQ ID NO: 1: the sequence of GENBANK® Accession No. NT_033899.8 truncated from nucleobases 20262640 to 20266603. In certain embodiments, an Apo CIII transcript comprises SEQ ID NO: 2: having the sequence of GENBANK® Accession No. NM_000040.1.
As used herein, "Apo CIII gene" means a gene that encodes an apoliprotein CIII protein and any apoliprotein CIII protein isoforms.

### B. Certain Compounds

In certain embodiments, the present invention provides compounds useful for studying, diagnosing, and/or treating a disease or disorder associated high triglycerides, high LDL, or diabetes. In certain embodiments, compounds of the present invention comprise an oligonucleotide and a conjugate and/or terminal group. In certain embodiments, compounds consist of an oligonucleotide.

In certain embodiments, an oligonucleotide of the present invention has a nucleobase sequence comprising a region that is complementary to an Apo CIII transcript. In certain embodiments, such oligonucleotides comprise one or more modifications.

### a. Certain 5'-Terminal Nucleosides

In certain embodiments, compounds of the present invention comprise oligonucleotides comprising a stabilized phosphate moiety at the 5'-terminus. In certain such embodiments, the phosphorus atom of the stabilized phosphate moiety is attached to the 5'-terminal nucleoside through a phosphorus-carbon bond. In certain embodiments, the carbon of that phosphorus -carbon bond is in turn bound to the 5'-position of the nucleoside.

In certain embodiments, the oligonucleotide comprises a 5'-stabilized phosphate moiety having the following formula: 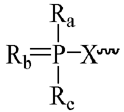 wherein:
Rₐ and R_{c} are each, independently, OH, SH, C₁-C₆ alkyl, substituted C₁-C₆ alkyl, C₁-C₆ alkoxy, substituted C₁-C₆ alkoxy, amino or substituted amino;
R_{b} is O or S;
X is substituted or unsubstitued C; and wherein X is attached to the 5'-terminal nucleoside. In certain embodiments, X is bound to an atom at the 5'-position of the the 5'-terminal nucleoside. In ceratin such embodiments, the 5'-atom is a carbon and the bond between X and the 5'-carbon of the the 5'-terminal nucleoside is a carbon-carbon single bond. In certain bemodiments, it is a carbon-carbon double bond. In certain nembodiments, it is a carbon-carbon triple bond. In certain embodiments, the 5'-carbon is substituted. In certain embodiments, X is substituted. In certain embodiments, X is unsubstituted.

In certain embodiments, the oligonucleotide comprises a 5'-stabilized phosphate moiety having the following formula: 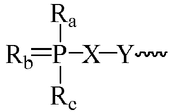 wherein:
Rₐ and R_{c} are each, independently, OH, SH, C₁-C₆ alkyl, substituted C₁-C₆ alkyl, C₁-C₆ alkoxy, substituted C₁-C₆ alkoxy, amino or substituted amino;
R_{b} is O or S;
X is substituted or unsubstitued C;
Y is selected from C, S, and N. In certain embodiments, Y is substituted or unsubstituted C. The bond between X and Y may be a single-, double-, or triple-bond.

In certain such embodiments, Y is the 5'-atom of the 5'-terminal nucleoside.
In certain embodiements, such oligonucleotides comprise a 5'terminal nucleoside having Formula I: 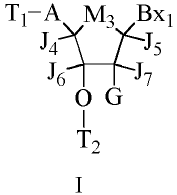 wherein:
T₁ is a phosphorus moiety;
T₂ is an internucleoside linking group linking the nucleoside of Formula I to the remainder of the oligonucleotide;
A has one of the formulas: 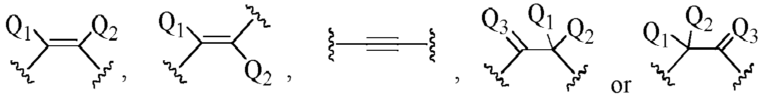
Q₁ and Q₂ are each, independently, H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl, C₁-C₆ alkoxy, substituted C₁-C₆ alkoxy or N(R₃)(R₄);
Q₃ is O, S, N(R₅) or C(R₆)(R₇);
each R₃, R₄ R₅, R₆ and R₇ is, independently, H, C₁-C₆ alkyl, substituted C₁-C₆ alkyl or C₁-C₆ alkoxy;
M₃ is O, S, NR₁₄, C(R₁₅)(R₁₆), C(R₁₅)(R₁₆)C(R₁₇)(R₁₈), C(R₁₅)=C(R₁₇), OC(R₁₅)(R₁₆) or OC(R₁₅)(Bx₂);
R₁₄ is H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
R₁₅, R₁₆, R₁₇ and R₁₈ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
one of Bx₁ and Bx₂ is a nucleobase and the other of Bx₁ and Bx₂, if present, is H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
J₄, J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
or J₄ forms a bridge with either J₅ or J₇ wherein said bridge comprises from 1 to 3 linked biradical groups selected from O, S, NR₁₉, C(R₂₀)(R₂₁), C(R₂₀)=C(R₂₁), C[=C(R₂₀)(R₂₁)] and C(=O) and the other two of J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
each R₁₉, R₂₀ and R₂₁ is, independently, H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
G is H, OH, halogen or O-[C(R₈)(R₉)]ₙ-[(C=O)ₘ-Xₗ]ⱼ-Z, or a conjugate group;
each R₈ and R₉ is, independently, H, halogen, C₁-C₆ alkyl or substituted C₁-C₆ alkyl;
X₁ is O, S or N(E₁);
Z is H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or N(E₂)(E₃);
E₁, E₂ and E₃ are each, independently, H, C₁-C₆ alkyl or substituted C₁-C₆ alkyl;
n is from 1 to about 6;
m is 0 or 1;
j is 0 or 1;
each substituted group comprises one or more optionally protected substituent groups independently selected from halogen, OJ₁, N(J₁)(J₂), =NJ₁, SJ₁, N₃, CN, OC(=X₂)J₁, OC(=X₂)N(J₁)(J₂) and C(=X₂)N(J₁)(J₂);
X₂ is O, S or NJ₃;
each J₁, J₂ and J₃ is, independently, H or C₁-C₆ alkyl; and
when j is 1 then Z is other than halogen or N(E₂)(E₃).

In certain embodiments, oligonucleotides comprise a 5'-terminal nucleoside having Formula II: 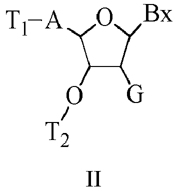 wherein:
Bx is a nucleobase;
T₁ is an phosphorus moiety;
T₂ is an internucleoside linking group linking the compound of Formula II to the remainder of the oligonucleotide;
A has one of the formulas: 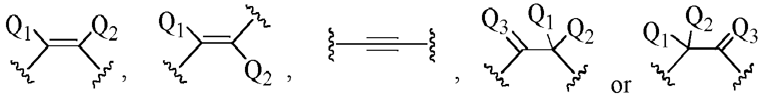
Q₁ and Q₂ are each, independently, H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl, C₁-C₆ alkoxy, substituted C₁-C₆ alkoxy or N(R₃)(R₄);
Q₃ is O, S, N(R₅) or C(R₆)(R₇);
each R₃, R₄ R₅, R₆ and R₇ is, independently, H, C₁-C₆ alkyl, substituted C₁-C₆ alkyl or C₁-C₆ alkoxy;
G is H, OH, halogen, O-[C(R₈)(R₉)]ₙ-[(C=O)ₘ-X]ⱼ-Z or a conjugate group;
each R₈ and R₉ is, independently, H, halogen, C₁-C₆ alkyl or substituted C₁-C₆ alkyl;
X is O, S or N(E₁);
Z is H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or N(E₂)(E₃);
E₁, E₂ and E₃ are each, independently, H, C₁-C₆ alkyl or substituted C₁-C₆ alkyl;
n is from 1 to about 6;
m is 0 or 1;
j is 0 or 1;
each substituted group comprises one or more optionally protected substituent groups independently selected from halogen, OJ₁, N(J₁)(J₂), =NJ₁, SJ₁, N₃, CN, OC(=L)J₁, OC(=L)N(J₁)(J₂) and C(=L)N(J₁)(J₂);
L is O, S or NJ₃;
each J₁, J₂ and J₃ is, independently, H or C₁-C₆ alkyl; and
when j is 1 then Z is other than halogen or N(E₂)(E₃).

In certain embodiments, oligonucleotides comprise a 5'-terminal nucleoside having Formula III: 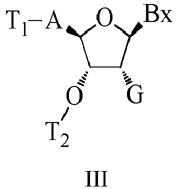 wherein:
Bx is a nucleobase;
T₁ is a phosphorus moiety;
T₂ is an internucleoside linking group linking the compound of Formula III to the remainder of the oligonucleotide;
A has one of the formulas: 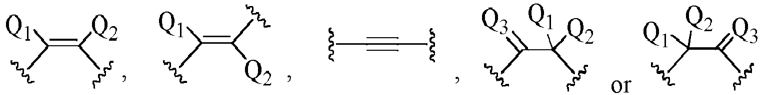
Q₁ and Q₂ are each, independently, H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl, C₁-C₆ alkoxy, substituted C₁-C₆ alkoxy or N(R₃)(R₄);
Q₃ is O, S, N(R₅) or C(R₆)(R₇);
each R₃, R₄ R₅, R₆ and R₇ is, independently, H, C₁-C₆ alkyl, substituted C₁-C₆ alkyl or C₁-C₆ alkoxy;
G is H, OH, halogen, O-[C(R₈)(R₉)]ₙ-[(C=O)ₘ-X]ⱼ-Z, or a conjugate group;
each R₈ and R₉ is, independently, H, halogen, C₁-C₆ alkyl or substituted C₁-C₆ alkyl;
X is O, S or N(E₁);
Z is H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or N(E₂)(E₃);
E₁, E₂ and E₃ are each, independently, H, C₁-C₆ alkyl or substituted C₁-C₆ alkyl;
n is from 1 to about 6;
m is 0 or 1;
j is 0 or 1;
each substituted group comprises one or more optionally protected substituent groups independently selected from halogen, OJ₁, N(J₁)(J₂), =NJ₁, SJ₁, N₃, CN, OC(=L)J₁, OC(=L)N(J₁)(J₂) and C(=L)N(J₁)(J₂);
L is O, S or NJ₃;
each J₁, J₂ and J₃ is, independently, H or C₁-C₆ alkyl; and
when j is 1 then Z is other than halogen or N(E₂)(E₃).

In certain embodiments, oligonucleotides comprise a 5'-terminal nucleoside having Formula IV:

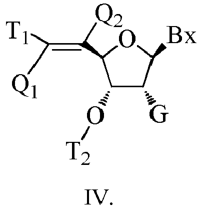

In certain embodiments, oligonucleotide are provided comprising a compound having Formula IV wherein Q₁ and Q₂ are each H. In certain embodiments, oligonucleotide are provided comprising a compound having Formula IV wherein G is O(CH₂)₂OCH₃.

In certain embodiments, oligonucleotides comprise a 5'-terminal nucleoside having Formula V:

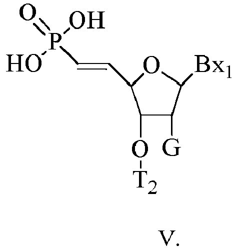

In certain embodiments, oligonucleotides comprise a nucleoside of Formula I, II, III, IV, or V. In certain such embodiments, the nucleoside of Formula I, II, III, IV, or V is at the 5'-terminus. In certain such embodiments, the remainder of the oligonucleotide comprises one or more modifications. Such modifications may include modified sugar moieties, modified nucleobases and/or modified internucleoside linkages. Certain such modifications which may be incorporated in an oligonucleotide comprising a nucleoside of Formula I, II, III, IV, or V at the 5'-terminus are known in the art.

### b. Certain Sugar Moieties

In certain embodiments, compounds of the invention comprise one or more modifed nucleosides comprising a modifed sugar moiety. Such compounds comprising one or more sugar-modified nucleosides may have desirable properties, such as enhanced nuclease stability or increased binding affinity with a target nucleic acid relative to an oligonucleotide comprising only nucleosides comprising naturally occurring sugar moieties. In certain embodiments, modified sugar moieties are substitued sugar moieties. In certain embodiments, modified sugar moieties are sugar surrogates. Such sugar surogates may comprise one or more substitutions corresponding to those of substituted sugar moieties.

In certain embodiments, modified sugar moieties are substituted sugar moieties comprising one or more non-bridging sugar substituent, including but not limited to substituents at the 2' and/or 5' positions. Examples of sugar substituents suitable for the 2'-position, include, but are not limited to: 2'-F, 2'-OCH₃ ("OMe" or "O-methyl"), and 2'-O(CH₂)₂OCH₃ ("MOE"). In certain embodiments, sugar substituents at the 2' position is selected from allyl, amino, azido, thio, O-allyl, O-C₁-C₁₀ alkyl, O-C₁-C₁₀ substituted alkyl; OCF₃, O(CH₂)₂SCH₃, O(CH₂)₂-O-N(Rm)(Rn), and O-CH₂-C(=O)-N(Rm)(Rn), where each Rm and Rn is, independently, H or substituted or unsubstituted C₁-C₁₀ alkyl. Examples of sugar substituents at the 5'-position, include, but are not limited to:, 5'-methyl (R or S); 5'-vinyl, and 5'-methoxy. In certain embodiments, substituted sugars comprise more than one non-bridging sugar substituent, for example, 2'-F-5'-methyl sugar moieties (*see,* e.g., PCT International Application WO 2008/101157, for additional 5', 2'-bis substituted sugar moieties and nucleosides).

Nucleosides comprising 2'-substituted sugar moieties are referred to as 2'-substituted nucleosides. In certain embodiments, a 2'- substituted nucleoside comprises a 2'-substituent group selected from halo, allyl, amino, azido, SH, CN, OCN, CF₃, OCF₃, O, S, or N(Rₘ)-alkyl; O, S, or N(Rₘ)-alkenyl; O, S or N(Rₘ)-alkynyl; O-alkylenyl-O-alkyl, alkynyl, alkaryl, aralkyl, O-alkaryl, O-aralkyl, O(CH₂)₂SCH₃, O-(CH₂)₂-ON(Rₘ)(Rₙ) or O-CH₂-C(=O)-N(Rₘ)(Rₙ), where each Rₘ and Rₙ is, independently, H, an amino protecting group or substituted or unsubstituted C₁-C₁₀ alkyl. These 2'-substituent groups can be further substituted with one or more substituent groups independently selected from hydroxyl, amino, alkoxy, carboxy, benzyl, phenyl, nitro (NO₂), thiol, thioalkoxy (S-alkyl), halogen, alkyl, aryl, alkenyl and alkynyl.

In certain embodiments, a 2'- substituted nucleoside comprises a 2'-substituent group selected from F, NH₂, N₃, OCF₃, O-CH₃, O(CH₂)₃NH₂, CH₂-CH=CH₂, O-CH₂-CH=CH₂, OCH₂CH₂OCH₃, O(CH₂)₂SCH₃, O-(CH₂)₂-O-N(Rₘ)(Rₙ), O(CH₂)₂O(CH₂)₂N(CH₃)₂, and N-substituted acetamide (O-CH₂-C(=O)-N(Rₘ)(Rₙ) where each Rₘ and Rₙ is, independently, H, an amino protecting group or substituted or unsubstituted C₁-C₁₀ alkyl.

In certain embodiments, a 2'- substituted nucleoside comprises a sugar moiety comprising a 2'-substituent group selected from F, OCF₃, O-CH₃, OCH₂CH₂OCH₃, O(CH₂)₂SCH₃, O-(CH₂)₂-O-N(CH₃)₂, -O(CH₂)₂O(CH₂)₂N(CH₃)₂, and O-CH₂-C(=O)-N(H)CH₃.

In certain embodiments, a 2'- substituted nucleoside comprises a sugar moiety comprising a 2'-substituent group selected from F, O-CH₃, and OCH₂CH₂OCH₃.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' sugar substituents, include, but are not limited to: -[C(Rₐ)(R_{b})]ₙ-, -[C(Rₐ)(R_{b})]ₙ-O-, -C(RₐR_{b})-N(R)-O- or, -C(RₐR_{b})-O-N(R)-; 4'-CH₂-2', 4'-(CH₂)₂-2', 4'-(CH₂)₃-2',. 4'-(CH₂)-O-2' (LNA); 4'-(CH₂)-S-2'; 4'-(CH₂)₂-O-2' (ENA); 4'-CH(CH₃)-O-2' (cEt) and 4'-CH(CH₂OCH₃)-O-2',and analogs thereof *(see, e.g.,* U.S. Patent 7,399,845, issued on July 15, 2008); 4'-C(CH₃)(CH₃)-O-2'and analogs thereof, *(see, e.g.,* WO2009/006478, published January 8, 2009); 4'-CH₂-N(OCH₃)-2' and analogs thereof *(see, e.g.,* WO2008/150729, published December 11, 2008); 4'-CH₂-O-N(CH₃)-2' *(see, e.g.,* US2004/0171570, published September 2, 2004); 4'-CH₂-O-N(R)-2', and 4'-CH₂-N(R)-O-2'-, wherein each Ris, independently, H, a protecting group, or C₁-C₁₂ alkyl; 4'-CH₂-N(R)-O-2', wherein R is H, C₁-C₁₂ alkyl, or a protecting group *(see,* U.S. Patent 7,427,672, issued on September 23, 2008); 4'-CH₂-C(H)(CH₃)-2' *(see, e.g.,* Chattopadhyaya, et al., J. Org. Chem.,2009, 74, 118-134); and 4'-CH₂-C(=CH₂)-2' and analogs thereof *(see,* published PCT International Application WO 2008/154401, published on December 8, 2008).

In certain embodiments, such 4' to 2' bridges independently comprise from 1 to 4 linked groups independently selected from -[C(Rₐ)(R_{b})]ₙ-, -C(Rₐ)=C(R_{b})-, -C(Rₐ)=N-, -C(=NRₐ)-, -C(=O)-, -C(=S)-, -O-,-Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Nucleosides comprising bicyclic sugar moieties are referred to as bicyclic nucleosides or BNAs. Bicyclic nucleosides include, but are not limited to, (A) α-L-Methyleneoxy (4'-CH₂-O-2') BNA, (B) β-D-Methyleneoxy (4'-CH₂-O-2') BNA (also referred to as locked nucleic acid or LNA), (C) Ethyleneoxy (4'-(CH₂)₂-O-2') BNA, (D) Aminooxy (4'-CH₂-O-N(R)-2') BNA, (E) Oxyamino (4'-CH₂-N(R)-O-2') BNA, (F) Methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH2-N(R)-2') BNA, (I) methyl carbocyclic (4'-CH₂-CH(CH₃)-2') BNA, (J) propylene carbocyclic (4'-(CH₂)₃-2') BNA, and (K) Methoxy(ethyleneoxy) (4'-CH(CH₂OMe)-O-2') BNA (also referred to as constrained MOE or cMOE) as depicted below. 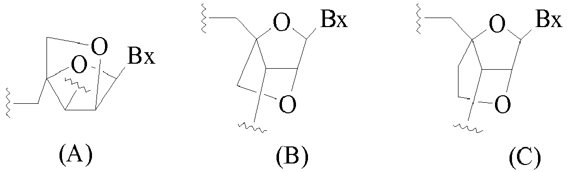 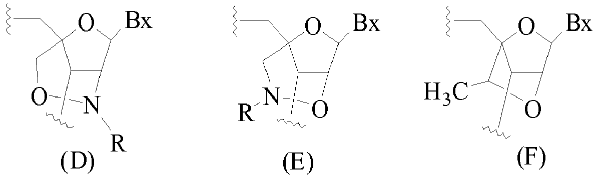 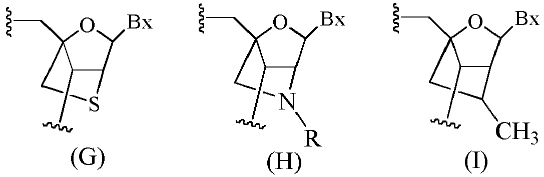 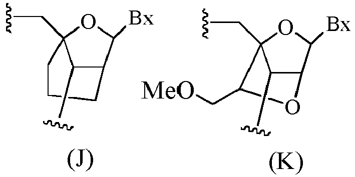 wherein Bx is a nucleobase moiety and R is, independently, H, a protecting group, or C₁-C₁₂ alkyl.

Additional bicyclic sugar moieties are known in the art, for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 129(26) 8362-8379 (Jul. 4, 2007); Elayadi et al., Curr. Opinion Invens. Drugs, 2001, 2, 5561; Braasch et al., Chem. Biol., 2001, 8, 1-7; Orum et al., Curr. Opinion Mol. Ther., 2001, 3, 239-243; U.S. Patent Nos. 7,053,207, 6,268,490, 6,770,748, 6,794,499, 7,034,133, 6,525,191, 6,670,461, and 7,399,845; WO 2004/106356, WO 1994/14226, WO 2005/021570, and WO 2007/134181; U.S. Patent Publication Nos. US2004/0171570, US2007/0287831, and US2008/0039618; U.S. Patent Serial Nos. 12/129,154, 60/989,574, 61/026,995, 61/026,998, 61/056,564, 61/086,231, 61/097,787, and 61/099,844; and PCT International Applications Nos. PCT/US2008/064591, PCT/US2008/066154, and PCT/US2008/068922.

In certain embodiments, bicyclic sugar moieties and nucleosides incorporating such bicyclic sugar moieties are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') bicyclic nucleosides have been incorporated into antisense oligonucleotides that showed antisense activity (Frieden et al., Nucleic Acids Research, 2003, 21, 6365-6372).

In certain embodiments, substituted sugar moieties comprise one or more non-bridging sugar substituent and one or more bridging sugar substituent (e.g., 5'-substituted and 4'-2' bridged sugars). *(see,* PCT International Application WO 2007/134181, published on 11/22/07, wherein LNA is substituted with, for example, a 5'-methyl or a 5'-vinyl group).

In certain embodiments, modified sugar moieties are sugar surrogates. In certain such embodiments, the oxygen atom of the naturally occuring sugar is substituted, e.g., with a sulfer, carbon or nitrogen atom. In certain such embodiments, such modified sugar moiety also comprises bridging and/or non-bridging substituents as described above. For example, certain sugar surogates comprise a 4'-sulfer atom and a substitution at the 2'-position (*see*,e.g., published U.S. Patent Application US2005/0130923, published on June 16, 2005) and/or the 5' position. By way of additional example, carbocyclic bicyclic nucleosides having a 4'-2' bridge have been described *(see, e.g.,* Freier et al., Nucleic Acids Research, 1997, 25(22), 4429-4443 and Albaek et al., J. Org. Chem., 2006, 71, 7731-7740).

In certain embodiments, sugar surrogates comprise rings having other than 5-atoms. For example, in certain embodiments, a sugar surrogate comprises a six-membered tetrahydropyran. Such tetrahydropyrans may be further modified or substituted. Nucleosides comprising such modified tetrahydropyrans include, but are not limited to, hexitol nucleic acid (HNA), anitol nucleic acid (ANA), manitol nucleic acid (MNA) *(see* Leumann, CJ. Bioorg. & Med. Chem. (2002) 10:841-854), fluoro HNA (F-HNA), and those compounds having Formula VII: 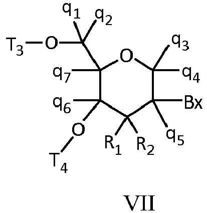 wherein independently for each of said at least one tetrahydropyran nucleoside analog of Formula VII:
Bx is a nucleobase moiety;
T₃ and T₄ are each, independently, an internucleoside linking group linking the tetrahydropyran nucleoside analog to the antisense compound or one of T₃ and T₄ is an internucleoside linking group linking the tetrahydropyran nucleoside analog to the antisense compound and the other of T₃ and T₄ is H, a hydroxyl protecting group, a linked conjugate group, or a 5' or 3'-terminal group;
   q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J_{1,} J₂, and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is F. In certain embodiments, R₁ is fluoro and R₂ is H, R₁ is methoxy and R₂ is H, and R₁ is methoxyethoxy and R₂ is H.

Many other bicyclo and tricyclo sugar surrogate ring systems are also known in the art that can be used to modify nucleosides for incorporation into antisense compounds *(see, e.g.,* review article: Leumann, J. C, Bioorganic & Medicinal Chemistry, 2002, 10, 841-854).

Combinations of modifications are also provided without limitation, such as 2'-F-5'-methyl substituted nucleosides (see PCT International Application WO 2008/101157 Published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

In certain embodiments, the present invention provides oligonucleotides comprising modified nucleosides. Those modified nucleotides may include modified sugars, modified nucleobases, and/or modified linkages. The specific modifications are selected such that the resulting oligonucleotides possess desireable characteristics. In certain embodmiments, oligonucleotides comprise one or more RNA-like nucleosides. In certain embodiments, oligonucleotides comprise one or more DNA-like nucleotides.

### c. Certain Nucleobases

In certain embodiments, nucleosides of the present invention comprise one or more unmodified nucleobases. In certain embodiments, nucleosides of the present invention comprise one or more modifed nucleobases.

In certain embodiments, modified nucleobases are selected from: universal bases, hydrophobic bases, promiscuous bases, size-expanded bases, and fluorinated bases as defined herein. 5-substituted pyrimidines, 6-azapyrimidines and N-2, N-6 and O-6 substituted purines, including 2-aminopropyladenine, 5-propynyluracil; 5-propynylcytosine; 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine, universal bases, hydrophobic bases, promiscuous bases, size-expanded bases, and fluorinated bases as defined herein. Further modified nucleobases include tricyclic pyrimidines such as phenoxazine cytidine([5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, Kroschwitz, J.I., Ed., John Wiley & Sons, 1990, 858-859; those disclosed by Englisch et al., Angewandte Chemie, International Edition, 1991, 30, 613; and those disclosed by Sanghvi, Y.S., Chapter 15, Antisense Research and Applications, Crooke, S.T. and Lebleu, B., Eds., CRC Press, 1993, 273-288.

Representative United States patents that teach the preparation of certain of the above noted modified nucleobases as well as other modified nucleobases include without limitation, U.S. 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121; 5,596,091; 5,614,617; 5,645,985; 5,681,941; 5,750,692; 5,763,588; 5,830,653 and 6,005,096, certain of which are commonly owned with the instant application, and each of which is herein incorporated by reference in its entirety.

### d. Certain Internucleoside Linkages

In certain embodiments, the present invention provides oligonucleotides comprising linked nucleosides. In such embodiments, nucleosides may be linked together using any internucleoside linkage. The two main classes of internucleoside linking groups are defined by the presence or absence of a phosphorus atom. Representative phosphorus containing internucleoside linkages include, but are not limited to, phosphodiesters (P=O), phosphotriesters, methylphosphonates, phosphoramidate, and phosphorothioates (P=S). Representative non-phosphorus containing internucleoside linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)₂-O-); and N,N'-dimethylhydrazine (-CH₂-N(CH₃)-N(CH₃)-). Modified linkages, compared to natural phosphodiester linkages, can be used to alter, typically increase, nuclease resistance of the oligonucleotide. In certain embodiments, internucleoside linkages having a chiral atom can be prepared as a racemic mixture, or as separate enantiomers. Representative chiral linkages include, but are not limited to, alkylphosphonates and phosphorothioates. Methods of preparation of phosphorous-containing and non-phosphorous-containing internucleoside linkages are well known to those skilled in the art.

The oligonucleotides described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids etc. Included in the antisense compounds provided herein are all such possible isomers, as well as their racemic and optically pure forms.

Neutral internucleoside linkages include without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### e. Certain Motifs

In certain embodiments, the present invention provides compounds comprising oligonucleotides. In certain embodiments, such oligonucleotides comprise one or more chemical modification. In certain embodiments, chemically modified oligonucleotides comprise one or more modified sugars. In certain embodiments, chemically modified oligonucleotides comprise one or more modified nucleobases. In certain embodiments, chemically modified oligonucleotides comprise one or more modified internucleoside linkages. In certain embodiments, the chemical modifications (sugar modifications, nucleobase modifications, and/or linkage modifications) define a pattern or motif. In certain embodiments, the patterns of chemical modifications of sugar moieties, internucleoside linkages, and nucleobases are each independent of one another. Thus, an oligonucleotide may be described by its sugar modification motif, internucleoside linkage motif and/or nucleobase modification motif (as used herein, nucleobase modification motif describes the chemical modifications to the nucleobases independent of the sequence of nucleobases).

### i. Certain sugar motifs

In certain embodiments, oligonucleotides comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having uniform sugar modifications. In certain such embodiments, each nucleoside of the region comprises the same RNA-like sugar modification. In certain embodiments, each nucleoside of the region is a 2'-F nucleoside. In certain embodiments, each nucleoside of the region is a 2'-OMe nucleoside. In certain embodiments, each nucleoside of the region is a 2'-MOE nucleoside. In certain embodiments, each nucleoside of the region is a cEt nucleoside. In certain embodiments, each nucleoside of the region is an LNA nucleoside. In certain embodiments, the uniform region constitutes all or essentially all of the oligonucleotide. In certain embodiments, the region constitutes the entire oligonucleotide except for 1-4 terminal nucleosides.

In certain embodiments, oligonucleotides of the present invention comprise one or more regions of alternating sugar modifications, wherein the nucleosides alternate between nucleosides having a sugar modification of a first type and nucleosides having a sugar modification of a second type. In certain embodiments, nucleosides of both types are RNA-like nucleosides. In certain embodiments the alternating nucleosides are selected from: 2'-Ome, 2'-F, 2'-MOE, LNA, and cEt. In certain embodiments, the alternating modifications are 2'-F and 2'-Ome. Such regions may be contiguous or may be interupted by differently modified nucleosides or conjugated nucleosides.

In certain embodiments, the alternating region of alternating modifications each consist of a single nucleoside (i.e., the patern is (AB)ₓA_{y} wheren A is a nucleoside having a sugar modification of a first type and B is a nucleoside having a sugar modification of a second type; x is 1-20 and y is 0 or 1). In certan embodiments, one or more alternating regions in an alternating motif includes more than a single nucleoside of a type. For example, oligonucleotides of the present invention may include one or more regions of any of the following nucleoside motifs:
AABBAA;
ABBABB;
AABAAB;
ABBABAABB;
ABABAA;
AABABAB;
ABABAA;
ABBAABBABABAA;
BABBAABBABABAA;
ABABBAABBABABAA; or
ABABABABABABABABAB;
wherein A is a nucleoside of a first type and B is a nucleoside of a second type. In certain embodiments, A and B are each selected from 2'-F, 2'-Ome, BNA, and MOE.

In certain embodiments, oligonucleotides having such an alternating motif also comprise a 5' terminal nucleoside of Formula I, II, III, IV, or V.

In certain embodiments, oligonucleotides of the present invention comprise a region having a 2-2-3 motif. Such regions comprises the following motif:

-(A)₂-(B)ₓ-(A)₂-(C)_{y}-(A)₃-

wherein: A is a first type of modifed nucleoside;
B and C, are nucleosides that are differently modified than A, however, B and C may have the same or different modifications as one another;
x and y are from 1 to 15.

In certain embodiments, A is a 2'-Ome modified nucleoside. In certain embodiments, B and C are both 2'-F modified nucleosides. In certain embodiments, A is a 2'-Ome modified nucleoside and B and C are both 2'-F modified nucleosides.

It is to be understood, that certain of the above described motifs and modifications may be combined. Since a motif may comprise only a few nucleosides, a particular oligonucleotide may comprise two or more motifs. By way of non-limiting example, in certain embodiments, oligonucleotides may have nucleoside motifs as described in the table below. In the table below, the term "None" indicates that a particular feature is not present in the oligonucleotide. For example, "None" in the column labeled "5' motif/modification" indicates that the 5' end of the oligonucleotide comprises the first nucleoside of the central motif.

| 5' motif/modification | Central Motif | 3'-motif |
|---|---|---|
| Compound of Formula I, II, III, IV, or V | Alternating | 2 MOE nucleosides |
| Compound of Formula I, II, III, IV, or V | 2-2-3 motif | 2 MOE nucleosides |
| Compound of Formula I, II, III, IV, or V | Uniform | 2 MOE nucleosides |
| Compound of Formula I, II, III, IV, or V | Alternating | 2 MOE nucleosides |
| Compound of Formula I, II, III, IV, or V | Alternating | 2 MOE A's |
| Compound of Formula I, II, III, IV, or V | 2-2-3 motif | 2 MOE A's |
| Compound of Formula I, II, III, IV, or V | Uniform | 2 MOE A's |
| Compound of Formula I, II, III, IV, or V | Alternating | 2 MOE U's |
| Compound of Formula I, II, III, IV, or V | 2-2-3 motif | 2 MOE U's |
| Compound of Formula I, II, III, IV, or V | Uniform | 2 MOE U's |
| Compound of Formula I, II, III, IV, or V | Alternating | 2 MOE nucleosides |
| Compound of Formula I, II, III, IV, or V | 2-2-3 motif | 2 MOE nucleosides |
| Compound of Formula I, II, III, IV, or V | Uniform | 2 MOE nucleosides |

In certain embodiments, oligonucleosides have the following sugar motif:

5'-(Q)-(E)_{w}-(A)₂-(B)ₓ-(A)₂-(C)_{y}-(A)₃(D)_{z}

wherein:
Q is a nucleoside comprising a stabilized phosphate moiety. In certain embodiments, Q is a nucleoside having Formula I, II, III, IV, or V;
A is a first type of modifed nucleoside;
B, C, D, and E are nucleosides that are differently modified than A, however, B, C, D, and E may have the same or different modifications as one another;
w and z are from 0 to 15;
x and y are from 1 to 15.
In certain embodiments, the sum of w, x, and y is 5-25.

In certain embodiments, oligonucleosides have the following sugar motif:

5'- (Q)- (AB)ₓA_{y}-(D)_{z}

wherein:
Q is a nucleoside comprising a stabilized phosphate moiety. In certain embodiments, Q is a nucleoside having Formula I, II, III, IV, or V;
A is a first type of modifed nucleosde;
B is a second type of modified nucleoside;
D is a modified nucleoside comprising a modification different from the nucleoside adjacent to it. Thus, if y is 0, then D must be differently modified than B and if y is 1, then D must be differently modified than A. In certain embodiments, D differs from both A and B.
X is 5-15;
Y is 0 or 1;
Z is 0-4.

In certain embodiments, oligonucleosides have the following sugar motif:

5'-(Q)-(A)ₓ-(D)_{z}

wherein:
Q is a nucleoside comprising a stabilized phosphate moiety. In certain embodiments, Q is a nucleoside having Formula I, II, III, IV, or V;
A is a first type of modifed nucleoside;
D is a modified nucleoside comprising a modification different from A.
X is 11-30;
Z is 0-4.

In certain embodiments A, B, C, and D in the above motifs are selected from : 2'-Ome, 2'-F, 2'-MOE, LNA, and cEt. In certain embodiments, D represents terminal nucleosides. In certain embodiments, such terminal nucleosides are not designed to hybridize to the target nucleic acid (though one or more might hybridize by chance). In certain embodiments, the nucleobase of each D nucleoside is adenine, regardless of the identity of the nucleobase at the corresponding position of the target nucleic acid. In certain embodiments the nucleobase of each D nucleoside is thymine.

### ii. Certain Internucleoside Linkage Motifs

In certain embodiments, oligonucleotides comprise modified internucleoside linkages arranged along the oligonucleotide or region thereof in a defined pattern or modified internucleoside linkage motif. In certain embodiments, oligonucleotides comprise a region having an alternating internucleoside linkage motif. In certain embodiments, oligonucleotides of the present invention comprise a region of uniformly modified internucleoside linkages. In certain such embodiments, the oligonucleotide comprises a region that is uniformly linked by phosphorothioate internucleoside linkages. In certain embodiments, the oligonucleotide is uniformly linked by phosphorothioate internucleoside linkages. In certain embodiments, each internucleoside linkage of the oligonucleotide is selected from phosphodiester and phosphorothioate. In certain embodiments, each internucleoside linkage of the oligonucleotide is selected from phosphodiester and phosphorothioate and at least one internucleoside linkage is phosphorothioate.

In certain embodiments, the oligonucleotide comprises at least 6 phosphorothioate internucleoside linkages. In certain embodiments, the oligonucleotide comprises at least 8 phosphorothioate internucleoside linkages. In certain embodiments, the oligonucleotide comprises at least 10 phosphorothioate internucleoside linkages. In certain embodiments, the oligonucleotide comprises at least one block of at least 6 consecutive phosphorothioate internucleoside linkages. In certain embodiments, the oligonucleotide comprises at least one block of at least 8 consecutive phosphorothioate internucleoside linkages. In certain embodiments, the oligonucleotide comprises at least one block of at least 10 consecutive phosphorothioate internucleoside linkages. In certain embodiments, the oligonucleotide comprises at least one block of at least one 12 consecutive phosphorothioate internucleoside linkages. In certain such embodiments, at least one such block is located at the 3' end of the oligonucleotide. In certain such embodiments, at least one such block is located within 3 nucleosides of the 3' end of the oligonucleotide.

Oligonucleotides having any of the various sugar motifs described herein, may have any linkage motif. For example, the oligonucleotides, including but not limited to those described above, may have a linkage motif selected from non-limiting the table below:

| 5' most linkage | Central region | 3'-region |
|---|---|---|
| PS | Alternating PO/PS | 6 PS |
| PS | Alternating PO/PS | 7 PS |
| PS | Alternating PO/PS | 8 PS |

### iii. Certain Nucleobase Modification Motifs

In certain embodiments, oligonucleotides comprise chemical modifications to nucleobases arranged along the oligonucleotide or region thereof in a defined pattern or nucleobases modification motif. In certain such embodiments, nucleobase modifications are arranged in a gapped motif. In certain embodiments, nucleobase modifications are arranged in an alternating motif. In certain embodiments, each nucleobase is modified. In certain embodiments, none of the nucleobases is chemically modified.

In certain embodiments, oligonucleotides comprise a block of modified nucleobases. In certain such embodiments, the block is at the 3'-end of the oligonucleotide. In certain embodiments the block is within 3 nucleotides of the 3'-end of the oligonucleotide. In certain such embodiments, the block is at the 5'-end of the oligonucleotide. In certain embodiments the block is within 3 nucleotides of the 5'-end of the oligonucleotide.

In certain embodiments, nucleobase modifications are a function of the natural base at a particular position of an oligonucleotide. For example, in certain embodiments each purine or each pyrimidine in an oligonucleotide is modified. In certain embodiments, each adenine is modified. In certain embodiments, each guanine is modified. In certain embodiments, each thymine is modified. In certain embodiments, each cytosine is modified. In certain embodiments, each uracil is modified.

In certain embodiments, some, all, or none of the cytosine moieties in an oligonucleotide are 5-methyl cytosine moieties. Herein, 5-methyl cytosine is not a "modified nucleobase." Accordingly, unless otherwise indicated, unmodified nucleobases include both cytosine residues having a 5-methyl and those lacking a 5 methyl. In certain embodiments, the methylation state of all or some cytosine nucleobases is specified.

### a. Certain Overall Lengths

In certain embodiments, the present invention provides oligonucleotides of any of a variety of ranges of lengths. In certain embodiments, the invention provides oligonucleotides consisting of X to Y linked nucleosides, where X represents the fewest number of nucleosides in the range and Y represents the largest number of nucleosides in the range. In certain such embodiments, X and Y are each independently selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50; provided that X≤Y. For example, in certain embodiments, the invention provides oligonucleotides consisting of 8 to 9, 8 to 10, 8 to 11, 8 to 12, 8 to 13, 8 to 14, 8 to 15, 8 to 16, 8 to 17, 8 to 18, 8 to 19, 8 to 20, 8 to 21, 8 to 22, 8 to 23, 8 to 24, 8 to 25, 8 to 26, 8 to 27, 8 to 28, 8 to 29, 8 to 30, 9 to 10, 9 to 11, 9 to 12, 9 to 13, 9 to 14, 9 to 15, 9 to 16, 9 to 17, 9 to 18, 9 to 19, 9 to 20, 9 to 21, 9 to 22, 9 to 23, 9 to 24, 9 to 25, 9 to 26, 9 to 27, 9 to 28, 9 to 29, 9 to 30, 10 to 11, 10 to 12, 10 to 13, 10 to 14, 10 to 15, 10 to 16, 10 to 17, 10 to 18, 10 to 19, 10 to 20, 10 to 21, 10 to 22, 10 to 23, 10 to 24, 10 to 25, 10 to 26, 10 to 27, 10 to 28, 10 to 29, 10 to 30, 11 to 12, 11 to 13, 11 to 14, 11 to 15, 11 to 16, 11 to 17, 11 to 18, 11 to 19, 11 to 20, 11 to 21, 11 to 22, 11 to 23, 11 to 24, 11 to 25, 11 to 26, 11 to 27, 11 to 28, 11 to 29, 11 to 30, 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 29 to 30 linked nucleosides. In embodiments where the number of nucleosides of an oligonucleotide of a compound is limited, whether to a range or to a specific number, the compound may, nonetheless further comprise additional other substituents. For example, an oligonucleotide comprising 8-30 nucleosides excludes oligonucleotides having 31 nucleosides, but, unless otherwise indicated, such an oligonucleotide may further comprise, for example one or more conjugates, terminal groups, or other substituents.

Further, where an oligonucleotide is described by an overall length range and by regions having specified lengths, and where the sum of specified lengths of the regions is less than the upper limit of the overall length range, the oligonucleotide may have additional nucleosides, beyond those of the specified regions, provided that the total number of nucleosides does not exceed the upper limit of the overall length range.

### b. Certain Oligonucleotides

In certain embodiments, oligonucleotides of the present invention are characterized by their sugar motif, internucleoside linkage motif, nucleobase modification motif and overall length. In certain embodiments, such parameters are each independent of one another. Thus, each internucleoside linkage of an oligonucleotide having a gapmer sugar motif may be modified or unmodified and may or may not follow the gapmer modification pattern of the sugar modifications. Thus, the internucleoside linkages within the wing regions of a sugar-gapmer may be the same or different from one another and may be the same or different from the internucleoside linkages of the gap region. Likewise, such sugar-gapmer oligonucleotides may comprise one or more modified nucleobase independent of the gapmer pattern of the sugar modifications. One of skill in the art will appreciate that such motifs may be combined to create a variety of oligonucleotides, such as those provided in the non-limiting table below. As is apparent from the above, non-limiting tables, the lengths of the regions defined by a nucleoside motif and that of a linkage motif need not be the same. To further illustrate, and not to limit in any way, nucleoside motifs and sequence motifs are combined to show five non-limiting examples in the table below. The first column of the table lists nucleosides and linkages by position from N1 (the first nucleoside at the 5'-end) to N20 (the 20^{th} position from the 5'-end). In certain embodiments, oligonucleotides of the present invention are longer than 20 nucleosides (the table is merely exemplary). Certain positions in the table recite the nucleoside or linkage "none" indicating that the oligonucleotide has no nucleoside at that position.

| Pos | A | B | C | D | E |
|---|---|---|---|---|---|
| N1 | Formula I, II, III, IV, or V | Formula I, II, III, IV, or V | Formula I, II, III, IV, or V | Formula I, II, III, IV, or V | Formula I, II, III, IV, or V |
| L1 | PS | PS | PS | PS | PO |
| N2 | 2'-F | 2'-F | 2'-F | 2'-Ome | MOE |
| L2 | PS | PS | PS | PO | PS |
| N3 | 2'-Ome | 2'-F | 2'-F | 2'-F | 2'-F |
| L3 | PO | PS | PS | PS | PS |
| N4 | 2'-F | 2'-F | 2'-F | 2'-Ome | 2'-F |
| L4 | PS | PS | PS | PO | PS |
| N5 | 2'-Ome | 2'-F | 2'-F | 2'-F | 2'-Ome |
| L5 | PO | PS | PS | PS | PO |
| N6 | 2'-F | 2'-Ome | 2'-F | 2'-Ome | 2'-Ome |
| L6 | PS | PO | PS | PO | PO |
| N7 | 2'-Ome | 2'-Ome | 2'-F | 2'-F | 2'-Ome |
| L7 | PO | PO | PS | PS | PO |
| N8 | 2'-F | 2'-F | 2'-F | 2'-Ome | 2'-F |
| L8 | PS | PS | PS | PO | PS |
| N9 | 2'-Ome | 2'-F | 2'-F | 2'-F | 2'-F |
| L9 | PO | PS | PS | PS | PS |
| N10 | 2'-F | 2'-Ome | 2'-F | 2'-Ome | 2'-Ome |
| L10 | PS | PO | PS | PO | PO |
| N11 | 2'-Ome | 2'-Ome | 2'-F | 2'-F | 2'Ome |
| L11 | PO | PO | PS | PS | PO |
| N12 | 2'-F | 2'-F | 2'-F | 2'-F | 2'-F |
| L12 | PS | PS | PS | PO | PS |
| N13 | 2'-Ome | 2'-F | 2'-F | 2'-F | 2'-F |
| L13 | PO | PS | PS | PS | PS |
| N14 | 2'-F | 2'-Ome | 2'-F | 2'-F | 2'-F |
| L14 | PS | PS | PS | PS | PS |
| N15 | 2'-Ome | 2'Ome | 2'-F | 2'-F | 2'-MOE |
| L15 | PS | PS | PS | PS | PS |
| N16 | 2'-F | 2'Ome | 2'-F | 2'-F | 2'-MOE |
| L16 | PS | PS | PS | PS | PS |
| N17 | 2'-Ome | 2'-MOE U | 2'-F | 2'-F | 2'-MOE |
| L17 | PS | PS | PS | PS | None |
| N18 | 2'-F | 2'-MOE U | 2'-F | 2'-Ome | None |
| L18 | PS | None | PS | PS | None |
| N19 | 2'-MOE U | None | 2'-MOE U | 2'-MOE A | None |
| L19 | PS | None | PS | PS | None |
| N20 | 2'-MOE U | None | 2'-MOE U | 2'-MOE A | None |

In the above, non-limiting examples:
Column A represent an oligonucleotide consisting of 20 linked nucleosides, wherein the oligonucleotide comprises: a modified 5'-terminal nucleoside of Formula I, II, III, IV, or V; a region of alternating nucleosides; a region of alternating linkages; two 3'-terminal MOE nucleosides, each of which comprises a uracil base; and a region of six phosphorothioate linkages at the 3'-end.
Column B represents an oligonucleotide consisting of 18 linked nucleosides, wherein the oligonucleotide comprises: a modified 5'-terminal nucleoside of Formula Formula I, II, III, IV, or V; a 2-2-3 motif wherein the modified nucleoside of the 2-2-3 motif are 2'O-Me and the remaining nucleosides are all 2'-F; two 3'-terminal MOE nucleosides, each of which comprises a uracil base; and a region of six phosphorothioate linkages at the 3'-end.
Column C represents an oligonucleotide consisting of 20 linked nucleosides, wherein the oligonucleotide comprises: a modified 5'-terminal nucleoside of Formula I, II, III, IV, or V; a region of uniformly modified 2'-F nucleosides; two 3'-terminal MOE nucleosides, each of which comprises a uracil base; and wherein each internucleoside linkage is a phosphorothioate linkage.
Column D represents an oligonucleotide consisting of 20 linked nucleosides, wherein the oligonucleotide comprises: a modified 5'-terminal nucleoside of Formula I, II, III, IV, or V; a region of alternating 2'-Ome/2'-F nucleosides; a region of uniform 2'F nucleosides; a region of alternating phosphorothioate/phosphodiester linkages; two 3'-terminal MOE nucleosides, each of which comprises an adenine base; and a region of six phosphorothioate linkages at the 3'-end.
Column E represents an oligonucleotide consisting of 17 linked nucleosides, wherein the oligonucleotide comprises: a modified 5'-terminal nucleoside of Formula I, II, III, IV, or V; a 2-2-3 motif wherein the modified nucleoside of the 2-2-3 motif are 2'F and the remaining nucleosides are all 2'-Ome; three 3'-terminal MOE nucleosides.

The above examples are provided solely to illustrate how the described motifs may be used in combination and are not intended to limit the invention to the particular combinations or the particular modifications used in illustrating the combinations. Further, specific examples herein, including, but not limited to those in the above table are intended to encompass more generic embodiments. For example, column A in the above table exemplifies a region of alternating 2'-Ome and 2'-F nucleosides. Thus, that same disclosure also exemplifies a region of alternating different 2'-modifications. It also exemplifies a region of alternating 2'-O-alkyl and 2'-halogen nucleosides. It also exemplifies a region of alternating differently modified nucleosides. All of the examples throughout this specification contemplate such generic interpretation.

It is also noted that the lengths of the oligonucleotides, such as those exemplified in the above tables, can be easily manipulated by lengthening or shortening one or more of the described regions, without disrupting the motif.

In certain embodiments, the invention provides oligonucleotides wherein the 5'-terminal nucleoside (position 1) is a compound of Formula I, II, III, IV, or V and the position 2 nucleoside comprises a 2'-modification. In certain such embodiments, the 2'-modification of the position 2 nucleoside is selected from halogen, alkyl, and substituted alkyl. In certain embodiments, the 2'-modification of the position 2 nucleoside is selected from 2'-F and 2'-alkyl. In certain embodiments, the 2'-modification of the position 2 nucleoside is 2'-F. In certain embodiments, the 2'-substitued of the position 2 nucleoside is an unmodified OH (as in naturally occurring RNA).

In certain embodiments, the position 3 nucleoside is a modified nucleoside. In certain embodiments, the position 3 nucleoside is a bicyclic nucleoside. In certain embodiments, the position 3 nucleoside comprises a sugar surrogate. In certain such embodiments, the sugar surrogate is a tetrahydropyran. In certain embodiments, the sugar of the position 3 nucleoside is a F-HNA.
In certain embodiments, an antisense compound comprises an oligonucleotide comprising 10 to 30 linked nucleosides wherein the oligonucleotide comprises: a position 1 modified nucleoside of Formula I, II, III, IV, or V; a position 2 nucleoside comprising a sugar moiety which is differently modified compared to the sugar moiety of the position 1 modified nucleoside; and from 1 to 4 3'-terminal group nucleosides each comprising a 2'-modification; and wherein at least the seven 3'-most internucleoside linkages are phosphorothioate linkages.

### c. Certain Conjugate Groups

In certain embodiments, oligonucleotides are modified by attachment of one or more conjugate groups. In general, conjugate groups modify one or more properties of the attached oligonucleotide, including but not limited to pharmacodynamics, pharmacokinetics, stability, binding, absorption, cellular distribution, cellular uptake, charge and clearance. Conjugate groups are routinely used in the chemical arts and are linked directly or via an optional conjugate linking moiety or conjugate linking group to a parent compound such as an oligonucleotide. Conjugate groups include without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

In certain embodiments, further conjugate groups and ss-RNA motifs have been described previously, for example: WO 2013/033230 which is hereby incorporated by reference in its entirety.

In certain embodiments, a conjugate group comprises an active drug substance, for example, aspirin, warfarin, phenylbutazone, ibuprofen, suprofen, fen-bufen, ketoprofen, (*S*)-(+)-pranoprofen, carprofen, dansylsarcosine, 2,3,5-triiodobenzoic acid, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

In certain embodiments, conjugate groups are directly attached to oligonucleotides. In certain embodiments, conjugate groups are attached to oligonucleotides by a conjugate linking group. In certain such embodiments, conjugate linking groups, including, but not limited to, bifunctional linking moieties such as those known in the art are amenable to the compounds provided herein. Conjugate linking groups are useful for attachment of conjugate groups, such as chemical stabilizing groups, functional groups, reporter groups and other groups to selective sites in a parent compound such as for example an oligonucleotide. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the conjugate linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like.

Some nonlimiting examples of conjugate linking moieties include pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

Conjugate groups may be attached to either or both ends of an oligonucleotide (terminal conjugate groups) and/or at any internal position.

In certain embodiments, conjugate groups are at the 3'-end of an oligonucleotide. In certain embodiments, conjugate groups are near the 3'-end. In certain embodiments, conjugates are attached at the 3'end of an oligonucleotide, but before one or more terminal group nucleosides. In certain embodiments, conjugate groups are placed within a terminal group. In certain embodiments, a conjugate group is attatched to the 3'-terminal nucleoside. In certain such embodiment, it is attached at the 3'-poition of the 3'-terminal nucleoside. In certain embodiments, it is attached at the 2'-poition of the 3'-terminal nucleoside.

In certain embodiments, compounds comprise an oligonucleotide. In certain embodiments, an compound comprises an oligonucleotide and one or more conjugate and/or terminal groups. Such conjugate and/or terminal groups may be added to oligonucleotides having any of the chemical motifs discussed above. Thus, for example, a compound comprising an oligonucleotide having region of alternating nucleosides may comprise a terminal group.

In certain embodiments, a conjugate is attached at the 2'-position of a nucleoside. In certain embodiments, a conjugate is attached to a nucleoside at one or more of: position 1,6 or 8 of the oligonucleotide, counting from the 5'-end. In certain embodiments a conjugate is attached to a nucleoside at one or more of : positon 13, 15, or 20 of the oligonucleotide, counting from the 3'-end.

In certain embodiments, conjugates interupt motifs. For example, in certain embodiments, oligonucleotides of the present invention have an alternating motif that spans positions 1-19 and a conjugate at position 8 (from the 5'-end) as follows:
Po-ABABABAXABABABABABA-
Wherein A represents nucleosides of a first-type;
B represents nucleosides of a second type; and
X represents a nucleoside to which a conjugate is attached.

In certain embodiments, A and B are 2'-modifications and X is a conjugate attached at the 2'-position. Thus, the motif of alternating 2'-modifications is interupted by the conjugate. Such an oligonucleotide may, nevertheless be described as having an alternating motif.

In certain embodiments, conjugates interupt motifs. For example, in certain embodiments, oligonucleotides of the present invention have an alternating motif that spans positions 1-19 and a conjugate at position 8 (from the 5'-end) as follows:
Pv-ABABABAXABABABABABA-
Wherein "Pv" at the 5'-end indicates a 5'-*(E)-*vinylphosphonate group, (PO(OH)₂(CH=CH)-;
A represents nucleosides of a first-type;
B represents nucleosides of a second type; and
X represents a nucleoside to which a conjugate is attached.

In certain embodiments, A and B are 2'-modifications and X is a conjugate attached at the 2'-position. In certain embodiments, X is a C₁₆ conjugate attached at the 2'-position. Thus, the motif of alternating 2'-modifications is interupted by the conjugate. Such an oligonucleotide may, nevertheless be described as having an alternating motif.

In certain embodiments, conjugates interupt motifs. For example, in certain embodiments, oligonucleotides of the present invention have an alternating motif that spans positions 1-19 and a conjugate at position 8 (from the 5'-end) as follows:
Pv-CABABABAXABABABABABA-
Wherein "Pv" at the 5'-end indicates a 5'-(E)-vinylphosphonate group, (PO(OH)₂(CH=CH)-;
A represents nucleosides of a first-type;
B represents nucleosides of a second type;
C represents a nucleosides of a first, second, or third type; and
X represents a nucleoside to which a conjugate is attached.

In certain embodiments, A and B are 2'-modifications and X is a conjugate attached at the 2'-position. In certain embodiments, X is a C₁₆ conjugate attached at the 2'-position. In certain embodiments, C is a T residue with a 5'-*(E)*-vinylphosphonate group. Thus, the motif of alternating 2'-modifications is interupted by the conjugate. Such an oligonucleotide may, nevertheless be described as having an alternating motif.

In certain embodiments, conjugates interupt motifs. For example, in certain embodiments, oligonucleotides of the present invention have an alternating motif that spans positions 1-19 and a conjugate at position 1 (from the 5'-end) as follows:
Pv-CXABABABAXABABABABABA-
Wherein "Pv" at the 5'-end indicates a 5'-*(E)*-vinylphosphonate group, (PO(OH)₂(CH=CH)-;
A represents nucleosides of a first-type;
B represents nucleosides of a second type;
C represents a nucleosides of a first, second, or third type; and
X represents a nucleoside to which a conjugate is attached.

In certain embodiments, A and B are 2'-modifications and X is a conjugate attached at the 2'-position. In certain embodiments, X is a C₁₆ conjugate attached at the 2'-position. In certain embodiments, C is a T residue with a 5'-*(E)*-vinylphosphonate group. Thus, the motif of alternating 2'-modifications is interupted by the conjugate. Such an oligonucleotide may, nevertheless be described as having an alternating motif.

### i. Certain Conjugates

In certain embodiments, a conjugate group comprises a cleavable moiety. In certain embodiments, a conjugate group comprises one or more cleavable bond. In certain embodiments, a conjugate group comprises a linker. In certain embodiments, a linker comprises a protein binding moiety. In certain embodiments, a conjugate group comprises a cell-targeting moiety (also referred to as a cell-targeting group).

### iv. Certain Cleavable Moieties

In certain embodiments, a cleavable moiety is a cleavable bond. In certain embodiments, a cleavable moiety comprises a cleavable bond. In certain embodiments, the conjugate group comprises a cleavable moiety. In certain such embodiments, the cleavable moiety attaches to the antisense oligonucleotide. In certain such embodiments, the cleavable moiety attaches directly to the cell-targeting moiety. In certain such embodiments, the cleavable moiety attaches to the conjugate linker. In certain embodiments, the cleavable moiety comprises a phosphate or phosphodiester. In certain embodiments, the cleavable moiety is a cleavable nucleoside or nucleoside analog. In certain embodiments, the nucleoside or nucleoside analog comprises an optionally protected heterocyclic base selected from a purine, substituted purine, pyrimidine or substituted pyrimidine. In certain embodiments, the cleavable moiety is a nucleoside comprising an optionally protected heterocyclic base selected from uracil, thymine, cytosine, 4-N-benzoylcytosine, 5-methylcytosine, 4-N-benzoyl-5-methylcytosine, adenine, 6-N-benzoyladenine, guanine and 2-N-isobutyrylguanine. In certain embodiments, the cleavable moiety is 2'-deoxy nucleoside that is attached to the 3' position of the antisense oligonucleotide by a phosphodiester linkage and is attached to the linker by a phosphodiester or phosphorothioate linkage. In certain embodiments, the cleavable moiety is 2'-deoxy adenosine that is attached to the 3' position of the antisense oligonucleotide by a phosphodiester linkage and is attached to the linker by a phosphodiester or phosphorothioate linkage. In certain embodiments, the cleavable moiety is 2'-deoxy adenosine that is attached to the 3' position of the antisense oligonucleotide by a phosphodiester linkage and is attached to the linker by a phosphodiester linkage.

In certain embodiments, the cleavable moiety is attached to the 3' position of the antisense oligonucleotide. In certain embodiments, the cleavable moiety is attached to the 5' position of the antisense oligonucleotide. In certain embodiments, the cleavable moiety is attached to a 2' position of the antisense oligonucleotide. In certain embodiments, the cleavable moiety is attached to the antisense oligonucleotide by a phosphodiester linkage. In certain embodiments, the cleavable moiety is attached to the linker by either a phosphodiester or a phosphorothioate linkage. In certain embodiments, the cleavable moiety is attached to the linker by a phosphodiester linkage. In certain embodiments, the conjugate group does not include a cleavable moiety.

In certain embodiments, the cleavable moiety is cleaved after the complex has been administered to an animal only after being internalized by a targeted cell. Inside the cell the cleavable moiety is cleaved thereby releasing the active antisense oligonucleotide. While not wanting to be bound by theory it is believed that the cleavable moiety is cleaved by one or more nucleases within the cell. In certain embodiments, the one or more nucleases cleave the phosphodiester linkage between the cleavable moiety and the linker. In certain embodiments, the cleavable moiety has a structure selected from among the following: 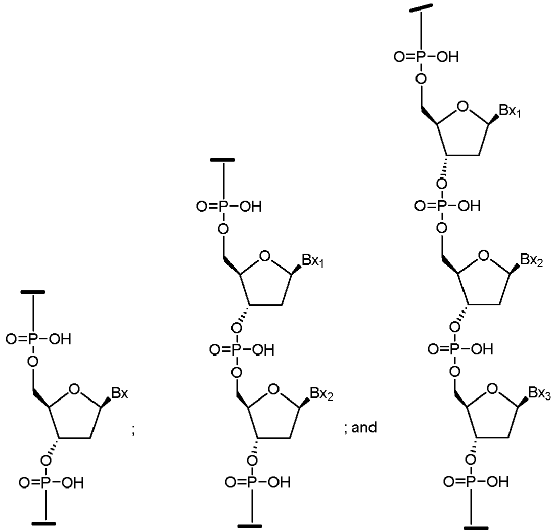 wherein each of Bx, Bx₁, Bx₂, and Bx₃ is independently a heterocyclic base moiety. In certain embodiments, the cleavable moiety has a structure selected from among the following:

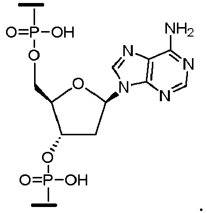

In certain embodiments, the cleavable moiety is covalently attached to the 3'-end of the sense strand of a double-stranded siRNA compound. In certain embodiments, the cleavable moiety is covalently attached to the 5'-end of the sense strand of a double-stranded siRNA compound.

### v. Certain Linkers

In certain embodiments, the conjugate groups comprise a linker. In certain such embodiments, the linker is covalently bound to the cleavable moiety. In certain such embodiments, the linker is covalently bound to the antisense oligonucleotide. In certain embodiments, the linker is covalently bound to a cell-targeting moiety. In certain embodiments, the linker further comprises a covalent attachment to a solid support. In certain embodiments, the linker further comprises a covalent attachment to a protein binding moiety. In certain embodiments, the linker further comprises a covalent attachment to a solid support and further comprises a covalent attachment to a protein binding moiety. In certain embodiments, the linker includes multiple positions for attachment of tethered ligands. In certain embodiments, the linker includes multiple positions for attachment of tethered ligands and is not attached to a branching group. In certain embodiments, the linker further comprises one or more cleavable bond. In certain embodiments, the conjugate group does not include a linker.

In certain embodiments, the linker includes at least a linear group comprising groups selected from alkyl, amide, disulfide, polyethylene glycol, ether, thioether (-S-) and hydroxylamino (-O-N(H)-) groups. In certain embodiments, the linear group comprises groups selected from alkyl, amide and ether groups. In certain embodiments, the linear group comprises groups selected from alkyl and ether groups. In certain embodiments, the linear group comprises at least one phosphorus linking group. In certain embodiments, the linear group comprises at least one phosphodiester group. In certain embodiments, the linear group includes at least one neutral linking group. In certain embodiments, the linear group is covalently attached to the cell-targeting moiety and the cleavable moiety. In certain embodiments, the linear group is covalently attached to the cell-targeting moiety and the antisense oligonucleotide. In certain embodiments, the linear group is covalently attached to the cell-targeting moiety, the cleavable moiety and a solid support. In certain embodiments, the linear group is covalently attached to the cell-targeting moiety, the cleavable moiety, a solid support and a protein binding moiety. In certain embodiments, the linear group includes one or more cleavable bond.

In certain embodiments, the linker includes the linear group covalently attached to a scaffold group. In certain embodiments, the scaffold includes a branched aliphatic group comprising groups selected from alkyl, amide, disulfide, polyethylene glycol, ether, thioether and hydroxylamino groups. In certain embodiments, the scaffold includes a branched aliphatic group comprising groups selected from alkyl, amide and ether groups. In certain embodiments, the scaffold includes at least one mono or polycyclic ring system. In certain embodiments, the scaffold includes at least two mono or polycyclic ring systems. In certain embodiments, the linear group is covalently attached to the scaffold group and the scaffold group is covalently attached to the cleavable moiety and the linker. In certain embodiments, the linear group is covalently attached to the scaffold group and the scaffold group is covalently attached to the cleavable moiety, the linker and a solid support. In certain embodiments, the linear group is covalently attached to the scaffold group and the scaffold group is covalently attached to the cleavable moiety, the linker and a protein binding moiety. In certain embodiments, the linear group is covalently attached to the scaffold group and the scaffold group is covalently attached to the cleavable moiety, the linker, a protein binding moiety and a solid support. In certain embodiments, the scaffold group includes one or more cleable bond.

In certain embodiments, the linker includes a protein binding moiety. In certain embodiments, the protein binding moiety is a lipid such as for example including but not limited to cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine), a vitamin (e.g., folate, vitamin A, vitamin E, biotin, pyridoxal), a peptide, a carbohydrate (e.g., monosaccharide, disaccharide, trisaccharide, tetrasaccharide, oligosaccharide, polysaccharide), an endosomolytic component, a steroid (e.g., uvaol, hecigenin, diosgenin), a terpene (e.g., triterpene, e.g., sarsasapogenin, friedelin, epifriedelanol derivatized lithocholic acid), or a cationic lipid. In certain embodiments, the protein binding moiety is a C16 to C22 long chain saturated or unsaturated fatty acid, cholesterol, cholic acid, vitamin E, adamantane or 1-pentafluoropropyl.

In certain embodiments, a linker has a structure selected from among: 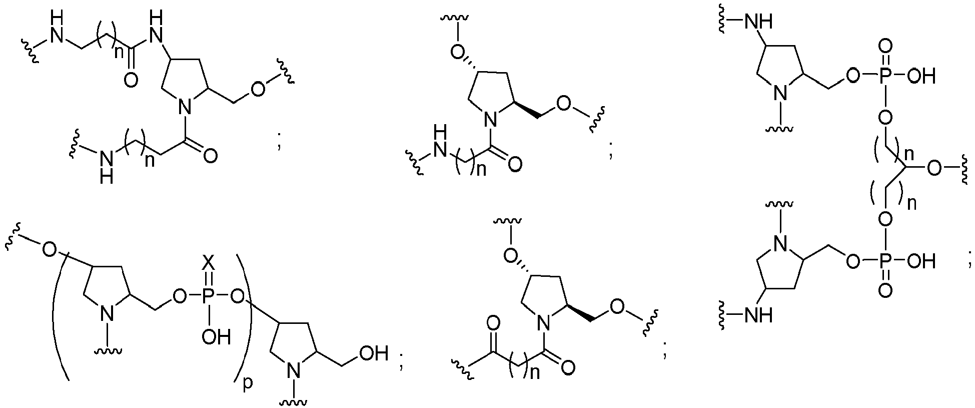 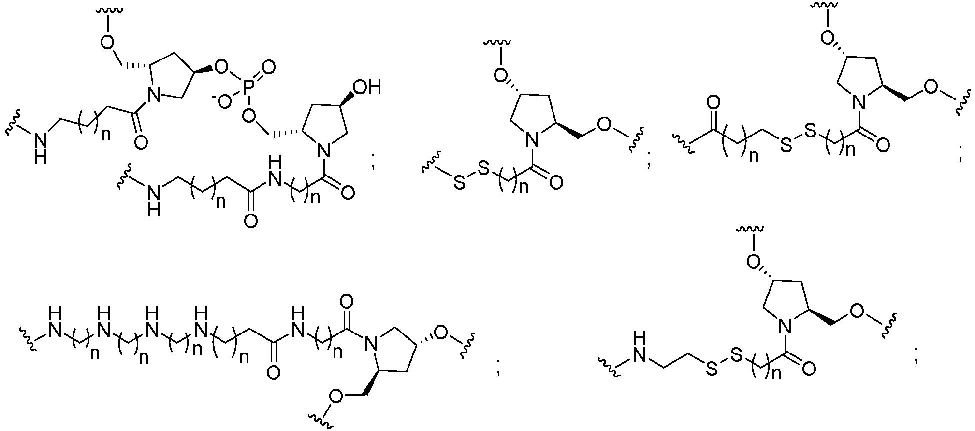 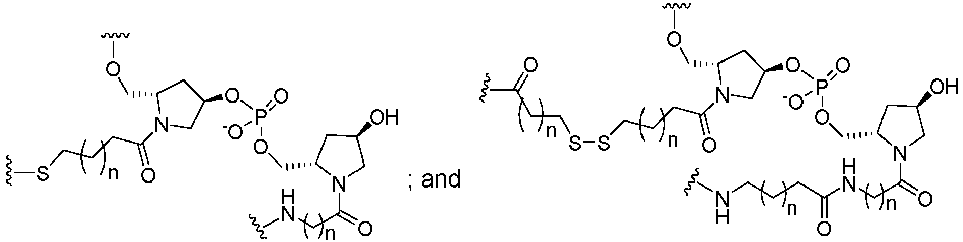 wherein each n is, independently, from 1 to 20; and p is from 1 to 6.

In certain embodiments, a linker has a structure selected from among: 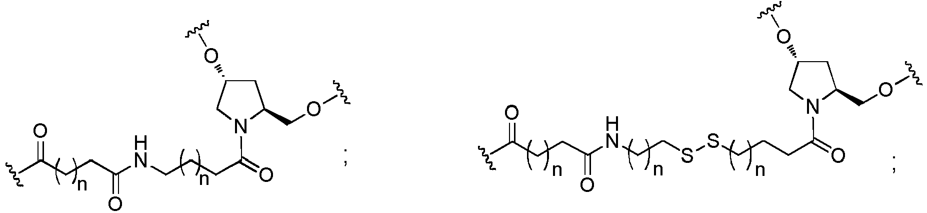 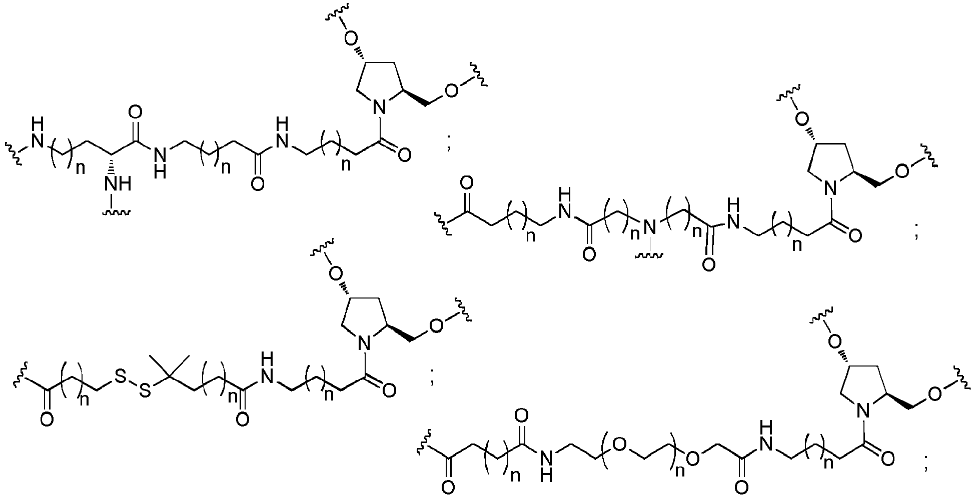 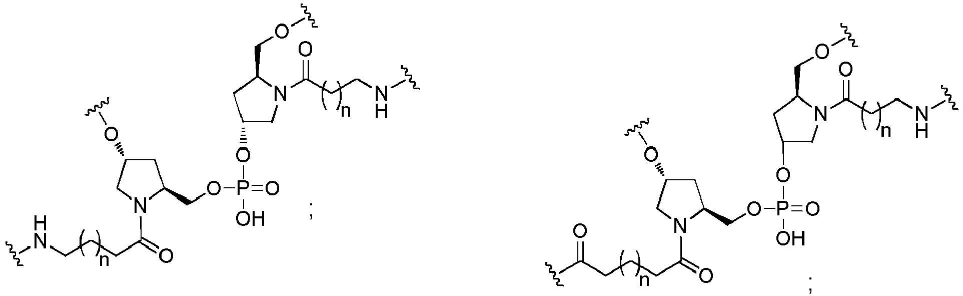 and 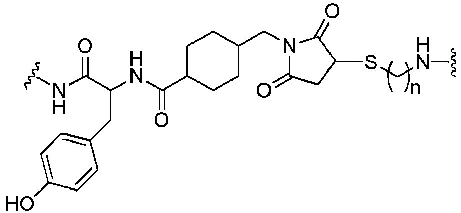 wherein each n is, independently, from 1 to 20.

In certain embodiments, a linker has a structure selected from among: 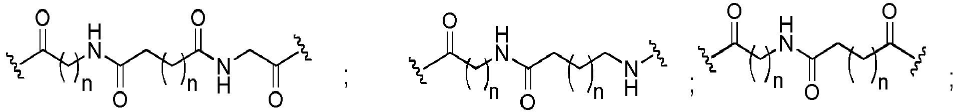 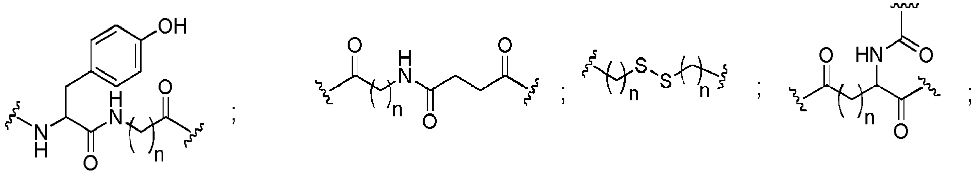 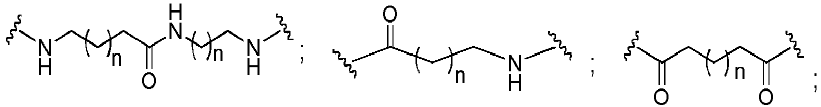 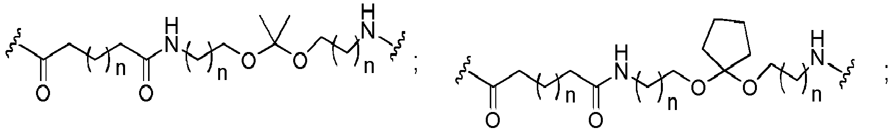 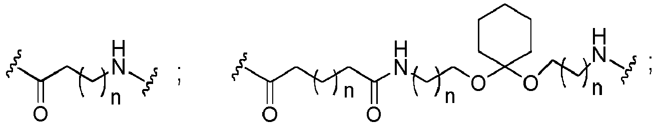 and 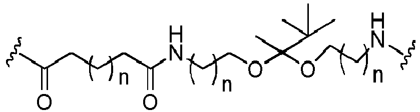 wherein n is from 1 to 20.

In certain embodiments, a linker has a structure selected from among: 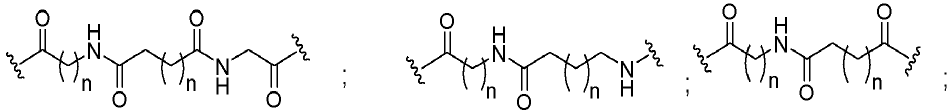 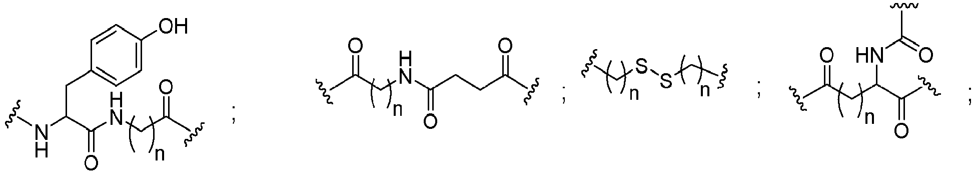 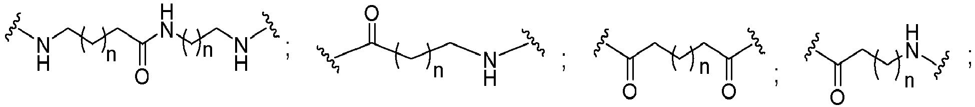 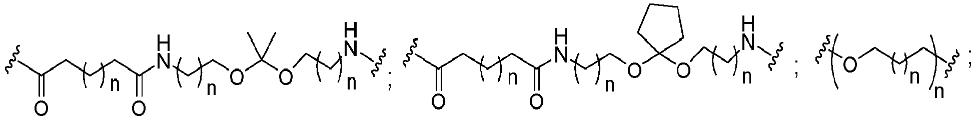 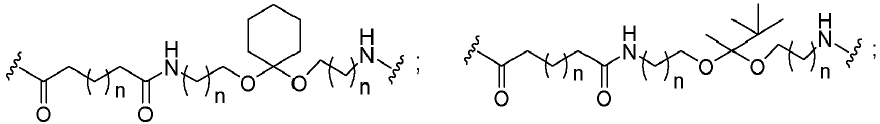 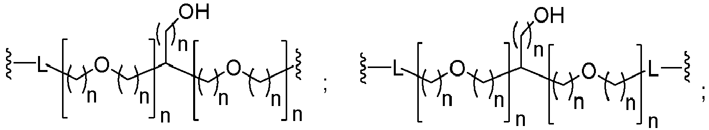 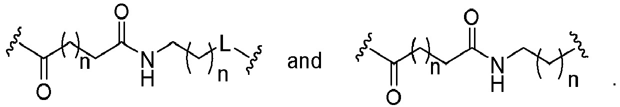
wherein each L is, independently, a phosphorus linking group or a neutral linking group; and
each n is, independently, from 1 to 20.

In certain embodiments, a linker has a structure selected from among: 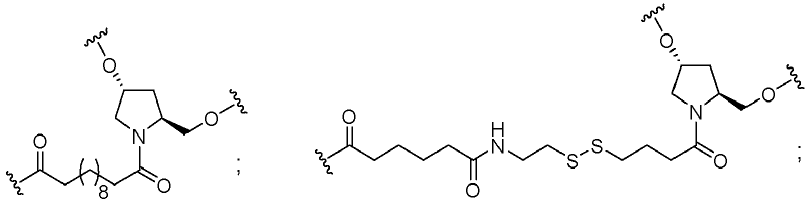 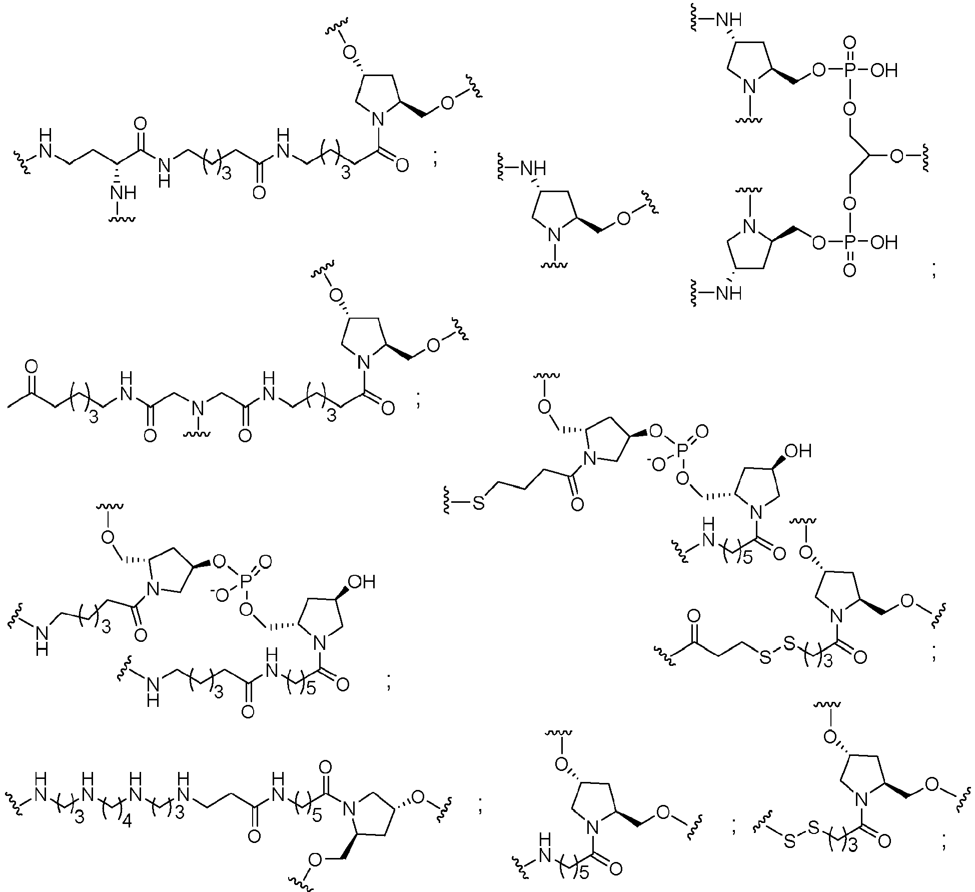 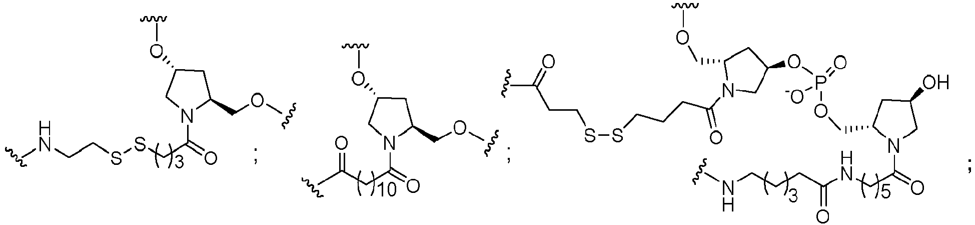 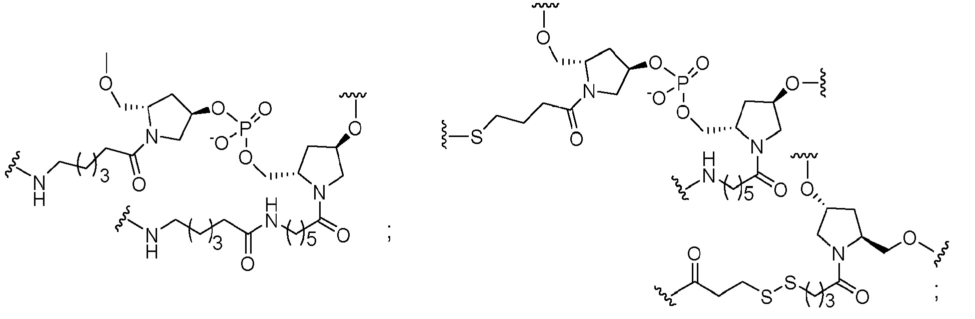 and

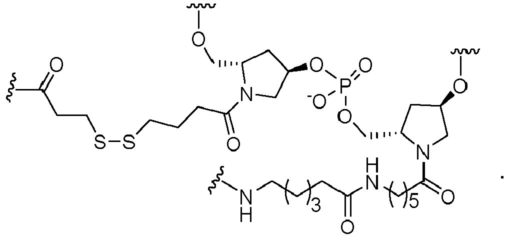

In certain embodiments, a linker has a structure selected from among: 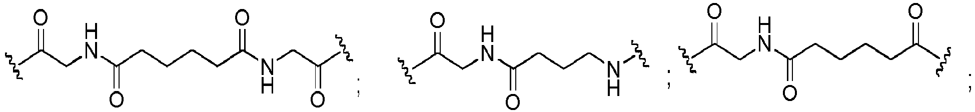 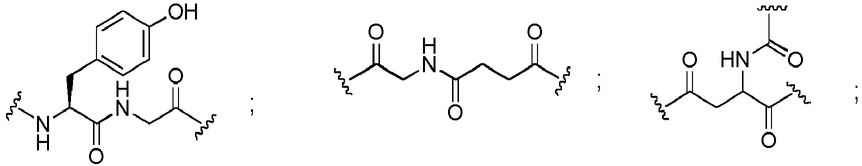 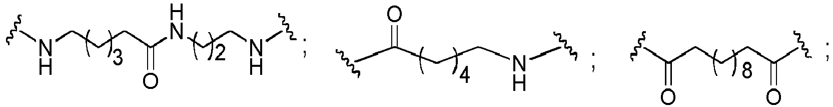 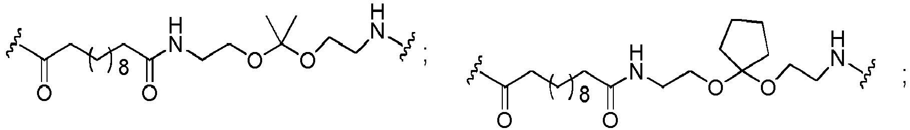 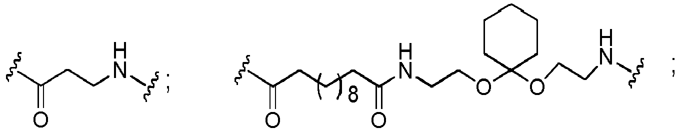 and

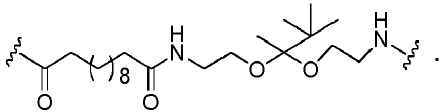

In certain embodiments, a linker has a structure selected from among: 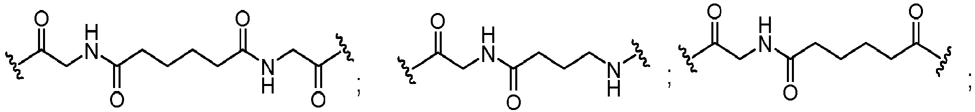 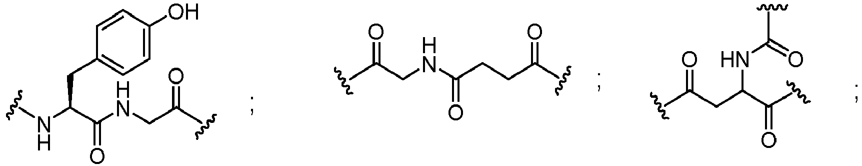 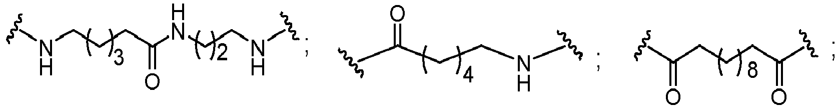 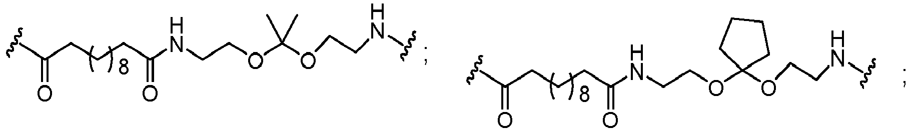 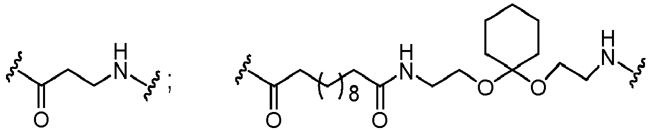 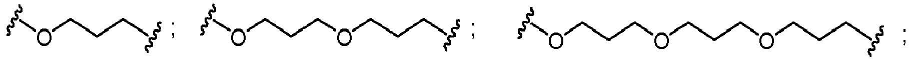 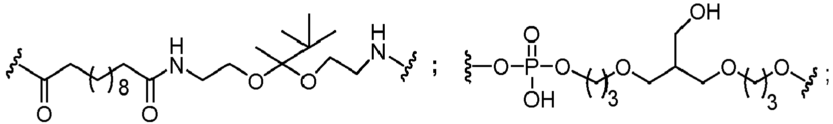 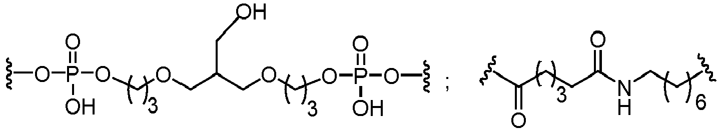 and

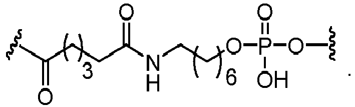

In certain embodiments, a linker has a structure selected from among: 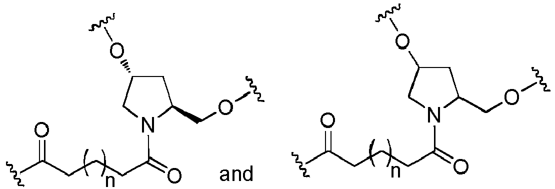 wherein n is from 1 to 20.

In certain embodiments, a linker has a structure selected from among:

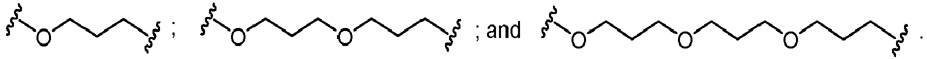

In certain embodiments, a linker has a structure selected from among:

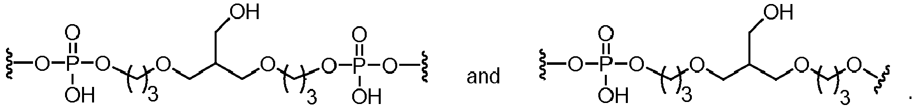

In certain embodiments, a linker has a structure selected from among:

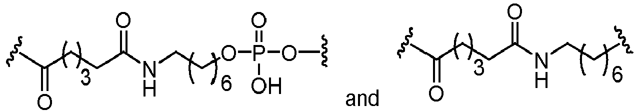

In certain embodiments, the conjugate linker has the structure:

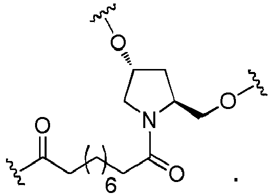

### vi. Certain Cell-Targeting Moieties

In certain embodiments, conjugate groups comprise cell-targeting moieties. Certain such cell-targeting moieties increase cellular uptake of antisense compounds. In certain embodiments, cell-targeting moieties comprise a branching group, one or more tether, and one or more ligand. In certain embodiments, cell-targeting moieties comprise a branching group, one or more tether, one or more ligand and one or more cleavable bond.

### 1. Certain Branching Groups

In certain embodiments, the conjugate groups comprise a targeting moiety comprising a branching group and at least two tethered ligands. In certain embodiments, the branching group attaches the conjugate linker. In certain embodiments, the branching group attaches the cleavable moiety. In certain embodiments, the branching group attaches the antisense oligonucleotide. In certain embodiments, the branching group is covalently attached to the linker and each of the tethered ligands. In certain embodiments, the branching group comprises a branched aliphatic group comprising groups selected from alkyl, amide, disulfide, polyethylene glycol, ether, thioether and hydroxylamino groups. In certain embodiments, the branching group comprises groups selected from alkyl, amide and ether groups. In certain embodiments, the branching group comprises groups selected from alkyl and ether groups. In certain embodiments, the branching group comprises a mono or polycyclic ring system. In certain embodiments, the branching group comprises one or more cleavable bond. In certain embodiments, the conjugate group does not include a branching group.

In certain embodiments, a branching group has a structure selected from among: 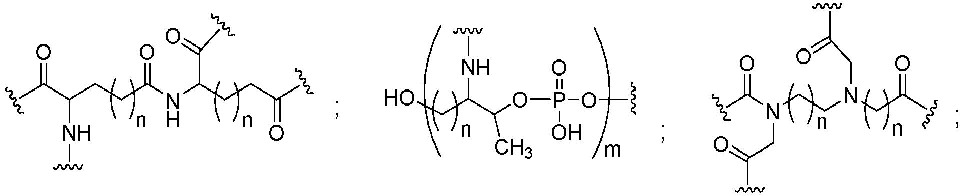 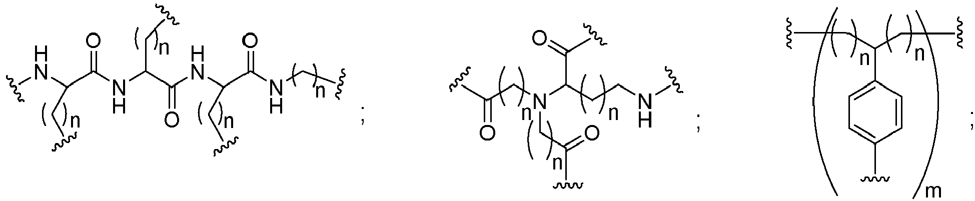 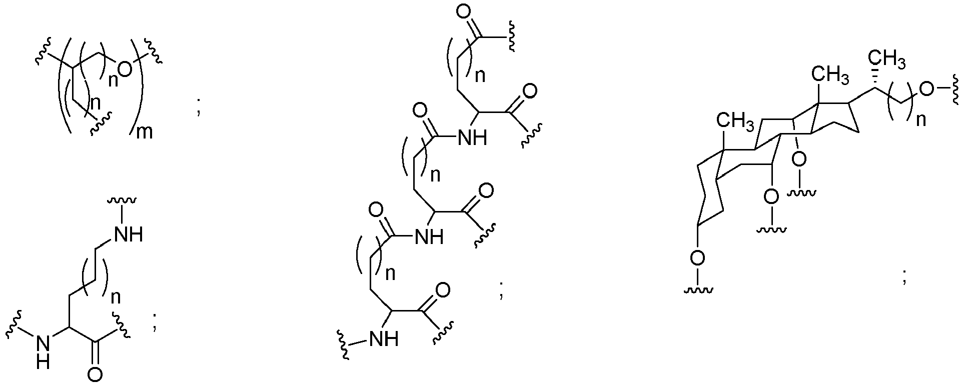 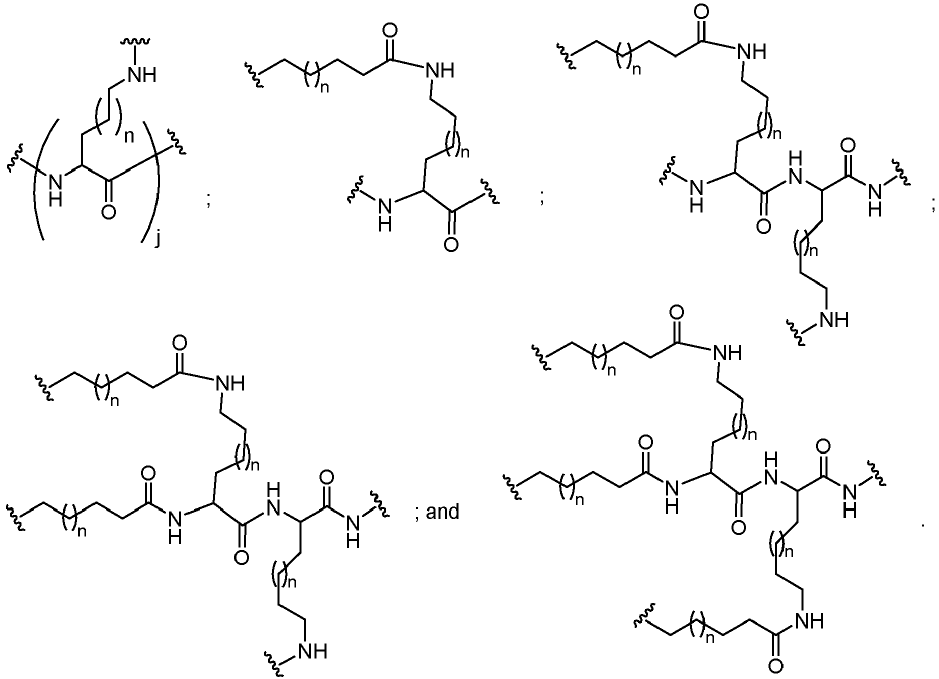
wherein each n is, independently, from 1 to 20;
j is from 1 to 3; and
m is from 2 to 6.

In certain embodiments, a branching group has a structure selected from among: 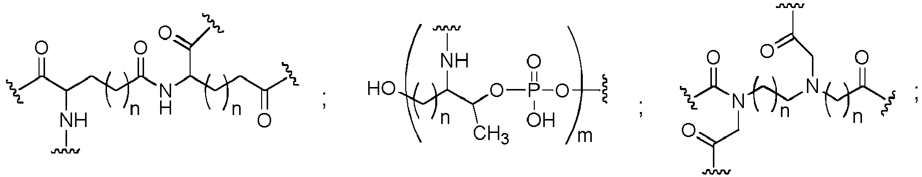 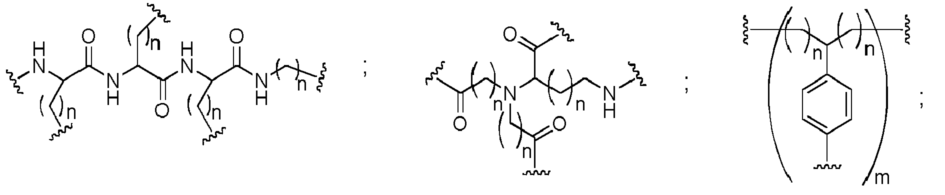 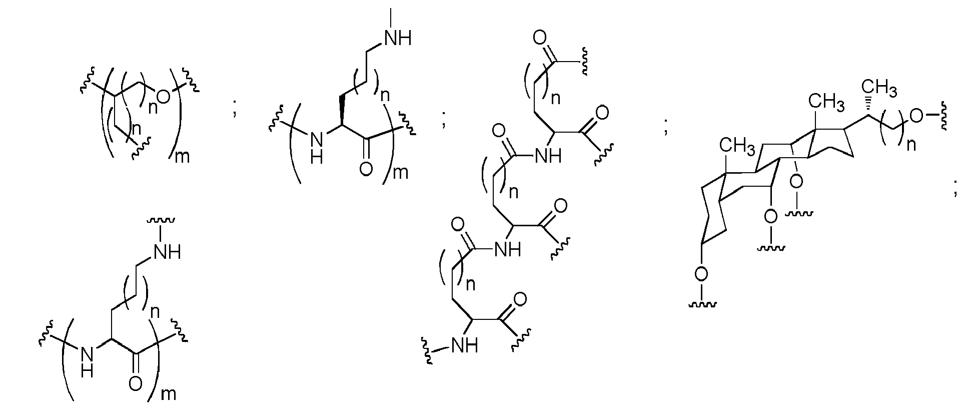 and
wherein each n is, independently, from 1 to 20; and
m is from 2 to 6.

In certain embodiments, a branching group has a structure selected from among:

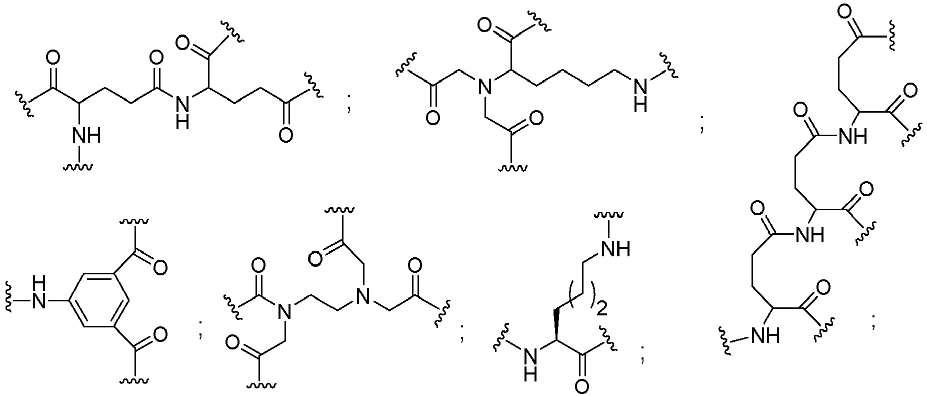

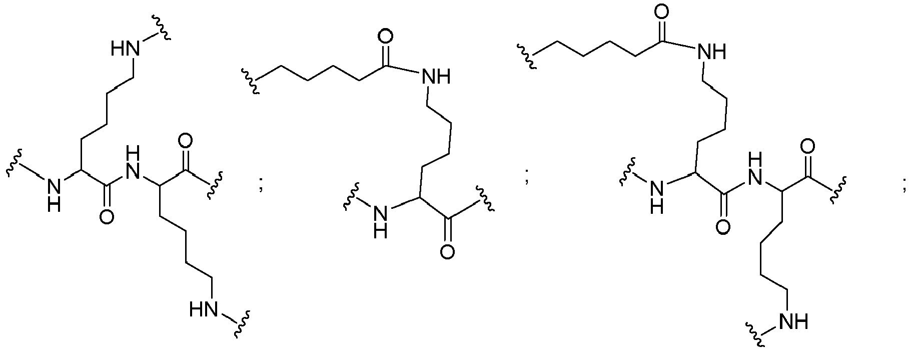

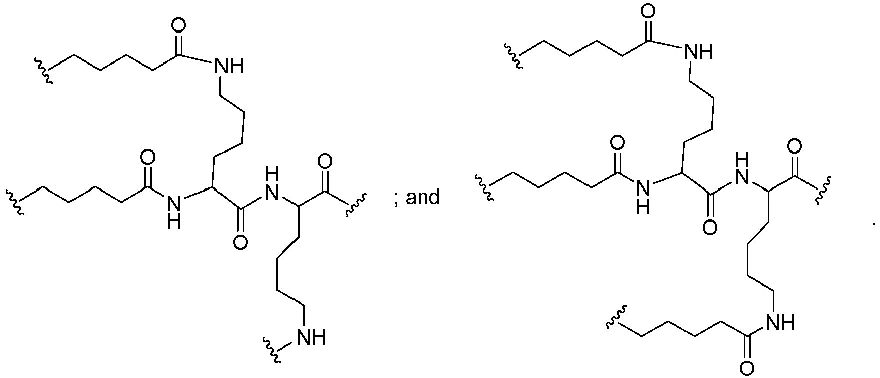

In certain embodiments, a branching group has a structure selected from among: 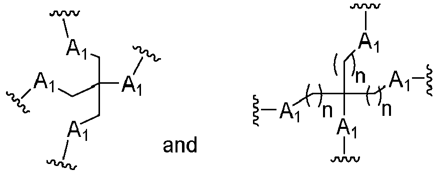
wherein each A₁ is independently, O, S, C=O or NH; and
each n is, independently, from 1 to 20.

In certain embodiments, a branching group has a structure selected from among: 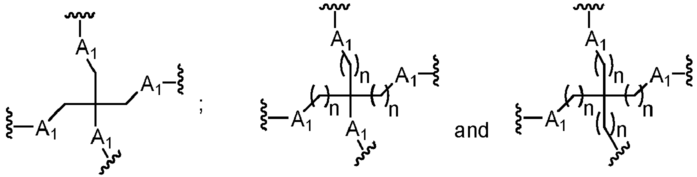
wherein each A₁ is independently, O, S, C=O or NH; and
each n is, independently, from 1 to 20.

In certain embodiments, a branching group has a structure selected from among: 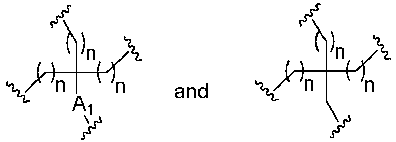
wherein A₁ is O, S, C=O or NH; and
each n is, independently, from 1 to 20.

In certain embodiments, a branching group has a structure selected from among:

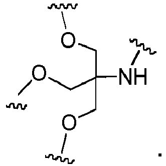

In certain embodiments, a branching group has a structure selected from among:

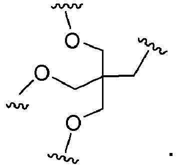

In certain embodiments, a branching group has a structure selected from among:

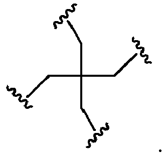

### 2. Certain Tethers

In certain embodiments, conjugate groups comprise one or more tethers covalently attached to the branching group. In certain embodiments, conjugate groups comprise one or more tethers covalently attached to the linking group. In certain embodiments, each tether is a linear aliphatic group comprising one or more groups selected from alkyl, ether, thioether, disulfide, amide and polyethylene glycol groups in any combination. In certain embodiments, each tether is a linear aliphatic group comprising one or more groups selected from alkyl, substituted alkyl, ether, thioether, disulfide, amide, phosphodiester and polyethylene glycol groups in any combination. In certain embodiments, each tether is a linear aliphatic group comprising one or more groups selected from alkyl, ether and amide groups in any combination. In certain embodiments, each tether is a linear aliphatic group comprising one or more groups selected from alkyl, substituted alkyl, phosphodiester, ether and amide groups in any combination. In certain embodiments, each tether is a linear aliphatic group comprising one or more groups selected from alkyl and phosphodiester in any combination. In certain embodiments, each tether comprises at least one phosphorus linking group or neutral linking group.

In certain embodiments, the tether includes one or more cleabable bond. In certain embodiments, the tether is attached to the branching group through either an amide or an ether group. In certain embodiments, the tether is attached to the branching group through a phosphodiester group. In certain embodiments, the tether is attached to the branching group through a phosphorus linking group or neutral linking group. In certain embodiments, the tether is attached to the branching group through an ether group. In certain embodiments, the tether is attached to the ligand through either an amide or an ether group. In certain embodiments, the tether is attached to the ligand through an ether group. In certain embodiments, the tether is attached to the ligand through either an amide or an ether group. In certain embodiments, the tether is attached to the ligand through an ether group.

In certain embodiments, each tether comprises from about 8 to about 20 atoms in chain length between the ligand and the branching group. In certain embodiments, each tether group comprises from about 10 to about 18 atoms in chain length between the ligand and the branching group. In certain embodiments, each tether group comprises about 13 atoms in chain length.

In certain embodiments, a tether has a structure selected from among: 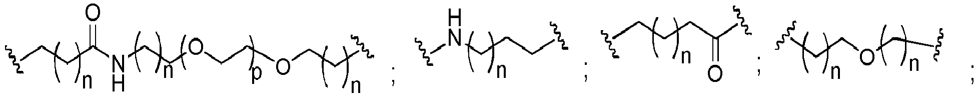 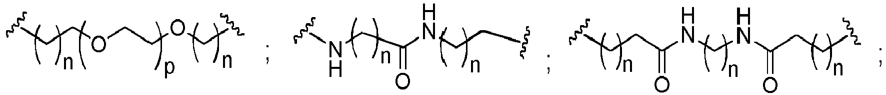 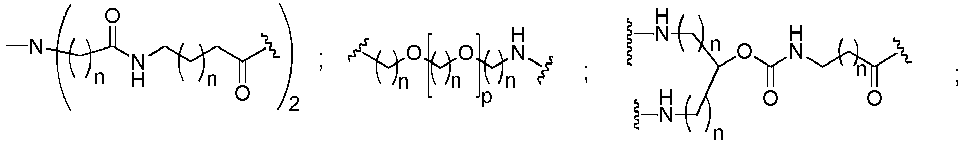 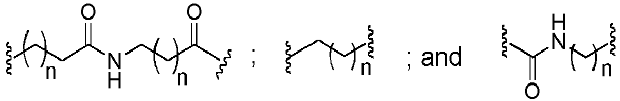
wherein each n is, independently, from 1 to 20; and
each p is from 1 to about 6.

In certain embodiments, a tether has a structure selected from among:

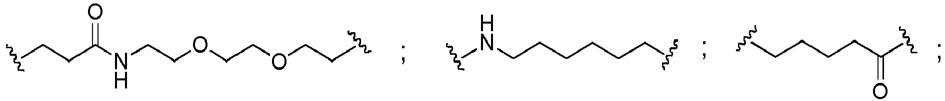

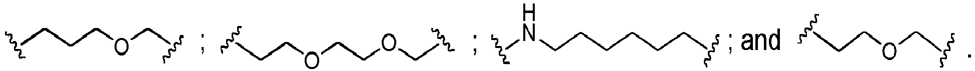

In certain embodiments, a tether has a structure selected from among: 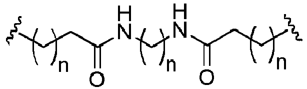 wherein each n is, independently, from 1 to 20.

In certain embodiments, a tether has a structure selected from among: 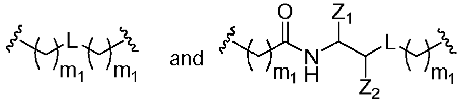
wherein L is either a phosphorus linking group or a neutral linking group;
Z₁ is C(=O)O-R₂;
Z₂ is H, C₁-C₆ alkyl or substituted C₁-C₆ alky;
R₂ is H, C₁-C₆ alkyl or substituted C₁-C₆ alky; and
each m₁ is, independently, from 0 to 20 wherein at least one m₁ is greater than 0 for each tether.

In certain embodiments, a tether has a structure selected from among:

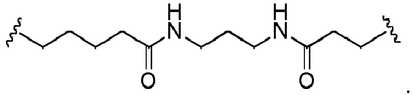

In certain embodiments, a tether has a structure selected from among: 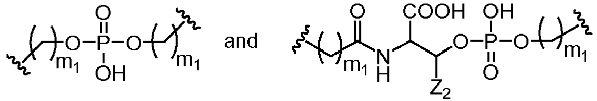
wherein Z₂ is H or CH₃; and
each m₁ is, independently, from 0 to 20 wherein at least one m₁ is greater than 0 for each tether.

In certain embodiments, a tether comprises a phosphorus linking group. In certain embodiments, a tether does not comprise any amide bonds. In certain embodiments, a tether comprsises a phosphorus linking group and does not comprise any amide bonds.

### 3. Certain Ligands

In certain embodiments, the present disclosure provides ligands wherein each ligand is covalently attached to a tether. In certain embodiments, each ligand is selected to have an affinity for at least one type of receptor on a target cell. In certain embodiments, ligands are selected that have an affinity for at least one type of receptor on the surface of a mammalian liver cell. In certain embodiments, ligands are selected that have an affinity for the hepatic asialoglycoprotein receptor (ASGP-R). In certain embodiments, each ligand is a carbohydrate. In certain embodiments, each ligand is, independently selected from galactose, N-acetyl galactoseamine, mannose, glucose, glucosamone and fucose. In certain embodiments, each ligand is N-acetyl galactoseamine (GalNAc). In certain embodiments, the targeting moiety comprises 2 to 6 ligands. In certain embodiments, the targeting moiety comprises 3 ligands. In certain embodiments, the targeting moiety comprises 3 N-acetyl galactoseamine ligands.

In certain embodiments, the ligand is a carbohydrate, carbohydrate derivative, modified carbohydrate, multivalent carbohydrate cluster, polysaccharide, modified polysaccharide, or polysaccharide derivative. In certain embodiments, the ligand is an amino sugar or a thio sugar. For example, amino sugars may be selected from any number of compounds known in the art, for example glucosamine, sialic acid, α-D-galactosamine, N-Acetylgalactosamine, 2-acetamido-2-deoxy-D-galactopyranose (GalNAc), 2-Amino-3-*O-*[(*R*)-1-carboxyethyl]-2-deoxy-β-D-glucopyranose (β-muramic acid), 2-Deoxy-2-methylamino-L-glucopyranose, 4,6-Dideoxy-4-formamido-2,3-di-*O*-methyl-D-mannopyranose, 2-Deoxy-2-sulfoamino-D-glucopyranose and *N*-sulfo-D-glucosamine, and *N*-Glycoloyl-α-neuraminic acid. For example, thio sugars may be selected from the group consisting of 5-Thio-β-D-glucopyranose, Methyl 2,3,4-tri-*O-*acetyl-1-thio-6-*O*-trityl-α-D-glucopyranoside, 4-Thio-β-D-galactopyranose, and ethyl 3,4,6,7-tetra-*O*-acetyl-2-deoxy-1,5-dithio-α-D-*gluco*-heptopyranoside.

In certain embodiments, "GalNac" or "Gal-NAc" refers to 2-(Acetylamino)-2-deoxy-D-galactopyranose, commonly referred to in the literature as N-acetyl galactosamine. In certain embodiments, "N-acetyl galactosamine" refers to 2-(Acetylamino)-2-deoxy-D-galactopyranose. In certain embodiments, "GalNac" or "Gal-NAc" refers to 2-(Acetylamino)-2-deoxy-D-galactopyranose. In certain embodiments, "GalNac" or "Gal-NAc" refers to 2-(Acetylamino)-2-deoxy-D-galactopyranose, which includes both the β-form: 2-(Acetylamino)-2-deoxy-β-D-galactopyranose and α-form: 2-(Acetylamino)-2-deoxy-D-galactopyranose. In certain embodiments, both the β-form: 2-(Acetylamino)-2-deoxy-β-D-galactopyranose and α-form: 2-(Acetylamino)-2-deoxy-D-galactopyranose may be used interchangeably. Accordingly, in structures in which one form is depicted, these structures are intended to include the other form as well. For example, where the structure for an α-form: 2-(Acetylamino)-2-deoxy-D-galactopyranose is shown, this structure is intended to include the other form as well. In certain embodiments, In certain preferred embodiments, the β-form 2-(Acetylamino)-2-deoxy-D-galactopyranose is the preferred embodiment.

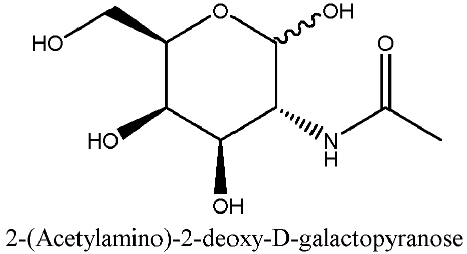

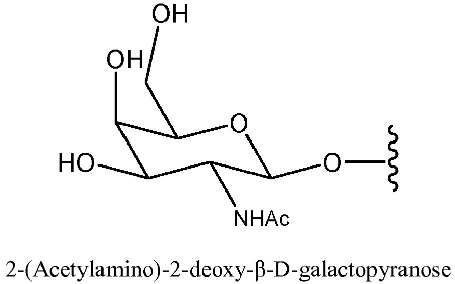

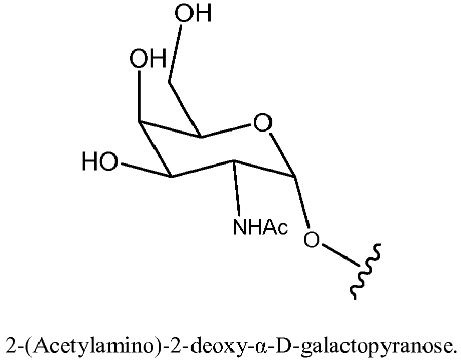

In certain embodimenst one or more ligand has a structure selected from among: 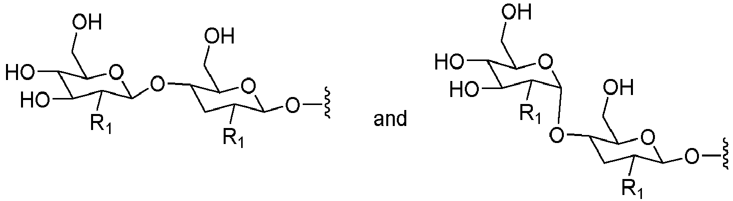 wherein each R₁ is selected from OH and NHCOOH.

In certain embodimenst one or more ligand has a structure selected from among: 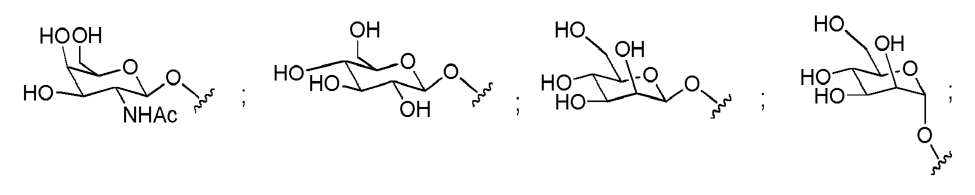 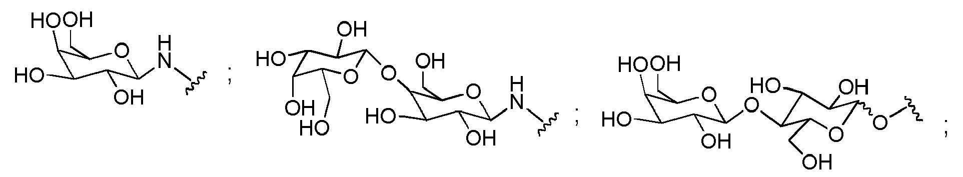 and

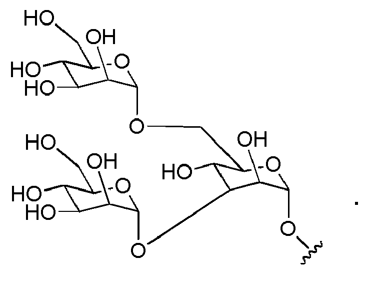

In certain embodimenst one or more ligand has a structure selected from among:

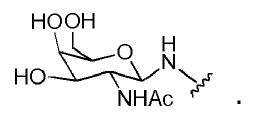

In certain embodimenst one or more ligand has a structure selected from among:

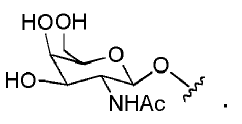

In certain embodiments, conjugate groups comprise the structural features above. In certain such embodiments, conjugate groups have the following structure:

In certain such embodiments, conjugate groups have the following structure: 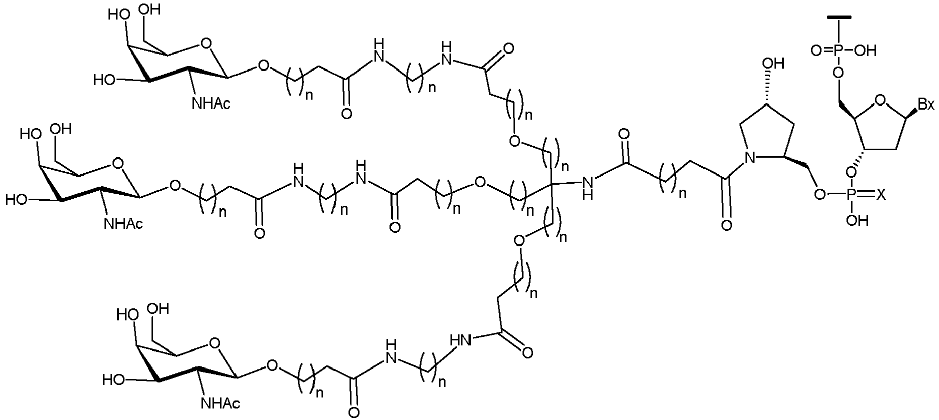
wherein each n is, independently, from 1 to 20;
Z is H or a linked solid support;
Q is an antisense compound;
X is O or S; and
Bx is a heterocyclic base moiety.

In certain such embodiments, conjugate groups have the following structure:

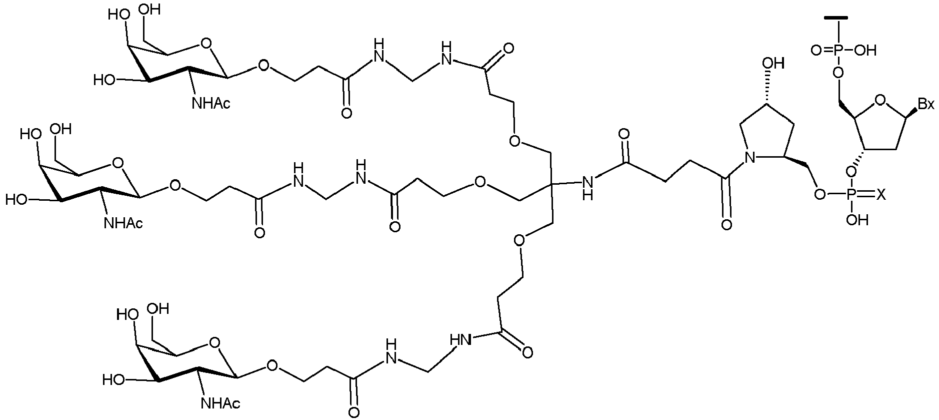

In certain such embodiments, conjugate groups have the following structure:

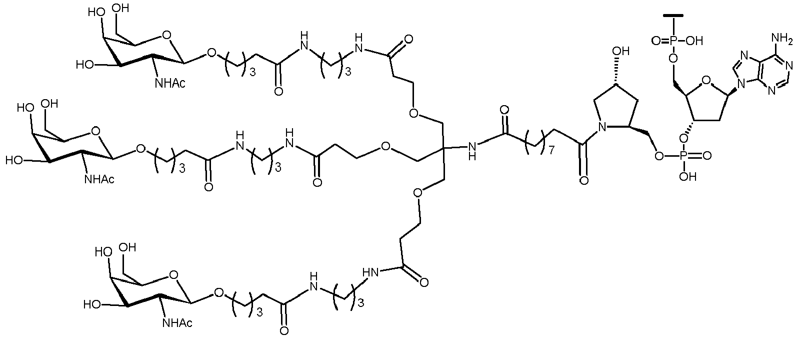

In certain such embodiments, conjugate groups have the following structure:

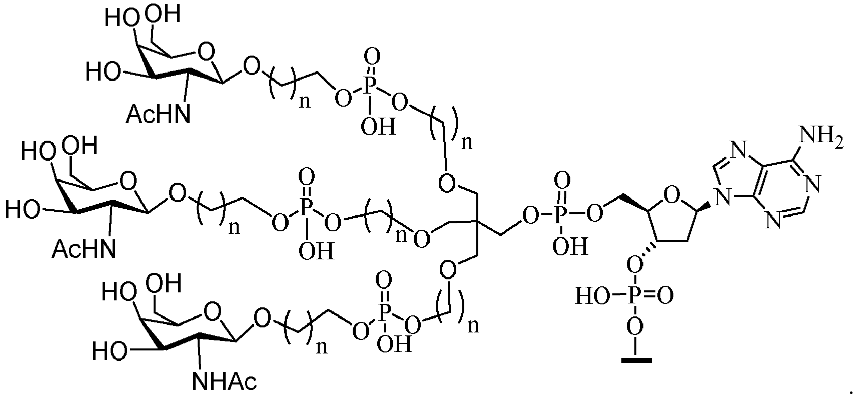

In certain such embodiments, conjugate groups have the following structure:

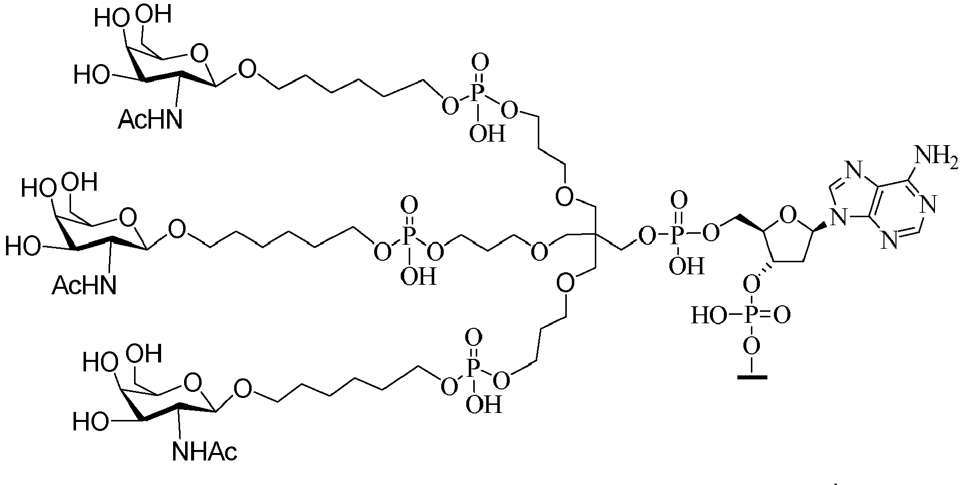

In certain such embodiments, conjugate groups have the following structure:

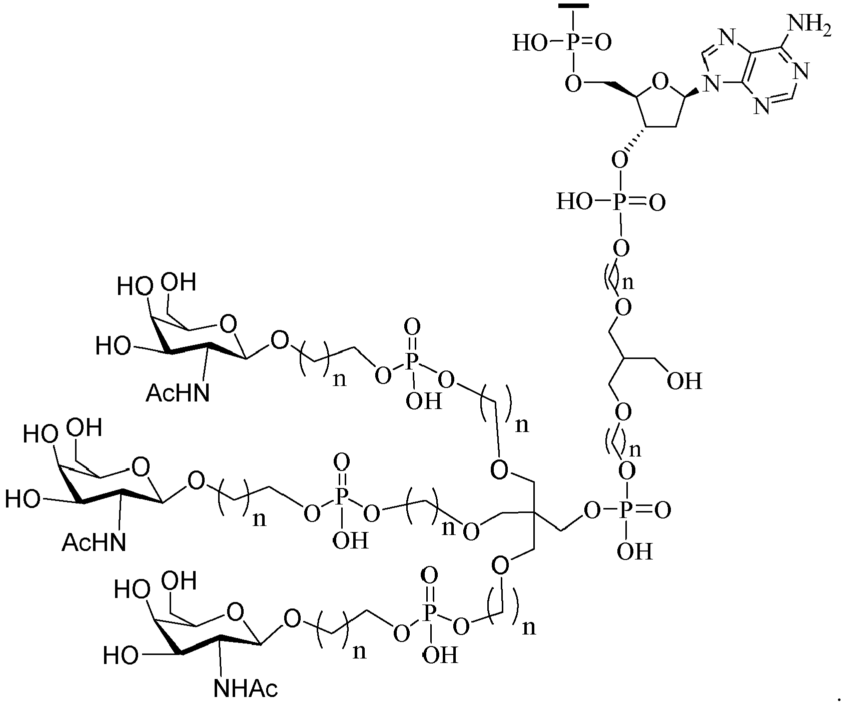

In certain such embodiments, conjugate groups have the following structure:

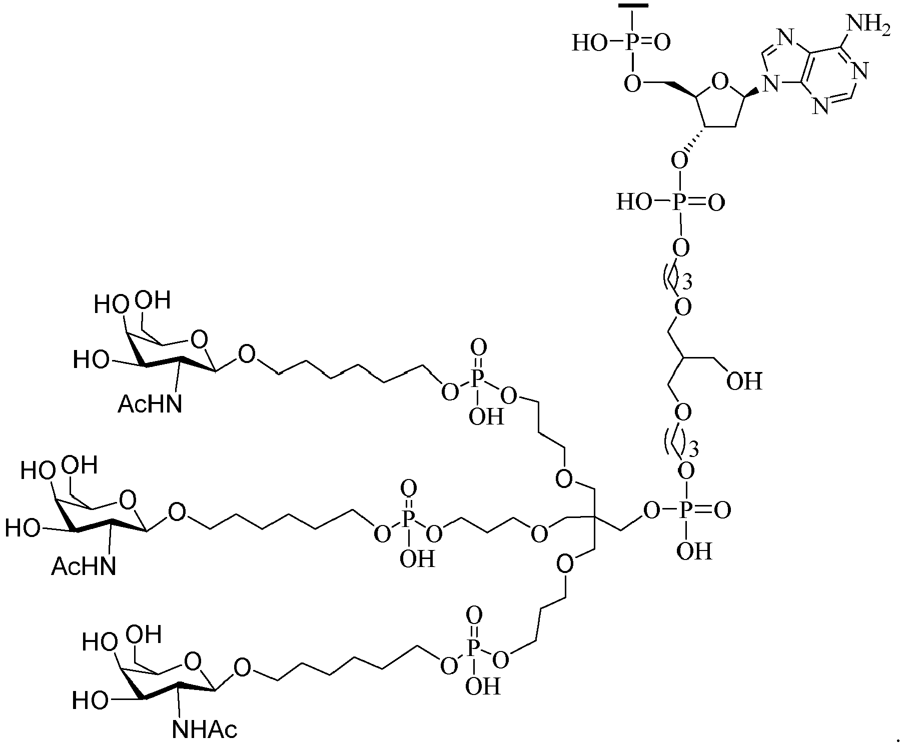

In certain embodiments, conjugates do not comprise a pyrolidine.

In certain embodiments, conjugate groups comprise cell-targeting moieties. In certain embodiments, cell-targeting moieties provide one or more properties to an antisense compound. In certain embodiments, cell-targeting moieties increase the tissue distribution of antisense compounds. In certain embodiments, cell-targeting moieties increase cellular uptake of antisense compounds. In certain embodiments, cell-targeting moieties comprise a branching group, one or more tether, and one or more ligand. In certain embodiments, cell-targeting moieties comprise a branching group, one or more tether, one or more ligand and one or more cleavable bond.

In certain embodiments, cell-targeting moieties have the following structure: 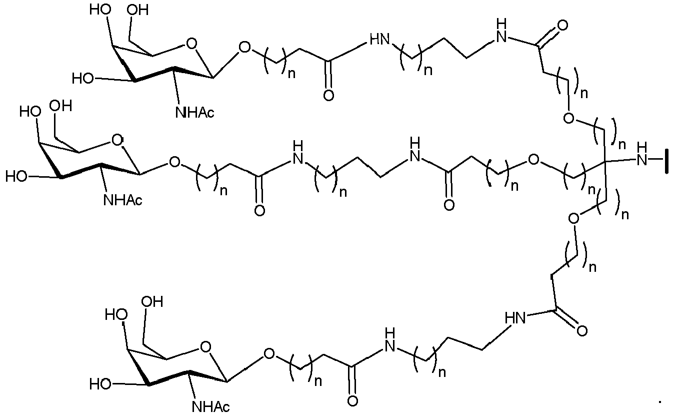 wherein each n is, independently, from 1 to 20.

In certain embodiments, cell-targeting moieties have the following structure:

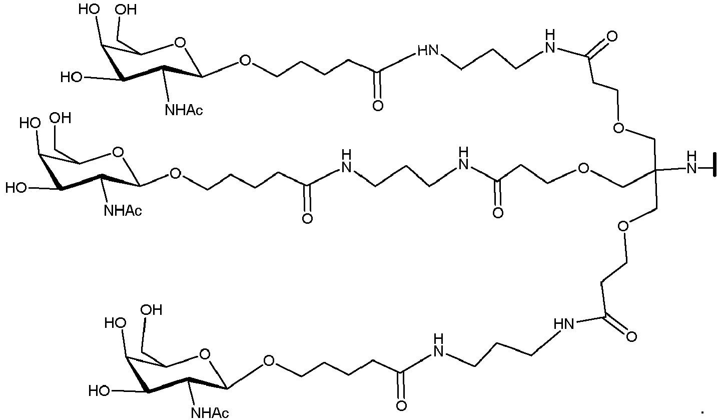

In certain embodiments, cell-targeting moieties have the following structure:

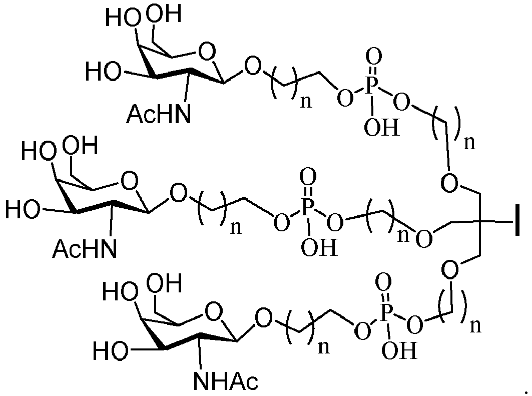

In certain embodiments, cell-targeting moieties have the following structure:

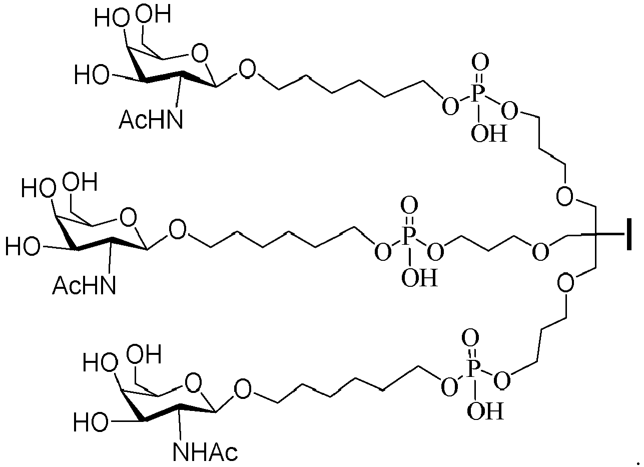

In certain embodiments, cell-targeting moieties have the following structure: 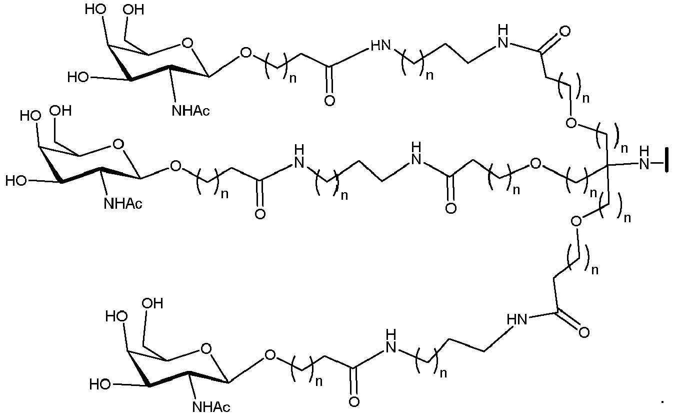 wherein each n is, independently, from 1 to 20.

In certain embodiments, cell-targeting moieties have the following structure:

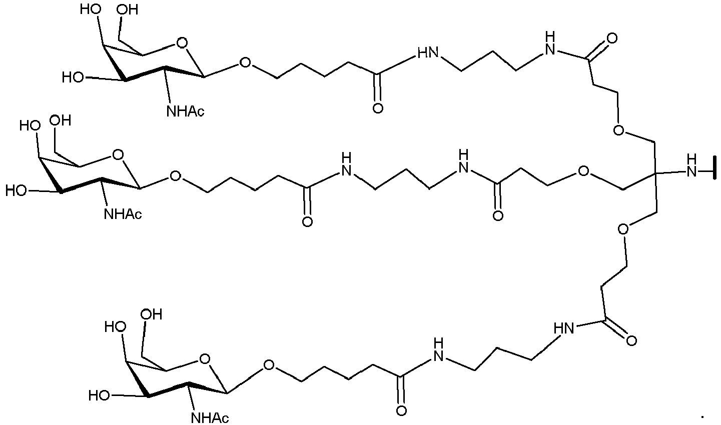

In certain embodiments, cell-targeting moieties have the following structure:

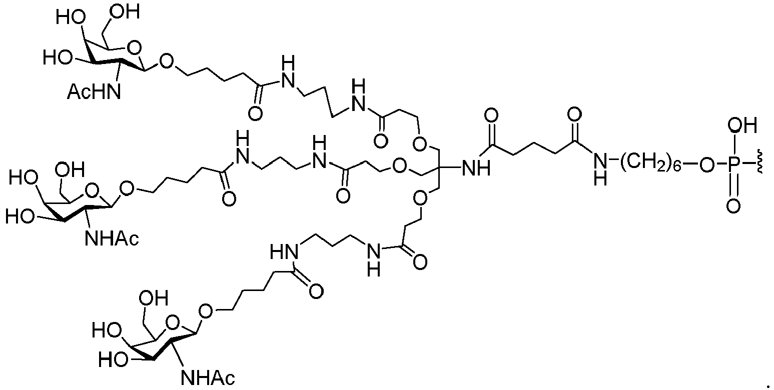

In certain embodiments, cell-targeting moieties have the following structure:

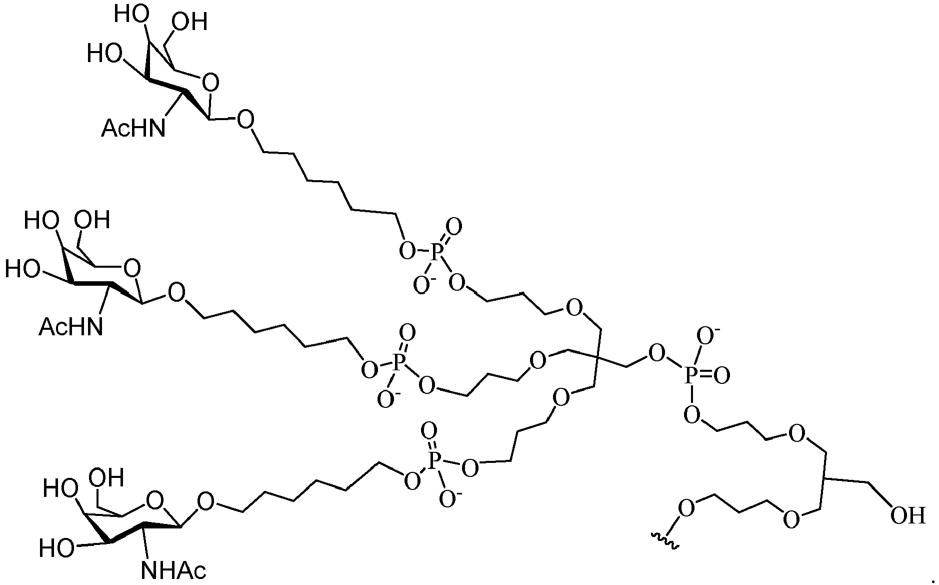

In certain embodiments, cell-targeting moieties have the following structure:

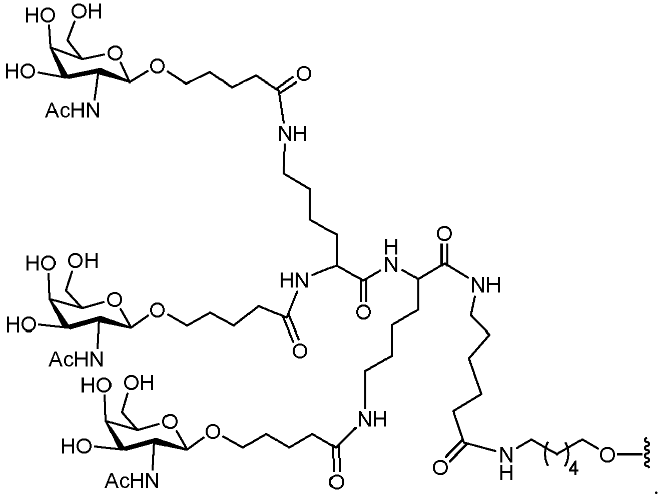

In certain embodiments, cell-targeting moieties have the following structure:

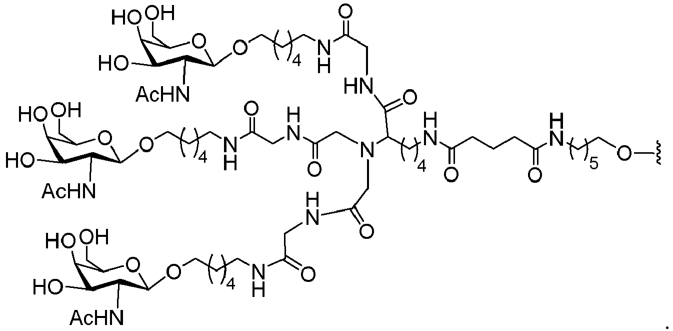

In certain embodiments, cell-targeting moieties have the following structure:

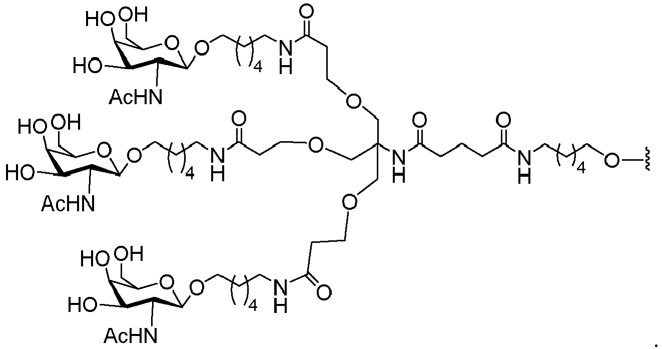

In certain embodiments, cell-targeting moieties have the following structure:

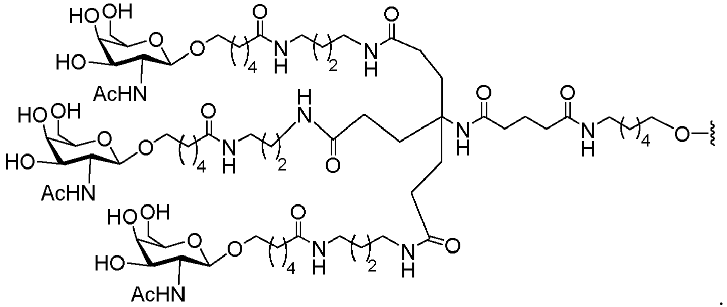

In certain embodiments, cell-targeting moieties have the following structure:

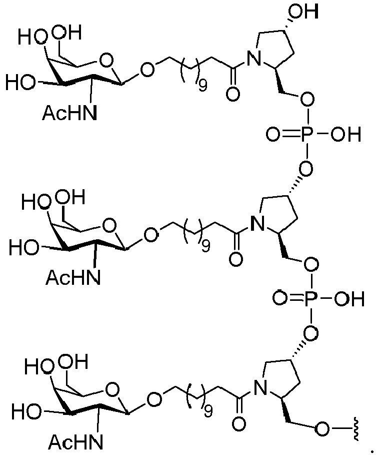

In certain embodiments, cell-targeting moieties have the following structure:

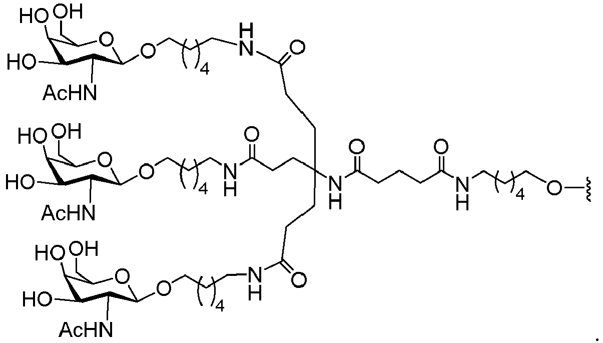

In certain embodiments, cell-targeting moieties have the following structure:

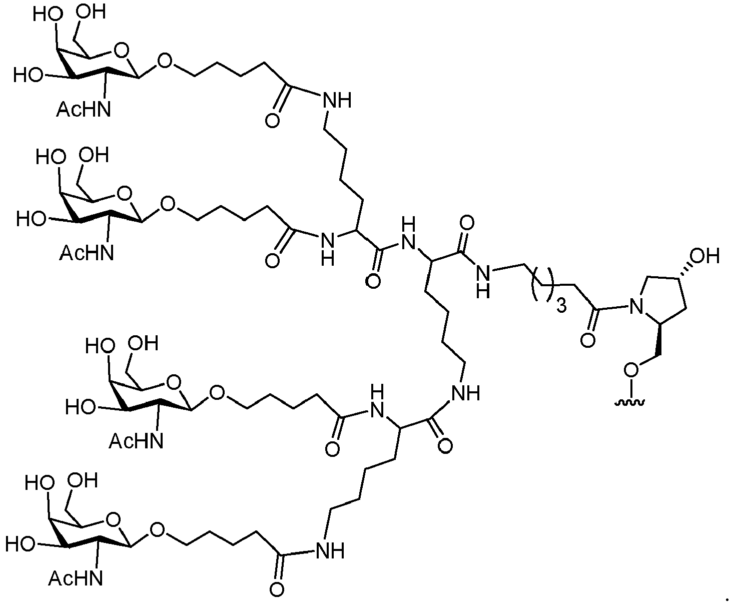

In certain embodiments, conjugate groups comprise the structural features above. In certain such embodiments, conjugate groups have the following structure: 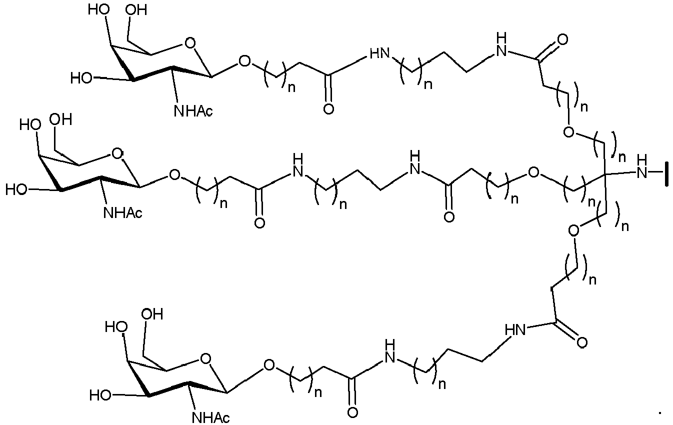 wherein each n is, independently, from 1 to 20.

In certain such embodiments, conjugate groups have the following structure:

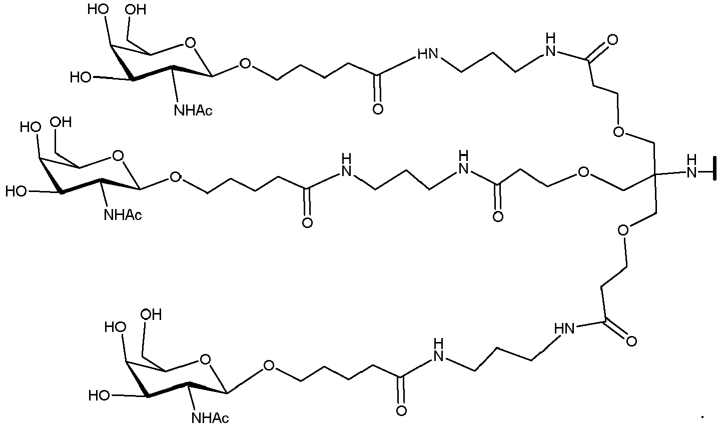

In certain such embodiments, conjugate groups have the following structure: 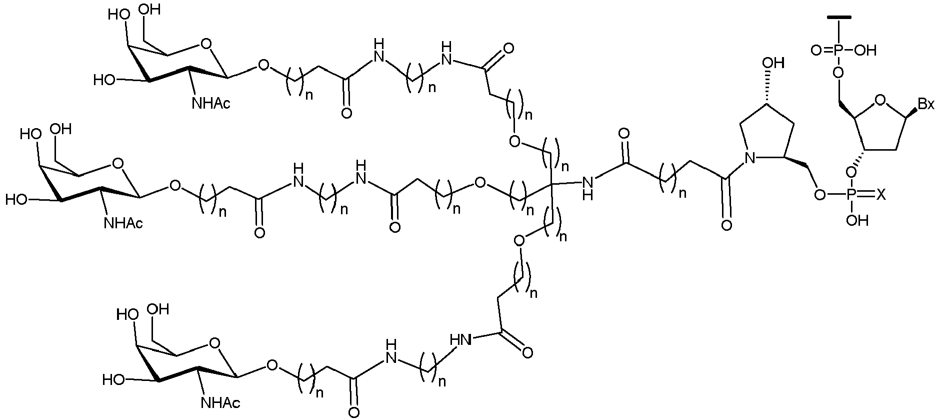
wherein each n is, independently, from 1 to 20;
Z is H or a linked solid support;
Q is an antisense compound;
X is O or S; and
Bx is a heterocyclic base moiety.

In certain such embodiments, conjugate groups have the following structure:

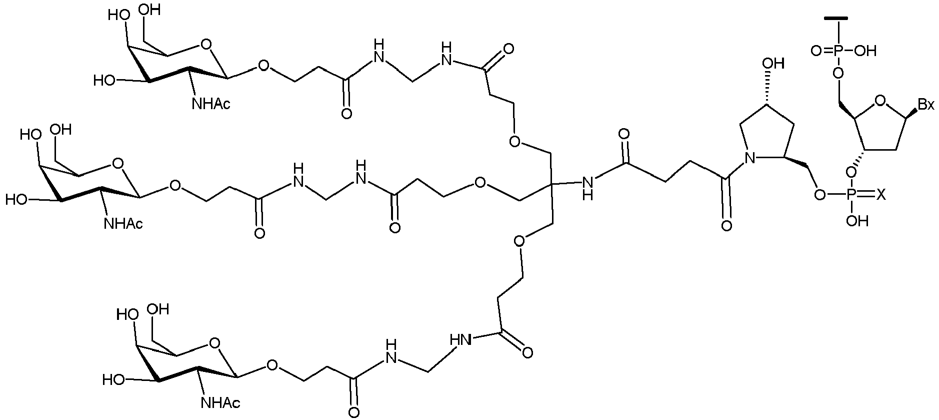

In certain such embodiments, conjugate groups have the following structure:

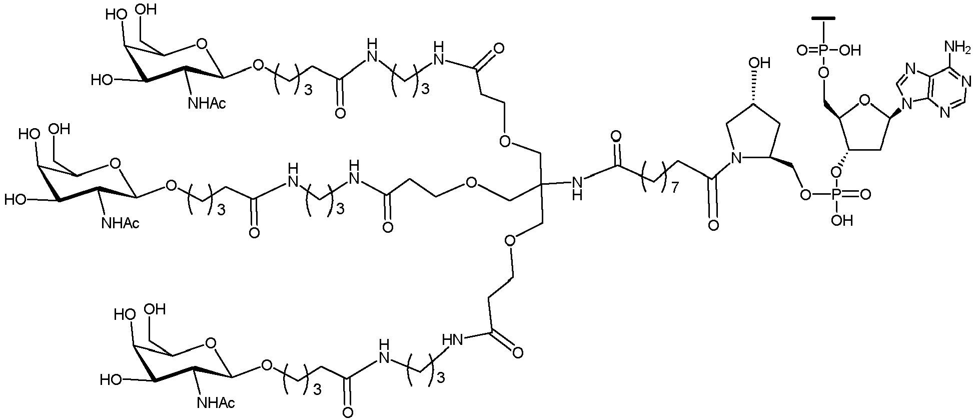

In certain such embodiments, conjugate groups have the following structure:

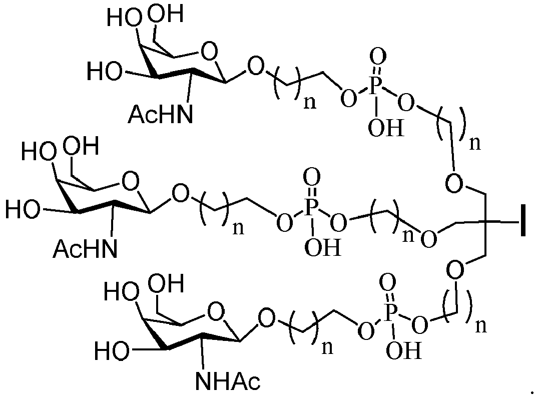

In certain such embodiments, conjugate groups have the following structure:

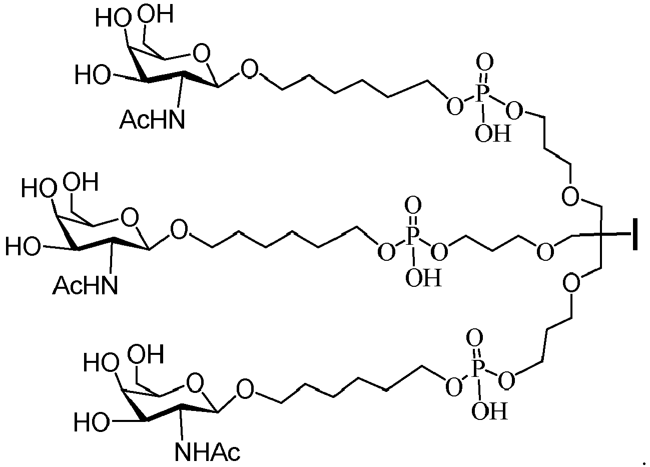

In certain such embodiments, conjugate groups have the following structure:

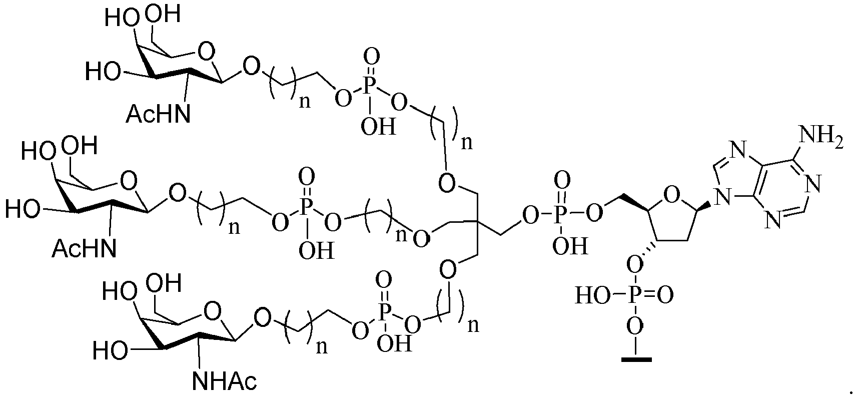

In certain such embodiments, conjugate groups have the following structure:

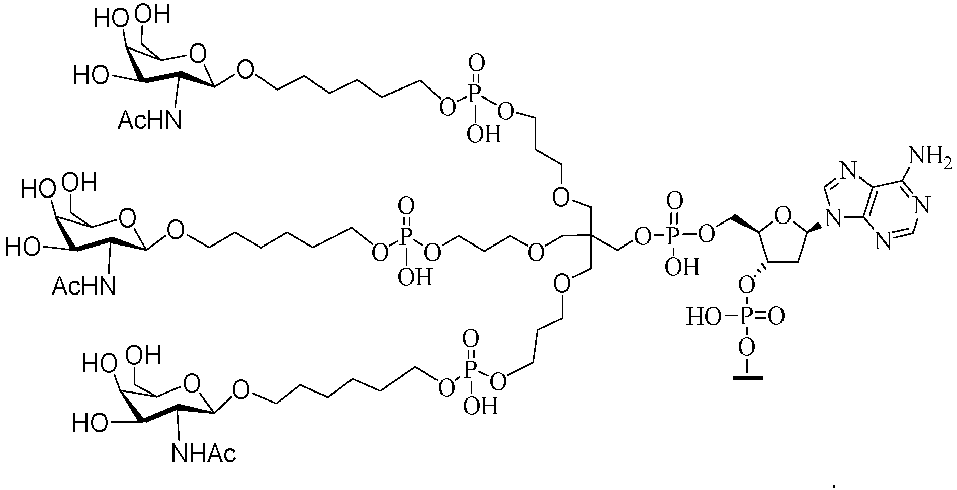

In certain such embodiments, conjugate groups have the following structure:

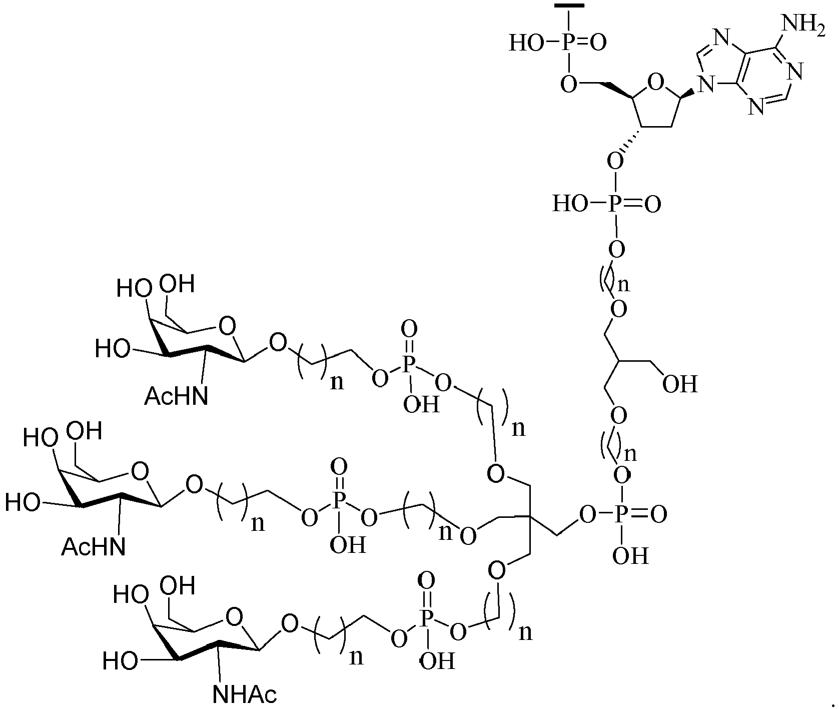

In certain such embodiments, conjugate groups have the following structure:

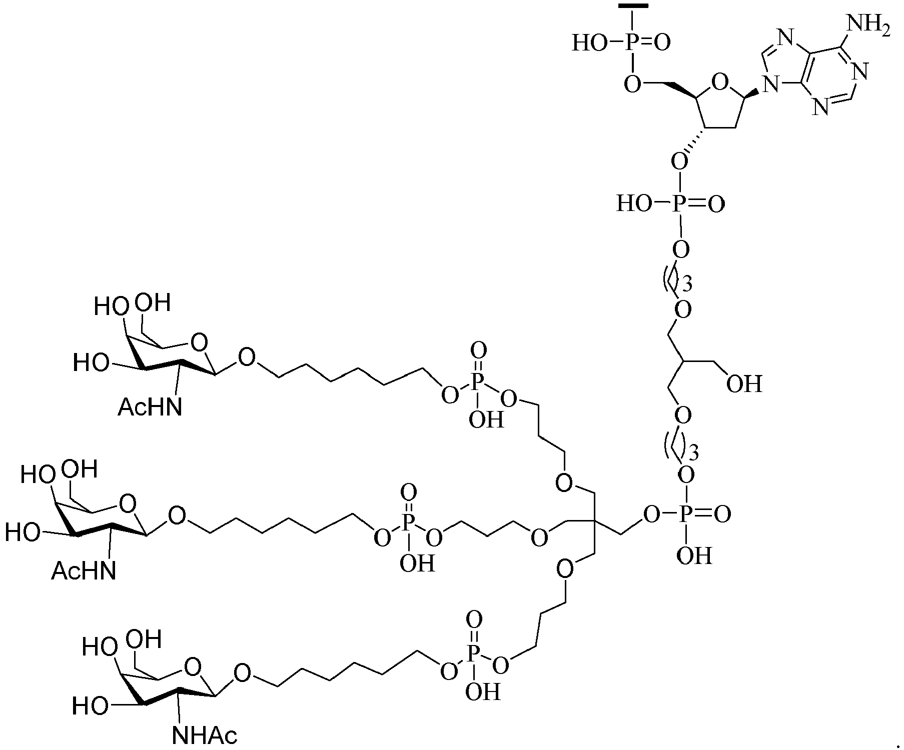

In certain embodiments, conjugates do not comprise a pyrrolidine.

In certain such embodiments, conjugate groups have the following structure:

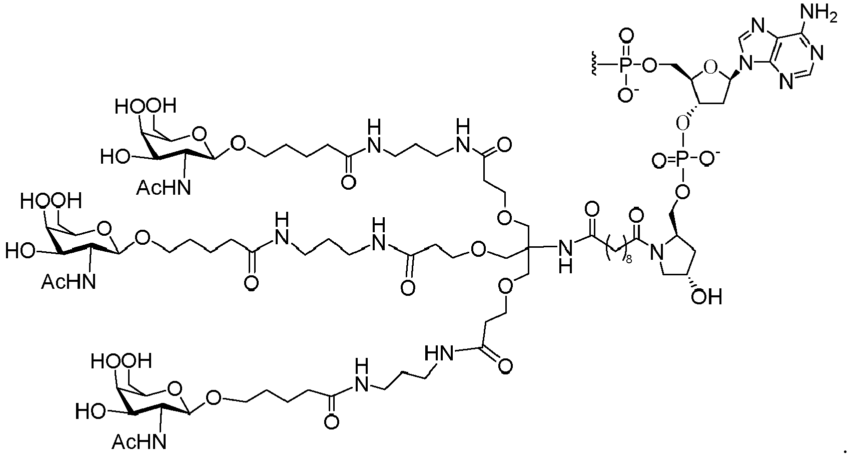

In certain such embodiments, conjugate groups have the following structure:

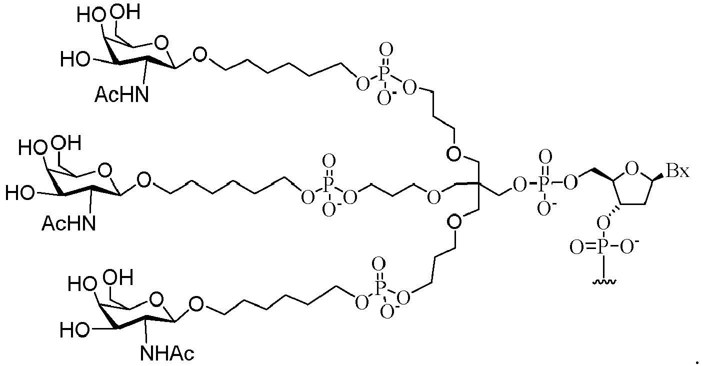

In certain such embodiments, conjugate groups have the following structure:

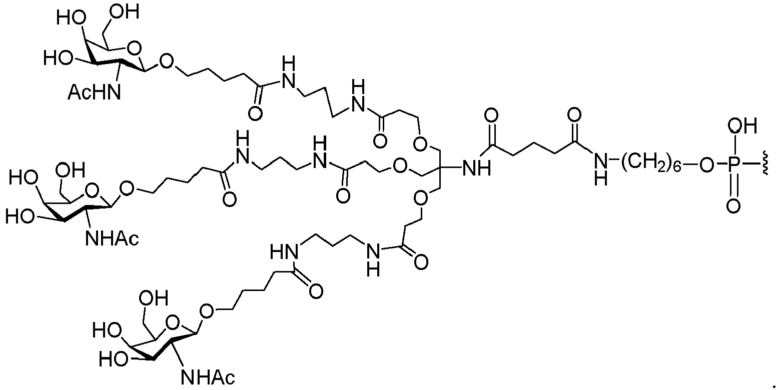

In certain such embodiments, conjugate groups have the following structure:

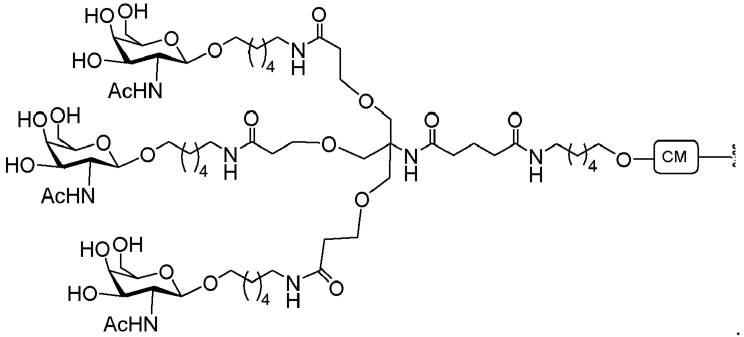

In certain such embodiments, conjugate groups have the following structure:

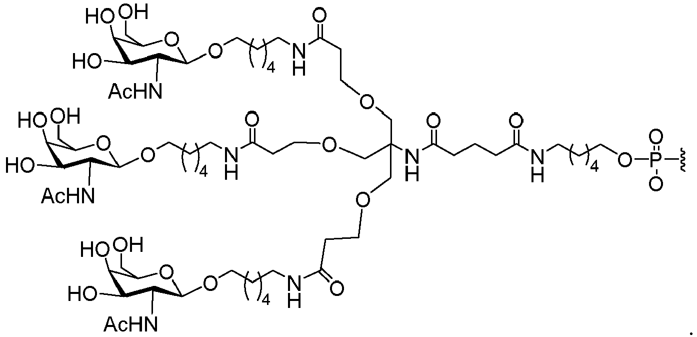

In certain such embodiments, conjugate groups have the following structure:

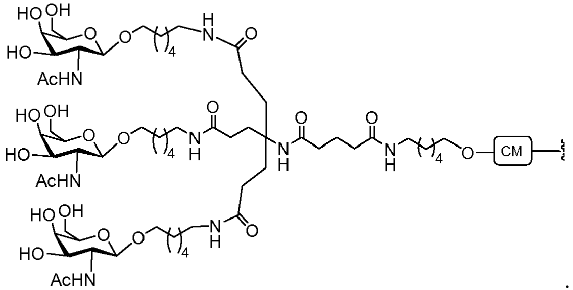

In certain such embodiments, conjugate groups have the following structure:

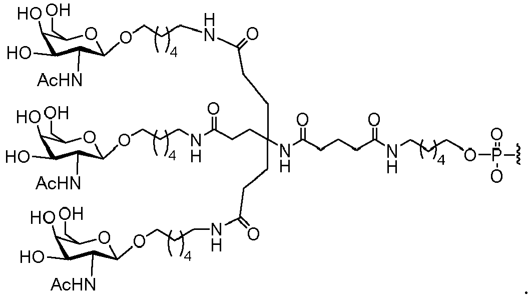

In certain such embodiments, conjugate groups have the following structure:

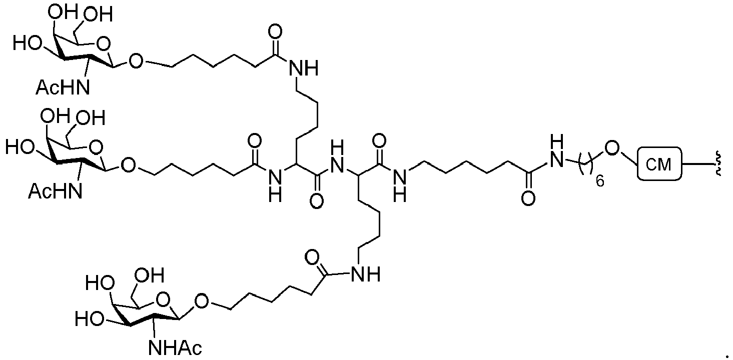

In certain such embodiments, conjugate groups have the following structure:

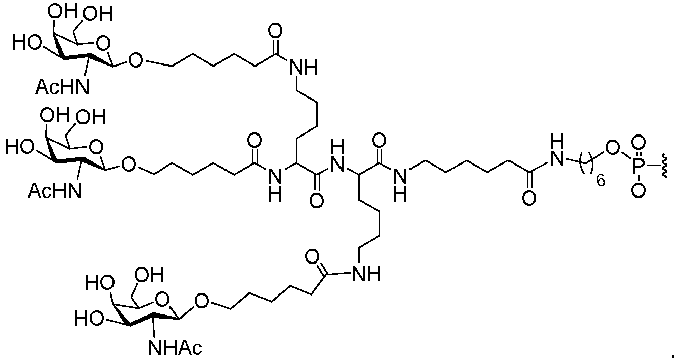

In certain such embodiments, conjugate groups have the following structure:

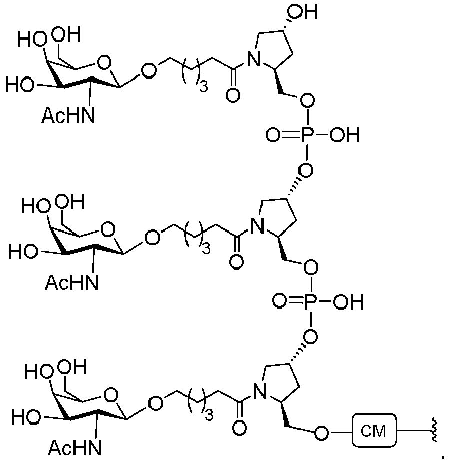

In certain such embodiments, conjugate groups have the following structure:

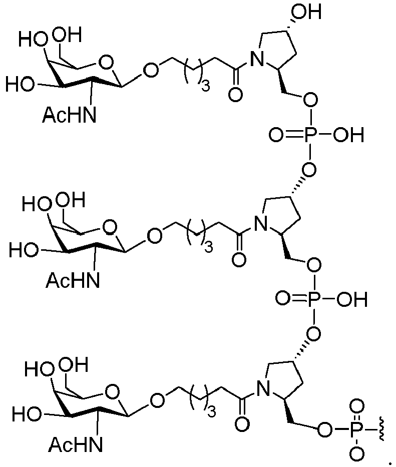

In certain embodiments, the cell-targeting moiety of the conjugate group has the following structure: 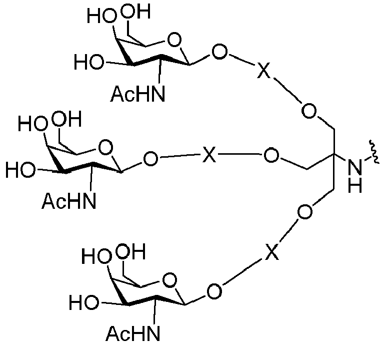 wherein X is a substituted or unsubstituted tether of six to eleven consecutively bonded atoms.

In certain embodiments, the cell-targeting moiety of the conjugate group has the following structure: 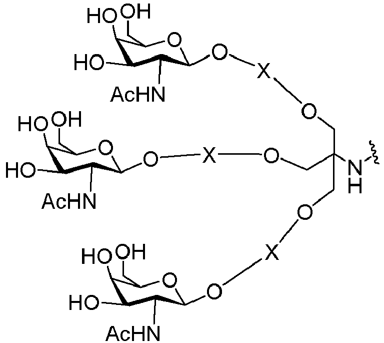 wherein X is a substituted or unsubstituted tether of ten consecutively bonded atoms.

In certain embodiments, the cell-targeting moiety of the conjugate group has the following structure: 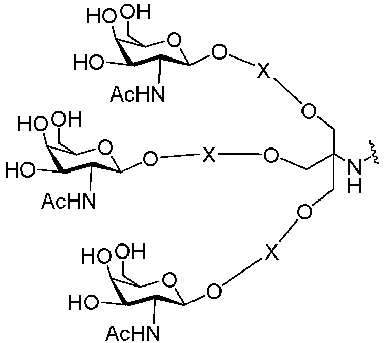 wherein X is a substituted or unsubstituted tether of four to eleven consecutively bonded atoms and wherein the tether comprises exactly one amide bond.

In certain embodiments, the cell-targeting moiety of the conjugate group has the following structure: 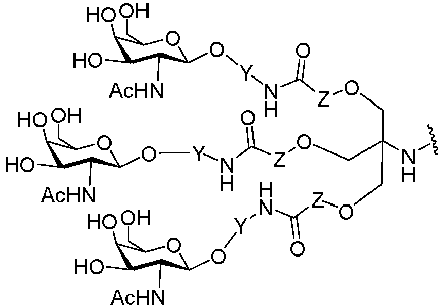 wherein Y and Z are independently selected from a C₁-C₁₂ substituted or unsubstituted alkyl, alkenyl, or alkynyl group, or a group comprising an ether, a ketone, an amide, an ester, a carbamate, an amine, a piperidine, a phosphate, a phosphodiester, a phosphorothioate, a triazole, a pyrrolidine, a disulfide, or a thioether.

In certain such embodiments, the cell-targeting moiety of the conjugate group has the following structure: 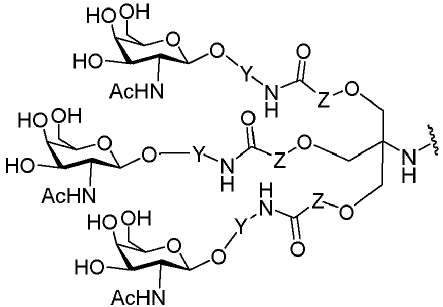 wherein Y and Z are independently selected from a C₁-C₁₂ substituted or unsubstituted alkyl group, or a group comprising exactly one ether or exactly two ethers, an amide, an amine, a piperidine, a phosphate, a phosphodiester, or a phosphorothioate.

In certain such embodiments, the cell-targeting moiety of the conjugate group has the following structure: 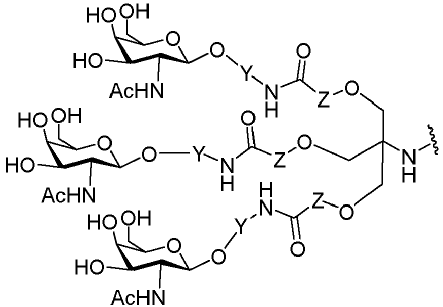 wherein Y and Z are independently selected from a C₁-C₁₂ substituted or unsubstituted alkyl group.

In certain such embodiments, the cell-targeting moiety of the conjugate group has the following structure: 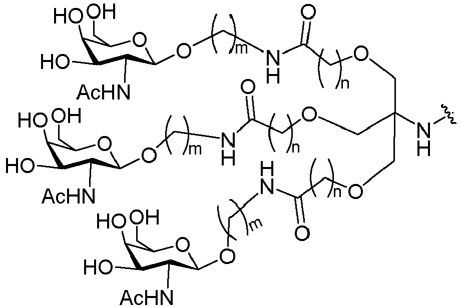 wherein m and n are independently selected from 1, 2, 3, 4, 5, 6, 7, 8, 9, 10, 11, and 12.

In certain such embodiments, the cell-targeting moiety of the conjugate group has the following structure: 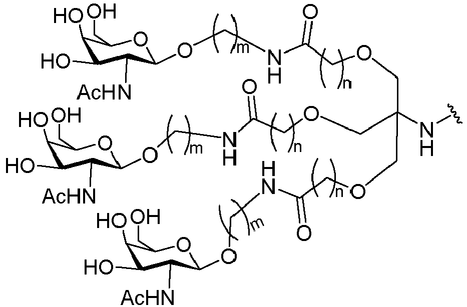 wherein m is 4, 5, 6, 7, or 8, and n is 1, 2, 3, or 4.

In certain embodiments, the cell-targeting moiety of the conjugate group has the following structure: 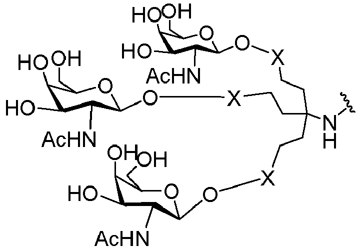 wherein X is a substituted or unsubstituted tether of four to thirteen consecutively bonded atoms, and wherein X does not comprise an ether group.

In certain embodiments, the cell-targeting moiety of the conjugate group has the following structure: 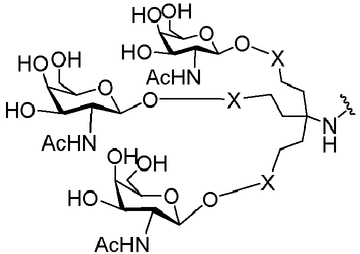 wherein X is a substituted or unsubstituted tether of eight consecutively bonded atoms, and wherein X does not comprise an ether group.

In certain embodiments, the cell-targeting moiety of the conjugate group has the following structure: 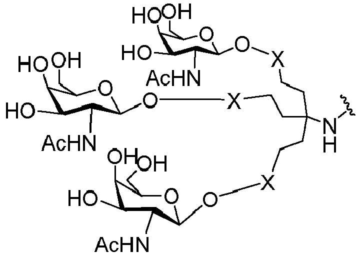 wherein X is a substituted or unsubstituted tether of four to thirteen consecutively bonded atoms, and wherein the tether comprises exactly one amide bond, and wherein X does not comprise an ether group.

In certain embodiments, the cell-targeting moiety of the conjugate group has the following structure: 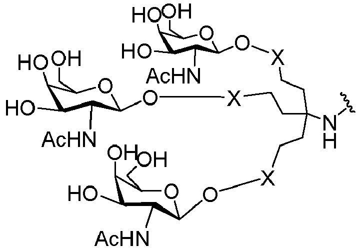 wherein X is a substituted or unsubstituted tether of four to thirteen consecutively bonded atoms and wherein the tether consists of an amide bond and a substituted or unsubstituted C₂-C₁₁ alkyl group.

In certain embodiments, the cell-targeting moiety of the conjugate group has the following structure: 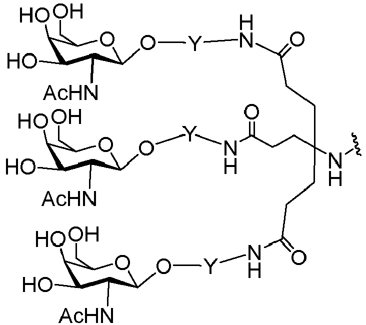 wherein Y is selected from a C₁-C₁₂ substituted or unsubstituted alkyl, alkenyl, or alkynyl group, or a group comprising an ether, a ketone, an amide, an ester, a carbamate, an amine, a piperidine, a phosphate, a phosphodiester, a phosphorothioate, a triazole, a pyrrolidine, a disulfide, or a thioether.

In certain such embodiments, the cell-targeting moiety of the conjugate group has the following structure: 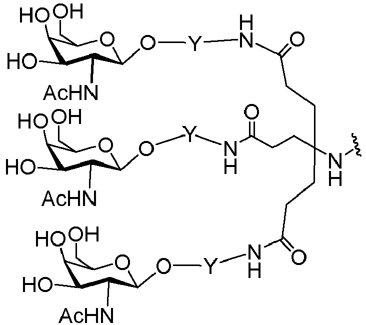 wherein Y is selected from a C₁-C₁₂ substituted or unsubstituted alkyl group, or a group comprising an ether, an amine, a piperidine, a phosphate, a phosphodiester, or a phosphorothioate.

In certain such embodiments, the cell-targeting moiety of the conjugate group has the following structure: 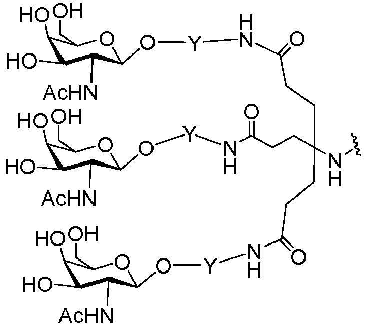 wherein Y is selected from a C₁-C₁₂ substituted or unsubstituted alkyl group.

In certain such embodiments, the cell-targeting moiety of the conjugate group has the following structure: 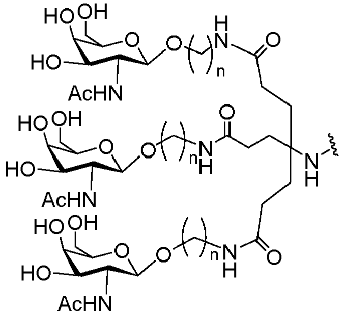 Wherein n is 1, 2, 3, 4, 5, 6, 7, 8, 9, 10, 11, or 12.

In certain such embodiments, the cell-targeting moiety of the conjugate group has the following structure: 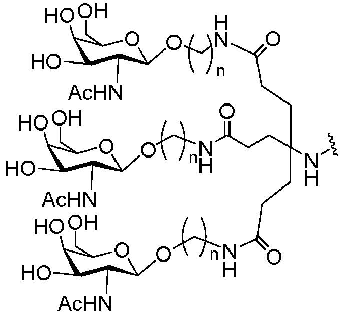 wherein n is 4, 5, 6, 7, or 8.

### d. Antisense Compounds

In certain embodiments, compounds of the present invention are antisense compounds. Such antisense compounds are capable of hybridizing to a target nucleic acid, resulting in at least one antisense activity. In certain embodiments, antisense compounds specifically hybridize to one or more target nucleic acid. In certain embodiments, a specifically hybridizing antisense compound has a nucleobase sequence comprising a region having sufficient complementarity to a target nucleic acid to allow hybridization and result in antisense activity and insufficient complementarity to any non-target so as to avoid or reduce nonspecific hybridization to non-target nucleic acid sequences under conditions in which specific hybridization is desired (e.g., under physiological conditions for *in vivo* or therapeutic uses, and under conditions in which assays are performed in the case of *in vitro* assays). In certain embodiments, oligonucleotides are selective between a target and non-target, even though both target and non-target comprise the target sequence. In such embodiments, selectivity may result from relative accessability of the target region of one nucleic acid molecule compared to the other.

In certain embodiments, the present invention provides antisense compounds comprising oligonucleotides that are fully complementary to the target nucleic acid over the entire length of the oligonucleotide. In certain embodiments, oligonucleotides are 99% complementary to the target nucleic acid. In certain embodiments, oligonucleotides are 95% complementary to the target nucleic acid. In certain embodiments, oligonucleotides are 90% complementary to the target nucleic acid.

In certain embodiments, oligonucleotides are 85% complementary to the target nucleic acid. In certain embodiments, oligonucleotides are 80% complementary to the target nucleic acid. In certain embodiments, an antisense compound comprises a region that is fully complementary to a target nucleic acid and is at least 80% complementary to the target nucleic acid over the entire length of the oligonucleotide. In certain such embodiments, the region of full complementarity is from 6 to 14 nucleobases in length.

In certain embodiments, oligonucleotides comprise a hybridizing region and a terminal region. In certain such embodiments, the hybridizing region consists of 12-30 linked nucleosides and is fully complementary to the target nucleic acid. In certain embodiments, the hybridizing region includes one mismatch relative to the target nucleic acid. In certain embodiments, the hybridizing region includes two mismatches relative to the target nucleic acid. In certain embodiments, the hybridizing region includes three mismatches relative to the target nucleic acid. In certain embodiments, the hybridizing region includes four mismatches relative to the target nucleic acid. In certain embodiments, the terminal region consists of 1-4 terminal nucleosides. In certain emdobiments, the terminal nucleosides are at the 3' end. In certain embodiments, one or more of the terminal nucleosides are not complementary to the target nucleic acid.

Antisense mechanisms include any mechanism involving the hybridization of an oligonucleotide with target nucleic acid, wherein the hybridization results in a biological effect. In certain embodiments, such hybridization results in either target nucleic acid degradation or occupancy with concomitant inhibition or stimulation of the cellular machinery involving, for example, translation, transcription, or splicing of the target nucleic acid.

One type of antisense mechanism involving degradation of target RNA is Rnase H mediated antisense. Rnase H is a cellular endonuclease which cleaves the RNA strand of an RNA:DNA duplex. It is known in the art that single-stranded antisense compounds which are "DNA-like" elicit Rnase H activity in mammalian cells. Activation of Rnase H, therefore, results in cleavage of the RNA target, thereby greatly enhancing the efficiency of DNA-like oligonucleotide-mediated inhibition of gene expression.

Antisense mechanisms also include, without limitation RNAi mechanisms, which utilize the RISC pathway. Such RNAi mechanisms include, without limitation siRNA, ssRNA and microRNA mechanisms.

In certain embodiments, antisense compounds of the present invention are RNAi compounds. In certain embodiments, antisense compounds of the present invention are ssRNA compounds. In certain embodiments, antisense compounds of the present invention are paired with a second oligonucleotide to form an siRNA. In certain such embodiments, the second oligonucleotide is also a compound of the present invention. In certain embodiments, the second oligonucleotide is any modified or unmodified oligonucleotide. In certain embodiments, the oligonucleotide of the present invention is the antisense strand in an siRNA compound. In certain embodiments, the oligonucleotide of the present invention is the sense strand in an siRNA compound.

### ii. Single-stranded RNAi compounds

In certain embodiments, oligonucleotides of the present invention are particularly suited for use as single-stranded antisense compounds. In certain such embodiments, such oligonucleotides are single-stranded RNAi compounds. In certain embodiments, such oligonucleotides are ssRNA compounds or microRNA mimics. Certain 5'-terminal nucleosides described herein are suited for use in such single-stranded oligonucleotides. In certain embodiments, such 5'-terminal nucleosides stabilize the 5'-phosphorous moiety. In certain embodiments, 5'-terminal nucleosides of the present invention are resistant to nucleases. In certain embodiments, the motifs of the present invention are particularly suited for use in single-stranded oligonucleotides. For further description of single-stranded RNAi compounds, see, e.g., WO 2010/048585, WO 2010/048549, and PCT/US2011/033968.

Use of single-stranded RNAi compounds has been limited. In certain instances, single stranded RNAi compounds are quickly degraded and/or do not load efficiently into RISC. Design of single-stranded RNAi compounds for use in cells and/or for use in vivo presents several challenges. For example, the compound must be chemically stable, resistant to nuclease degradation, capable of entering cells, capable of loading into RISC (e.g., binding Ago1 or Ago2), capable of hybridizing with a target nucleic acid, and not toxic to cells or animals. In certain instances, a modification or motif that improves one such feature may worsen another feature, rendering a compound having such modification or motif unsuitable for use as an RNAi compound. For example, certain modifications, particularly if placed at or near the 5'-end of an oligonucleotide, may make the compound more stable and more resistant to nuclease degradation, but may also inhibit or prevent loading into RISC by blocking the interaction with RISC components, such as Ago1 or Ago2. Despite its improved stability properties, such a compound would be unsuitable for use in RNAi.

In certain instances, a single-stranded oligonucleotide comprising a 5'-phosphorous moiety is desired. For example, in certain embodiments, such 5'-phosphorous moiety is necessary or useful for RNAi compounds, particularly, single-stranded RNAi compounds. In such instances, it is further desirable to stabilize the phosphorous moiety against degradation or de-phosphorylation, which may inactivate the compound. Further, it is desirable to stabilize the entire 5'-nucleoside from degradation, which could also inactivate the compound. Thus, in certain embodiments, oligonucleotides in which both the 5'-phosphorous moiety and the 5'-nucleoside have been stabilized are desired. In certain embodiments, provided are modified nucleosides that may be placed at the 5'-end of an oligonucleotide, resulting in a stabilized phosphorous and stabilized nucleoside. In certain such embodiments, the phosphorous moiety is resistant to removal in biological systems, relative to unmodified nucleosides and/or the 5'-nucleoside is resistant to cleavage by nucleases. In certain embodiments, such nucleosides are modified at one, at two or at all three of: the 2'-position, the 5'-position, and at the phosphorous moiety. Such modified nucleosides may be incorporated at the 5'-end of an oligonucleotide.

Although certain oligonucleotides described herein have particular use as single-stranded compounds, such compounds may also be paired with a second strand to create a double-stranded compound. In such embodiments, the second strand of the double-stranded duplex may or may not also be an oligonucleotide as described herein.

In certain embodiments, oligonucleotides as described herein interact with an argonaute protein (Ago). In certain embodiments, such oligonucleotides first enter the RISC pathway by interacting with another member of the pathway (e.g., dicer). In certain embodiments, oligonucleotides first enter the RISC pathway by interacting with Ago. In certain embodiments, such interaction ultimately results in antisense activity. In certain embodiments, provided are methods of activating Ago comprising contacting Ago with an oligonucleotide. In certain embodiments, such oligonucleotides comprise a modified 5'-phosphate group. In certain embodiments, provided are methods of modulating the expression or amount of a target nucleic acid in a cell comprising contacting the cell with an oligonucleotide capable of activating Ago, ultimately resulting in cleavage of the target nucleic acid. In certain embodiments, the cell is in an animal. In certain embodiments, the cell is in vitro. In certain embodiments, the methods are performed in the presence of manganese. In certain embodiments, the manganese is endogenous. In certain embodiments, the methods are performed in the absence of magnesium. In certain embodiments, the Ago is endogenous to the cell. In certain such embodiments, the cell is in an animal. In certain embodiments, the Ago is human Ago. In certain embodiments, the Ago is Ago2. In certain embodiments, the Ago is human Ago2.

In certain embodiments, provided are oligonucleotides having motifs (nucleoside motifs and/or linkage motifs) that result in improved properties. Certain such motifs result in single-stranded oligonucleotides with improved stability and/or cellular uptake properties while retaining antisense activity. For example, oligonucleotides having an alternating nucleoside motif and seven phosphorothioate linkages at the 3'-terminal end have improved stability and activity. Similar compounds that comprise phosphorothioate linkages at each linkage have further improved stability, but are not active as RNAi compounds, presumably because the additional phosphorothioate linkages interfere with the interaction of the oligonucleotide with the RISC pathway components (e.g., with Ago). In certain embodiments, the oligonucleotides having motifs herein result in single-stranded RNAi compounds having desirable properties. In certain embodiments, such oligonucleotides may be paired with a second strand to form a double-stranded RNAi compound. In such embodiments, the second strand of such double-stranded RNAi compounds may comprise a motif as described herein, may comprise another motif of modifications or may be unmodified.

It has been shown that in certain circumstances for single-stranded RNA comprising a 5'-phosphate group has RNAi activity but has much less RNAi activity if it lacks such 5'-phosphate group. The present inventors have recognized that in certain circumstances unmodified 5'-phophate groups may be unstable (either chemically or enzymatically). Accordingly, in certain circumstances, it is desirable to modify the oligonucleotide to stabilize the 5'-phosphate. In certain embodiments, this is achieved by modifying the phosphate group. In certain embodiments, this is achieved by modifying the sugar of the 5'-terminal nucleoside. In certain embodiments, this is achieved by modifying the phosphate group and the sugar. In certain embodiments, the sugar is modified at the 5'-position, the 2'-position, or both the 5'-position and the 2'-position. As with motifs, above, in embodiments in which RNAi activity is desired, a phosphate stabilizing modification must not interfere with the ability of the oligonucleotide to interact with RISC pathway components (e.g., with Ago).

In certain embodiments, provided are oligonucleotides comprising a phosphate-stabilizing modification and a motif described herein. In certain embodiments, such oligonucleotides are useful as single-stranded RNAi compounds having desirable properties. In certain embodiments, such oligonucleotides may be paired with a second strand to form a double-stranded RNAi compound. In such embodiments, the second strand may comprise a motif as described herein, may comprise another motif of modifications or may be unmodified RNA.

In certain embodiments, provided are compounds and methods for antisense activity in a cell. In certain embodiments, the cell is in an animal. In certain embodiments, the animal is a human. In certain embodiments, provided are methods of administering a compound as described herein to an animal to modulate the amount or activity or function of one or more target nucleic acid.

In certain embodiments oligonucleotides comprise one or more motifs as described herein, but do not comprise a phosphate stabilizing modification. In certain embodiments, such oligonucleotides are useful for in vitro applications.

### iii. Certain conjugated compounds

In certain embodiments, the conjugate groups described herein are bound to a nucleoside on an antisense oligonucleotide, a single-stranded RNAi compound, or a double-stranded RNAi compound at the 2', 3', or 5' position of the nucleoside. In certain embodiments, a conjugated compound has the following structure: 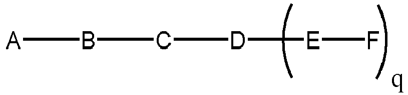 wherein
A is selected from among an antisense oligonucleotide, a single-stranded RNAi compound, or a double-stranded RNAi compound;
B is the cleavable moiety
C is the conjugate linker
D is the branching group
each E is a tether;
each F is a ligand; and
q is an integer between 1 and 5.

In certain embodiments, a conjugated compound has the following structure: 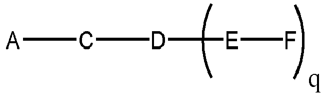 wherein
A is selected from among an antisense oligonucleotide, a single-stranded RNAi compound, or a double-stranded RNAi compound;
C is the conjugate linker
D is the branching group
each E is a tether;
each F is a ligand; and
q is an integer between 1 and 5.

In certain such embodiments, the conjugate linker comprises at least one cleavable bond.

In certain such embodiments, the branching group comprises at least one cleavable bond.

In certain embodiments each tether comprises at least one cleavable bond.

In certain embodiments, the conjugates are bound to a nucleoside of the conjugated compound at the 2', 3', of 5' position of the nucleoside.

In certain embodiments, a conjugated compound has the following structure: 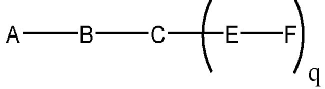 wherein
A is selected from among an antisense oligonucleotide, a single-stranded RNAi compound, or a double-stranded RNAi compound;
B is the cleavable moiety
C is the conjugate linker
each E is a tether;
each F is a ligand; and
q is an integer between 1 and 5.

In certain embodiments, the conjugates are bound to a nucleoside of the conjugated compound at the 2', 3', of 5' position of the nucleoside. In certain embodiments, a conjugated compound has the following structure: 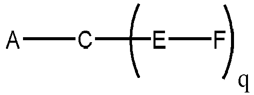 wherein
A is selected from among an antisense oligonucleotide, a single-stranded RNAi compound, or a double-stranded RNAi compound;
C is the conjugate linker
each E is a tether;
each F is a ligand; and
q is an integer between 1 and 5.

In certain embodiments, a conjugated compound has the following structure: 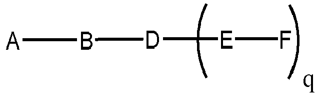 wherein
A is selected from among an antisense oligonucleotide, a single-stranded RNAi compound, or a double-stranded RNAi compound;
B is the cleavable moiety
D is the branching group
each E is a tether;
each F is a ligand; and
q is an integer between 1 and 5.

In certain embodiments, a conjugated compound has the following structure: 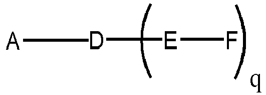 wherein

In the above diagram and in similar diagrams herein, the branching group "D" branches as many times as is necessary to accommodate the number of (E-F) groups as indicated by "q". Thus, where q = 1, the formula is: 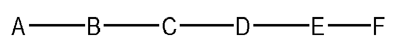 where q = 2, the formula is: 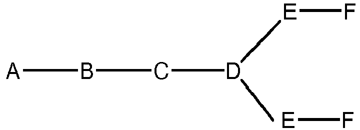 where q = 3, the formula is: 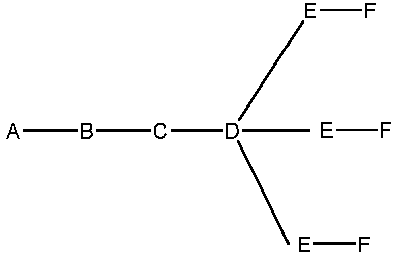 where q = 4, the formula is: 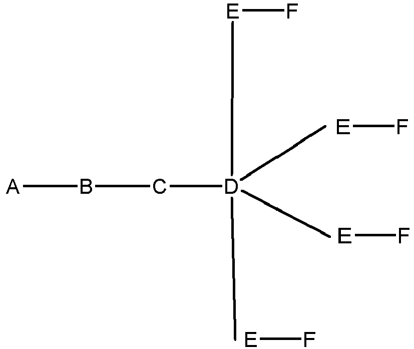 where q = 5, the formula is: 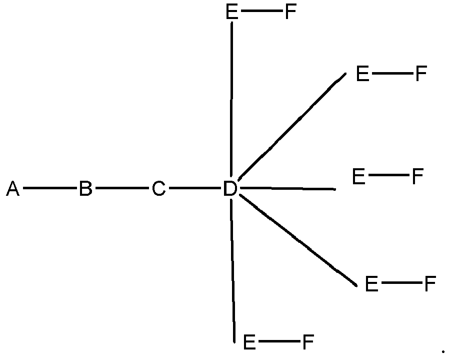
A is selected from among an antisense oligonucleotide, a single-stranded RNAi compound, or a double-stranded RNAi compound;
D is the branching group
each E is a tether;
each F is a ligand; and
q is an integer between 1 and 5.

In certain such embodiments, the conjugate linker comprises at least one cleavable bond.

In certain embodiments each tether comprises at least one cleavable bond.

In certain embodiments, a conjugated compound has a structure selected from among the following: 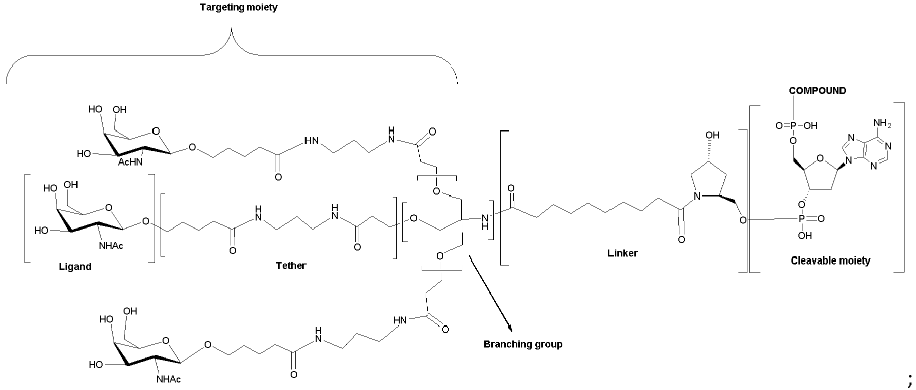 wherein compound represents an antisense oligonucleotide, a single-stranded RNAi compound, or a double-stranded RNAi compound.

In certain embodiments, a conjugated compound has a structure selected from among the following: 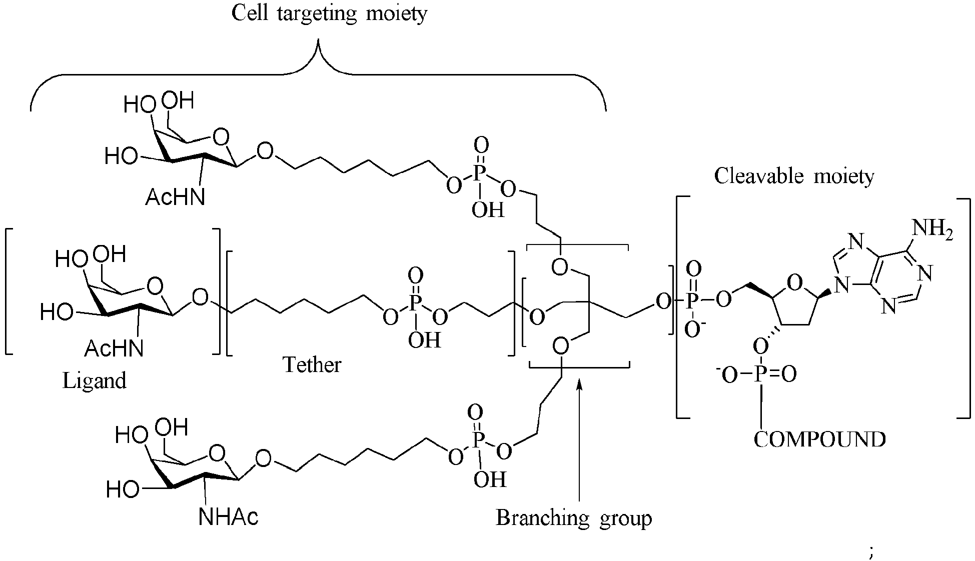 wherein compound represents an antisense oligonucleotide, a single-stranded RNAi compound, or a double-stranded RNAi compound.

In certain embodiments, a conjugated compound has a structure selected from among the following: 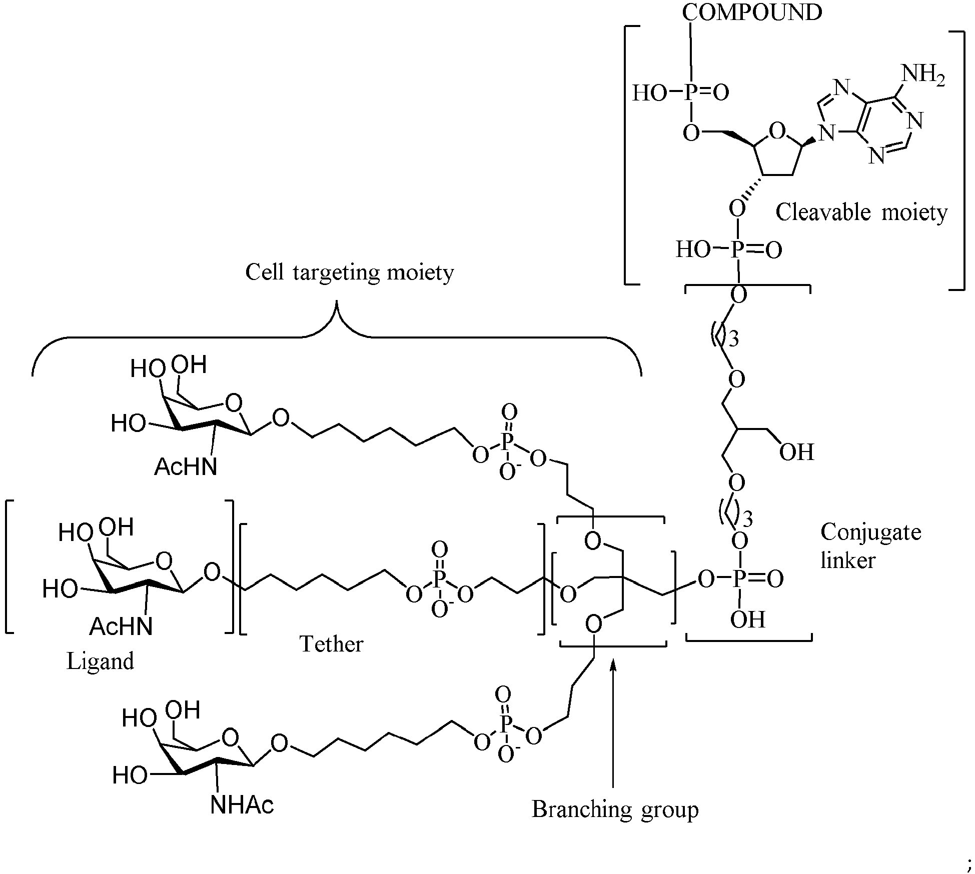 wherein compound represents an antisense oligonucleotide, a single-stranded RNAi compound, or a double-stranded RNAi compound.

Representative United States patents, United States patent application publications, and international patent application publications that teach the preparation of certain of the above noted conjugates, conjugated antisense compounds, tethers, linkers, branching groups, ligands, cleavable moieties as well as other modifications include without limitation, US 5,994,517, US 6,300,319, US 6,660,720, US 6,906,182, US 7,262,177, US 7,491,805, US 8,106,022, US 7,723,509, US 2006/0148740, US 2011/0123520, WO 2013/033230 and WO 2012/037254, each of which is incorporated by reference herein in its entirety.

Representative publications that teach the preparation of certain of the above noted conjugates, conjugated antisense compounds, tethers, linkers, branching groups, ligands, cleavable moieties as well as other modifications include without limitation, BIESSEN et al., "The Cholesterol Derivative of a Triantennary Galactoside with High Affinity for the Hepatic Asialoglycoprotein Receptor: a Potent Cholesterol Lowering Agent" J. Med. Chem. (1995) 38:1846-1852, BIESSEN et al., "Synthesis of Cluster Galactosides with High Affinity for the Hepatic Asialoglycoprotein Receptor" J. Med. Chem. (1995) 38:1538-1546, LEE et al., "New and more efficient multivalent glyco-ligands for asialoglycoprotein receptor of mammalian hepatocytes" Bioorganic & Medicinal Chemistry (2011) 19:2494-2500, RENSEN et al., "Determination of the Upper Size Limit for Uptake and Processing of Ligands by the Asialoglycoprotein Receptor on Hepatocytes in Vitro and in Vivo" J. Biol. Chem. (2001) 276(40):37577-37584, RENSEN et al., "Design and Synthesis of Novel N-Acetylgalactosamine-Terminated Glycolipids for Targeting of Lipoproteins to the Hepatic Asialoglycoprotein Receptor" J. Med. Chem. (2004) 47:5798-5808, SLIEDREGT et al., "Design and Synthesis of Novel Amphiphilic Dendritic Galactosides for Selective Targeting of Liposomes to the Hepatic Asialoglycoprotein Receptor" J. Med. Chem. (1999) 42:609-618, and Valentijn et al., "Solid-phase synthesis of lysine-based cluster galactosides with high affinity for the Asialoglycoprotein Receptor" Tetrahedron, 1997, 53(2), 759-770, each of which is incorporated by reference herein in its entirety.

### e. Certain Target Nucleic Acids, Regions, and Segments

### a. Apolipoprotein C-III (ApoCIII)

ApoCIII is a constituent of HDL and of triglyceride (TG)-rich lipoproteins. Elevated ApoCIII levels are associated with elevated TG levels and diseases such as cardiovascular disease, metabolic syndrome, obesity and diabetes. Elevated TG levels are associated with pancreatitis. ApoCIII slows clearance of TG-rich lipoproteins by inhibiting lipolysis through inhibition of lipoprotein lipase (LPL) and through interfering with lipoprotein binding to cell-surface glycosaminoglycan matrix. Antisense compounds targeting ApoCIII have been previously disclosed in WO2004/093783 and WO2012/149495, each herein incorporated by reference in its entirety. Currently, an antisense oligonucleobase targeting ApoCIII, ISIS-APOCIIIRx, is in Phase II clinical trials to assess its effectiveness in the treatment of diabetes or hypertriglyceridemia. However, there is still a need to provide patients with additional and more potent treatment options.

### Certain Conjugated Antisense Compounds Targeted to an ApoCIII Nucleic Acid

In certain embodiments, conjugated antisense compounds are targeted to an ApoCIII nucleic acid having the sequence of GENBANK® Accession No. NT_033899.8 truncated from nucleobases 20262640 to 20266603, incorporated herein as SEQ ID NO: 1. In certain such embodiments, a conjugated antisense compound is at least 90%, at least 95%, or 100% complementary to SEQ ID NO: 1.

In certain embodiments, conjugated antisense compounds are targeted to an ApoCIII nucleic acid having the sequence of GENBANK® Accession No. NM_000040.1, incorporated herein as SEQ ID NO: 2. In certain such embodiments, a conjugated antisense compound is at least 90%, at least 95%, or 100% complementary to SEQ ID NO: 2.

### ApoCIII Therapeutic Indications

In certain embodiments, the invention provides methods for using a conjugated antisense compound targeted to an ApoCIII nucleic acid for modulating the expression of ApoCIII in a subject. In certain embodiments, the expression of ApoCIII is reduced.

In certain embodiments, the invention provides methods for using a conjugated antisense compound targeted to an ApoCIII nucleic acid in a pharmaceutical composition for treating a subject. In certain embodiments, the subject has a cardiovascular and/or metabolic disease, disorder or condition. In certain embodiments, the subject has hypertriglyceridemia, non-familial hypertriglyceridemia, familial hypertriglyceridemia, heterozygous familial hypertriglyceridemia, homozygous familial hypertriglyceridemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hyperlipidemia, hypercholesterolemia, hyperfattyacidemia, hepatic steatosis, non-alcoholic steatohepatitis, pancreatitis and/or non-alcoholic fatty liver disease.

In certain embodiments, the invention provides methods for using a conjugated antisense compound targeted to an ApoCIII nucleic acid in the preparation of a medicament.

### C. Certain Nucleic Acid GalNAc Conjugates

In certain embodiments, conjugated antisense compounds comprise double stranded siRNA (ds-siRNA) compounds targeted to coding and non-coding regions of hApoC III (SEQ ID NO: 2). In certain embodiments, conjugated antisense compounds comprise double stranded siRNA (ds-siRNA) compounds targeted to coding and non-coding regions of hApoC III (SEQ ID NO: 2) and attached to a GalNAc conjugate. In certain embodiments, a GalNAc conjugate is covalently attached at the 3'-end of the sense strand of the double stranded siRNA. In certain embodiments, a GalNAc conjugate is covalently attached at the 5'-end of the sense strand of the double stranded siRNA. In certain embodiments, conjugated ds-siRNA compounds targeted to hApoCIII have the nucleobase sequences and modifications of the ds-siRNA compounds in Table 16 below, described in published PCT application WO 2012/177947, hereby incorporated by reference, with an attached GalNAc conjugate. The ds-siRNAs can be prepared using procedures described in published PCT application WO 2012/177947, and the GalNAc conjugates can be prepared as described in Example 11 or via procedures known in the art. In the table below entitled "Modified ds-siRNAs attached to a GalNAc conjugate targeting hApoC III" only, lowercase "g", "a", "u", and "c" represent 2'-O-methyl nucleosides; lowercase "s" between two nucleosides indicates a phosphorothioate internucleoside linkage; lowercase "dT" represents a 2'-deoxythymidine nucleoside; and "Gf", "Af", "Uf", and "Cf" represent 2'-fluoro nucleosides.

**Modified ds-siRNAs attached to a GalNAc conjugate targeting hApoC III**

| SEQ ID No. | Sense Sequence | SEQ ID No. | Antisense Sequence |
|---|---|---|---|
| 87 | UCCCUGAAAGACUACUGGA | 111 | UCCAGUAGUCUUUCAGGGA |
| 88 | UfGGGUfGACfCfGAUfGGCfUfUfCfAdTsdT | 112 | UGAAGCCfAUCGGUCfACCCfAdTsdT |
| 89 | GAUfGGCfUfUfCfAGUfUfCfCfCfUfGAdTsdT | 113 | UCfAGGGAACUGAAGCCfAUCdTsdT |
| 90 | UGCAGCCCCGGGUACUCCUdTsdT | 114 | AGGAGUACCCGGGGCUGCAdTsdT |
| 91 | GCAGCCCCGGGUACUCCUUdTsdT | 115 | AAGGAGUACCCGGGGCUGCdTsdT |
| 88 | UGGGUGACCGAUGGCUUCAdTsdT | 112 | UGAAGCCAUCGGUCACCCAdTsdT |
| 92 | CfcGfaUfgGfcUfuCfaGfuUfcCfcUfdTsdT | 116 | aGfgGfaAfcUfgAfaGfcCfaUfcGfgdTsdT |
| 93 | AfuGfgCfuUfcAfgUfuCfcCfuGfaAfdTsdT | 117 | uUfcAfgGfgAfaCfuGfaAfgCfcAfudTsdT |
| 94 | UGGCUUCAGUUCCCUGAAAdTsdT | 118 | UUUCAGGGAACUGAAGCCAdTsdT |
| 95 | CUGAAAGACUACUGGAGCAdTsdT | 119 | UGCUCCAGUAGUCUUUCAGdTsdT |
| 96 | AGCACCGUUAAGGACAAGUdTsdT | 120 | ACUUGUCCUUAACGGUGCUdTsdT |
| 97 | GCACCGUUAAGGACAAGUUdTsdT | 121 | AACUUGUCCUUAACGGUGCdTsdT |
| 98 | GCUGCCUGAGACCUCAAUAdTsdT | 122 | UAUUGAGGUCUCAGGCAGCdTsdT |
| 98 | GfcUfgCfcUfgAfgAfcCfuCfaAfuAfdTsdT | 122 | uAfuUfgAfgGfuCfuCfaGfgCfaGfcdTsdT |
| 99 | CUGAGACCUCAAUACCCCAdTsdT | 123 | UGGGGUAUUGAGGUCUCAGdTsdT |
| 100 | GCUGCCCCUGUAGGUUGCUdTsdT | 124 | AGCAACCUACAGGGGCAGCdTsdT |
| 101 | GCUUAAAAGGGACAGUAUUdTsdT | 125 | AAUACUGUCCCUUUUAAGCdTsdT |
| 102 | CUGGACAAGAAGCUGCUAUdTsdT | 126 | AUAGCAGCUUCUUGUCCAGdTsdT |
| 103 | CfcCfuGfuAfgGfuUfgCfuUfaAfaAfdTsdT | 127 | uUfuUfaAfgCfaAfcCfuAfcAfgGfgdTsdT |
| 90 | UfGCfAGCfCfCfCfGGGUfACfUfCfCfUfdTsdT | 114 | AGGAGUfACCCGGGGCUGCfAdTsdT |
| 91 | GCfAGCfCfCfCfGGGUfACfUfCfCfUfUfdTsdT | 115 | AAGGAGUfACCCGGGGCUGCdTsdT |
| 104 | CAAGACCGCCAAGGAUGCAdTsdT | 128 | UGCAUCCUUGGCGGUCUUGdTsdT |
| 105 | GGUfGACfCfGAUfGGCfUfUfCfAGUfdTsdT | 129 | ACUGAAGCCfAUCGGUCfACCdTsdT |
| 105 | GGUGACCGAUGGCUUCAGUdTsdT | 129 | ACUGAAGCCAUCGGUCACCdTsdT |
| 105 | GfgUfgAfcCfgAfuGfgCfuUfcAfgUfdTsdT | 129 | aCfuGfaAfgCfcAfuCfgGfuCfaCfcdTsdT |
| 92 | CfCfGAUfGGCfUfUfCfAGUfUfCfCfCfUfdTsdT | 116 | AGGGAACUGAAGCCfAUCGGdTsdT |
| 92 | CCGAUGGCUUCAGUUCCCUdTsdT | 116 | AGGGAACUGAAGCCAUCGGdTsdT |
| 89 | GAUGGCUUCAGUUCCCUGAdTsdT | 113 | UCAGGGAACUGAAGCCAUCdTsdT |
| 93 | AUGGCUUCAGUUCCCUGAAdTsdT | 117 | UUCAGGGAACUGAAGCCAUdTsdT |
| 94 | uGGcuucAGuucccuGAAAdTsdT | 118 | UUUcAGGGAACUGAAGCcAdTsdT |
| 94 | UfGGCfUfUfCfAGUfUfCfCfCfUfGAAAdTsdT | 118 | UUUCfAGGGAACUGAAGCCfAdTsdT |
| 94 | UfgGfcUfuCfaGfuUfcCfcUfgAfaAfdTsdT | 118 | uUfuCfaGfgGfaAfcUfgAfaGfcCfadTsdT |
| 106 | GcuucAGuucccuGAAAGAdTsdT | 130 | UCUUUcAGGGAACUGAAGCdTsdT |
| 106 | GCfUfUfCfAGUfUfCfCfCfUfGAAAGAdTsdT | 130 | UCUUUCfAGGGAACUGAAGCdTsdT |
| 106 | GCUUCAGUUCCCUGAAAGAdTsdT | 130 | UCUUUCAGGGAACUGAAGCdTsdT |
| 95 | cuGAAAGAcuAcuGGAGcAdTsdT | 119 | UGCUCcAGuAGUCUUUcAGdTsdT |
| 95 | CfUfGAAAGACfUfACfUfGGAGCfAdTsdT | 119 | UGCUCCfAGUfAGUCUUUCfAGdTsdT |
| 96 | AGCfACfCfGUfUfAAGGACfAAGUfdTsdT | 120 | ACUUGUCCUUfAACGGUGCUdTsdT |
| 97 | GcAccGuuAAGGAcAAGuudTsdT | 121 | AACUUGUCCUuAACGGUGCdTsdT |
| 97 | GCfACfCfGUfUfAAGGACfAAGUfUfdTsdT | 121 | AACUUGUCCUUfAACGGUGCdTsdT |
| 97 | GfcAfcCfgUfuAfaGfgAfcAfaGfuUfdTsdT | 121 | aAfcUfuGfuCfcUfuAfaCfgGfuGfcdTsdT |
| 97 | GcAccGuuAAGGAcAAGuudTsdT | 121 | AACuUGUCCuuAACGGugcdTsdT |
| 107 | CfCfUfCfAAUfACfCfCfCfAAGUfCfCfAdTsdT | 131 | UGGACUUGGGGUfAUUGAGGdTsdT |
| 107 | CCUCAAUACCCCAAGUCCAdTsdT | 131 | UGGACUUGGGGUAUUGAGGdTsdT |
| 108 | AGGUfUfGCfUfUfAAAAGGGACfAdTsdT | 132 | UGUCCCUUUUfAAGCfAACCUdTsdT |
| 109 | UfGCfUfUfAAAAGGGACfAGUfAUfdTsdT | 133 | AUfACUGUCCCUUUUfAAGCfAdTsdT |
| 109 | UGCUUAAAAGGGACAGUAUdTsdT | 133 | AUACUGUCCCUUUUAAGCAdTsdT |
| 109 | UfgCfuUfaAfaAfgGfgAfcAfgUfaUfdTsdT | 133 | aUfaCfuGfuCfcCfuUfuUfaAfgCfadTsdT |
| 101 | GcuuAAAAGGGAcAGuAuudTsdT | 125 | AAuACUGUCCCUUUuAAGCdTsdT |
| 101 | GCfUfUfAAAAGGGACfAGUfAUfUfdTsdT | 125 | AAUfACUGUCCCUUUUfAAGCdTsdT |
| 101 | GfcUfuAfaAfaGfgGfaCfaGfuAfuUfdTsdT | 125 | aAfuAfcUfgUfcCfcUfuUfuAfaGfcdTsdT |
| 102 | cuGGAcAAGAAGcuGcuAudTsdT | 126 | AuAGcAGCUUCUUGUCcAGdTsdT |
| 110 | AGACfUfACfUfGGAGCfACfCfGUfUfdTsdT | 134 | AACGGUGCUCCfAGUfAGUCUdTsdT |
| 110 | AfgAfcUfaCfuGfgAfgCfaCfcGfuUfdTsdT | 134 | aAfcGfgUfgCfuCfcAfgUfaGfuCfudTsdT |
| 103 | CfCfCfUfGUfAGGUfUfGCfUfUfAAAAdTsdT | 127 | UUUUfAAGCfAACCUfACfAGGGdTsdT |
| 103 | CfcCfuGfuAfgGfuUfgCfuUfaAfaAfdTsdT | 127 | uUfuUfaAfgCfaAfcCfuAfcAfgGfgdTsdT |
| 103 | cccuGuAGGuuGcuuAAAAdTsdT | 127 | UuUuAAGCAACCuACAgggdTsdT |

In certain embodiments, double-stranded compounds have the following modification motifs: sense strand: 5'-N_{f}NₘN_{f}NₘN_{f}NₘN_{f}NₘN_{f}N_{f}N_{f}NₘN_{f}NₘNₘNₘN_{f}NₘN_{f}NₘN_{f}-X; antisense: 5'-NₘN_{f}NₘN_{f}NₘN_{f}N_{f}N_{f}NₘN_{f}NₘNₘNₘN_{f}NₘN_{f}NₘN_{f}NₘN_{f}NₘₛN_{fs}Nₘ-3'; wherein "N" represents a nucleobase, subscript "m" indicates 2'-O-methyl nucleotides; Nf (e.g., Af) indicates a 2'-fluoro nucleotide; s indicates a phosphothiorate linkage; and "X" indicates a GalNAc ligand. If not indicated by an "s" the internucleoside linkage is a phosphodiester. In certain embodiments, "X" indicates a GalNAc₃ ligand.

In certain embodiments, double-stranded compounds have the following modification motifs: sense strand: 5'-NₓN_{y}NₓN_{y}NₓN_{y}NₓN_{y}NₓNₓNₓN_{y}NₓN_{y}N_{y}N_{y}NₓN_{y}NₓN_{y}Nₓ-X; antisense: 5 ' - N_{y}NₓN_{y}NₓN_{y}NₓNₓNₓN_{y}NₓN_{y}N_{y}N_{y}NₓN_{y}NₓN_{y}NₓN_{y}NₓN_{ys}N_{x s}N_{y}-3'; wherein "N" represents a nucleobase, subscript "y" indicates a 2'-modification selected from among 2'-O-methyl, 2'-MOE, 2'-NMA, 2'-OH, and 2'-H. In certain embodiments, subscript "y" indicates a nucleobase modification selected from among 2'-fluoro nucleotide, BNA, cMOE, ENA, LNA, cEt, LNA, 2'-Ome, 2'-MOE; s indicates a phosphothiorate linkage; and uppercase "X" indicates a GalNAc ligand. If not indicated by an "s" the internucleoside linkage is a phosphodiester. In certain embodiments, "X" indicates a GalNAc₃ ligand.

### D. Certain Pharmaceutical Compositions

In certain embodiments, provided herein are pharmaceutical compositions comprising one or more antisense compound. In certain embodiments, such pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one or more antisense compound and sterile water. In certain embodiments, the sterile saline is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile phosphate-buffered saline (PBS). In certain embodiments, the sterile saline is pharmaceutical grade PBS.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

Pharmaceutical compositions comprising antisense compounds encompass any pharmaceutically acceptable salts, esters, or salts of such esters. In certain embodiments, pharmaceutical compositions comprising antisense compounds comprise one or more oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an oligonucleotide which are cleaved by endogenous nucleases within the body, to form the active antisense oligonucleotide.

Lipid moieties have been used in nucleic acid therapies in a variety of methods. In certain such methods, the nucleic acid is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions provided herein comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition provided herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition provided herein comprises one or more tissue-specific delivery molecules designed to deliver the one or more pharmaceutical agents as described herein to specific tissues or cell types. For example, in certain embodiments, pharmaceutical compositions include liposomes coated with a tissue-specific antibody.

In certain embodiments, a pharmaceutical composition provided herein comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80™ and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80™; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition provided herein is prepared for oral administration. In certain embodiments, pharmaceutical compositions are prepared for buccal administration.

In certain embodiments, a pharmaceutical composition is prepared for administration by injection or infusion (e.g., intravenous, subcutaneous, intramuscular, intrathecal, intracerebroventricular etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition provided herein comprises an oligonucleotide in a therapeutically effective amount. In certain embodiments, the therapeutically effective amount is sufficient to prevent, alleviate or ameliorate symptoms of a disease or to prolong the survival of the subject being treated.

In certain embodiments, one or more modified oligonucleotide provided herein is formulated as a prodrug. In certain embodiments, upon *in vivo* administration, a prodrug is chemically converted to the biologically, pharmaceutically or therapeutically more active form of an oligonucleotide. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, provided herein are compositions and methods for reducing the amount or activity of a target nucleic acid in a cell. In certain embodiments, the cell is in an animal. In certain embodiments, the animal is a mammal. In certain embodiments, the animal is a rodent. In certain embodiments, the animal is a primate. In certain embodiments, the animal is a non-human primate. In certain embodiments, the animal is a human.

In certain embodiments, provided herein are methods of administering a pharmaceutical composition comprising an oligonucleotide as described herein to an animal. Suitable administration routes include, but are not limited to, oral, rectal, transmucosal, intestinal, enteral, topical, suppository, through inhalation, intrathecal, intracerebroventricular, intraperitoneal, intranasal, intraocular, intratumoral, and parenteral (e.g., intravenous, intramuscular, intramedullary, and subcutaneous).

### Nonlimiting disclosure and incorporation by reference

While certain compounds, compositions and methods described herein have been described with specificity in accordance with certain embodiments, the following examples serve only to illustrate the compounds described herein and are not intended to limit the same. Each of the references, GenBank accession numbers, and the like recited in the present application is incorporated herein by reference in its entirety.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligonucleotide having the nucleobase sequence "ATCGATCG" encompasses any oligonucleotides having such nucleobase sequence, whether modified or unmodified, including, but not limited to, such compounds comprising RNA bases, such as those having sequence "AUCGAUCG" and those having some DNA bases and some RNA bases such as "AUCGATCG" and oligonucleotides having other modified bases, such as "AT^{me}CGAUCG," wherein ^{me}C indicates a cytosine base comprising a methyl group at the 5-position.

### EXAMPLES

### Non-limiting disclosure and incorporation by reference

While certain compounds, compositions and methods described herein have been described with specificity in accordance with certain embodiments, the following examples serve only to illustrate the compounds described herein and are not intended to limit the same. Each of the patents, applications, printed publications, and other published documents mentioned or referred to in this specification are herein incorporated by reference in their entirety.

### Example 1: Preparation of Compound 5

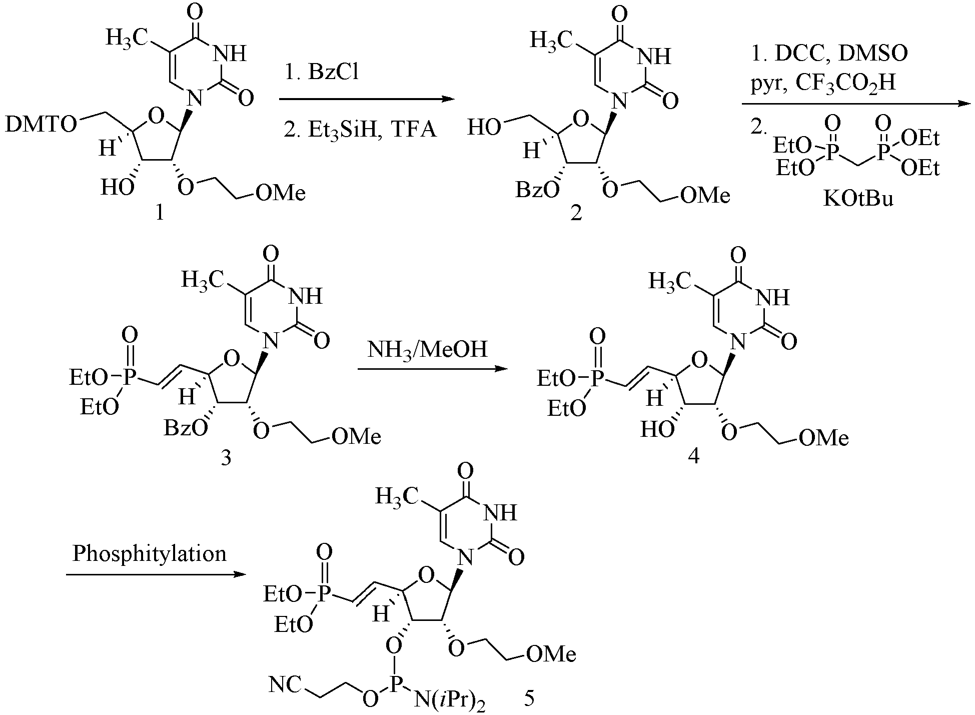

### a) Preparation of Compound 2

Compound 1 was prepared according to the procedures published in U.S. Patent 5,969,116. Benzoyl chloride (5.6 mL, 48.5 mmol) was added to solution of nucleoside Compound 1 (25 g, 40.5 mmol) in pyridine (100 mL). After stirring at room temperature for 3 hours, additional benzoyl chloride (2.5 mL) was added to the reaction. After an additional 60 minutes, the reaction was quenched with water and then partitioned between ethyl acetate and water. The organic layer was further washed with water, brine, dried (sodium sulfate) and concentrated to provide the crude benzoyl protected nucleoside which was used without any further protection.

Trifluoroacetic acid (5 mL) was added to a solution of the crude nucleoside from above and triethylsilane (12 mL) in dichloromethane. After 2 hours, additional trifluoroacetic acid (5 mL) and triethylsilane (5 mL) were added to the reaction and the stirring was continued for an additional 4 hours during which time the reaction turned light yellow from an initial bright orange. The solvent was removed on a rotary evaporator and the residue was dissolved in ethyl acetate and the organic layer was carefully washed with water, sodium bicarbonate, brine, dried (sodium sulfate) and concentrated. The resulting white solid was suspended in hexanes and collected by filtration and further washed with additional hexanes to provide nucleoside Compound 2 (14.9 g, 87% over 2 steps).

### b) Preparation of Compound 3

Dicyclohexylcarbodimide (1.5 g, 7.2 mmol) was added to a solution of Compound 2 (2.0 g, 4.8 mmol) and pyridinium trifluoroacetate (0.92 g, 4.8 mmol) in dimethylsulfoxide (48 mL) and the reaction mixture was allowed to stir at room temperature for 6 hours. In a separate flask, a solution of potassium tert-butoxide (10 mL of a 1M solution in THF) was added to a solution of tetraethylmethylenediphosphonate (2.4 mL, 9.6 mmol) in THF (20 mL). After stirring for 10 minutes at room temperature, this flask was cooled in an ice bath and the DMSO solution was added *via* a cannula. After stirring at room temperature for 2 hours, the reaction was diluted with ethyl acetate and the organic layer was washed with water, brine, dried (sodium sulfate) and concentrated. Purification by column chromatography (silica gel, eluting with 20 to 40% acetone in dichloromethane) provided the vinyl nucleoside Compound 3 (1.25 g, 47%).

### c) Preparation of Compound 4

A solution of vinyl nucleoside Compound 3 (110 mg, 0.2 mmol) and 7 N ammonia in methanol (2 mL) were aged at room temperature for 6 hours and the solvent was removed on a rotary evaporator. Purification of the residue by chromatography (silica gel, eluting with 70 to 90% acetone in dichloromethane) provided Compound 4 (84 mg, 95%).

### d) Preparation of Compound 5

(2-Cyanoethoxy)-tetraisopropylphosphordiamidite (0.084 mL, 0.28 mmol) was added to a solution of Compound 4 (84 mg, 0.19 mmol), tetrazole (12 mg, 0.15 mmol) and N-methylimidazole (1 drop) in dimethylformamide (1 mL). After stirring at room temperature for 3 hours, the reaction was diluted with ethyl acetate and the organic layer was washed with brine (2x), dried (sodium sulfate) and concentrated. Purification by column chromatography (silica gel, eluting with 2 to 4% methanol in dichloromethane) provided amidite Compound 5 (113 mg, 90%).

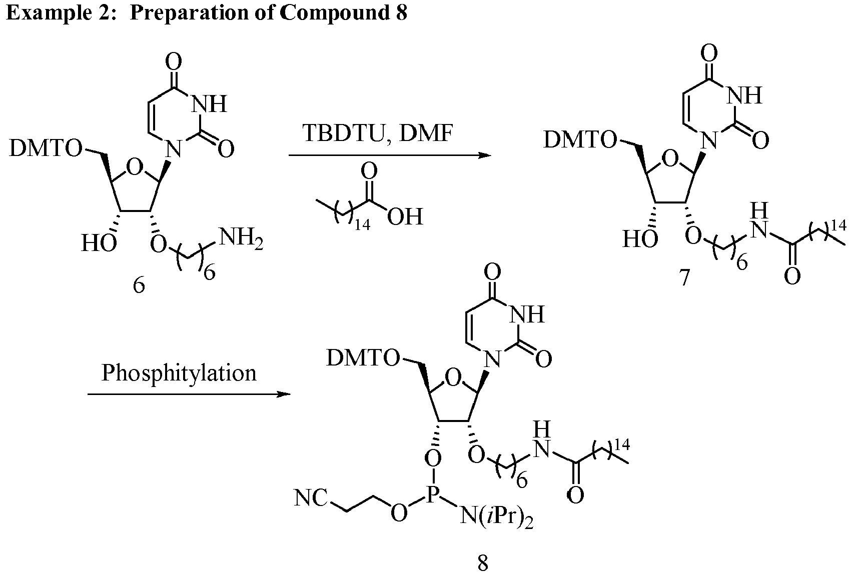

Compound 6 was prepared as per the procedures illustrated in Example 1. Spectral analysis for Compound 8 was consistent with the structure.

### Example 3: Preparation of Compound 12

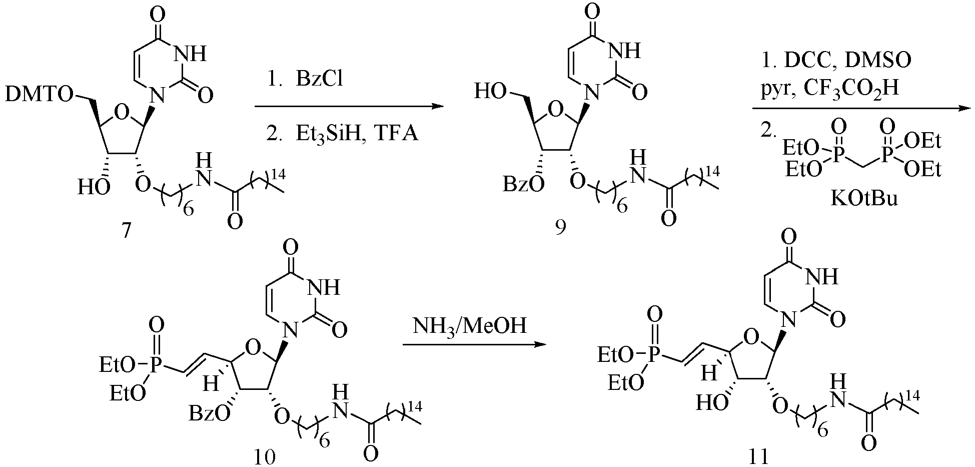

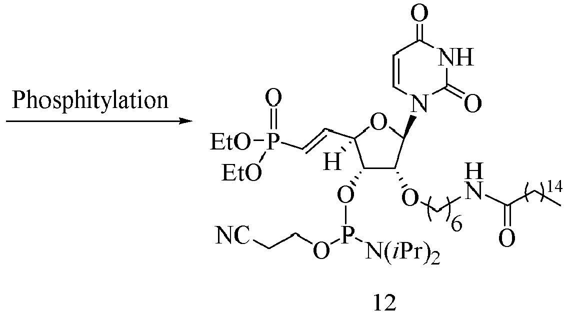

Compound 7 was prepared as per the procedures illustrated in Example 2. Spectral analysis for Compound 12 was consistent with the structure.

### Example 4: Preparation of Compounds 13-16

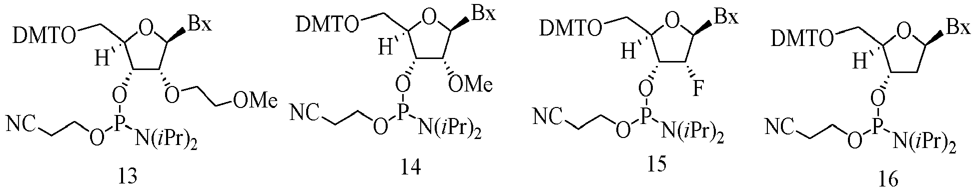

Compounds 13-16 were prepared as per the procedures well known in the art as described in the specification herein (see Seth et al., Bioorg. Med. Chem., 2011, 21(4), 1122-1125, J. Org. Chem., 2010, 75(5), 1569-1581, Nucleic Acids Symposium Series, 2008, 52(1), 553-554); and also see published PCT 20 International Applications (WO 2011/115818, WO 2010/077578, WO2010/036698, WO2009/143369, WO 2009/006478, and WO 2007/090071), and US patent 7,569,686).

### Example 5: General preparation of single stranded-small interfering RNAs (ss-siRNAs) comprising 5'-(E)-Vinylphosphonate and C16 conjugate at 5' terminus, Compound 20

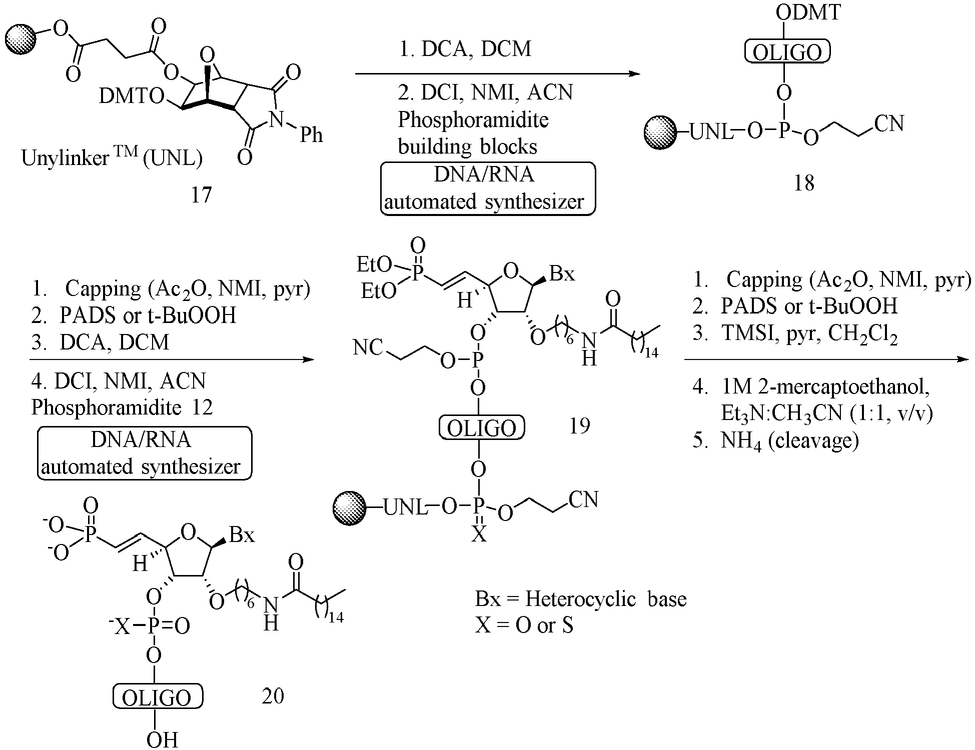

The Unylinker™ 17 is commercially available. Phosphoramidite 12 is prepared using similar procedures as illustrated in Example 3. Conjugated ss-siRNA, Compound 20 is prepared using standard procedures in automated DNA/RNA synthesis (see Swayze et al., WO 2006/031461 and Dupouy et al., Angew. Chem. Int. Ed., 2006, 45, 3623-3627). Phosphoramidite building blocks, Compounds 5, 8 and 12-16 were prepared as per the procedures illustrated in Examples 1-4. The phosphoramidites illustrated are meant to be representative and not intended to be limiting as other phosphoramidite building blocks can be used to prepare ss-siRNAs having a predetermined sequence and composition. The order and quantity of phosphoramidites added to the solid support can be adjusted to prepare the ss-siRNAs as described herein. Such ss-siRNAs can have predetermined composition and base sequence as dictated by any given target.

### Example 6: General method for the preparation of ss-siRNAs comprising 5'-(E)-Vinylphosphonate and/or 2'-C16 conjugate

Unless otherwise stated, all reagents and solutions used for the synthesis of ss-siRNAs were purchased from commercial sources. Standard phosphoramidites and solid support were used for incorporation of A, U, G, ^{me}C and C residues. A 0.1 M solution of 2'-F and 2'-O-Me phosphoramidites in anhydrous acetonitrile (CH₃CN) along with 2'-O-MOE-5'-vinylphosphonate 3'-phosphoramidites and 2'-C16-5'-vinylphosphonate 3'-phosphoramidites in 30% dichloromethane (CH₂Cl₂) in anhydrous CH₃CN were used for the synthesis. The ss-siRNAs were synthesized on VIMAD UnyLinker™ solid support and the appropriate amounts of solid support were packed in the column for synthesis. Dichloroacetic acid (6%) in toluene was used as detritylating reagent. 4,5-Dicyanoimidazole in the presence of *N*-methylimidazole or 1*H*-tetrazole in CH₃CN was used as activator during the coupling step. The synthesis of ss-siRNAs was performed either on an ÄKTAOligopilot synthesizer (GE Healthcare Bioscience) or an ABI394 synthesizer (Applied Biosystems) on a 2-200 µmol scale using the procedures set forth below.

A solid support preloaded with the Unylinker™ was loaded into a synthesis column after closing the column bottom outlet and CH₃CN was added to form a slurry. The swelled support-bound Unylinker™ was treated with a detritylating reagent containing 6% dichloroacetic acid in toluene to provide the free hydroxyl groups. During the coupling step, four to fourteen equivalents of phosphoramidite solutions were delivered with coupling for 10 minutes. All of the other steps followed standard protocols. Phosphorothioate linkages were introduced by sulfurization with a 0.05 M solution of DDTT (3-((dimethylamino-methylidene)amino)-3H-1,2,4-dithiazole-3-thione) in 1:1 pyridine/CH₃CN for a contact time of 3 minutes. Phosphite triester internucleoside linkages were oxidized to phosphate diester internucleoside linkages using a solution of *tert-*butyl hydroperoxide/ CH₃CN/water (10:87:3) over 12 minutes.

After the desired sequence was assembled, the solid support bound ss-siRNA was washed with CH₂Cl₂ and dried under high vacuum. After 4 hrs, the dried solid support was suspended in a solution of iodotrimethylsilane (TMSI) and pyridine in CH₂Cl₂ to remove the 5'-phosphonate protecting group (ethyl ether or methyl ether). The deprotection solution was prepared by dissolving 0.75 mL TMSI and 0.53 mL pyridine in 28.2 mL CH₂Cl₂ (used 0.5 mL/µmol of solid support). After 30 min at room temperature, the reaction was quenched with 1M 2-mercaptoethanol in 1:1 TEA/CH₃CN (used 0.5 mL/µmol of solid support). The supernatant was decanted and the solid-support was washed with additional 2-mercaptoethanol solution. After 45 minutes at room temperature the wash step with additional 2-mercaptoethanol solution was repeated. The supernatant was decanted and the solid-support bound oligomeric compound was suspended in ammonia (28-30 wt%) in 1M 2-mercaptoethanol (used 0.75 mL/µmol of solid support) and heated at 55 °C for 2 hrs to cleave the oligomeric compound from the solid support.

The cleaved solution was allowed to cool to ambient temperature (20 °C) for 24 hrs. The unbound oligomeric compound was then filtered and the support was rinsed and filtered with water:ethanol (1:1) followed by water. The filtrate was combined and concentrated to dryness. The residue obtained was purified by HPCL on a reverse phase column (Waters X-Bridge C-18 5µm, 19 x 250 mm, A = 5 mM tributylammonium acetate in 5% aqueous CH₃CN, B = CH₃CN, 0 to 90 % B in 80 min, flow 7 mL min⁻¹, λ = 260 nm). Fractions containing full-length oligomeric compound were pooled together (assessed by LC/MS analysis >95%) and the tributylammonium counter ion was exchanged to sodium by HPLC on a strong anion exchange column (GE Healthcare Bioscience, Source 30Q, 30 µm, 2.54 x 8 cm, A = 100 mM ammonium acetate in 30% aqueous CH₃CN, B = 1.5 M NaBr in A, 0-40% of B in 60 min, flow 14 mL min⁻¹). The residue was desalted by HPLC on a reverse phase column to yield the oligomeric compound in an isolated yield of 15-20% based on solid-support loading. The unbound oligomeric compound was characterized by ion-pair-HPLC-MS analysis with Agilent 1100 MSD system.

ss-siRNAs not comprising a conjugate were synthesized using standard oligonucleotide synthesis procedures well known in the art.

Using these methods, several ss-siRNAs targeting ApoC III were prepared and described in Table 1, below. Each of the six antisense compounds targeting ApoC III had the same nucleobase sequence as ISIS 572735 or 572746. ISIS 572735 had a 5'-phosphate-2'-MOE at the 5' terminus; ISIS 594230 or 594231 was the same as ISIS 572735, except that it had a 5'-phosphonate-2'-MOE group or a 5'-phosphonate-2'-C16 conjugate at its 5' end. Further, ISIS 572746 had a 5'-phosphate-2'-MOE at the 5' terminus; ISIS 594232 was the same as ISIS 572746, except that it had a 5'-phosphonate-2'-MOE; and ISIS 594290 was the same as ISIS 572746, except that it had a C16-conjugate at position 8, counting from the 5' end.

**Table 1**

| **Modified ss-siRNAs comprising** | | | |
|---|---|---|---|
| **5'-*(E)*-vinylphosphonate and/or 2'-C16 conjugate at position 1 or 8 targeting human ApoC III** | | | |
| ISIS No. | Composition (5' to 3') | Chemistry | SEQ ID No. |
| 572735 | Po-TesCfsAmoCfsUmoGfsAmoGfsAmoAfs UmoAfsCmoUfsGmsUfsCmsCfsCmsAesAe | 5'-Phosphate-2'-MOE | 3 |
| 594230 | Pv-TesCfsAmoCfsUmoGfsAmoGfsAmoAfs UmoAfsCmoUfsGmsUfsCmsCfsCmsAesAe | 5'-*(E)*-vinylphosphonate-2'-MOE | 3 |
| 594231 | Pv-TC16sCfsAmoCfsUmoGfsAmoGfsAmoAfs UmoAfsCmoUfsGmsUfsCmsCfsCmsAesAe | 5'-*(E)*-vinylphosphonate-2'-C16 at position 1 | 3 |
| 572746 | Po-TesAfsGmoCfsUmoUfsCmoUfsUmoGfs UmoCfsCmoAfsGmsCfsUmsUfsUmsAesAe | 5'-Phosphate-2'-MOE | 14 |
| 594232 | Pv-TesAfsGmoCfsUmoUfsCmoUfsUmoGfs UmoCfsCmoAfsGmsCfsUmsUfsUmsAesAe | 5'-*(E)*-vinylphosphonate-2'-MOE | 14 |
| 594290 | Pv-TesAfsGmoCfsUmoUfsCmoUC16sUmoGfs UmoCfsCmoAfsGmsCfsUmsUfsUmsAesAe | 5'-*(E)*-vinylphosphonate-2'-MOE with C16 conjugate at position 8 | 14 |

Subscripts: "s" between two nucleosides indicates a phosphorothioate internucleoside linkage; "o" between two nucleosides indicates a phosphodiester internucleoside linkage; "Pv" at the 5'-end indicates a 5'-*(*E*)*-vinylphosphonate group, (PO(OH)₂(CH=CH)-; "f" indicates a 2'-fluoro modified nucleoside; "m" indicates a 2'-O-methyl modified nucleoside; "e" indicates a 2'-O-methoxyethyl (MOE) modified nucleoside. Underlined nucleoside indicates the conjugate position.

### Example 7: Modified ss-siRNAs comprising 5'-phosphate at the 5' terminus

A series of modified ss-siRNAs were designed to target coding and non-coding regions of human ApoC III (hApoC III) and were screened for their inhibitory effect in reducing hApoC III *in vitro.* For ease of synthesis, these modified ss-siRNAs were designed by introducing a 5'-phosphate group at the 5' terminus.

The ss-siRNAs were prepared using similar procedures as illustrated in Example 6 and are described in Table 2, below. A subscript "s" between two nucleosides indicates a phosphorothioate internucleoside linkage. A subscript "o" between two nucleosides indicates a phosphodiester internucleoside linkage. A "Po" at the 5'-end indicates a 5'-phosphate group, (PO(OH)₂)-. Nucleosides followed by a subscript "f", "m", "e", or "k" are sugar modified nucleosides. A subscript "f" indicates a 2'-fluoro modified nucleoside; a subscript "m" indicates a 2'-O-methyl modified nucleoside; a subscript "e" indicates a 2'-O-methoxyethyl (MOE) modified nucleoside; and a subscript "k" indicates a constrained ethyl bicyclic nucleoside (cEt). "^{m}C" indicates 5-methyl cytosine.

Primary hepatocyte cells from transgenic mice at a density of 25,000 cells per well were electroporated at 20 µM concentration of modified ss-siRNA. After a treatment period of approximately 16 hours, RNA was isolated from the cells and mRNA levels were measured by quantitative real-time PCR. Primer probe set hApoC III or RTS1392 was used to measure mRNA levels. Human ApoC III mRNA levels were adjusted according to total RNA content, as measured by RIBOGREEN. Results are presented as percent of hApoC III mRNA expression, relative to untreated control levels and is denoted as "% UTC."

hApoC III primer probe set (forward sequence 5'-GCCGTGGCTGCCTGAG-3', designated herein as SEQ ID NO: 4; reverse sequence 5'-AGGAGCTCGCAGGATGGAT-3', designated herein as SEQ ID NO: 5; probe sequence 5'-CCTCAATACCCCAAGTCCACCTGCC-3', designated herein as SEQ ID NO: 6).

As illustrated in Table 3, the majority of the tested ss-siRNAs comprising 5'-phosphate demonstrated inhibition of hApoC III mRNA levels under the conditions specified above.

**Table 2**

| **Modified ss-siRNAs comprising 5'-phosphate at 5' terminus targeting hApoC III** | | |
|---|---|---|
| ISIS No. | Composition (5' to 3') | SEQ ID No. |
| 555559 | Po-Gₑₛ^{m}CₖₛAₖₛ^{m}C_{ds}T_{ds}G_{ds}A_{ds}G_{ds}A_{ds}A_{ds}T_{ds}A_{ds}^{m}C_{ds}TₖₛGₖₛTₑ | 7 |
| 572735 | Po-TₑₛC_{fs}AₘₒC_{fs}UₘₒG_{fs}AₘₒG_{fs}AₘₒA_{fs}UₘₒA_{fs}CₘₒU_{fs}GₘₛU_{fs}CₘₛC_{fs}CₘₛAₑₛAₑ | 3 |
| 572729 | Po-TₑₛG_{fs}AₘₒA_{fs}UₘₒA_{fs}CₘₒU_{fs}GₘₒU_{fs}CₘₒC_{fs}CₘₒU_{fs}UₘₛU_{fs}UₘₛA_{fs}A_{fs}AₑₛAₑ | 8 |
| 572730 | Po-TₑₛA_{fs}GₘₒA_{fs}AₘₒU_{fs}AₘₒC_{fs}UₘₒG_{fs}UₘₒC_{fs}CₘₒC_{fs}UₘₛU_{fs}UₘₛU_{fs}AₘₛAₑₛAₑ | 9 |
| 572731 | Po-TₑₛG_{fs}AₘₒG_{fs}AₘₒA_{fs}UₘₒA_{fs}CₘₒU_{fs}GₘₒU_{fs}CₘₒC_{fs}CₘₛU_{fs}UₘₛU_{fs}UₘₛAₑₛAₑ | 10 |
| 572733 | Po-TₑₛC_{fs}UₘₒG_{fs}AₘₒG_{fs}AₘₒA_{fs}UₘₒA_{fs}CₘₒU_{fs}GₘₒU_{fs}CₘₛC_{fs}CₘₛU_{fs}UₘₛAₑₛAₑ | 11 |
| 572732 | Po-TₑₛU_{fs}GₘₒA_{fs}GₘₒA_{fs}AₘₒU_{fs}AₘₒC_{fs}UₘₒG_{fs}UₘₒC_{fs}CₘₛC_{fs} UₘₛU_{fs}UₘₛAₑₛAₑ | 12 |
| 572736 | Po-TₑₛG_{fs}CₘₒA_{fs}CₘₒU_{fs}GₘₒA_{fs}GₘₒA_{fu}AₘₒU_{fs}AₘₒC_{fs}UₘₛG_{fs}UₘₛC_{fu}CₘₛAₑₛAₑ | 13 |
| 572746 | Po-TₑₛA_{fs}GₘₒC_{fs}UₘₒU_{fs}CₘₒU_{fs}UₘₒG_{fs}UₘₒC_{fs}CₘₒA_{fs}GₘₛC_{fs}UₘₛU_{fs}UₘₛAₑₛAₑ | 14 |
| 572734 | Po-TₑₛA_{fs}CₘₒU_{fs}GₘₒA_{fs}GₘₒA_{fs}AₘₒU_{fs}AₘₒC_{fs}UₘₒG_{fs}UₘₛC_{fs}CₘₛC_{fs}UₘₛAₑₛAₑ | 15 |
| 572738 | Po-TₑₛG_{fs}UₘₒC_{fs}CₘₒA_{fs}GₘₒC_{fs}UₘₒU_{fs}UₘₒA_{fs}UₘₒU_{fs}GₘₛG_{fs}GₘₛA_{fs}GₘₛAₑₛAₑ | 16 |
| 572709 | Po-TₑₛU_{fs}GₘₒU_{fs}CₘₒC_{fs}UₘₒU_{fs}AₘₒA_{fs}CₘₒG_{fs}GₘₒU_{fs}GₘₛC_{fs}UₘₛC_{fs}CₘₛAₑₛAₑ | 17 |
| 572728 | Po-TₑₛA_{fs}AₘₒU_{fs}AₘₒC_{fs}UₘₒG_{fs}UₘₒC_{fs}CₘₒC_{fs}UₘₒU_{fs}UₘₛU_{fs}AₘₛA_{fs}GₘₛAₑₛAₑ | 18 |
| 572742 | Po-TₑₛU_{fs}CₘₒU_{fs}UₘₒG_{fs}UₘₒC_{fs}CₘₒA_{fs}GₘₒC_{fs}UₘₒU_{fs}UₘₛA_{fs}UₘₛU_{fs}GₘₛAₑₛAₑ | 19 |
| 572749 | Po-TₑₛA_{fs}GₘₒC_{fs}AₘₒG_{fs}CₘₒU_{fs}UₘₒC_{fs}UₘₒU_{fe}GₘₒU_{fs}CₘₛC_{fe}AₘₛG_{fs}CₘₛAₑₛAₑ | 20 |
| 572739 | Po-TₑₛU_{fs}GₘₒU_{fs}CₘₒC_{fs}AₘₒG_{fs}CₘₒU_{fs}UₘₒU_{fs}AₘₒU_{fs}UₘₛG_{fs}GₘₛG_{fs}AₘₛAₑₛAₑ | 21 |
| 572741 | P-TₑₛC_{fs}UₘₒU_{fs}GₘₒU_{fs}CₘₒC_{fs}AₘₒG_{fs}CₘₒU_{fs}UₘₒU_{fs}AₘₛU_{fs}UₘₛG_{fs}GₘₛAₑₛAₑ | 22 |
| 572743 | Po-TₑₛU_{fs}UₘₒC_{fs}UₘₒU_{fs}GₘₒU_{fs}CₘₒC_{fs}AₘₒG_{fs}CₘₒU_{fs}UₘₛU_{fs}AₘₛU_{fs}UₘₛAₑₛAₑ | 23 |
| 572698 | Po-TₑₛG_{fs}UₘₒC_{fs}UₘₒU_{fs}UₘₒC_{fs}AₘₒG_{fs}GₘₒG_{fs}AₘₒA_{fs}CₘₛU_{fs}GₘₛA_{fs}AₘₛAₑₛAₑ | 24 |
| 572751 | Po-TₑₛA_{fs}UₘₒA_{fs}GₘₒC_{fs}AₘₒG_{fs}CₘₒU_{fs}UₘₒC_{fs}UₘₒU_{fs}GₘₛU_{fs}CₘₛC_{fs}AₘₛAₑₛAₑ | 25 |
| 572711 | Po-TₑₛA_{fs}AₘₒC_{fs}UₘₒU_{fs}GₘₒU_{fs}CₘₒC_{fs}UₘₒU_{fs}AₘₒA_{fs}CₘₛG_{fs}GₘₛU_{fs}GₘₛAₑₛAₑ | 26 |
| 572744 | Po-TₑₛC_{fs}UₘₒU_{fs}CₘₒU_{fs}UₘₒG_{fs}UₘₒC_{fs}CₘₒA_{fs}GₘₒC_{fs} UₘₛU_{fs}UₘₛA_{fs}UₘₛAₑₛAₑ | 27 |
| 572727 | Po-TₑₛA_{fs}UₘₒA_{fs}CₘₒU_{fs}GₘₒU_{fs}CₘₒC_{fs}CₘₒU_{fs}UₘₒU_{fs}UₘₛA_{fs}AₘₛG_{fs}CₘₛAₑₛAₑ | 28 |
| 572688 | Po-TₑₛG_{fs}GₘₒC_{fs}CₘₒA_{fs}CₘₒC_{fs}UₘₒG_{fs}GₘₒG_{fs}AₘₒC_{fs}UₘₛC_{fs}CₘₛU_{fs}GₘₛAₑₛAₑ | 29 |
| 572681 | Po-TₑₛC_{fs}CₘₒU_{fs}CₘₒU_{fs}GₘₒU_{fs}UₘₒC_{fs}CₘₒU_{fs}GₘₒG_{fs}AₘₛG_{fs}CₘₛA_{fs}GₘₛAₑₛAₑ | 30 |
| 572748 | Po-TₑₛG_{fs}CₘₒA_{fs}GₘₒC_{fs}UₘₒU_{fs}CₘₒU_{fs}UₘₒG_{fs}UₘₒC_{fs}CₘₒA_{fs}GₘₛC_{fs}UₘₛAₑₛAₑ | 31 |
| 572694 | Po-TₑₛG_{fs}AₘₒA_{fs}CₘₒU_{fs}GₘₒA_{fs}AₘₒG_{fs}CₘₒC_{fs}AₘₒU_{fs}CₘₛG_{fs}GₘₛU_{fs}CₘₛAₑₛAₑ | 32 |
| 572747 | Po-TₑₛC_{fs}AₘₒG_{fs}CₘₒU_{fs}UₘₒC_{fs}UₘₒU_{fs}GₘₒU_{fs}CₘₒC_{fs}AₘₛG_{fs}CₘₛU_{fs}UₘₛAₑₛAₑ | 33 |
| 572679 | Po-TₑₛU_{fs}GₘₒG_{fs}AₘₒG_{fs}CₘₒA_{fs}GₘₒC_{fs}UₘₒG_{fs}CₘₒC_{fs}UₘₛC_{fs}UₘₛA_{fs}GₘₛAₑₛAₑ | 34 |
| 572689 | Po-TₑₛU_{fs}GₘₒG_{fs}CₘₒC_{fs}UₘₒG_{fs}CₘₒU_{fs}GₘₒG_{fs}GₘₒC_{fs}CₘₛA_{fs}CₘₛC_{fs}UₘₛAₑₛAₑ | 35 |
| 572697 | Po-TₑₛC_{fs}UₘₒU_{fs}UₘₒC_{fs}AₘₒG_{fs}GₘₒG_{fs}AₘₒA_{fs}CₘₒU_{fs}GₘₛA_{fs}AₘₛG_{fs}CₘₛAₑₛAₑ | 36 |
| 572696 | Po-TₑₛC_{fs}AₘₒG_{fs}GₘₒG_{fs}AₘₒAₘₒCₘₒU_{fs}GₘₒA_{fs}AₘₒG_{fs}CₘₛC_{fs}AₘₛU_{fs}CₘₛAₑₛAₑ | 37 |
| 572693 | Po-TₑₛA_{fs}CₘₒU_{fs}GₘₒA_{fs}AₘₒG_{fs}CₘₒC_{fs}AₘₒU_{fs}CₘₒG_{fs}GₘₛU_{fs}CₘₛA_{fs}CₘₛAₑₛAₑ | 38 |
| 572752 | Po-TₑₛC_{fs}AₘₒU_{fs}AₘₒG_{fs}CₘₒA_{fs}GₘₒC_{fs}UₘₒU_{fs}CₘₒU_{fs}UₘₛG_{fs}UₘₛC_{fs}CₘₛAₑₛAₑ | 39 |
| 572700 | Po-TₑₛA_{fs}GₘₒU_{fs}AₘₒG_{fs}UₘₒC_{fs}UₘₒU_{fs}UₘₒC_{fs}AₘₒG_{fs}GₘₛG_{fs}AₘₛA_{fs}CₘₛAₑₛAₑ | 40 |
| 572690 | Po-TₑₛG_{fs}CₘₒC_{fs}AₘₒU_{fs}CₘₒG_{fs}GₘₒU_{fs}CₘₒA_{fs}CₘₒC_{fs}CₘₛA_{fs}GₘₛC_{fs}CₘₛAₑₛAₑ | 41 |
| 572737 | Po-TₑₛU_{fs}CₘₒC_{fs}AₘₒG_{fs}CₘₒU_{fs}UₘₒU_{fs}AₘₒU_{fs}UₘₒG_{fs}GₘₛG_{fs}AₘₛG_{fs}GₘₛAₑₛAₑ | 42 |
| 572740 | Po-TₑₛU_{fs}UₘₒG_{fs}UₘₒC_{fs}CₘₒA_{fs}GₘₒC_{fs}UₘₒU_{fs}UₘₒA_{fs}UₘₛU_{fs}GₘₛG_{fs}GₘₛAₑₛAₑ | 43 |
| 572692 | Po-TₑₛU_{fs}GₘₒA_{fs}AₘₒG_{fs}CₘₒC_{fs}AₘₒU_{fs}CₘₒG_{fs}GₘₒU_{fs}CₘₛA_{fs}CₘₛC_{fs}CₘₛAₑₛAₑ | 44 |
| 572701 | Po-TₑₛC_{fs}CₘₒA_{fs}GₘₒU_{fs}AₘₒG_{fs}UₘₒC_{fs}UₘₒU_{fs}UₘₒC_{fs}AₘₛG_{fs}GₘₛG_{fs}AₘₛAₑₛAₑ | 45 |
| 572745 | Po-TₑₛG_{fs}CₘₒU_{fs}UₘₒC_{fs}UₘₒU_{fs}GₘₒU_{fs}CₘₒC_{fs}AₘₒG_{fs}CₘₛU_{fs}UₘₛU_{fs}AₘₛAₑₛAₑ | 46 |
| 572726 | Po-TₑₛU_{fs}AₘₒC_{fs}UₘₒG_{fs}UₘₒC_{fs}CₘₒC_{fs}UₘₒU_{fs}UₘₒU_{fs}AₘₛA_{fs}GₘₛC_{fs}AₘₛAₑₛAₑ | 47 |
| 572699 | Po-TₑₛU_{fs}AₘₒG_{fs}UₘₒC_{fs}UₘₒU_{fs}UₘₒC_{fs}AₘₒG_{fs}GₘₒG_{fs}AₘₛA_{fs}CₘₛU_{fs}GₘₛAₑₛAₑ | 48 |
| 572714 | Po-TₑₛG_{fs}GₘₒU_{fs}AₘₒU_{fs}UₘₒG_{fs}AₘₒG_{fs}GₘₒU_{fs}CₘₒU_{fs}CₘₛA_{fs}GₘₛG_{fs}CₘₛAₑₛAₑ | 49 |
| 572691 | Po-TₑₛA_{fs}AₘₒG_{fs}CₘₒC_{fs}AₘₒU_{fs}CₘₒG_{fs}GₘₒU_{fs}CₘₒA_{fs}CₘₛC_{fs}CₘₛA_{fs}GₘₛAₑₛAₑ | 50 |
| 572680 | Po-TₑₛU_{fs}GₘₒU_{fs}UₘₒC_{fs}CₘₒU_{fs}GₘₒG_{fs}AₘₒG_{fs}CₘₒA_{fs}GₘₛC_{fs}UₘₛG_{fs}CₘₛAₑₛAₑ | 51 |
| 572750 | Po-TₑₛU_{fs}AₘₒG_{fs}CₘₒA_{fs}GₘₒC_{fs}UₘₒU_{fs}CₘₒU_{fs}UₘₒG_{fs}UₘₛC_{fs}CₘₛA_{fs}GₘₛAₑₛAₑ | 52 |
| 572695 | Po-TₑₛG_{fs}GₘₒG_{fs}AₘₒA_{fs}CₘₒU_{fs}GₘₒA_{fs}AₘₒG_{fs}CₘₒC_{fs}AₘₛU_{fs}CₘₛG_{fs}GₘₛAₑₛAₑ | 53 |
| 572717 | Po-TₑₛU_{fs}UₘₒU_{fs}UₘₒA_{fs}AₘₒG_{fs}CₘₒA_{fs}AₘₒC_{fs}CₘₒU_{fs}AₘₛC_{fs}AₘₛG_{fs}GₘₛAₑₛAₑ | 54 |
| 572702 | Po-TₑₛC_{fs}UₘₒC_{fs}CₘₒA_{fs}GₘₒU_{fs}AₘₒG_{fs}UₘₒC_{fs}UₘₒU_{fs}UₘₛC_{fs}AₘₛG_{fs}GₘₛAₑₛAₑ | 55 |
| 572703 | Po-TₑₛU_{fs}GₘₒC_{fs}UₘₒC_{fs}CₘₒA_{fs}GₘₒU_{fs}AₘₒG_{fs}UₘₒC_{fs}UₘₛU_{fs}UₘₛC_{fs}AₘₛAₑₛAₑ | 56 |
| 572705 | Po-TₑₛA_{fs}CₘₒG_{fs}GₘₒU_{fs}GₘₒC_{fs}UₘₒC_{fs}CₘₒA_{fs}GₘₒU_{fs}AₘₛG_{fs}UₘₛC_{fs}UₘₛAₑₛAₑ | 57 |
| 572725 | Po-TₑₛA_{fs}CₘₒU_{fs}GₘₒU_{fs}CₘₒC_{fs}CₘₒU_{fs}UₘₒU_{fs}UₘₒA_{fs}AₘₛG_{fs}CₘₛA_{fs}AₘₛAₑₛAₑ | 58 |
| 572708 | Po-TₑₛU_{fs}CₘₒC_{fs}UₘₒU_{fs}AₘₒA_{fs}CₘₒG_{fs}GₘₒU_{fs}GₘₒC_{fs}UₘₛC_{fs}CₘₛA_{fs}GₘₛAₑₛAₑ | 59 |
| 572704 | Po-TₑₛG_{fs}GₘₒU_{fs}GₘₒC_{fs}UₘₒC_{fs}CₘₒA_{fs}GₘₒU_{fs}AₘₒG_{fs}UₘₛC_{fs}UₘₛU_{fs}UₘₛAₑₛAₑ | 60 |
| 572706 | Po-TₑₛU_{fs}AₘₒA_{fs}CₘₒG_{fs}GₘₒU_{fs}GₘₒC_{fs}UₘₒC_{fs}CₘₒA_{fs}GₘₛU_{fs}AₘₛG_{fs}UₘₛAₑₛAₑ | 61 |
| 572716 | Po-TₑₛU_{fs}UₘₒU_{fs}AₘₒA_{fs}GₘₒC_{fs}AₘₒA_{fs}CₘₒC_{fs}UₘₒA_{fs}CₘₛA_{fs}GₘₛG_{fs}GₘₛAₑₛAₑ | 62 |
| 572724 | Po-TₑₛC_{fs}UₘₒG_{fs}UₘₒC_{fs}CₘₒC_{fs}UₘₒU_{fs}UₘₒU_{fs}AₘₒA_{fs}GₘₛC_{fs}AₘₛA_{fs}CₘₛAₑₛAₑ | 63 |
| 572713 | Po-TₑₛU_{fs}AₘₒU_{fs}UₘₒG_{fs}AₘₒG_{fs}GₘₒU_{fs}CₘₒU_{fs}CₘₒA_{fs}GₘₛG_{fs}CₘₛA_{fs}GₘₛAₑₛAₑ | 64 |
| 572710 | Po-TₑₛC_{fs}UₘₒU_{fs}GₘₒU_{fs}CₘₒC_{fs}UₘₒU_{fs}AₘₒA_{fs}CₘₒG_{fs}GₘₛU_{fs}GₘₛC_{fs}UₘₛAₑₛAₑ | 65 |
| 572707 | Po-TₑₛC_{fs}UₘₒU_{fs}AₘₒA_{fs}CₘₒG_{fs}GₘₒU_{fs}GₘₒC_{fs}UₘₒC_{fs}CₘₛA_{fs}GₘₛU_{fs}AₘₛAₑₛAₑ | 66 |
| 572721 | Po-TₑₛU_{fs}CₘₒC_{fs}CₘₒU_{fs}UₘₒU_{fs}UₘₒA_{fs}AₘₒG_{fs}CₘₒA_{fs}AₘₛC_{fs}CₘₛU_{fs}AₘₛAₑₛAₑ | 67 |
| 572720 | Po-TₑₛC_{fs}CₘₒC_{fs}UₘₒU_{fs}UₘₒU_{fs}AₘₒA_{fs}GₘₒC_{fs}AₘₒA_{fs}CₘₛC_{fs}UₘₛA_{fs}CₘₛAₑₛAₑ | 68 |
| 572682 | Po-TₑₛU_{fs}CₘₒC_{fs}UₘₒC_{fs}GₘₒG_{fs}CₘₒC_{fs}UₘₒC_{fs}UₘₒG_{fs}AₘₛA_{fs}GₘₛC_{fs}UₘₛAₑₛAₑ | 69 |
| 572712 | Po-TₑₛU_{fs}UₘₒG_{fs}AₘₒG_{fs}GₘₒU_{fs}CₘₒU_{fs}CₘₒA_{fs}GₘₒG_{fs}CₘₛA_{fs}GₘₛC_{fs}CₘₛAₑₛAₑ | 70 |
| 572722 | Po-TₑₛG_{fs}UₘₒC_{fs}CₘₒC_{fs}UₘₒU_{fs}UₘₒU_{fs}AₘₒA_{fs}GₘₒC_{fs}AₘₛA_{fs}CₘₛC_{fs}UₘₛAₑₛAₑ | 71 |
| 572719 | Po-TₑₛC_{fs}CₘₒU_{fs}UₘₒU_{fs}UₘₒA_{fs}AₘₒG_{fs}CₘₒA_{fs}AₘₒC_{fs}CₘₛU_{fs}AₘₛC_{fs}AₘₛAₑₛAₑ | 72 |
| 572715 | Po-TₑₛU_{fs}GₘₒC_{fs}AₘₒG_{fs}GₘₒA_{fs}CₘₒC_{fs}CₘₒA_{fs}AₘₒG_{fs}GₘₛA_{fs}GₘₛC_{fs}UₘₛAₑₛAₑ | 73 |
| 572718 | Po-TₑₛC_{fs}UₘₒU_{fs}UₘₒU_{fs}AₘₒA_{fs}GₘₒC_{fs}AₘₒA_{fs}CₘₒC_{fs}UₘₛA_{fs}CₘₛA_{fs}GₘₛAₑₛAₑ | 74 |
| 572678 | Po-TₑₛG_{fs}AₘₒG_{fs}CₘₒA_{fs}GₘₒC_{fs}UₘₒG_{fs}CₘₒC_{fs}UₘₒC_{fs}UₘₛA_{fs}GₘₛG_{fs}GₘₛAₑₛAₑ | 75 |
| 572676 | Po-TₑₛA_{fs}GₘₒC_{fs}UₘₒG_{fs}CₘₒC_{fs}UₘₒC_{fs}UₘₒA_{fs}GₘₒG_{fs}GₘₛA_{fs}UₘₛG_{fs}AₘₛAₑₛAₑ | 76 |
| 572675 | Po-TₑₛC_{fs}UₘₒG_{fs}CₘₒC_{fs}UₘₒC_{fs}UₘₒA_{fs}GₘₒG_{fs}GₘₒA_{fs}UₘₛG_{fs}AₘₛA_{fs}CₘₛAₑₛAₑ | 77 |
| 572677 | Po-TₑₛG_{fs}CₘₒA_{fs}GₘₒC_{fs}UₘₒG_{fs}CₘₒC_{fs}UₘₒC_{fs}UₘₒA_{fs}GₘₛG_{fs}GₘₛA_{fs}UₘₛAₑₛAₑ | 78 |
| 572723 | Po-TₑₛU_{fs}GₘₒU_{fs}CₘₒC_{fs}CₘₒU_{fs}UₘₒU_{fs}UₘₒA_{fs}AₘₒG_{fs}CₘₛA_{fs}AₘₛC_{fs}CₘₛAₑₛAₑ | 79 |
| 572685 | Po-TₑₛC_{fs}AₘₒU_{fs}CₘₒC_{fs}UₘₒU_{fs}GₘₒG_{fs}CₘₒG_{fs}GₘₒU_{fs}CₘₛU_{fs}UₘₛG_{fs}GₘₛAₑₛAₑ | 80 |
| 572684 | Po-TₑₛU_{fs}CₘₒC_{fs}UₘₒU_{fs}GₘₒG_{fs}CₘₒG_{fs}GₘₒU_{fs}CₘₒU_{fs}UₘₛG_{fs}GₘₛU_{fs}GₘₛAₑₛ Aₑ | 81 |
| 572687 | Po-TₑₛU_{fs}CₘₒA_{fs}GₘₒU_{fs}GₘₒC_{fs}AₘₒU_{fs}CₘₒC_{fs}UₘₒU_{fs}GₘₛG_{fs}CₘₛG_{fs}GₘₛAₑₛAₑ | 82 |
| 572686 | Po-TₑₛA_{fs}GₘₒU_{fs}GₘₒC_{fs}AₘₒU_{fs}CₘₒC_{fs}UₘₒU_{fs}GₘₒG_{fs}CₘₛG_{fs}GₘₛU_{fs}CₘₛAₑₛAₑ | 83 |
| 572683 | Po-TₑₛC_{fs}UₘₒU_{fs}GₘₒG_{fs}CₘₒG_{fs}GₘₒU_{fs}CₘₒU_{fs}UₘₒG_{fs}GₘₛU_{fs}GₘₛG_{fs}CₘₛAₑₛAₑ | 84 |
| 18076 | ^{m}CₑₛTₑₛTₑₛTₑₛ^{m}CₑₛC_{ds}G_{ds}T_{ds}T_{ds}G_{ds}G_{ds}A_{ds}C_{ds}C_{ds}^{m}Cₑₛ^{m}CₑₛTₑₛGₑₛGₑₛGₑ | 85 |
| 18078 | GₑₛTₑₛGₑₛ^{m}CₑₛGₑₛC_{ds}G_{ds}C_{ds}G_{ds}A_{ds}G_{ds}C_{ds}C_{ds}C_{ds}GₑₛAₑₛAₑₛAₑₛTₑₛ^{m}Cₑ | 86 |

**Table 3**

| **Inhibitory effect of 5'-phosphate ss-siRNAs on hApoC III mRNA levels using primer probe set hApoC III** | | |
|---|---|---|
| ISIS No. | hApoC III % UTC | SEQ ID No. |
| 555559 | 3.39 | 7 |
| 572735 | 7.45 | 3 |
| 572729 | 7.69 | 8 |
| 572730 | 10.71 | 9 |
| 572731 | 10.81 | 10 |
| 572733 | 12.60 | 11 |
| 572732 | 12.67 | 12 |
| 572736 | 14.70 | 13 |
| 572746 | 30.87 | 14 |
| 572734 | 33.06 | 15 |
| 572738 | 32.02 | 16 |
| 572709 | 38.67 | 17 |
| 572728 | 37.21 | 18 |
| 572742 | 37.15 | 19 |
| 572749 | 41.34 | 20 |
| 572739 | 44.26 | 21 |
| 572741 | 50.54 | 22 |
| 572743 | 26.68 | 23 |
| 572698 | 51.10 | 24 |
| 572751 | 44.28 | 25 |
| 572711 | 48.01 | 26 |
| 572744 | 53.50 | 27 |
| 572727 | 54.68 | 28 |
| 572688 | 60.22 | 29 |
| 572681 | 52.84 | 30 |
| 572748 | 57.48 | 31 |
| 572694 | 65.20 | 32 |
| 572747 | 61.79 | 33 |
| 572679 | 61.99 | 34 |
| 572689 | 77.50 | 35 |
| 572697 | 63.28 | 36 |
| 572696 | 67.52 | 37 |
| 572693 | 71.22 | 38 |
| 572752 | 58.01 | 39 |
| 572700 | 76.3 | 40 |
| 572690 | 70.34 | 41 |
| 572737 | 71.28 | 42 |
| 572740 | 64.20 | 43 |
| 572692 | 78.22 | 44 |
| 572701 | 86.53 | 45 |
| 572745 | 71.58 | 46 |
| 572726 | 81.89 | 47 |
| 572699 | 87.02 | 48 |
| 572714 | 78.31 | 49 |
| 572691 | 84.5 | 50 |
| 572680 | 73.78 | 51 |
| 572750 | 87.61 | 52 |
| 572695 | 86.70 | 53 |
| 572717 | 89.51 | 54 |
| 572702 | 93.01 | 55 |
| 572703 | 90.53 | 56 |
| 572705 | 88.87 | 57 |
| 572725 | 93.93 | 58 |
| 572708 | 102.46 | 59 |
| 572704 | 99.52 | 60 |
| 572706 | 97.31 | 61 |
| 572716 | 99.38 | 62 |
| 572724 | 101.99 | 63 |
| 572713 | 99.07 | 64 |
| 572710 | 108.35 | 65 |
| 572707 | 119.09 | 66 |
| 572721 | 94.72 | 67 |
| 572720 | 92.43 | 68 |
| 572682 | 111.31 | 69 |
| 572712 | 124.24 | 70 |
| 572722 | 127.51 | 71 |
| 572719 | 119.29 | 72 |
| 572715 | 131.82 | 73 |
| 572718 | 150.78 | 74 |
| 572678 | 162.04 | 75 |
| 572676 | 124.96 | 76 |
| 572675 | >125 | 77 |
| 572677 | >125 | 78 |
| 18076 | 95.11 | 85 |
| 18078 | 121.90 | 86 |

### Example 8: Inhibitory effect of ss-siRNAs on hApoC III expression in vitro

Several modified ss-siRNAs from Table 2, each targeting hApoC III were selected and further evaluated in a dose-response study for their ability to inhibit hApoC III expression *in vitro.*

Primary hepatocyte cells from transgenic mice at a density of 25,000 cells per well were electroporated at 0.03,0.08, 0.25, 0.74, 2.22, 6.67 and 20 µM concentration of modified ss-siRNA. After a treatment period of approximately 16 hours, RNA was isolated from the cells and mRNA levels were measured by quantitative real-time PCR. Primer probe set hApoC III was used to measure mRNA levels. Human ApoC III mRNA levels were adjusted according to total RNA content, as measured by RIBOGREEN.

The half maximal inhibitory concentration (IC₅₀) of each ss-siRNA was measured by plotting the concentrations of ss-siRNAs used versus the percent inhibition of hApoC III expression achieved at each concentration, and noting the concentration of ss-siRNA at which 50% inhibition of hApoC III mRNA expression was achieved compared to the control. Only the IC₅₀ values are reported and the results are presented in Table 4, below.

As illustrated, ISIS 572735, 572736 and 572746 demonstrated greater potency in reducing hApoC III mRNA levels than their counterparts.

**Table 4**

| **Inhibitory effect of modified ss-siRNAs on hApoC III mRNA levels** | | |
|---|---|---|
| ISIS No. | IC₅₀ (µM) | SEQ ID No. |
| 572735 | 0.26 | 3 |
| 572729 | 1.25 | 8 |
| 572730 | 1.92 | 9 |
| 572731 | 1.66 | 10 |
| 572733 | 1.64 | 11 |
| 572732 | 1.19 | 12 |
| 572736 | 0.79 | 13 |
| 572746 | 0.22 | 14 |
| 572734 | 2.14 | 15 |
| 572738 | 2.88 | 16 |
| 572728 | 17.33 | 18 |

### Example 9: Inhibitory effect of ss-siRNAs on hApoC III expression in vitro

Additional ss-siRNAs were designed based on the parent compounds identified from the previous screens, ISIS 572735 and 572746 (see Table 1). The newly designed ss-siRNAs comprise a 5'-vinylphosphonate-2'-MOE, a 5'-phosphonate-2'-C16 conjugate at position 1, or a 5'-vinylphosphonate-2'-MOE with 2'-C16 at position 8. The ss-siRNAs were tested and evaluated in a dose-reponse study for hApoC III inhibition in hepatocytes. ISIS 572735, and 572746 were included in the study for comparison.

Primary hepatocyte cells from transgenic mice at a density of 25,000 cells per well were electroporated at 0.03,0.08, 0.25, 0.74, 2.22, 6.67 and 20 µM concentration of modified ss-siRNA. After a treatment period of approximately 16 hours, RNA was isolated from the cells and mRNA levels were measured by quantitative real-time PCR. Primer probe set hApoC III was used to measure mRNA levels. Human ApoC III mRNA levels were adjusted according to total RNA content, as measured by RIBOGREEN.

The IC₅₀ of each ss-siRNA was measured in the same manner as described in Example 8. The IC₅₀ for ISIS 594230, 594231, 497687, and 594232 are presented as the average IC₅₀ measured from multiple independent studies. As illustrated in Tables 5 and 6, reduction in potency was observed for C16 conjugated ss-siRNAs compared to the parent ss-siRNAs lacking the conjugate. Moreover, ISIS 594231 comprising C16 at position 1 demonstrated greater *in vitro* potency compared to ISIS 594290 with C16 conjugate at position 8.

**Table 5**

| **Inhibitory effect of modified ss-siRNAs comprising 5'-*(E)*-vinylphosphonate-2'-C16 conjugate at position 1 targeting hApoC III** | | | |
|---|---|---|---|
| ISIS No. | IC₅₀ (µM) | Chemistry | SEQ ID No. |
| 572735 (parent) | 0.26 | 5'-Phosphate-2'-MOE | 3 |
| 594230 | 0.23 | 5'-*(E)*-vinylphosphonate-2'-MOE | 3 |
| 594231 | 2.17 | 5'-*(E)*-vinylphosphonate-2'-C16 at position 1 counting from 5' end | 3 |

**Table 6**

| **Inhibitory effect of modified ss-siRNAs comprising 5'-*(E)*-vinylphosphonate-2'-MOE with C16 conjugate at position 8 targeting hApoC III** | | | |
|---|---|---|---|
| ISIS No. | IC₅₀ (µM) | Chemistry | SEQ ID No. |
| 572746 (parent) | 0.22 | 5'-Phosphate-2'-MOE | 14 |
| 594232 | 1.25 | 5'*-(E)*-vinylphosphonate-2'-MOE | 14 |
| 594290 | >20 | 5'-*(E)*-vinylphosphonate-2'-MOE with C16 conjugate at position 8 counting from 5' end | 14 |

### Example 10: Effect of ss-siRNAs on inhibition of human ApoC III in hApoC III transgenic mice

ISIS 594230, 594231, 594232, and 594290, each targeting human ApoC III and are described in Table 1, above, were separately tested and evaluated for hApoC III inhibition in hApoC III transgenic mice.

### Treatment

Male human ApoCIII transgenic mice were maintained on a 12-hour light/dark cycle and fed ad libitum Teklad lab chow. Animals were acclimated for at least 7 days in the research facility before initiation of the experiment. ss-siRNAs were prepared in PBS and sterilized by filtering through a 0.2 micron filter. ss-siRNAs were dissolved in 0.9% PBS for injection.

Male human ApoC III transgenic mice were injected subcutaneously twice a week for three weeks with ISIS 594231, 594290, and 497687 at the dosage presented in Table 7, below or with PBS as a control. For parent compounds lacking C16-conjugate, ISIS 594230 and 594232, the animals were dosed twice a day at 25 mg/kg for two days (100 mg/kg total). Each treatment group consisted of 4 animals. Forty-eight hours after the administration of the last dose, blood was drawn from each mouse and the mice were sacrificed and tissues were collected.

### ApoC III mRNA Analysis

ApoC III mRNA levels in the mice's livers were determined using real-time PCR and RIBOGREEN® RNA quantification reagent (Molecular Probes, Inc. Eugene, OR) according to standard protocols. ApoC III mRNA levels were determined relative to total RNA (using Ribogreen), prior to normalization to PBS-treated control. The results below are presented as the average percent of ApoC III mRNA levels for each treatment group, normalized to PBS-treated control and are denoted as "% PBS". The half maximal effective dosage (ED₅₀) of the ss-siRNAs was measured using the standard method and is presented in Table 7, below. "N/A" indicates not applicable.

ISIS 594231 has the same nucleobase sequence as ISIS 594230, except it has a C16 conjugate at position 1. ISIS 594290 has the same nucleobase sequence as ISIS 594232, except it has a C16 conjugate at position 8. As illustrated, treatment with ss-siRNAs demonstrated inhibition of hApoC III mRNA levels compared to PBS treated control. Moreover, treatment with C16 conjugated ss-siRNAs demonstrated inhibition of hApoC III mRNA levels in a dose-dependent manner. Greater *in vivo* potency was observed for C16 conjugated ss-siRNA at position 1 compared to position 8.

**Table 7**

| **Effect of ss-siRNA treatment on hApoC III mRNA levels in transgenic mice** | | | | | |
|---|---|---|---|---|---|
| ss-siRNA | Dose (mg/kg) | % PBS | ED₅₀ (mg/kg) | Chemistry | SEQ ID No. |
| PBS | 0 | 99.89 | N/A | | |
| ISIS 594230 (parent) | 25 mg/kg twice/day (100 mg/kg total) | 20.21 | N/A | 5'-*(E)*-vinylphosphonate-2'-MOE | 3 |
| ISIS 594231 | 6 | 97.56 | 10 | 5'-*(E)*-vinylphosphonate-2'-C16 at position 1 counting from 5' end | 3 |
| | 14 | 33.97 | | | |
| | 36 | 12.65 | | | |
| | 88 | 10.52 | | | |
| ISIS 594232 (parent) | 25 mg/kg twice/day (100 mg/kg total) | 82.28 | N/A | 5'-*(E)*-vinylphosphonate-2'-MOE | 14 |
| ISIS 594290 | 6 | 104.00 | 20 | 5'-*(E)*-vinylphosphonate-2'-MOE with C16 conjugate at position 8 counting from 5' end | 14 |
| | 14 | 67.25 | | | |
| | 36 | 39.46 | | | |
| | 88 | 22.35 | | | |

### ApoC III Protein Analysis (Turbidometric Assay)

Plasma ApoC III protein analysis was determined using procedures reported by Graham et al, Circulation Research, published online before print March 29, 2013.

Approximately 100 µl of plasma isolated from mice was analyzed without dilution using an Olympus Clinical Analyzer and a commercially available turbidometric ApoC III assay (Kamiya, Cat# KAI-006, Kamiya Biomedical, Seattle, WA). The assay protocol was performed as described by the vendor.

ISIS 594231 has the same nucleobase sequence as ISIS 594230, except it has a C16 conjugate at position 1. ISIS 594290 has the same nucleobase sequence as ISIS 594232, except it has a C16 conjugate at position 8. "N/A" indicates not applicable.

As illustrated, treatment with ss-siRNAs demonstrated inhibition of hApoC III protein levels compared to PBS treated control. Moreover, treatment with C16 conjugated ss-siRNAs demonstrated inhibition of hApoC III protein levels in a dose-dependent manner. Greater *in vivo* potency was observed for C16 conjugated ss-siRNA at position 1 compared to position 8.

**Table 8**

| **Effect of ss-siRNA treatment on hApoC III plasma protein levels in transgenic mice** | | | | | |
|---|---|---|---|---|---|
| ss-siRNA | Dose (mg/kg) | % PBS | ED₅₀ (mg/kg) | Chemistry | SEQ ID No. |
| PBS | 0 | 105.92 | N/A | | |
| ISIS 594230 (parent) | 25 mg/kg twice/day (100 mg/kg total) | 6.98 | N/A | 5'-*(E)*-vinylphosphonate-2'-MOE | 3 |
| ISIS 594231 | 6 | 51.72 | 10 | 5'-*(E)*-vinylphosphonate-2'-C16 at position 1 counting from 5' end | 3 |
| | 14 | 24.79 | | | |
| | 36 | 10.02 | | | |
| | 88 | 4.74 | | | |
| ISIS 594232 (parent) | 25 mg/kg twice/day (100 mg/kg total) | 50.12 | N/A | 5'-*(E)*-vinylphosphonate-2'-MOE | 14 |
| ISIS 594290 | 6 | 95.54 | 20 | 5'-*(E)*-vinylphosphonate-2'-MOE with C16 conjugate at position 8 counting from 5' end | 14 |
| | 14 | 58.43 | | | |
| | 36 | 20.03 | | | |
| | 88 | 12.61 | | | |

Plasma triglycerides and cholesterol were extracted by the method of Bligh and Dyer (Bligh, E.G. and Dyer, W.J. Can. J. Biochem. Physiol. 37: 911-917, 1959)(Bligh, E and Dyer, W, Can J Biochem Physiol, 37, 911-917, 1959)(Bligh, E and Dyer, W, Can J Biochem Physiol, 37, 911-917, 1959) and measured by using a Beckmann Coulter clinical analyzer and commercially available reagents.

The triglyceride levels were measured relative to PBS injected mice and is denoted as "% PBS". Results are presented in Table 9. "N/A" indicates not applicable.

ISIS 594231 has the same nucleobase sequence as ISIS 594230, except it has a C16 conjugate at position 1. ISIS 594290 has the same nucleobase sequence as ISIS 594232, except it has a C16 conjugate at position 8. As illustrated, treatment with ss-siRNAs demonstrated substantial reduction in triglyceride levels compared to PBS treated control. Moreover, treatment with C16 conjugated ss-siRNAs demonstrated reduction in triglyceride levels in a dose-dependent manner. Greater *in vivo* potency was observed for C16 conjugated ss-siRNA at position 1 compared to position 8.

**Table 9**

| **Effect of ss-siRNA treatment on triglyceride levels in transgenic mice** | | | | | |
|---|---|---|---|---|---|
| ss-siRNA | Dose (mg/kg) | % PBS | ED₅₀ (mg/kg) | Chemistry | SEQ ID No. |
| PBS | 0 | 111.57 | N/A | | |
| ISIS 594230 (parent) | 25 mg/kg twice/day (100 mg/kg total) | 9.22 | N/A | 5'-*(E)*-vinylphosphonate-2'-MOE | 3 |
| ISIS 594231 | 6 | 46.90 | 8 | 5'-*(E)*-vinylphosphonate-2'-C16 at position 1 counting from 5' end | 3 |
| | 14 | 22.13 | | | |
| | 36 | 14.70 | | | |
| | 88 | 9.83 | | | |
| ISIS 594232 (parent) | 25 mg/kg twice/day (100 mg/kg total) | 44.97 | N/A | 5'-*(E)*-vinylphosphonate-2'-MOE | 14 |
| ISIS 594290 | 6 | 92.18 | 15 | 5'-*(E)*-vinylphosphonate-2'-MOE with C16 conjugate at position 8 counting from 5' end | 14 |
| | 14 | 55.68 | | | |
| | 36 | 19.45 | | | |
| | 88 | 13.76 | | | |

Plasma samples were analyzed by HPLC to determine the amount of total cholesterol and of different fractions of cholesterol (HDL and LDL). Results are presented in Tables 10, 11 and 12. "N/A" indicates not applicable.

ISIS 594231 has the same nucleobase sequence as ISIS 594230, except it has a C16 conjugate at position 1. ISIS 594290 has the same nucleobase sequence as ISIS 594232, except it has a C16 conjugate at position 8. As illustrated, treatment with ss-siRNAs lowered total cholesterol levels, lowered LDL levels, and increased HDL levels compared to PBS treated control. An increase in HDL and a decrease in LDL levels is a cardiovascular beneficial effect of ss-siRNA inhibition of ApoC III.

**Table 10**

| **Effect of ss-siRNA treatment on total cholesterol levels in transgenic mice** | | | | |
|---|---|---|---|---|
| ss-siRNA | Dose (mg/kg) | Total Cholesterol (mg/dL) | Chemistry | SEQ ID No. |
| PBS | 0 | 102.59 | | |
| ISIS 594230 (parent) | 25 mg/kg twice/day (100 mg/kg total) | 56.83 | 5'-*(E)*-vinylphosphonate-2'-MOE | 3 |
| ISIS 594231 | 6 | 74.63 | 5'-*(E)*-vinylphosphonate-2'-C16 at position 1 counting from 5' end | 3 |
| | 14 | 45.98 | | |
| | 36 | 53.21 | | |
| | 88 | 54.70 | | |
| ISIS 594232 (parent) | 25 mg/kg twice/day (100 mg/kg total) | 71.94 | 5'-*(E)*-vinylphosphonate-2'-MOE | 14 |
| ISIS 594290 | 6 | 90.78 | 5'-*(E)*-vinylphosphonate-2'-MOE with C16 conjugate at position 8 counting from 5' end | 14 |
| | 14 | 66.73 | | |
| | 36 | 48.96 | | |
| | 88 | 55.77 | | |

**Table 11**

| **Effect of ss-siRNA treatment on LDL levels in transgenic mice** | | | | |
|---|---|---|---|---|
| ss-siRNA | Dose (mg/kg) | LDL (mg/dL) | Chemistry | SEQ ID No. |
| PBS | 0 | 105.31 | | |
| ISIS 594230 (parent) | 25 mg/kg twice/day (100 mg/kg total) | 14.02 | 5'-*(E)*-vinylphosphonate-2'-MOE | 3 |
| ISIS 594231 | 6 | 92.92 | 5'-*(E)*-vinylphosphonate-2'-C16 at position 1 counting from 5' end | 3 |
| | 14 | 29.28 | | |
| | 36 | 17.96 | | |
| | 88 | 25.70 | | |
| ISIS 594232 (parent) | 25 mg/kg twice/day (100 mg/kg total) | 70.78 | 5'-*(E)*-vinylphosphonate-2'-MOE | 14 |
| ISIS 594290 | 6 | 98.70 | 5'-*(E)*-vinylphosphonate-2'-MOE with C16 conjugate at position 8 counting from 5' end | 14 |
| | 14 | 78.16 | | |
| | 36 | 33.59 | | |
| | 88 | 28.55 | | |

**Table 12**

| **Effect of ss-siRNA treatment on HDL levels in transgenic mice** | | | | |
|---|---|---|---|---|
| ss-siRNA | Dose (mg/kg) | HDL (mg/dL) | Chemistry | SEQ ID No. |
| PBS | 0 | 77.24 | | |
| ISIS 594230 (parent) | 25 mg/kg twice/day (100 mg/kg total) | 247.72 | 5'-*(E)-*vinylphosphonate-2'-MOE | 3 |
| ISIS 594231 | 6 | 151.53 | *5'-(E)-*vinylphosphonate-2'-C16 at position 1 counting from 5' end | 3 |
| | 14 | 159.43 | | |
| | 36 | 221.45 | | |
| | 88 | 235.64 | | |
| ISIS 594232 (parent) | 25 mg/kg twice/day (100 mg/kg total) | 200.91 | 5*'-(E)-*vinylphosphonate-2'-MOE | 14 |
| ISIS 594290 | 6 | 112.30 | *5'-(E)-*vinylphosphonate-2'-MOE with C16 conjugate at position 8 counting from 5' end | 14 |
| | 14 | 145.17 | | |
| | 36 | 171.50 | | |
| | 88 | 235.19 | | |

Liver transaminase levels, alanine aminotranferease (ALT) and aspartate aminotransferase (AST), in serum were measured relative to saline injected mice using standard protocols. Organ weights were also evaluated. The results demonstrated that no elevation in transaminase levels or organ weights was observed in mice treated with ss-siRNAs compared to PBS control.

**Table 13**

| **Effect of ss-siRNA treatment on ALT levels in transgenic mice** | | | | |
|---|---|---|---|---|
| ss-siRNA | Dose (mg/kg) | ALT (IU/L) | Chemistry | SEQ ID No. |
| PBS | 0 | 103.46 | | |
| ISIS 594230 (parent) | 25 mg/kg twice/day (100 mg/kg total) | 62.72 | 5'-*(E)*-vinylphosphonate-2'-MOE | 3 |
| ISIS 594231 | 6 | 72.19 | 5'-*(E)*-vinylphosphonate-2'-C16 at position 1 counting from 5' end | 3 |
| | 14 | 59.50 | | |
| | 36 | 69.15 | | |
| | 88 | 67.01 | | |
| ISIS 594232 (parent) | 25 mg/kg twice/day (100 mg/kg total) | 72.37 | 5'-*(E)*-vinylphosphonate-2'-MOE | 14 |
| ISIS 594290 | 6 | 84.15 | 5'-*(E)*-vinylphosphonate-2'-MOE with C16 conjugate at position 8 counting from 5' end | 14 |
| | 14 | 66.03 | | |
| | 36 | 71.27 | | |
| | 88 | 60.53 | | |

**Table 14**

| **Effect of ss-siRNA treatment on AST levels in transgenic mice** | | | | |
|---|---|---|---|---|
| ss-siRNA | Dose (mg/kg) | AST (IU/L) | Chemistry | SEQ ID No. |
| PBS | 0 | 95.02 | | |
| ISIS 594230 (parent) | 25 mg/kg twice/day (100 mg/kg total) | 72.47 | 5'-*(E)*-vinylphosphonate-2'-MOE | 3 |
| ISIS 594231 | 6 | 71.93 | 5'-*(E)*-vinylphosphonate-2'-C16 at position 1 counting from 5' end | 3 |
| | 14 | 66.03 | | |
| | 36 | 66.03 | | |
| | 88 | 69.66 | | |
| ISIS 594232 (parent) | 25 mg/kg twice/day (100 mg/kg total) | 84.15 | 5'-*(E)*-vinylphosphonate-2'-MOE | 14 |
| ISIS 594290 | 6 | 84.15 | 5'-*(E)*-vinylphosphonate-2'-MOE with C16 conjugate at position 8 counting from 5' end | 14 |
| | 14 | 66.03 | | |
| | 36 | 71.27 | | |
| | 88 | 80.53 | | |

### Pharmacokinetics Analysis (PK)

The PK of the ss-siRNAs was also evaluated. Liver samples were minced and extracted using standard protocols. Samples were analyzed on MSD1 utilizing IP-HPLC-MS. The tissue level (µg/g) of full-length ss-siRNAs was measured and the results are provided in Table 15. "N/A" indicates not applicable.

As illustrated, greater liver concentration was observed for C16-conjugated ss-siRNAs compared to unconjugated ss-siRNAs. The observed full-length ss-siRNAs identified for conjugated ss-siRNAs, ISIS 594231 and 594290 contained only the hexylamino linker. The lack of C16 conjugate was due to hydrolysis at the amide bond between the hexylamino linker and the conjugate.

**Table 15**

| **PK analysis of ss-siRNA treatment in male hApoC III transgenic mice** | | | | | |
|---|---|---|---|---|---|
| ss-siRNA | Dose (mg/kg) | Liver (µg/g) | Liver EC₅₀ (µg/g) | Chemistry | SEQ ID No. |
| PBS | 0 | 0 | N/A | | |
| ISIS 594230 (parent) | 25 mg/kg twice/day for two days (100 mg/kg total) | 235.95 | N/A | 5'-*(E)*-vinylphosphonate-2'-MOE | 3 |
| ISIS 594231 | 6 | 22.89 | 50 | 5'-*(E)*-vinylphosphonate-2'-C16 at position 1 counting from 5' end | 3 |
| | 14 | 74.09 | | | |
| | 36 | 153.00 | | | |
| | 88 | 400 | | | |
| ISIS 594232 (parent) | 25 mg/kg twice/day (100 mg/kg total) | 126.85 | N/A | 5'-*(E)*-vinylphosphonate-2'-MOE | 14 |
| ISIS 594290 | 6 | 27.40 | 150 | 5'-*(E)*-vinylphosphonate-2'-MOE with C16 conjugate at position 8 counting from 5' end | 14 |
| | 14 | 112.30 | | | |
| | 36 | 242.02 | | | |
| | 88 | 430.14 | | | |

### Example 11: General method for the preparation of ss-siRNAs comprising a GalNAc₃ conjugate

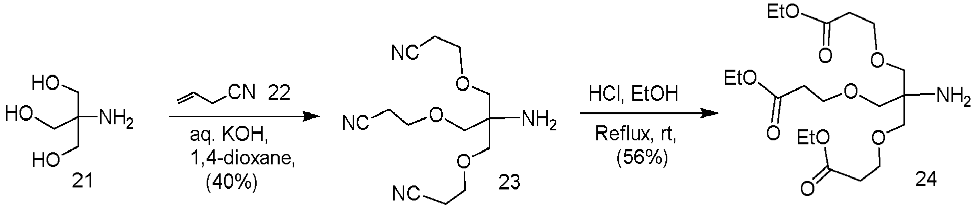

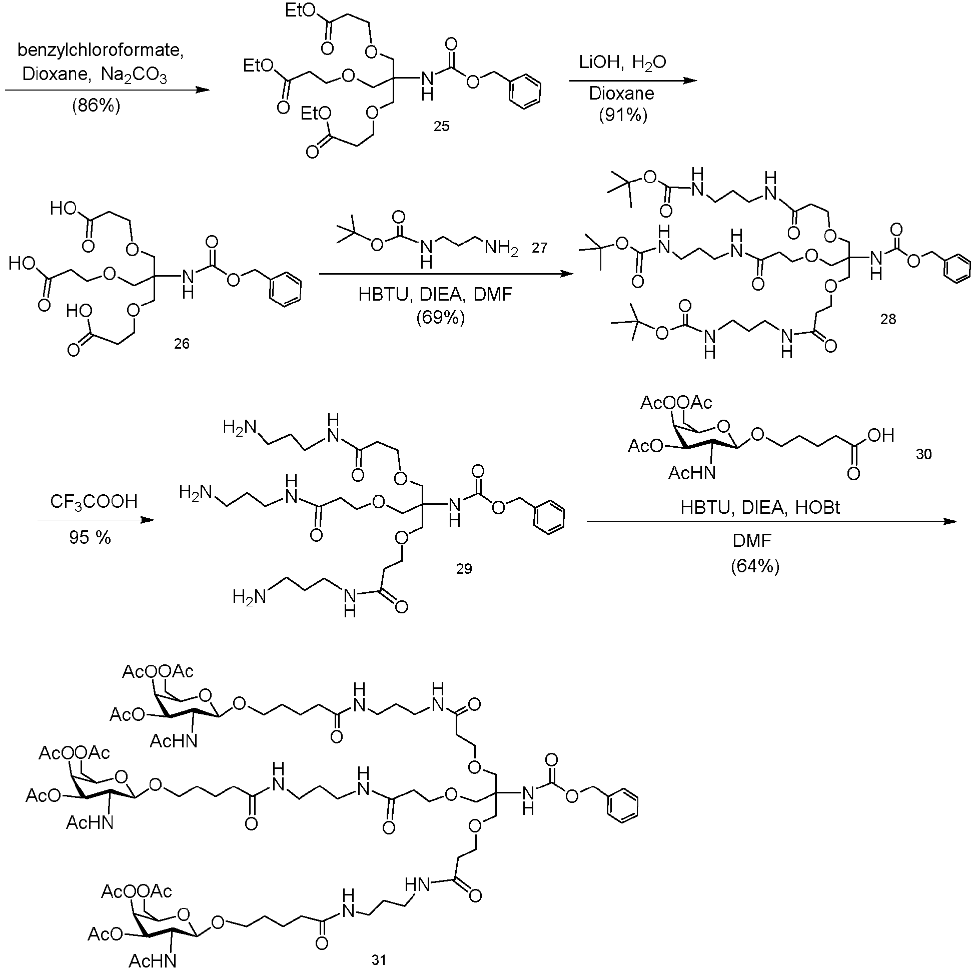

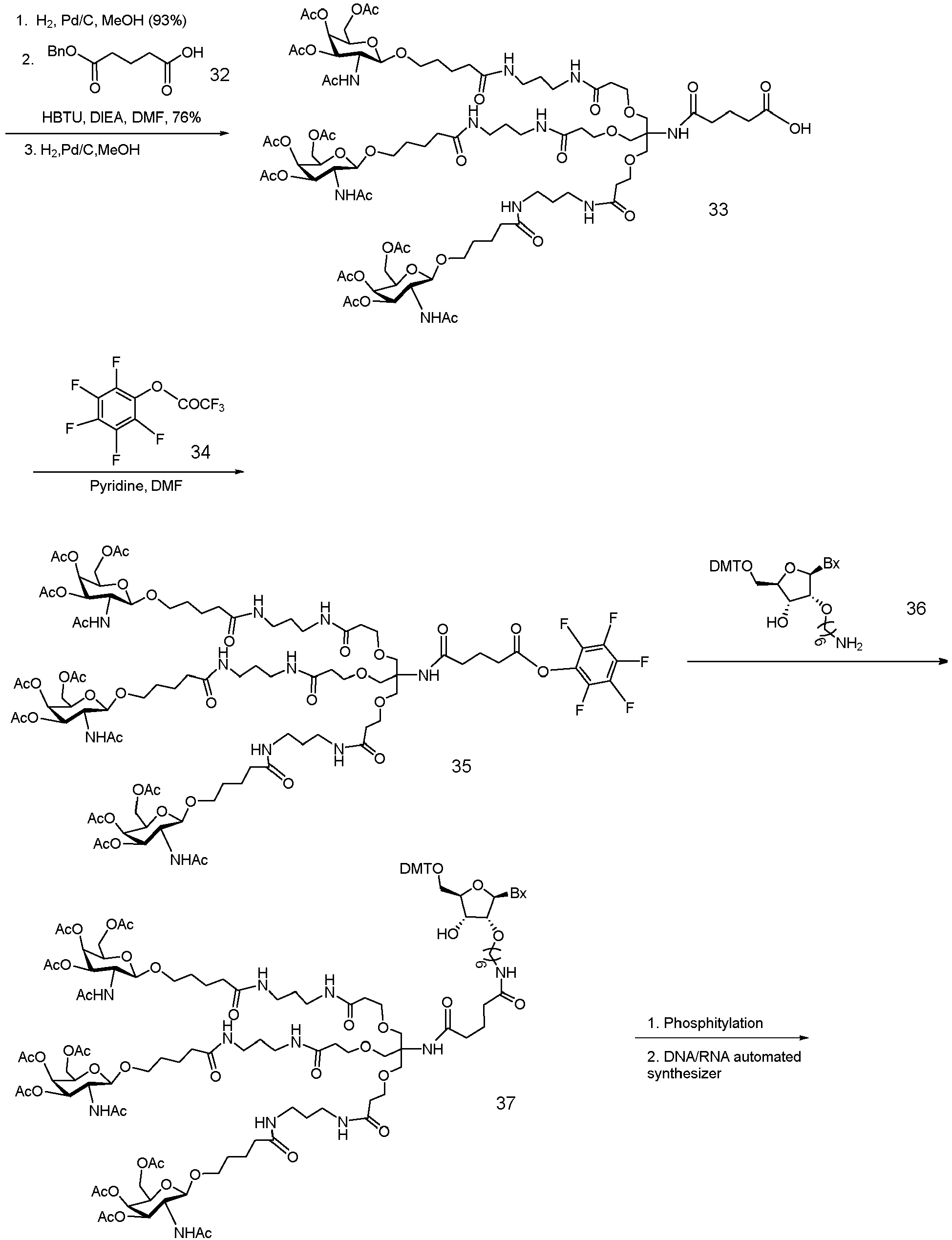

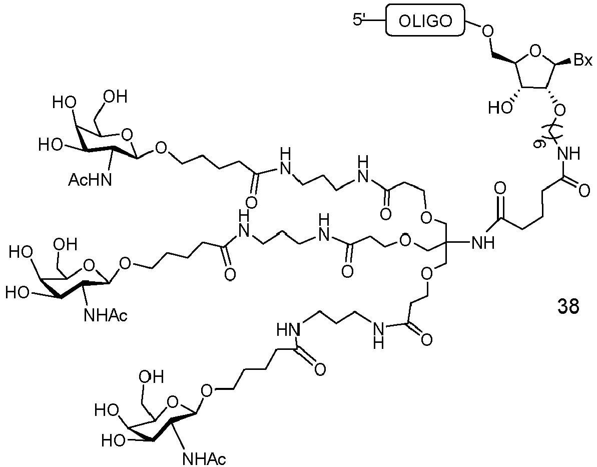

Compounds 21, 22, 27, 32, and 34 are commercially available. Compound 30 was prepared using similar procedures reported by Rensen et al., J. Med. Chem., 2004, 47, 5798-5808. Nucleotide 36 is prepared in a similar manner as compound 6. Oligonucleotide 38 can comprise a 5'-(E)-vinylphosphate by incorporating phosphoramidites such as compound 5 or compound 12 at the 5'-end of the oligonucleotide.

Using these methods, a GalNAc conjugated ss-siRNA targeting PTEN was prepared (see Table 16) for testing in mice. A similar ss-siRNA that does not comprise a GalNAc conjugate and a gapmer were also prepared as controls (see Table 16).

**Table 16**

| **Modified ss-siRNAs and gapmer targeting PTEN** | | |
|---|---|---|
| ISIS No. | Composition (5' to 3') | SEQ ID No. |
| 116847 | ^{m}CₑₛTₑₛGₑₛ^{m}CₑₛTₑₛA_{ds}G_{ds}^{m}C_{ds}^{m}C_{ds}T_{ds}^{m}C_{ds}T_{ds}G_{ds}G_{ds}A_{ds}TₑₛTₑₛTₑₛGₑₛAₑ | 135 |
| 522247 | Pv-TₑₛU_{fs}AₘₒU_{fs}CₘₒU_{fs}AₘₒU_{fs}AₘₒA_{fs}UₘₒG_{fs}AₘₒU_{fs}CₘₛA_{fs}GₘₛG_{fs}UₘₛAₑₛAₑ | 136 |
| 691564 | Pv-TₑₛU_{fs}AₘₒU_{fs}CₘₒU_{fs}AₘₒU_{fs}AₘₒA_{fs}UₘₒG_{fs}AₘₒU_{fs}CₘₛA_{fs}GₘₛG_{fs}UₘₛAₑₛAₑₒA_{do} T-GalNAc3 | 137 |

Subscripts: "s" between two nucleosides indicates a phosphorothioate internucleoside linkage; "o" between two nucleosides indicates a phosphodiester internucleoside linkage; "Pv" at the 5'-end indicates a 5'-*(E)*-vinylphosphonate group, (PO(OH)₂(CH=CH)-; "f" indicates a 2'-fluoro modified nucleoside; "m" indicates a 2'-O-methyl modified nucleoside; "e" indicates a 2'-O-methoxyethyl (MOE) modified nucleoside; and "GalNAc₃" indicates a 2'-O-(CH₂)₆-NH-GalNAc₃ conjugate group as described in Example 11. Superscript "m" indicates a 5-methyl nucleobase.

### Example 12: Effect of ss-siRNAs on inhibition of PTEN in vivo

The oligonucleotides described in Table 16 were tested and evaluated for PTEN inhibition in mice. Wild type mice were injected subcutaneously twice a day for two days with an oligonucleotide described in Table 16 or with saline as a control. Each treatment group consisted of 4 animals. Each dose of ISIS 116847 and 522247 was 25 mg/kg, for a total of 100 mg/kg. Each dose of ISIS 691564 was either 2.5 mg/kg, for a total of 10 mg/kg, or 7.5 mg/kg, for a total of 30 mg/kg. Forty-eight hours after the administration of the last dose, the mice were sacrificed and liver and kidney were collected.

PTEN mRNA levels in liver was determined using real-time PCR and RIBOGREEN® RNA quantification reagent (Molecular Probes, Inc. Eugene, OR) according to standard protocols. PTEN mRNA levels were determined relative to total RNA (using Ribogreen), prior to normalization to PBS-treated control. The results are presented in Table 17 as the average percent of PTEN mRNA levels for each treatment group, normalized to saline-treated control and are denoted as "% control". The results show that the GalNAc conjugated ss-siRNA (ISIS 691564) inhibited liver PTEN mRNA to nearly the same extent as the parent ss-siRNA (ISIS 522247) despite the fact that ISIS 691564 was administered at a 3-fold lower dose.

Liver transaminase levels, alanine aminotranferease (ALT) and aspartate aminotransferase (AST), in serum were measured relative to saline injected mice using standard protocols. Total bilirubin and organ weights were also evaluated. The average results for each treatment group are presented in Table 18 and show that no elevation in any of these markers was observed in mice treated with the ss-siRNAs compared to those treated with saline.

**Table 17**

| **PTEN mRNA levels** | | | |
|---|---|---|---|
| ISIS No. | Dose (mg/kg) | % control | SEQ ID No. |
| Saline | n/a | 100.0 | n/a |
| 116847 | 25 twice/day (100 total) | 21.6 | 135 |
| 522247 | 25 twice/day (100 total) | 60.1 | 136 |
| 691564 | 7.5 twice/day (30 total) | 71.1 | 137 |
| | 2.5 twice/day (10 total) | 100.3 | |

**Table 18**

| **Liver ALT, AST, and total bilirubin levels and organ weights** | | | | | | | | |
|---|---|---|---|---|---|---|---|---|
| ISIS No. | Total dose (mg/kg) | ALT (U/L) | AST (U/L) | T. Bil. (mg/dL) | Liver/Body weight | Kidney/Body weight | Spleen/Body weight | SEQ ID No. |
| Saline | n/a | 25 | 53 | 0.30 | 5.57 | 1.46 | 0.38 | n/a |
| 116847 | 100 | 35 | 73 | 0.25 | 6.60 | 1.42 | 0.44 | 135 |
| 522247 | 100 | 27 | 54 | 0.23 | 5.57 | 1.44 | 0.42 | 136 |
| 691564 | 30 | 23 | 75 | 0.23 | 5.80 | 1.58 | 0.40 | 137 |
| | 10 | 26 | 57 | 0.19 | 5.56 | 1.53 | 0.40 | |

### Example 13: Preparation of ss-siRNAs comprising a GalNAc₃ conjugate

A GalNAc conjugated ss-siRNA targeting Apo-CIII was prepared according to the procedures described in Example 11 above. A similar ss-siRNA that does not comprise a GalNAc conjugate and a gapmer were also prepared as controls (see Table 19).

**Table 19**

| **Modified ss-siRNAs and gapmer targeting APO-CIII** | | |
|---|---|---|
| Isis No. | Composition (5' to 3') | SEQ ID No. |
| 304801 | AₑₛGₑₛ^{m}CₑₛTₑₛTₑₛ^{m}C_{ds}T_{ds}T_{ds}G_{ds}T_{ds}^{m}C_{ds}^{m}C_{ds}A_{ds}G_{ds}^{m}C_{ds}TₑₛTₑₛTₑₛAₑTₑ | 138 |
| 594230 | Pv-*T*_{S}C_{fS}AₘₒC_{fS}TₘₒG_{fS}AₘₒG_{fS}AₘₒA_{fS}TₘₒA_{fS}CₘₒT_{fS}G_{mS}T_{fS}C_{mS}C_{fS}C_{mS}A_{eS}Aₑ | 139 |
| 722060 | Pv-TSCfSAmoCfSTmoGfSAmoGfSAmoAfSTmoAfSCmoTfSGmSTfSCmSCfSCmSAeSAe oAdoU-GalNac3 | 140 |

Subscripts: "s" between two nucleosides indicates a phosphorothioate internucleoside linkage; "o" between two nucleosides indicates a phosphodiester internucleoside linkage; "Pv" at the 5'-end indicates a 5'-*(E)*-vinylphosphonate group, (PO(OH)₂(CH=CH)-; "f" indicates a 2'-fluoro modified nucleoside; "m" indicates a 2'-O-methyl modified nucleoside; "e" indicates a 2'-O-methoxyethyl (MOE) modified nucleoside; and "GalNAc₃" indicates a 2'-O-(CH₂)₆-NH-GalNAc₃ conjugate group as described in Example 11. Superscript "m" indicates a 5-methyl nucleobase.

### Example 14: Inhibitory effect of ss-siRNAs on hApoC III expression in vitro

The modified ss-siRNAs and gapmer from Table 19, each targeting hApoC III, were evaluated in a dose-response study for their ability to inhibit hApoC III expression *in vitro.*

Primary hepatocyte cells from transgenic mice at a density of 15,000 cells per well were treated with concentrations of .0005, .002, .0078, .031, .125, .5, and 2 µM of modified ss-siRNA. After a treatment period of approximately 16 hours, RNA was isolated from the cells and mRNA levels were measured by quantitative real-time PCR. Human ApoC III mRNA levels were adjusted according to total RNA content, as measured by RIBOGREEN.

The half maximal inhibitory concentration (IC₅₀) of each ss-siRNA and the gapmer was measured by plotting the concentrations of ss-siRNAs used versus the percent inhibition of hApoC III expression achieved at each concentration, and noting the concentration of ss-siRNA at which 50% inhibition of hApoC III mRNA expression was achieved compared to the control. The (IC₅₀) of each ss-siRNA and the gapmer are shown in the table below.

**Table 20**

| **Modified ss-siRNAs and gapmer targeting APO-CIII** | |
|---|---|
| Isis # | IC50 (nM) |
| 304801 | 150 |
| 594230 | 70 |
| 722060 | 6 |

### Example 15: Effect of ss-siRNAs on inhibition of Apo-CIII in vivo

The oligonucleotides described in Table 19 were tested and evaluated for Apo-CIII inhibition in mice. Transgenic mice were injected subcutaneously with an oligonucleotide described in Table 19 or with saline as a control. Each treatment group consisted of 4 animals. Each treatment group of animals dosed with ISIS 304801 received a single dose of either 3, 10, or 30 mg/kg. Each treatment group of animals dosed with ISIS 594230 received doses as follows: (1) Dose of 10 mg/kg administered as a single dose of 10 mg/kg; (2) Dose of 25 mg/kg administered as a single dose of 25 mg/kg; (3) Dose of 100 mg/kg administered as a series of doses of 25 mg/kg given twice a day for two days (for a total of 100 mg/kg); (4) Dose of 300 mg/kg administered as a series of doses of 25 mg/kg given twice a day for six days (for a total of 300 mg/kg). Each treatment group of animals dosed with ISIS 722060 received a single dose of either 1, 3, 10, 30, or 90 mg/kg.

Seventy-two hours after the administration of the last dose, the mice were sacrificed and tissue was collected for analysis. Apo-CIII mRNA levels in liver were determined using real-time PCR and according to standard protocols and Apo-CIII mRNA levels were determined relative to total RNA (using Cyclophilin), prior to normalization to PBS-treated control. The results are presented in Table 21 as the average percent of Apo-CIII mRNA levels for each treatment group, normalized to saline-treated control and are denoted as "% control".

**Table 21**

| **Apo-CIII mRNA levels** | | | |
|---|---|---|---|
| ISIS No. | Dose (mg/kg) | % control | SEQ ID No. |
| Saline | n/a | 100.0 | n/a |
| 304801 | 3 | 76.5 | 138 |
| 304801 | 10 | 63.8 | 138 |
| 304801 | 30 | 26.4 | 138 |
| 594230 | 10 | 69.1 | 139 |
| 594230 | 25 | 31.1 | 139 |
| 594230 | 100 | 15.6 | 139 |
| 594230 | 300 | 8.2 | 139 |
| 722060 | 1 | 125.4 | 140 |
| 722060 | 3 | 99.4 | 140 |
| 722060 | 10 | 48.1 | 140 |
| 722060 | 30 | 34.6 | 140 |
| 722060 | 90 | 43.1 | 140 |

### NUMBERED EMBODIMENTS:

1. A compound comprising a single-stranded oligonucleotide consisting of 13 to 30 linked nucleosides and having a nucleobase sequence comprising at least 8 contiguous nucleobases complementary to an equal-length portion within a target region of a target nucleic acid, wherein the 5'-terminal nucleoside of the single-stranded oligonucleotide comprises a stabilized phosphate moiety and an internucleoside linking group linking the 5'-terminal nucleoside to the remainder of the oligonucleotide.
2. The compound of embodiment 1, wherein the target nucleic acid is Apolipoprotein C-III transcript.
3. The compound of embodiment 1 or 2, wherein the compound comprises a conjugate group.
4. The compound of embodiment 3, wherein the conjugate group is covalently attached to the oligonucleotide.
5. The compound of any of embodiments 1 to 4, wherein the conjugate group is attached to the oligonucleotide at a nucleoside at position 1, 2, 3, 4, 6, 7, 8, 9, 18, 19, 20, or 21 from the 5'-end of the oligonucleotide or at position 1, 2, 3, 12, 13, 14, 15, 17, 18, 19, 20, or 21 from the 3'-end of the oligonucleotide.
6. The compound of any of embodiments 1 to 5, wherein the conjugate group is attached to the oligonucleotide at a nucleoside at position 1 from the 5'-end of the oligonucleotide.
7. The compound of any of embodiments 1 to 6, wherein the conjugate group is attached to the oligonucleotide at a nucleoside at position 8 from the 5'-end of the oligonucleotide.
8. The compound of any of embodiments 1 to 7, wherein the Apolipoprotein C-III transcript comprises the nucleobase sequence as set forth in SEQ ID NO: 1.
9. The compound of any of embodiments 1 to 8, wherein the Apolipoprotein C-III transcript comprises the nucleobase sequence as set forth in SEQ ID NO: 2.
10. The compound of any of embodiments 1 to 9, wherein the complementary region comprises at least 10 contiguous nucleobases complementary to an equal-length portion within a target region of an Apolipoprotein C-III transcript.
11. The compound of any of embodiments 1 to 9, wherein the complementary region comprises at least 12 contiguous nucleobases complementary to an equal-length portion within a target region of an Apolipoprotein C-III transcript.
12. The compound of any of embodiments 1 to 9, wherein the complementary region comprises at least 14 contiguous nucleobases complementary to an equal-length portion within a target region of an Apolipoprotein C-III transcript.
13. The compound of any of embodiments 1 to 9, wherein the complementary region comprises at least 16 contiguous nucleobases complementary to an equal-length portion within a target region of an Apolipoprotein C-III transcript.
14. The compound of any of embodiments 1 to 9, wherein the complementary region comprises at least 18 contiguous nucleobases complementary to an equal-length portion within a target region of an Apolipoprotein C-III transcript.
15. The compound of any of embodiments 1 to 14, wherein the 5'-terminal nucleoside of the single-stranded oligonucleotide has Formula I: 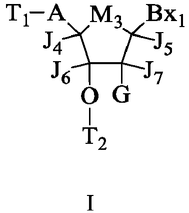 wherein:
   T₁ is a phosphorus moiety;
   T₂ is an internucleoside linking group linking the 5'-terminal nucleoside of Formula I to the remainder of the oligonucleotide;
   A has a formula selected from among: 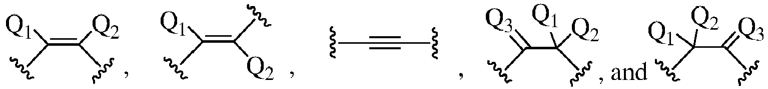
   Q₁ and Q₂ are each independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, substituted C₂-C₆ alkynyl, and N(R₃)(R₄);
   Q₃ is selected from among: O, S, N(Rs), and C(R₆)(R₇);
   each R₃, R₄ R₅, R₆ and R₇ is independently selected from among: H, C₁-C₆ alkyl, substituted C₁-C₆ alkyl, and C₁-C₆ alkoxy;
   M₃ is selected from among: O, S, NR₁₄, C(R₁₅)(R₁₆), C(R₁₅)(R₁₆)C(R₁₇)(R₁₈), C(R₁₅)=C(R₁₇), OC(R₁₅)(R₁₆), and OC(R₁₅)(Bx₂);
   R₁₄ is selected from among: H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   R₁₅, R₁₆, R₁₇ and R₁₈ are each independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   if Bx₂ is present, then Bx₂ is a nucleobase and Bx₁ is selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   if Bx₂ is not present, then Bx₁ is a nucleobase;
   either each of J₄, J₅, J₆ and J₇ is independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   or J₄ forms a bridge with one of J₅ or J₇ wherein the bridge comprises from 1 to 3 linked biradical groups selected from O, S, NR₁₉, C(R₂₀)(R₂₁), C(R₂₀)=C(R₂₁), C[=C(R₂₀)(R₂₁)] and C(=O) and the other two of J₅, J₆ and J₇ are independently selected from among: H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl, and substituted C₂-C₆ alkynyl;
   each R₁₉, R₂₀ and R₂₁ is independently selected from among: H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
   G is selected from among: H, OH, halogen, O-[C(R₈)(R₉)]ₙ-[(C=O)ₘ-X₁]ⱼ-Z, and a conjugate group;
   each R₈ and R₉ is independently selected from among: H, halogen, C₁-C₆ alkyl, and substituted C₁-C₆ alkyl;
   X₁ is O, S or N(E₁);
   Z is selected from among: H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl, and N(E₂)(E₃);
   E₁, E₂ and E₃ are each independently selected from among: H, C₁-C₆ alkyl, and substituted C₁-C₆ alkyl;
   n is from 1 to 6;
   m is 0 or 1;
   j is 0 or 1;
   provided that, if j is 1, then Z is other than halogen or N(E₂)(E₃);
   each substituted group comprises one or more optionally protected substituent groups independently selected from among: a halogen, OJ₁, N(J₁)(J₂), =NJ₁, SJ₁, N₃, CN, OC(=X₂)J₁, OC(=X₂)N(J₁)(J₂), and C(=X₂)N(J₁)(J₂);
   X₂ is O, S or NJ₃; and
   each J₁, J₂ and J₃ is independently selected from among: H and C₁-C₆ alkyl.
16. The compound of embodiment 15, wherein M₃ is selected from among: O, CH=CH, OCH₂, and OC(H)(Bx₂).
17. The compound of embodiment 15, wherein M₃ is O.
18. The compound of any of embodiments 15-17, wherein each of J₄, J₅, J₆ and J₇ is H.
19. The compound of any of embodiments 15-18, wherein J₄ forms a bridge with either J₅ or J₇.
20. The compound of any of embodiments 15-19, wherein A has the formula: 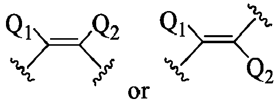 wherein:
   Q₁ and Q₂ are each independently selected from among: H, halogen, C₁-C₆ alkyl, substituted C₁-C₆ alkyl, C₁-C₆ alkoxy, and substituted C₁-C₆ alkoxy.
21. The compound of embodiment 20, wherein each of Q₁ and Q₂ is H.
22. The compound of embodiment 20, wherein Q₁ and Q₂ are each independently selected from among: H and a halogen.
23. The compound of embodiment 20, wherein one of Q₁ and Q₂ is H and the other of Q₁ and Q₂ is F, CH₃ or OCH₃.
24. The compound of any of embodiments 15 to 23, wherein T₁ has the formula: 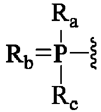 wherein:
   Rₐ and R_{c} are each independently selected from among: protected hydroxyl, protected thiol, C₁-C₆ alkyl, substituted C₁-C₆ alkyl, C₁-C₆ alkoxy, substituted C₁-C₆ alkoxy, protected amino or substituted amino; and
   R_{b} is O or S.
25. The compound of embodiment 24, wherein R_{b} is O and Rₐ and R_{c} are each, independently selected from among: OCH₃, OCH₂CH₃, OCH(CH₃)₂.
26. The compound of any of embodiments 15 to 25, wherein G is selected from among: a halogen, OCH₃, OCH₂F, OCHF₂, OCF₃, OCH₂CH₃, O(CH₂)₂F, OCH₂CHF₂, OCH₂CF₃, OCH₂-CH=CH₂, O(CH₂)₂-OCH₃, O(CH₂)₂-SCH₃, O(CH₂)₂-OCF₃, O(CH₂)₃-N(R₁₀)(R₁₁), O(CH₂)₂-ON(R₁₀)(R₁₁), O(CH₂)₂-O(CH₂)₂-N(R₁₀)(R₁₁), OCH₂C(=O)-N(R₁₀)(R₁₁), OCH₂C(=O)-N(R₁₂)-(CH₂)₂-N(R₁₀)(R₁₁), and O(CH₂)₂-N(R₁₂)-C(=NR₁₃)[N(R₁₀)(R₁₁)]; wherein R₁₀, R₁₁, R₁₂ and R₁₃ are each, independently, H or C₁-C₆ alkyl.
27. The compound of any of embodiments 15-26, wherein G is selected from among: a halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂, and OCH₂-N(H)-C(=NH)NH₂.
28. The compound of any of embodiments 15-27, wherein G is selected from among: F, OCH₃, and O(CH₂)₂-OCH₃.
29. The compound of embodiment 28, wherein G is O(CH₂)₂-OCH₃.
30. The compound of any of embodiments 15-25, wherein G is a conjugate group.
31. The compound of embodiment 30, wherein the conjugate of the conjugate group is selected from among: cholesterol, palmityl, stearoyl, lithocholic-oleyl, C₂₂ alkyl, C₂₀ alkyl, C₁₆ alkyl, C₁₈ alkyl, and C₁₀ alkyl.
32. The compound of embodiment 31, wherein the conjugate group comprises C₁₆ alkyl.
33. The compound of any of embodiments 30 to 32, wherein the conjugate group comprises a linker.
34. The compound of embodiment 33, wherein the linker is selected from among: hexanamide, 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C₁-C₁₀ alkyl, substituted or unsubstituted C₂-C₁₀ alkenyl, and substituted or unsubstituted C₂-C₁₀ alkynyl.
35. The compound of any of embodiments 15-34, wherein the nucleobase is a modified nucleobase.
36. The compound of any of embodiments 15-35, wherein the nucleobase is a pyrimidine, substituted pyrimidine, purine or substituted purine.
37. The compound of any of embodiments 15-36, wherein the nucleobase is uracil, thymine, cytosine, 5-methylcytosine, adenine or guanine.
38. The compound of any of embodiments 15-37, wherein the 5'-terminal nucleoside of the single-stranded oligonucleotide has Formula III: 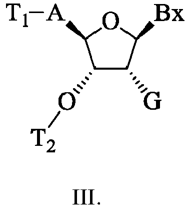
39. The compound of embodiment 38, wherein A has the formula: 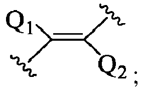 wherein Q₁ and Q₂ are each independently selected from among: H, a halogen, C₁-C₆ alkyl, substituted C₁-C₆ alkyl, C₁-C₆ alkoxy, and substituted C₁-C₆ alkoxy.
40. The compound of embodiment 39, wherein Q₁ and Q₂ are each independently selected from among: H, F, CH₃, and OCH₃.
41. The compound of any of embodiments 15-40, wherein the 5'-terminal nucleoside has Formula V: 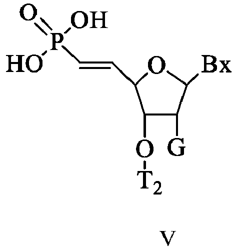 wherein:
   Bx is selected from among: uracil, thymine, cytosine, 5-methyl cytosine, adenine, and guanine;
   T₂ is a phosphorothioate internucleoside linking group linking the compound of Formula V to the remainder of the oligonucleotide; and
   G is selected from among: a halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂, OCH₂-N(H)-C(=NH)NH₂, and a conjugate group.
42. The compound of any of embodiments 1-41, wherein the remainder of the oligonucleotide comprises at least one RNA-like nucleoside.
43. The compound of embodiment 42, wherein essentially each nucleoside of the remainder of the oligonucleotide is an RNA-like nucleoside.
44. The compound of embodiment 43, wherein each nucleoside of the remainder of the oligonucleotide is an RNA-like nucleoside.
45. The compound of any of embodiments 42-44, wherein each RNA-like nucleoside is independently selected from among: a 2'-endo furanosyl nucleoside and an RNA-surrogate nucleoside.
46. The compound of embodiment 45, wherein each RNA-like nucleoside is a 2'-endo furanosyl nucleoside.
47. The compound of embodiment 46, wherein each RNA-like nucleoside is selected from among: 2'-F, 2'-MOE, 2'-OMe, LNA, F-HNA, and cEt.
48. The compound of any of embodiments 1-47, wherein the remainder of the oligonucleotide comprises at least one region having sugar motif:

   -[(A)ₓ-(B)_{y}-(A)_{z}]_{q}-

   wherein
   A is a modified nucleoside of a first type,
   B is a modified nucleoside of a second type;
   each x and each y is independently 1 or 2;
   z is 0 or 1;
   q is 1-15.
49. The compound of any of embodiments 1-47, wherein the remainder of the oligonucleotide comprises at least one region having sugar motif:

   -[(A)ₓ-(B)_{y}-(A)_{z}]_{q}-

   wherein
   A is a modified nucleoside of a first type,
   B is a modified nucleoside of a second type;
   each x and each y is independently 1 or 2;
   z is 0 or 1;
   q is 1-15.
50. The compound of any of embodiments 1-49, wherein the remainder of the oligonucleotide comprises at least one region having sugar motif:

   -[(A)ₓ-(A)_{y}-(A)_{z}]_{q}-

   wherein
   A is a modified nucleoside;
   each x and each y is independently 1 or 2;
   z is 0 or 1;
   q is 1-15.
51. The compound of any of embodiments 1-49, wherein the remainder of the oligonucleotide comprises at least one region having sugar motif:

   -[(B)ₓ-(B)_{y}-(B)_{z}]_{q}-

   wherein
   B is a modified nucleoside,
   each x and each y is independently 1 or 2;
   z is 0 or 1;
   q is 1-15.
52. The compound of embodiment 50 or 51, wherein A is a modified nucleoside selected from among: 2'-F, 2'-OMe, and F-HNA.
53. The compound of embodiment 50 or 51, wherein B is a modified nucleoside selected from among: 2'-F, 2'-OMe, and F-HNA.
54. The compound of any of embodiments 48 to 53, wherein the modifications of the first type and the modifications of the second type are selected from among: 2'-F, 2'-OMe, and F-HNA.
55. The compound of any of embodiments 48 to 53, wherein the modifications of the first type are 2'-F and the modifications of the second type are 2'-OMe.
56. The compound of any of embodiments 48 to 53, wherein the modifications of the first type are 2'-OMe and the modifications of the second type are 2'-F.
57. The compound of any of embodiments 48 to 56, wherein each x and each y is 1.
58. The compound of any of embodiments 1-57, wherein the remainder of the oligonucleotide comprises 1-4 3 'terminal nucleosides, each comprising the same sugar modification, wherein the sugar modification of the 1-4 3'terminal nucleosides is different from the sugar modification of the immediately adjacent nucleoside.
59. The compound of embodiment 58, wherein the 3'-terminal nucleosides are each 2'-MOE nucleosides.
60. The compound of embodiment 58 or 59 comprising two 3'-terminal nucleosides.
61. The compound of any of embodiments 1-60, comprising at least one modified internucleoside linkage.
62. The compound of embodiment 61, wherein each internucleoside linkage is selected from phosphorothioate and phosphodiester.
63. The compound of embodiment 61 or 62, wherein each of the 6-10 3'-most internucleoside linkages is phosphorothioate linkage.
64. The compound of any of embodiments 61 to 63, wherein the 5'-most internucleoside linkage is a phosphorothioate linkage.
65. The compound of any of embodiments 61 to 64, comprising a region of alternating linkages.
66. The compound of any of embodiments 1-65, comprising a 5'region having the motif:

   (Nucleoside of Formula I, III, or V) -s-(A-s-B-o-A)ₓ(-s-B)_{Y}

   wherein:
   A is a nucleoside of a first type;
   B is a nucleoside of a second type;
   s is a phosphorothioate linkage;
   o is a phosphodiester linkage;
   X is 1-8; and
   Y is 1 or 0.
67. The compound of any of embodiments 1-66, comprising a 3'region having the motif:

   -(A-s-B-s-A)_{z}(-s-B)_{q}-s-(D)-(s-D)ᵣ

   wherein:
   s is a phosphorothioate linkage;
   A is a nucleoside of a first type;
   B is a nucleoside of a second type;
   D is a nucleoside of a third type;
   Z is 1-5;
   q is 1 or 0; and
   and r is 0-3.
68. The compound embodiment 66 or 67, wherein A is a 2'-F nucleoside.
69. The compound of any of embodiments 66 to 68, wherein B is a 2'-OMe nucleoside.
70. The compound of any of embodiments 67 to 69, wherein D is a 2'-MOE nucleoside.
71. The compound of any of embodiments 67to 70, wherein the oligonucleotide comprises a hybridizing region and a 3'-terminal region, wherein the hybridizing region comprisies nucleosides A and B and the terminal region comprising nucleosides D, wherein the hybridizing region is complementary to a target region of an Apoliprotein CIII transcript.
72. The compound of any of embodiments 1-65, comprising the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s

   wherein:
   s is a phosphorothioate linkage;
   A is a nucleoside of a first type;
   B is a nucleoside of a second type; and
   D is a nucleoside of a third type.
73. The compound of any of embodiments 1-65, consisting of the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s

   wherein:
   s is a phosphorothioate linkage;
   A is a nucleoside of a first type;
   B is a nucleoside of a second type; and
   D is a nucleoside of a third type.
74. The compound of any of embodiments 1-65, comprising the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s-X

   wherein:
   s is a phosphorothioate linkage;
   A is a nucleoside of a first type;
   B is a nucleoside of a second type;
   D is a nucleoside of a third type; and
   X is a conjugate group.
75. The compound of any of embodiments 1-65, consisting of the motif:

   (Nucleoside of Formula V) -s-A-s-B-o-A-s-B-o-A-s-B-o-A-s-B-o-A-s-B-o-A-s-B-o-A-s-B-s-A-s-B-s-A-s-B-s-D-s-D-s-X

   wherein:
   s is a phosphorothioate linkage;
   A is a nucleoside of a first type;
   B is a nucleoside of a second type;
   D is a nucleoside of a third type; and
   X is a conjugate group.
76. The compound of any of embodiments 72 to 75, wherein A is a 2'-F nucleoside.
77. The compound of any of embodiments 72 to 76, wherein B is a 2'-OMe nucleoside.
78. The compound of any of embodiments 72 to 77, wherein D is a 2'-MOE nucleoside.
79. The compound of any of embodiments 1-75, wherein the oligonucleotide has two mismatches relative to a target region of the Apolipoprotein C-III transcript.
80. The compound of any of embodiments 1-79, wherein the oligonucleotide has three mismatches relative to a target region of the Apolipoprotein C-III transcript.
81. The compound of any of embodiments 1-79, wherein the oligonucleotide has four mismatches relative to a target region of the Apolipoprotein C-III transcript.
82. The compound of any of embodiments 1-81, wherein the oligonucleotide comprises a hybridizing region and 0-4 3'-terminal nucleosides.
83. The compound of any of embodiments 1-81, wherein the oligonucleotide comprises a hybridizing region and 1-4 3'-terminal nucleosides.
84. The compound of embodiment 82 or 83, wherein the hybridizing region is 100% complementary to a target region of the Apolipoprotein C-III transcript.
85. The compound of embodiment 82 or 83, wherein the hybridizing region has one mismatch relative to a target region of the Apolipoprotein C-III transcript.
86. The compound of embodiment 82 or 83, wherein the hybridizing region has two mismatches relative a target region of the Apolipoprotein C-III transcript.
87. The compound of embodiment 82 or 83, wherein the hybridizing region has three mismatches relative to a target region of the Apolipoprotein C-III transcript.
88. The compound of embodiment 82 or 83, wherein the hybridizing region has four mismatches relative to a target region of the Apolipoprotein C-III transcript.
89. The compound of any of embodiments 79 to 88, wherein one or more of the 3'-terminal nucleosides is not complementary to the target RNA.
90. The compound of any of embodiments 79 to 89, wherein the nucleobase of each 3'-terminal nucleoside is a purine.
91. The compound of embodiment 90, wherein the nucleobase of each 3'-terminal nucleoside is an adenine.
92. The compound of any of embodiments 1-91, wherein the oligonucleotide comprises at least one modified nucleobase.
93. The compound of any of embodiments 1-92, wherein each cytosine residue comprises a 5-methylcytosine.
94. The compound of any of embodiments 1-92, wherein the nucleobase sequence of the oligonucleotide comprises a nucleobase sequence selected from among: SEQ ID NO: 3, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, or 86.
95. The compound of any of embodiments 1-92, wherein the nucleobase sequence of the oligonucleotide comprises a nucleobase sequence selected from among: SEQ ID NO:87, 88, 89, 90, 91, 92, 93, 94, 95, 96, 97, 98, 99, 100, 101, 102, 103, 104, 105, 106, 107, 108, 109, or 110.
96. The compound of any of embodiments 1-92, wherein the nucleobase sequence of the oligonucleotide comprises the nucleobase sequence of SEQ ID NO: 3.
97. The compound of any of embodiments 1-92, wherein the nucleobase sequence of the oligonucleotide comprises the nucleobase sequence of SEQ ID NO: 14.
98. The compound of any of embodiments 1-92, wherein the nucleobase sequence of the oligonucleotide comprises the nucleobase sequence of SEQ ID NO: 140.
99. The compound of any of embodiments 1-93, wherein the nucleobase sequence of the oligonucleotide consists of a nucleobase sequence selected from among: SEQ ID NO: 3, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, or 86.
100. The compound of any of embodiments 1-93, wherein the nucleobase sequence of the oligonucleotide consists of a nucleobase sequence selected from among: SEQ ID NO:87, 88, 89, 90, 91, 92, 93, 94, 95, 96, 97, 98, 99, 100, 101, 102, 103, 104, 105, 106, 107, 108, 109, or 110.
101. The compound of any of embodiments 1-93, wherein the nucleobase sequence of the oligonucleotide consists of the nucleobase sequence of SEQ ID NO: 3.
102. The compound of any of embodiments 1-93, wherein the nucleobase sequence of the oligonucleotide consists of the nucleobase sequence of SEQ ID NO: 14.
103. The compound of any of embodiments 1-93, wherein the nucleobase sequence of the oligonucleotide consists of the nucleobase sequence of SEQ ID NO: 140.
104. The compound of embodiment 1, wherein the compound comprises ISIS No. 594290.
105. The compound of embodiment 1, wherein the compound comprises ISIS No. 594231.
106. The compound of embodiment 1, wherein the compound comprises ISIS No. 722060.
107. The compound of embodiment 1, wherein the single-stranded oligonucleotide is ISIS No. 594290.
108. The compound of embodiment 1, wherein the single-stranded oligonucleotide is ISIS No. 594231.
109. The compound of embodiment 1, wherein the single-stranded oligonucleotide is ISIS No. 722060.
110. The compound of any of embodiments 1-109, wherein the oligonucleotide comprises at least one conjugate group.
111. A double-stranded compound comprising a sense strand and an antisense strand, wherein the antisense strand comprises at least 12 contiguous nucleobases of a nucleobase sequence selected from SEQ ID NO: 3, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110; and at least one conjugate group.
112. The double-stranded compound of embodiment 111, wherein the sense strand comprises at least 12 contiguous nucleobases of a nucleobase sequence selected from SEQ ID NO: 111, 112, 113, 114, 115, 116, 117, 118, 119, 120, 121, 122, 123, 124, 125, 126, 127, 128, 129, 130, 131, 132, or 133.
113. The double-stranded compound of embodiment 112, wherein the conjugate group is covalently attached to the 5'-end of the sense strand.
114. The double-stranded compound of embodiment 113, wherein the conjugate group is covalently attached to the 3'-end of the sense strand.
115. The compound of any of embodiments 3 to 114, wherein the conjugate group comprises: 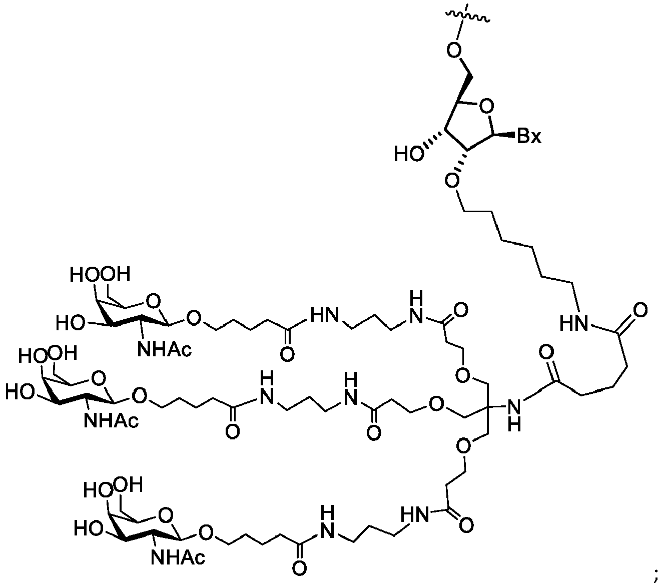 Wherein Bx is a naturally occurring nucleobase.
116. The compound of any of embodiments 3 to 114, wherein the conjugate group comprises: 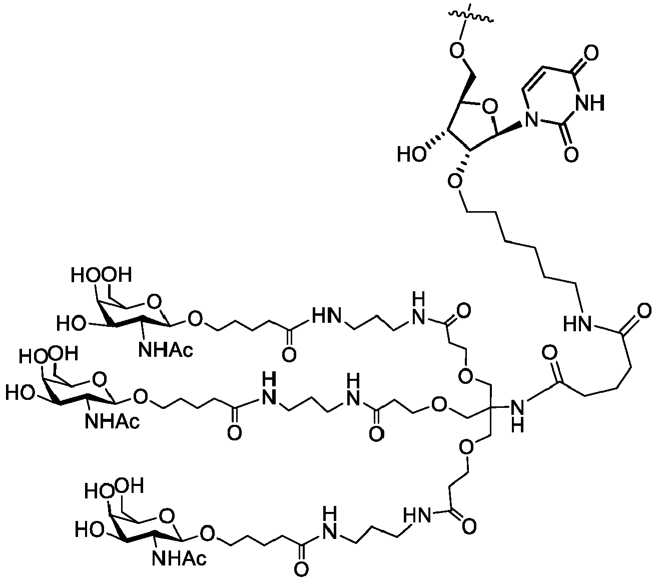
117. The compound of any of embodiments 3 to 114, wherein the conjugate group comprises: 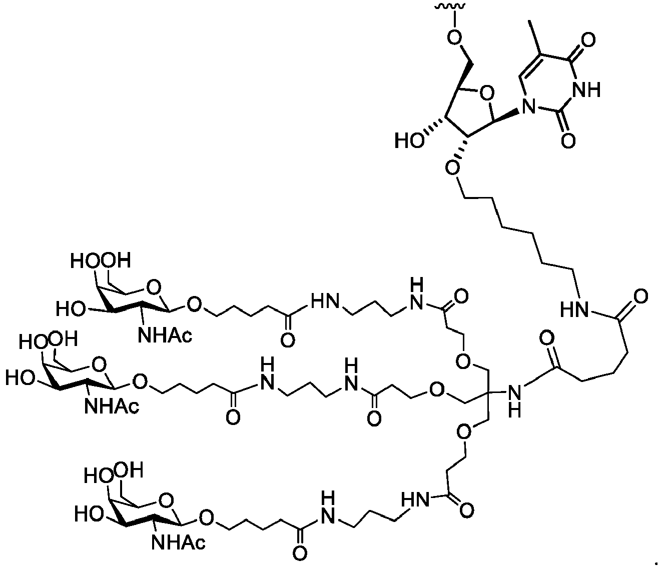
118. The compound of any of embodiments 3 to 114, wherein the conjugate group comprises a cell-targeting moiety having the following structure: 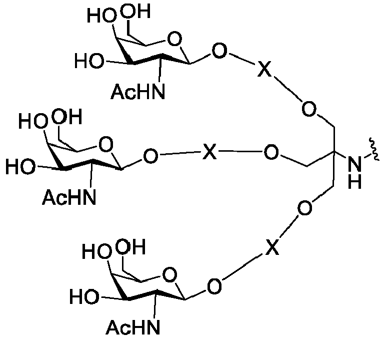 wherein X is a substituted or unsubstituted tether of six to eleven consecutively bonded atoms.
119. The compound of any of embodiments 3 to 114, wherein the conjugate group comprises a cell-targeting moiety having the following structure: 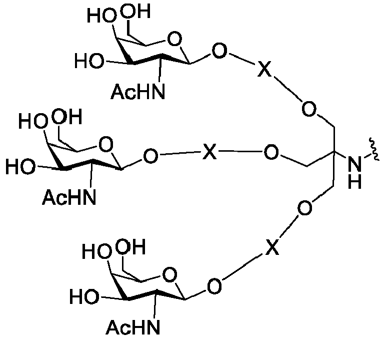 wherein X is a substituted or unsubstituted tether of ten consecutively bonded atoms.
120. The compound of any of embodiments 3 to 114, wherein the conjugate group comprises a cell-targeting moiety having the following structure: 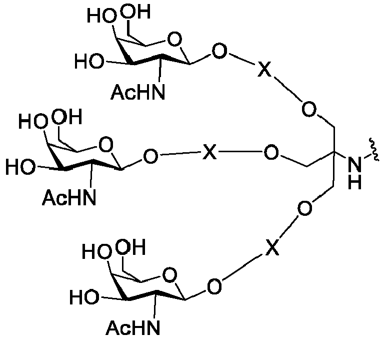 wherein X is a substituted or unsubstituted tether of four to eleven consecutively bonded atoms and wherein the tether comprises exactly one amide bond.
121. The compound of any of embodiments 3 to 114, wherein the conjugate group comprises a cell-targeting moiety having the following structure: 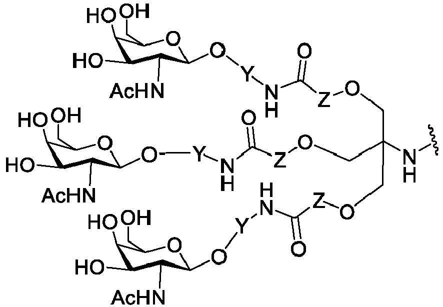 wherein Y and Z are independently selected from a C₁-C₁₂ substituted or unsubstituted alkyl, alkenyl, or alkynyl group, or a group comprising an ether, a ketone, an amide, an ester, a carbamate, an amine, a piperidine, a phosphate, a phosphodiester, a phosphorothioate, a triazole, a pyrrolidine, a disulfide, or a thioether.
122. The compound of any of embodiments 3 to 114, wherein the conjugate group comprises a cell-targeting moiety having the following structure: 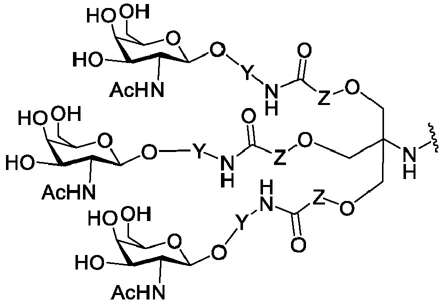 wherein Y and Z are independently selected from a C₁-C₁₂ substituted or unsubstituted alkyl group, or a group comprising exactly one ether or exactly two ethers, an amide, an amine, a piperidine, a phosphate, a phosphodiester, or a phosphorothioate.
123. The compound of any of embodiments 3 to 114, wherein the conjugate group comprises a cell-targeting moiety having the following structure: 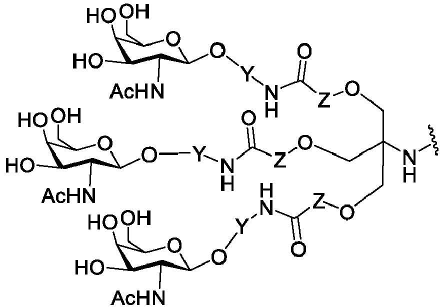 wherein Y and Z are independently selected from a C₁-C₁₂ substituted or unsubstituted alkyl group.
124. The compound of any of embodiments 3 to 114, wherein the conjugate group comprises a cell-targeting moiety having the following structure: 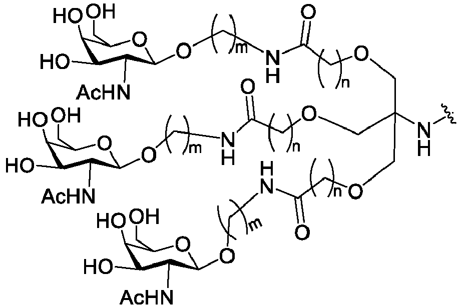 wherein m and n are independently selected from 1, 2, 3, 4, 5, 6, 7, 8, 9, 10, 11, and 12.
125. The compound of any of embodiments 3 to 114, wherein the conjugate group comprises a cell-targeting moiety having the following structure: 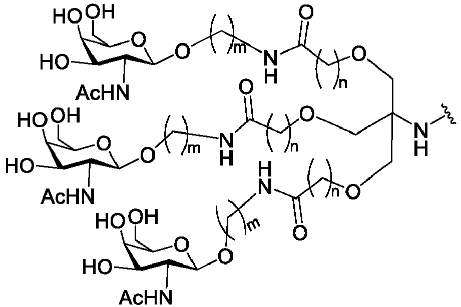 wherein m is 4, 5, 6, 7, or 8, and n is 1, 2, 3, or 4.
126. The compound of any of embodiments 3 to 114, wherein the conjugate group comprises a cell-targeting moiety having the following structure: 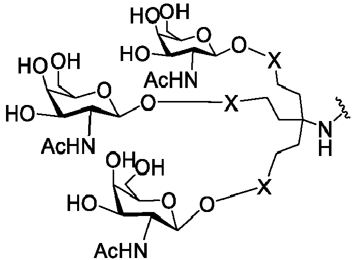 wherein X is a substituted or unsubstituted tether of four to thirteen consecutively bonded atoms, and wherein X does not comprise an ether group.
127. The compound of any of embodiments 3 to 114, wherein the conjugate group comprises a cell-targeting moiety having the following structure: 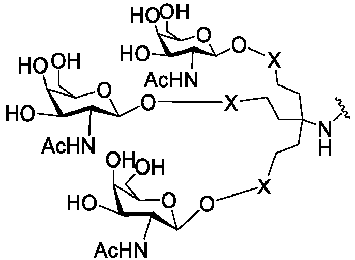 wherein X is a substituted or unsubstituted tether of eight consecutively bonded atoms, and wherein X does not comprise an ether group.
128. The compound of any of embodiments 3 to 114, wherein the conjugate group comprises a cell-targeting moiety having the following structure: 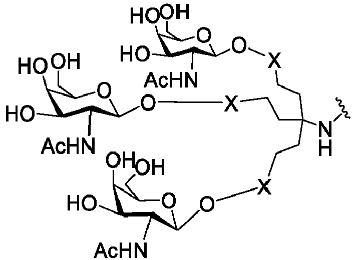 wherein X is a substituted or unsubstituted tether of four to thirteen consecutively bonded atoms, and wherein the tether comprises exactly one amide bond, and wherein X does not comprise an ether group.
129. The compound of any of embodiments 3 to 114, wherein the conjugate group comprises the following structure: 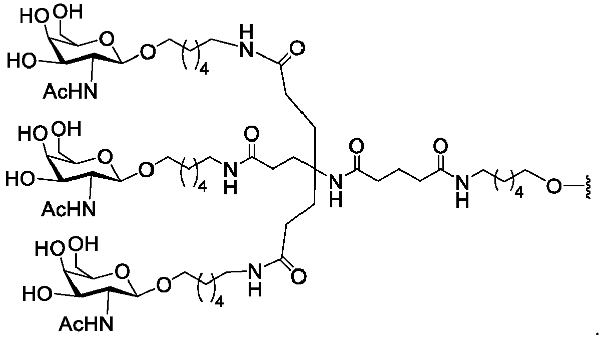
130. The compound of any of embodiments 3 to 114, wherein the conjugate group comprises a cell-targeting moiety having the following structure: 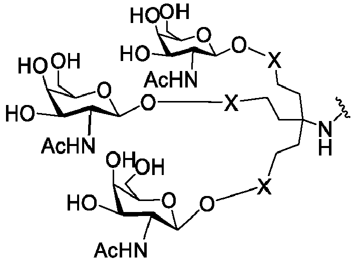 wherein X is a substituted or unsubstituted tether of four to thirteen consecutively bonded atoms and wherein the tether consists of an amide bond and a substituted or unsubstituted C₂-C₁₁ alkyl group.
131. The compound of any of embodiments 3 to 114, wherein the conjugate group comprises a cell-targeting moiety having the following structure: 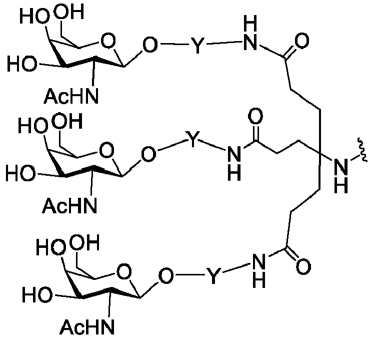 wherein Y is selected from a C₁-C₁₂ substituted or unsubstituted alkyl, alkenyl, or alkynyl group, or a group comprising an ether, a ketone, an amide, an ester, a carbamate, an amine, a piperidine, a phosphate, a phosphodiester, a phosphorothioate, a triazole, a pyrrolidine, a disulfide, or a thioether.
132. The compound of any of embodiments 3 to 114, wherein the conjugate group comprises a cell-targeting moiety having the following structure: 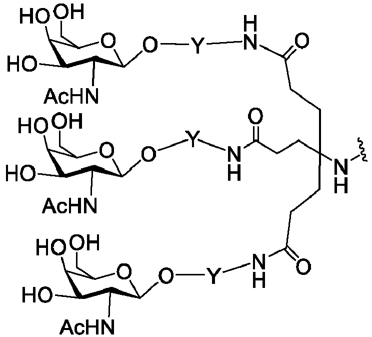 wherein Y is selected from a C₁-C₁₂ substituted or unsubstituted alkyl group, or a group comprising an ether, an amine, a piperidine, a phosphate, a phosphodiester, or a phosphorothioate.
133. The compound of any of embodiments 3 to 114, wherein the conjugate group comprises a cell-targeting moiety having the following structure: 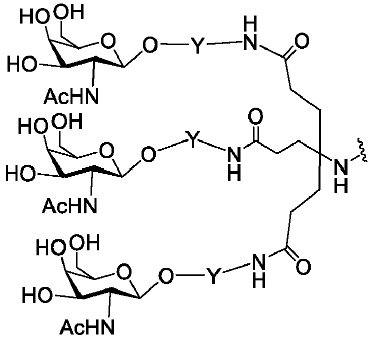 wherein Y is selected from a C₁-C₁₂ substituted or unsubstituted alkyl group.
134. The compound of any of embodiments 3 to 114, wherein the conjugate group comprises a cell-targeting moiety having the following structure: 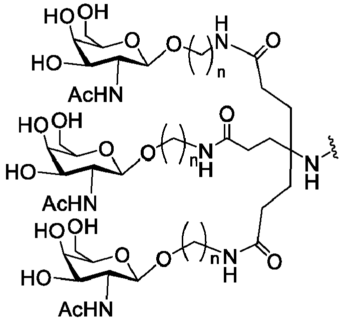 Wherein n is 1, 2, 3, 4, 5, 6, 7, 8, 9, 10, 11, or 12.
135. The compound of any of embodiments 3 to 114, wherein the conjugate group comprises a cell-targeting moiety having the following structure: 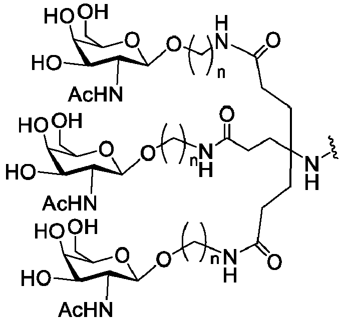 wherein n is 4, 5, 6, 7, or 8.
136. The compound of any of embodiments 3 to 114, wherein the conjugate group comprises a cell-targeting moiety having the following structure: 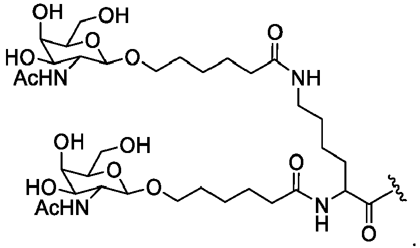
137. The compound of any of embodiments 3 to 114, wherein the conjugate group comprises a cell-targeting moiety having the following structure: 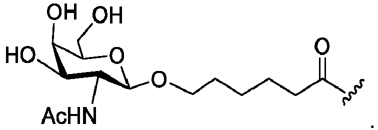
138. The compound of any of embodiments 3 to 114, wherein the conjugate comprises the following structure: 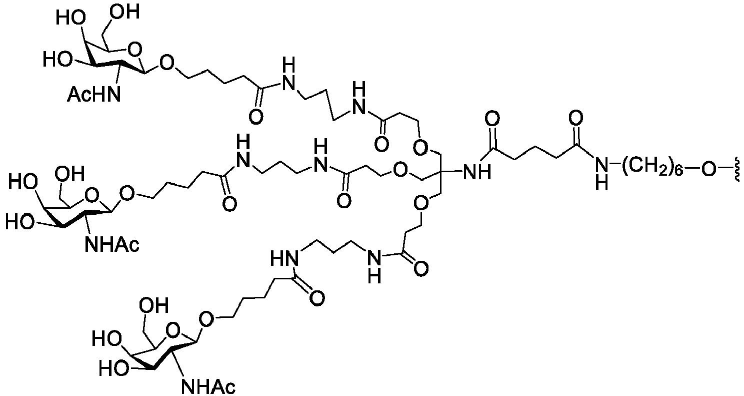
139. The compound of any of embodiments 3 to 114, wherein the conjugate comprises the following structure: 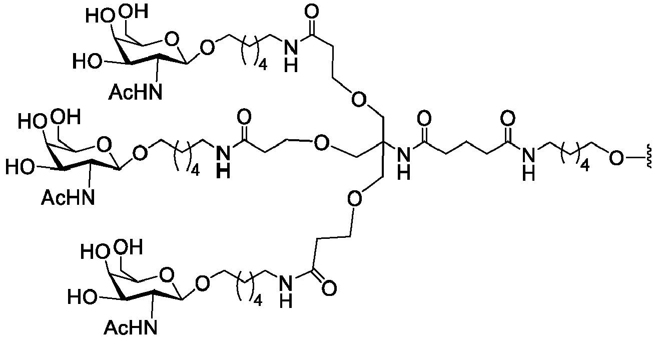
140. The compound of any of embodiments 3 to 114, wherein the conjugate comprises the following structure: 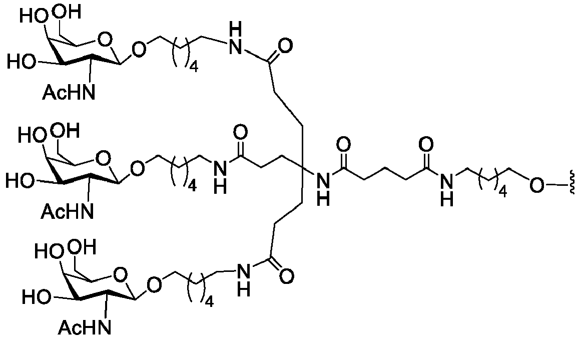
141. The compound of any of embodiments 3 to 114, wherein the conjugate comprises the following structure: 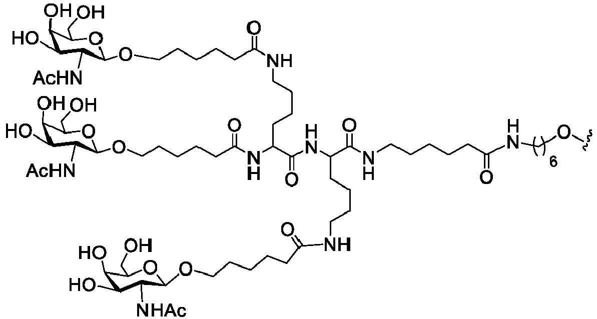
142. The compound of any of embodiments 3 to 114, wherein the conjugate comprises the following structure: 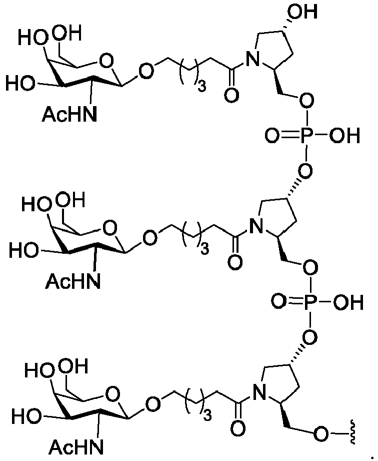
143. The compound of any of embodiments 3 to 114, wherein the conjugate comprises the following structure: 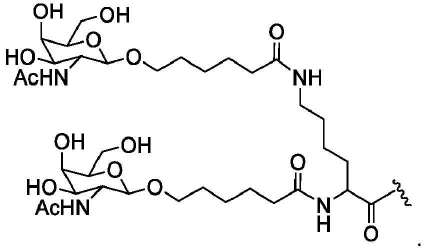
144. The compound of any of embodiments 3 to 114, wherein the conjugate comprises the following structure: 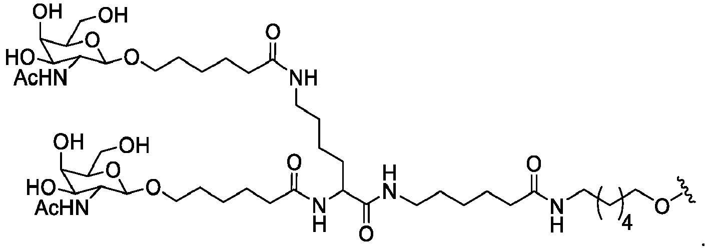
145. The compound of any of embodiments 3 to 114, wherein the conjugate comprises the following structure: 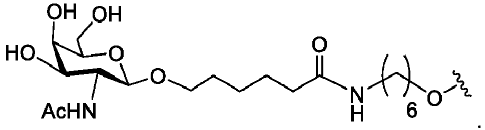
146. The compound of any of embodiments 3 to 114, wherein the conjugate comprises the following structure: 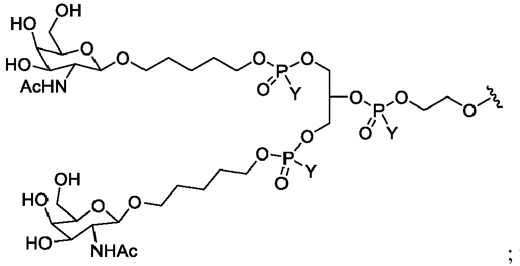 wherein Y is selected from O, S, a substituted or unsubstituted C₁-C₁₀ alkyl, amino, substituted amino, azido, alkenyl or alkynyl.
147. The compound of any of embodiments 3 to 114, wherein the conjugate comprises the following structure: 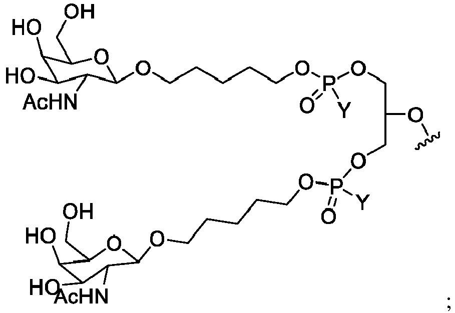 wherein Y is selected from O, S, a substituted or unsubstituted C₁-C₁₀ alkyl, amino, substituted amino, azido, alkenyl or alkynyl.
148. The compound of any of embodiments 3 to 114, wherein the conjugate comprises the following structure: 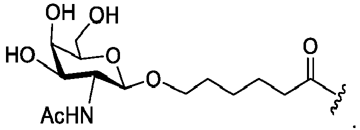
149. The compound of any of embodiments 3 to 114, wherein the conjugate group has the following structure: 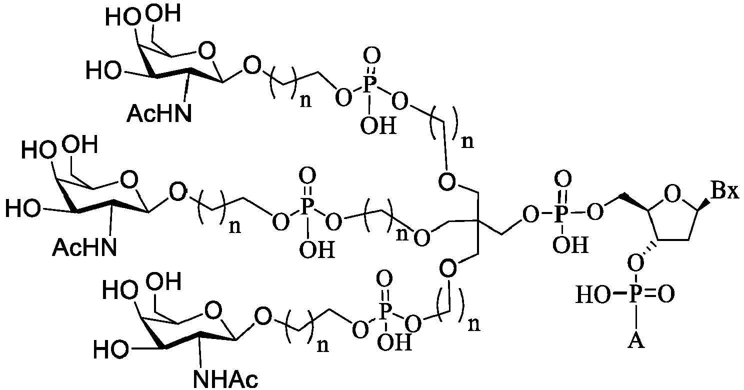
   wherein each n is, independently, from 1 to 20;
   A is the sense strand, the antisense strand, or the single-stranded oligonucleotide; and
   Bx is a heterocyclic base moiety.
150. The compound of any of embodiments 3 to 114, wherein the conjugate group has the following structure: 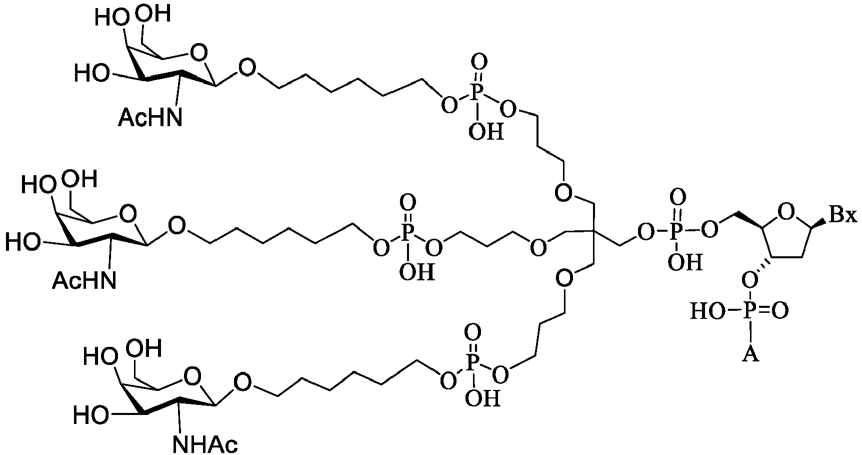
   wherein each n is, independently, from 1 to 20;
   A is the sense strand, the antisense strand, or the single-stranded oligonucleotide; and
   Bx is a heterocyclic base moiety.
151. The compound of any of embodiments 3 to 114, wherein the conjugate group has the following structure: 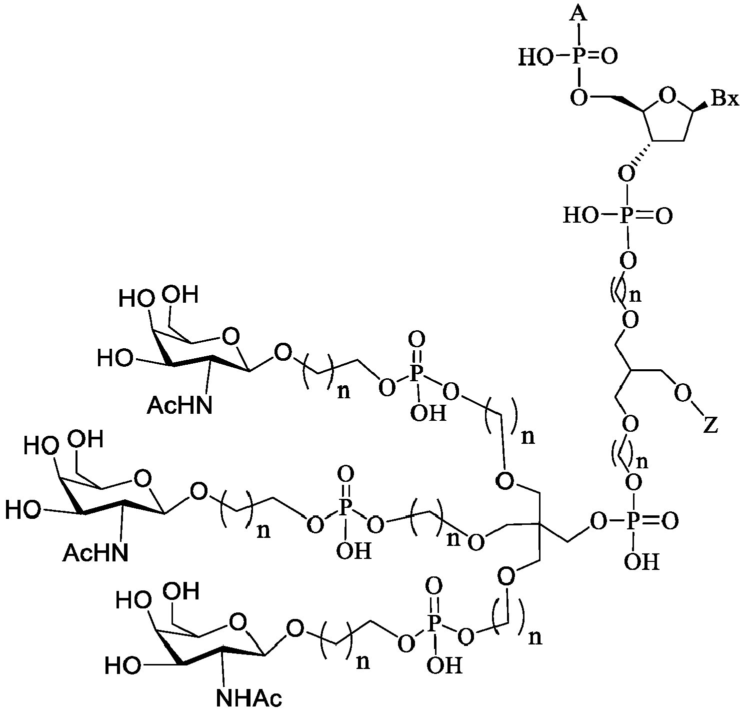
   wherein each n is, independently, from 1 to 20;
   A is the sense strand, the antisense strand, or the single-stranded oligonucleotide;
   Z is H or a linked solid support; and
   Bx is a heterocyclic base moiety.
152. The compound of any of embodiments 3 to 114, wherein the conjugate group has the following structure: 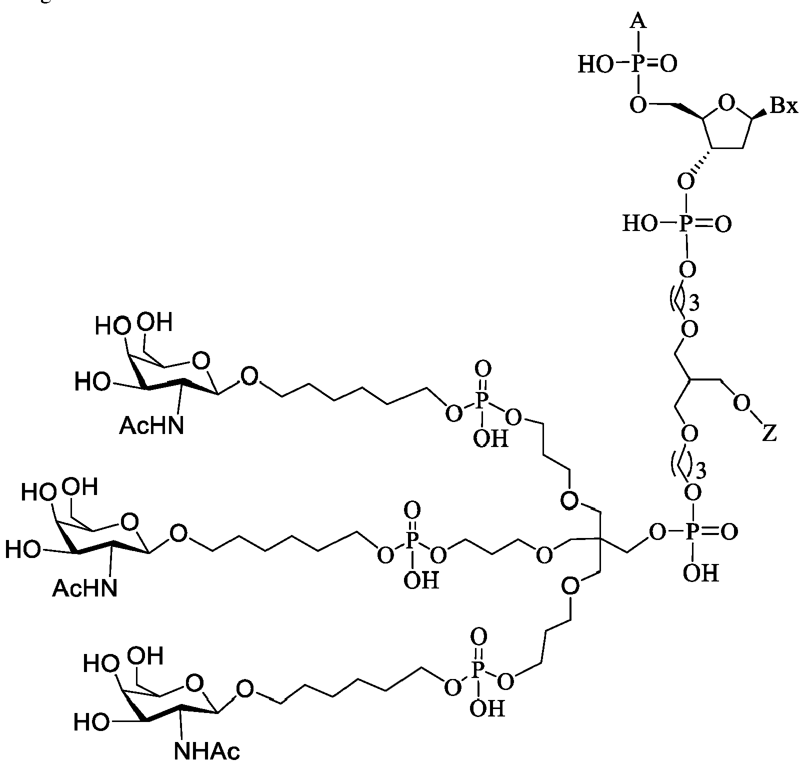
   wherein each n is, independently, from 1 to 20;
   A is the sense strand, the antisense strand, or the single-stranded oligonucleotide;
   Z is H or a linked solid support; and
   Bx is a heterocyclic base moiety.
153. The compound of any of embodiments 3 to 114, wherein the conjugate group has the following structure: 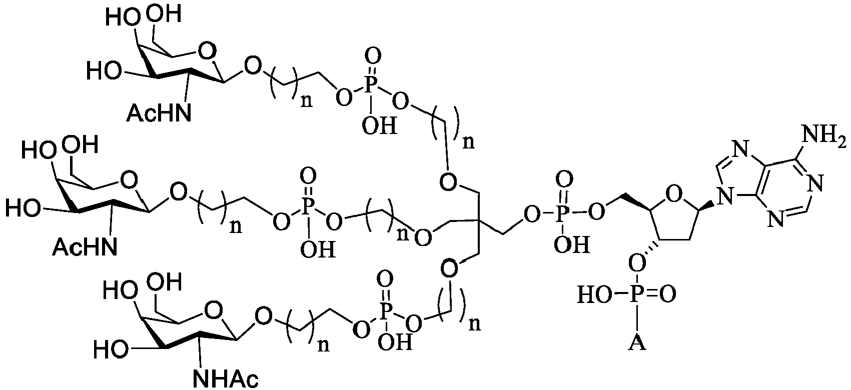 wherein A is the sense strand, the antisense strand, or the single-stranded oligonucleotide.
154. The compound of any of embodiments 3 to 114, wherein the conjugate group has the following structure: 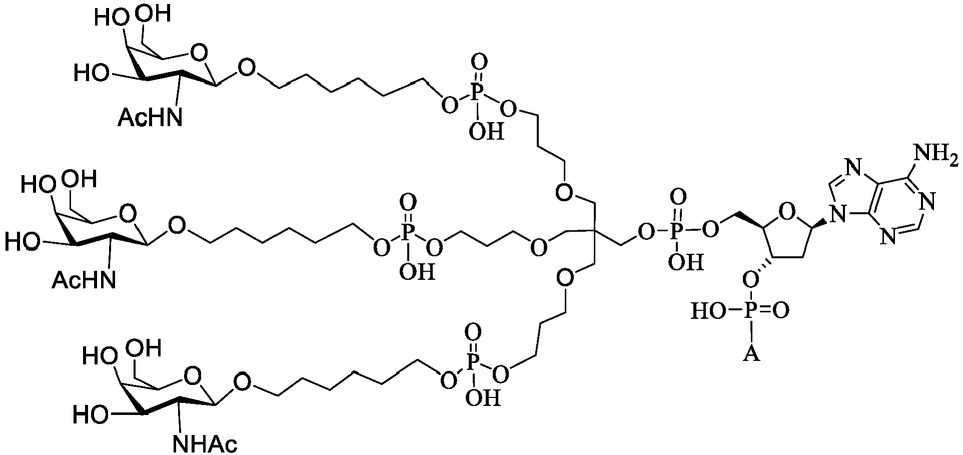 wherein A is the sense strand, the antisense strand, or the single-stranded oligonucleotide.
155. The compound of any of embodiments 3 to 114, wherein the conjugate group has the following structure: 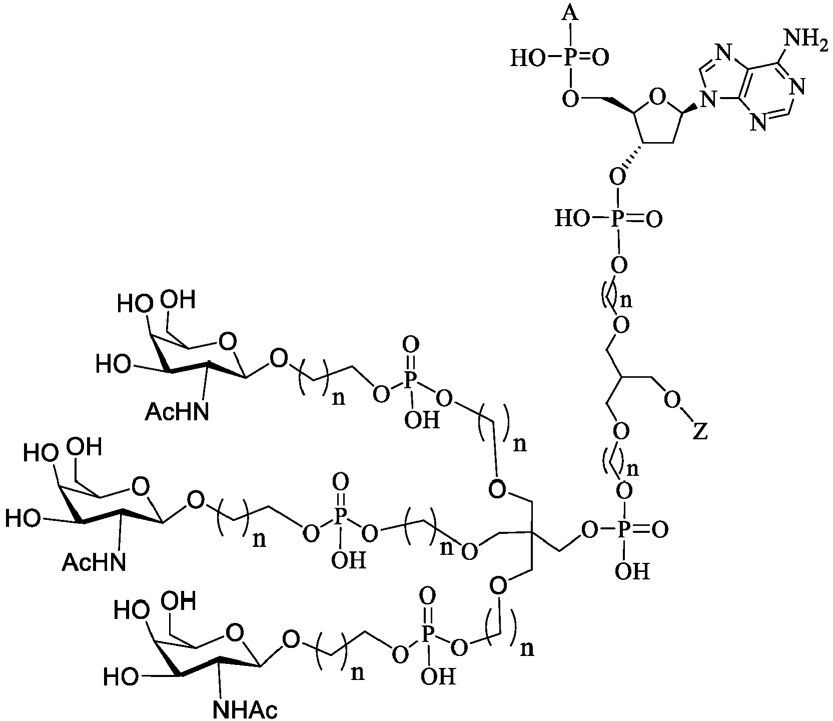
   wherein A is the sense strand, the antisense strand, or the single-stranded oligonucleotide; and
   Z is H or a linked solid support.
156. The compound of any of embodiments 3 to 114, wherein the conjugate group has the following structure: 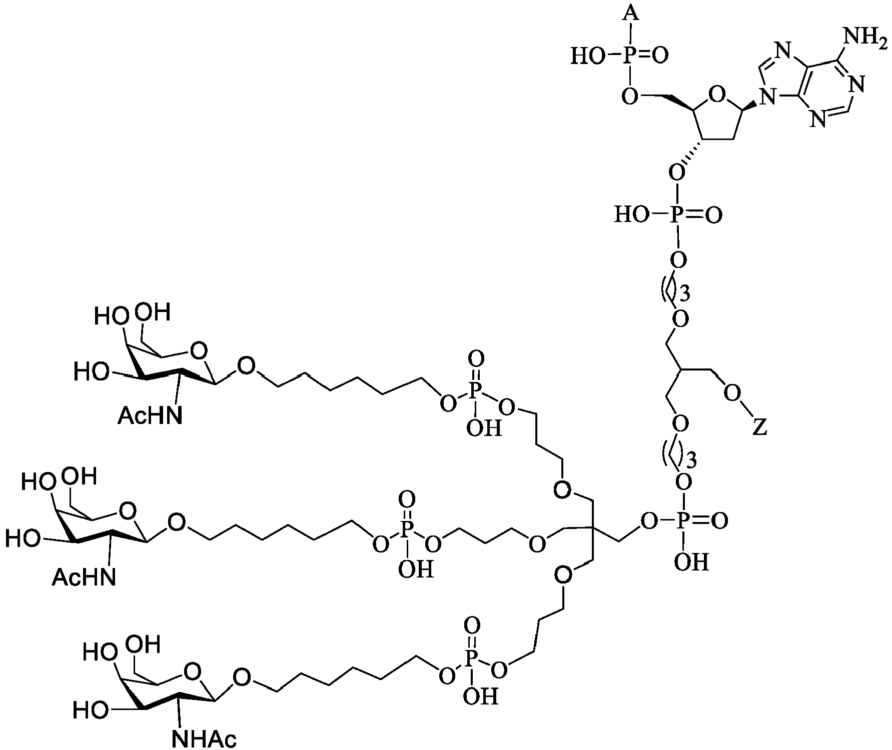
   wherein A is the sense strand, the antisense strand, or the single-stranded oligonucleotide; and
   Z is H or a linked solid support.
157. The compound of any of embodiments 3 to 114, wherein the conjugate group has the following structure: 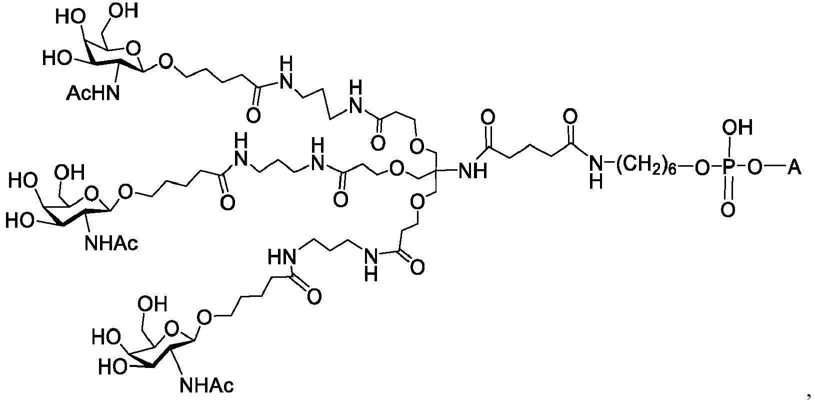 and wherein A is the sense strand, the antisense strand, or the single-stranded oligonucleotide.
158. The compound of any of embodiments 3 to 114, wherein the conjugate group has the following structure: 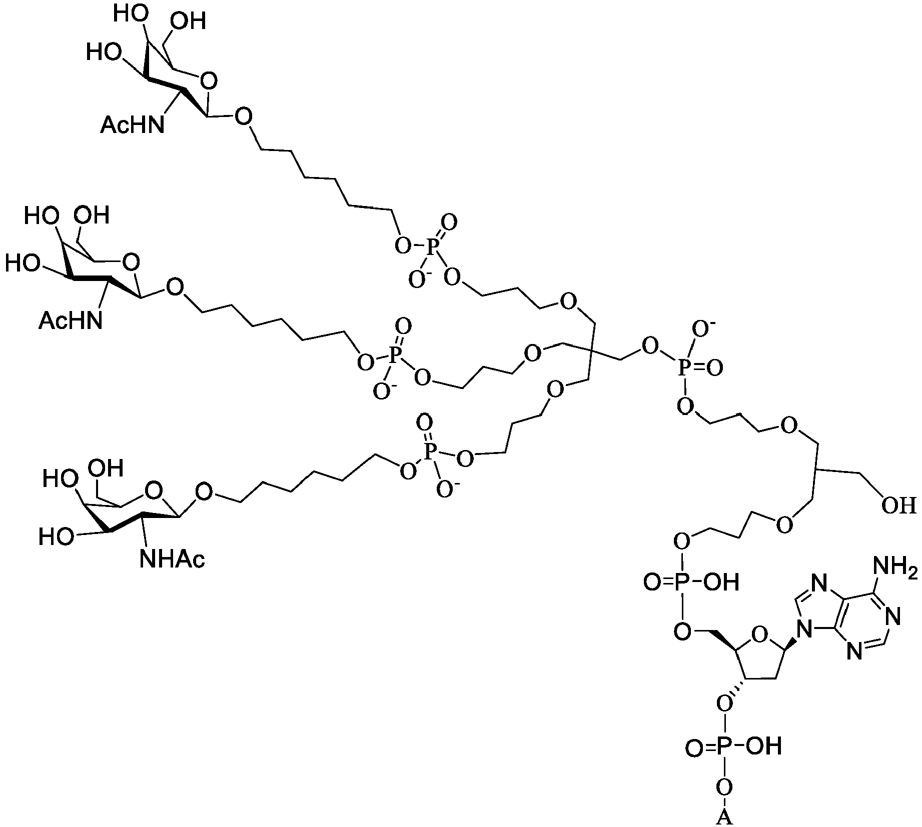 , and wherein A is the sense strand, the antisense strand, or the single-stranded oligonucleotide.
159. The compound of any of embodiments 3 to 114, wherein the conjugate group has the following structure: 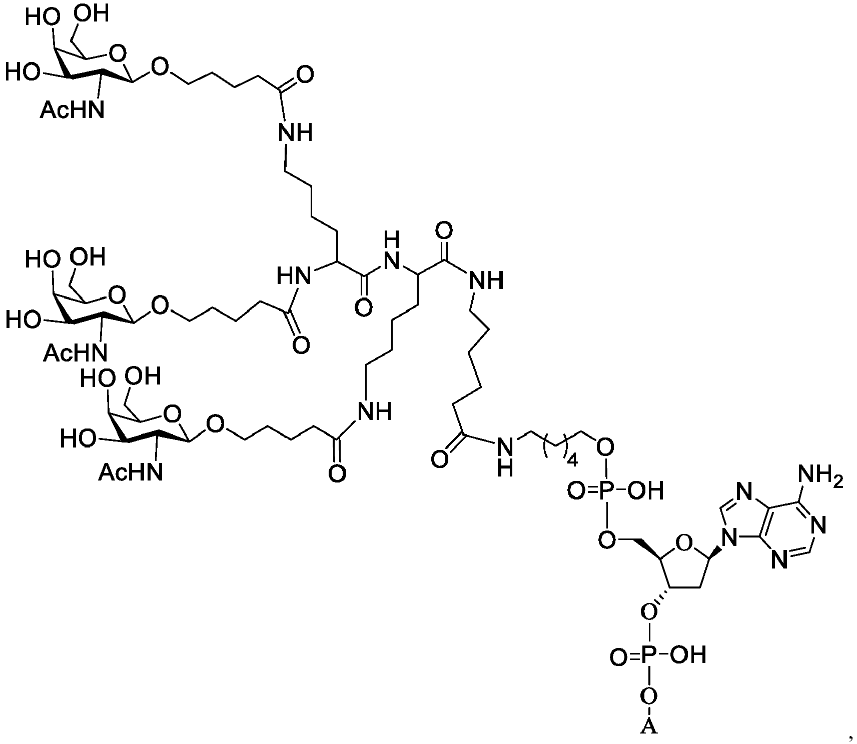 and wherein A is the sense strand, the antisense strand, or the single-stranded oligonucleotide.
160. The compound of any of embodiments 3 to 114, wherein the conjugate group has the following structure: 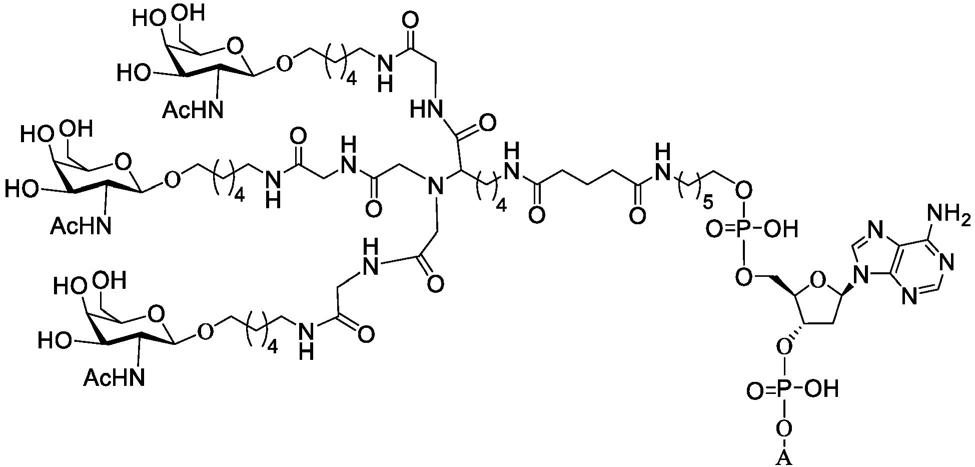 , and wherein A is the sense strand, the antisense strand, or the single-stranded oligonucleotide.
161. The compound of any of embodiments 3 to 114, wherein the conjugate group has the following structure: 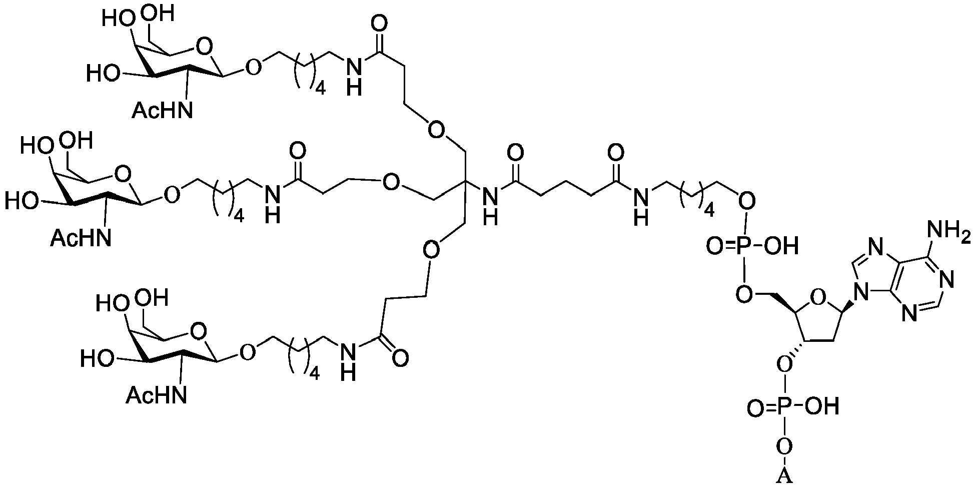 , and wherein A is the sense strand, the antisense strand, or the single-stranded oligonucleotide.
162. The compound of any of embodiments 3 to 114, wherein the conjugate group has the following structure: 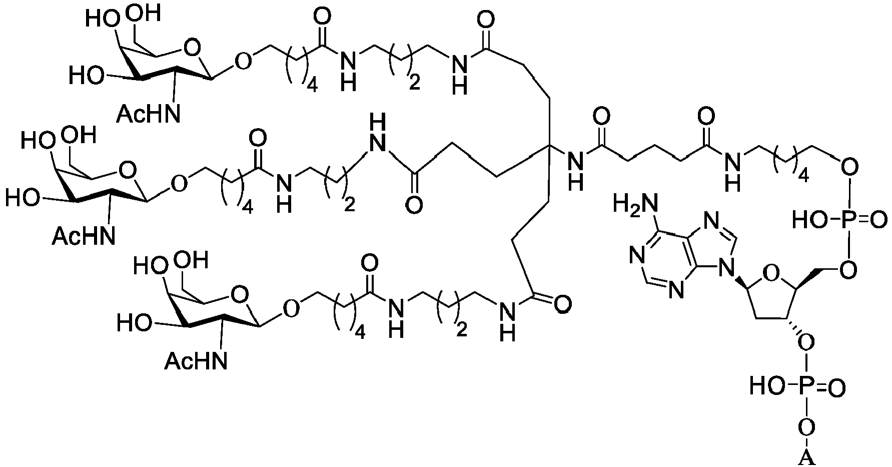 , and wherein A is the sense strand, the antisense strand, or the single-stranded oligonucleotide.
163. The compound of any of embodiments 3 to 114, wherein the conjugate group has the following structure: 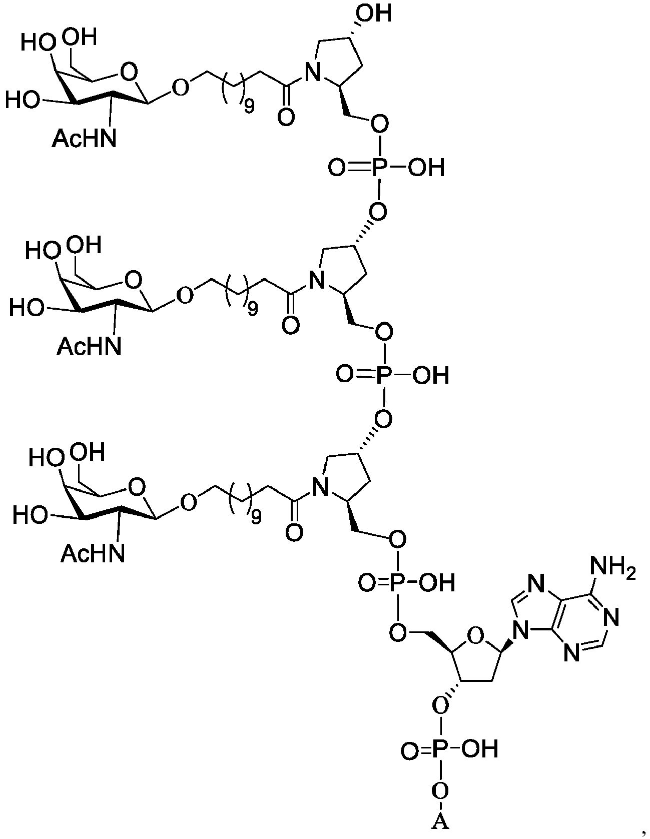 and wherein A is the sense strand, the antisense strand, or the single-stranded oligonucleotide.
164. The compound of any of embodiments 3 to 114, wherein the conjugate group has the following structure: 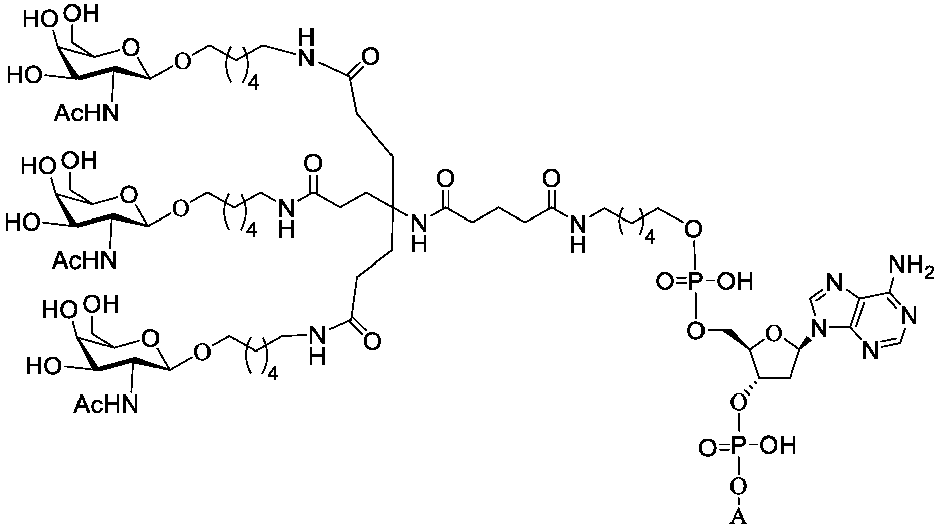 , and wherein A is the sense strand, the antisense strand, or the single-stranded oligonucleotide.
165. The compound of any of embodiments 3 to 114, wherein the conjugate group has the following structure: 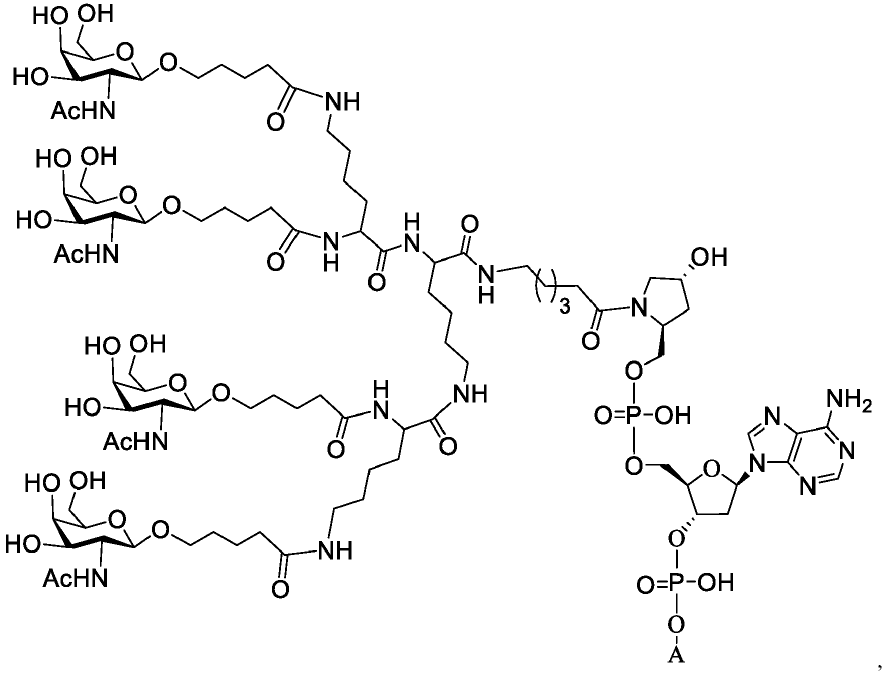 and wherein A is the sense strand, the antisense strand, or the single-stranded oligonucleotide.
166. The compound of any of embodiments 3 to 114, wherein the conjugate group has the following structure: 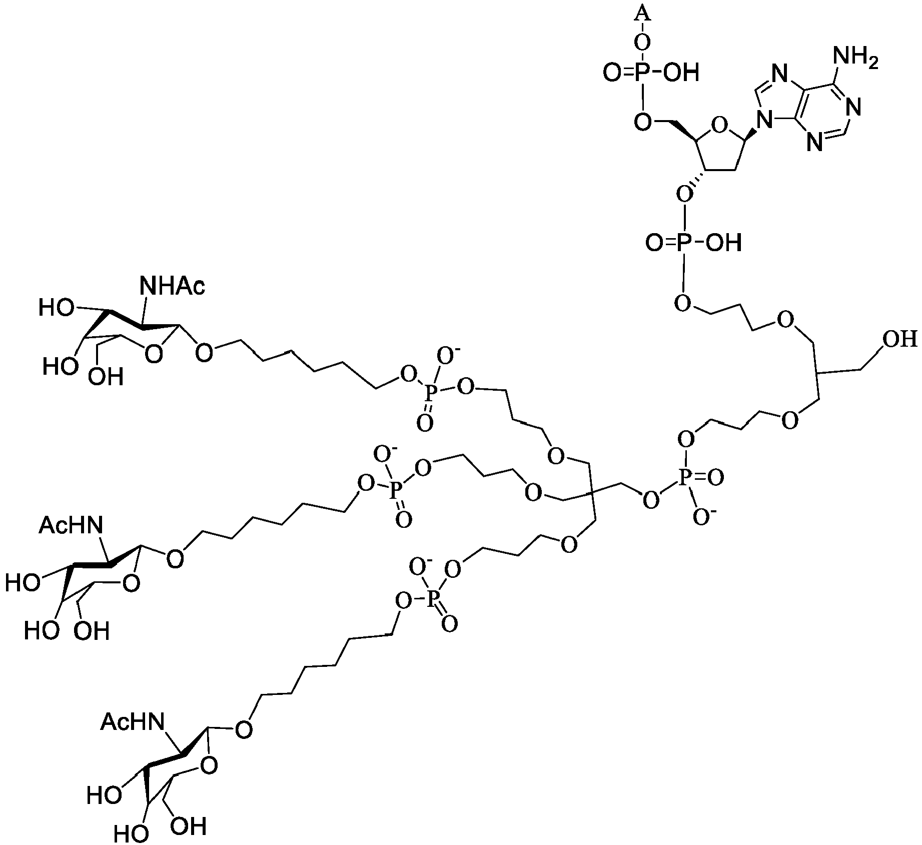 , and wherein A is the sense strand, the antisense strand, or the single-stranded oligonucleotide.
167. The compound of any of embodiments 3 to 114, wherein the conjugate group has the following structure: 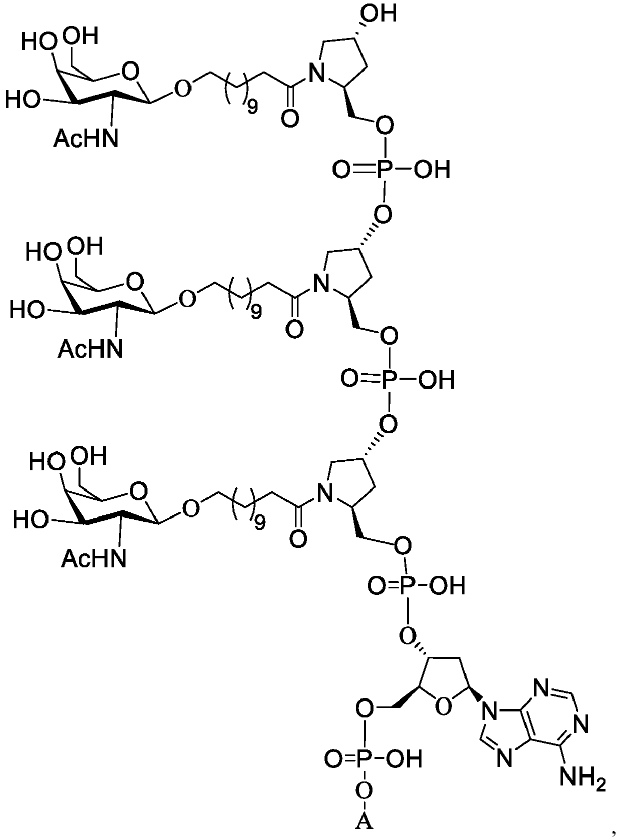 and wherein A is the sense strand, the antisense strand, or the single-stranded oligonucleotide.
168. The compound of any of embodiments 3 to 114, wherein the conjugate group has the following structure: 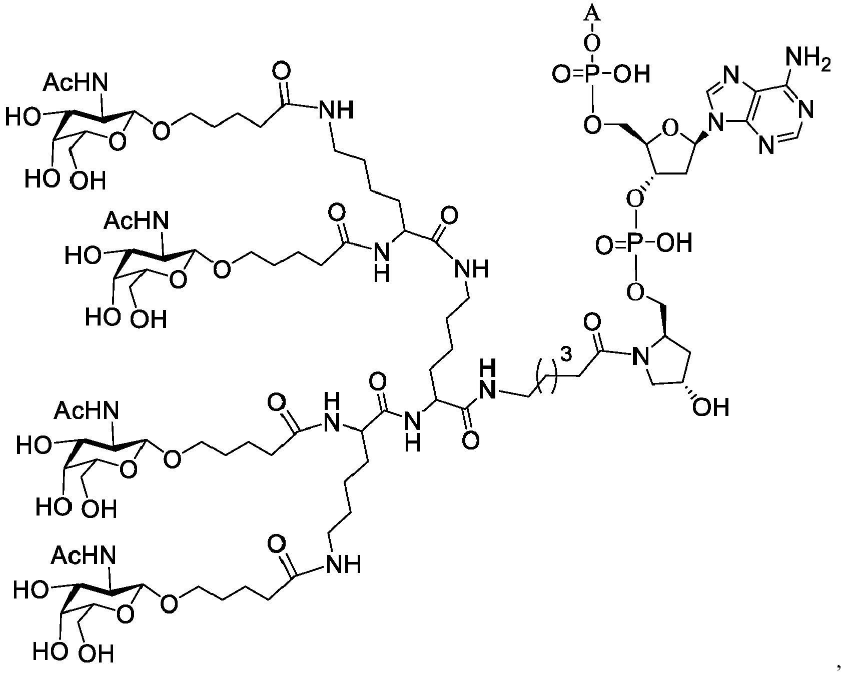 and wherein A is the sense strand of the double stranded compound, the antisense strand of the double-stranded compound, or the single-stranded oligonucleotide.
169. The compound of any of embodiments 3 to 114, wherein the conjugate of the conjugate group is selected from among: cholesterol, palmityl, stearoyl, lithocholic-oleyl, C₂₂ alkyl, C₂₀ alkyl, C₁₆ alkyl, C₁₈ alkyl, and C₁₀ alkyl.
170. The compound of embodiment 164, wherein the conjugate of the conjugate group is C₁₆ alkyl.
171. The compound of any of embodiments 3 to 114, wherein the conjugate comprises exactly one GalNAc ligand.
172. The compound of any of embodiments 3 to 114, wherein the conjugate comprises exactly two GalNAc ligands.
173. The compound of any of embodiments 3 to 114, wherein the conjugate comprises exactly three GalNAc ligands.
174. The compound of any of embodiments 3 to 148 or 169 to 173, wherein the conjugate group comprises a linker.
175. The compound of embodiment 174, wherein the linker is selected from among: hexanamide, 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C₁-C₁₀ alkyl, substituted or unsubstituted C₂-C₁₀ alkenyl, and substituted or unsubstituted C₂-C₁₀ alkynyl.
176. The compound of embodiment 175, wherein the linker is hexanamide.
177. The compound of embodiment 175, wherein the linker does not comprise pyrollidine.
178. The compound of embodiment 175, wherein the linker comprises pyrollidine.
179. A pharmaceutical composition comprising at least one compound of any of embodiments 1-178 and a pharmaceutically acceptable carrier or diluent.
180. A method of reducing the activity or amount of a nucleic acid transcript in a cell, comprising contacting a cell with at least one compound of any of embodiments 1 to 179; and thereby reducing the activity or amount of the nucleic acid transcript in the cell.
181. A method of reducing the activity or amount of an Apolipoprotein C-III transcript in a cell, comprising contacting a cell with at least one compound of any of embodiments 1 to 179; and thereby reducing the activity or amount of the Apolipoprotein C-III transcript in the cell.
182. The method of embodiment 181, wherein the Apolipoprotein C-III transcript is Apolipoprotein C-III pre-mRNA.
183. The method of embodiment 181, wherein the Apolipoprotein C-III transcript is Apolipoprotein C-III mRNA.
184. The method of any of embodiments 181 to 183, wherein the cell is in vitro.
185. The method of any of embodiments 181 to 183, wherein the cell is in an animal.
186. The method of embodiment 185, wherein the animal is a human.
187. A method of reducing the activity or amount of an Apolipoprotein C-III protein in a cell, comprising contacting a cell with at least one compound of any of embodiments 1 to 179; and thereby reducing the activity or amount of the Apolipoprotein C-III protein in the cell.
188. The method of embodiment 187, wherein the cell is in vitro.
189. The method of embodiment 187, wherein the cell is in an animal.
190. The method of embodiment 189, wherein the animal is a human.
191. A method of decreasing total cholesterol, comprising contacting a cell with at least one compound of any of embodiments 1 to 179; and thereby decreasing total cholesterol.
192. The method of embodiment 191, wherein the cell is in vitro.
193. The method of embodiment 191, wherein the cell is in an animal.
194. The method of embodiment 193, wherein the animal is a human.
195. A method of decreasing triglycerides, comprising contacting a cell with at least one compound of any of embodiments 1 to 179; and thereby decreasing triglycerides.
196. The method of embodiment 195, wherein the cell is in vitro.
197. The method of embodiment 195, wherein the cell is in an animal.
198. The method of embodiment 197, wherein the animal is a human.
199. A method of lowering LDL, comprising contacting a cell with at least one compound of any of embodiments 1 to 179; and thereby lowering LDL.
200. The method of embodiment 199, wherein the cell is in vitro.
201. The method of embodiment 199, wherein the cell is in an animal.
202. The method of embodiment 201, wherein the animal is a human.
203. A method of increasing HDL, comprising contacting a cell with at least one compound of any of embodiments 1 to 179; and thereby increasing HDL.
204. The method of embodiment 203, wherein the cell is in vitro.
205. The method of embodiment 203, wherein the cell is in an animal.
206. The method of embodiment 205, wherein the animal is a human.
207. Use of a compound of any of embodiments 1 to 178 or the pharmaceutical composition of embodiment 174 for the manufacture of a medicament for use in treatment of a disease.
208. A compound of any of embodiments 1 to 178, or the pharmaceutical composition of embodiment 174, for use in treatment of a disease.
209. A compound of any of embodiments 1 to 178, or the pharmaceutical composition of embodiment 174, for use in treatment of hypertriglyceridemia.

## Claims

1. A compound comprising an oligonucleotide having a nucleobase sequence comprising a region that is complementary to an ApoCIII transcript, and wherein the oligonucleotide comprises at least 10 contiguous nucleobases of a nucleobase sequence of SEQ ID NO:3.

2. The compound of claim 1, wherein the compound comprises at least one conjugate group.

3. The compound of claim 1 or 2, wherein the oligonucleotide comprises at least 12, at least 14, at least 16 or at least 18 contiguous nucleobases of a nucleobases sequence of SEQ ID NO:3.

4. The compound of any one of the preceding claims, wherein the oligonucleotide comprises a region that is fully complementary to the ApoCIII transcript and is at least 80% complementary to the ApoCIII transcript over the entire length of the oligonucleotide.

5. The compound of any one of the preceding claims, wherein the oligonucleotide is at least 85% complementary to the ApoCIII transcript over the entire length of the oligonucleotide.

6. The compound of any one of the preceding claims, wherein the complementary region comprises at least 12, at least 14, at least 16 or at least 18 contiguous nucleobases complementary to an equal-length portion of the ApoCIII transcript.

7. The compound of any one of the preceding claims, wherein the nucleobase sequence of the ApoCIII transcript is the nucleobase sequence as set forth in SEQ ID NO: 1 or SEQ ID NO:2.

8. The compound of any one of the preceding claims, wherein the oligonucleotide comprises one or more modified nucleotides, optionally wherein the modified nucleotide includes modified sugars, modified nucleobases and/or modified linkages.

9. The compound of claim 8, wherein:
(a) each nucleoside of the oligonucleotide is modified;
(b) the oligonucleotide comprises one or more RNA-like nucleosides, optionally wherein each nucleoside of the oligonucleotide is an RNA-like nucleoside;
(c) the modified sugar moiety is a substituted sugar moiety at the 2' position, optionally wherein the substituted sugar moiety is 2'-F, 2'-OCH₃ or 2'-O(CH₂)₂OCH₃,
(d) the oligonucleotide comprises one or more regions of alternating sugar modifications, wherein the nucleosides alternate between nucleosides having a sugar modification of a first type and nucleosides having a sugar modification of a second type, optionally wherein:
(i) the nucleosides having a sugar modification of a first type and nucleosides having a sugar modification of a second type are RNA-like nucleosides;
(ii) the alternating modifications are 2'F or 2'-OMe; and/or
(iii) the regions of alternating modifications have the pattern of (AB)ₓA_{y}, wherein:
A is 2'-OMe,
B is 2'-F,
X is 1-20; and
Y is 0 or 1,
optionally wherein X is 3-9;
(e) each linkage of the oligonucleotide is selected from phosphodiester or phosphorothioate; and/or
(f) the oligonucleotide comprises a 5'-*(E)*-vinylphosphonate group, (PO(OH)₂(CH=CH)-.

10. The compound of any one of the preceding claims, wherein the oligonucleotide consists of 13 to 30 or 21 to 30 linked nucleosides, optionally wherein the oligonucleotide consists of 21 linked nucleosides.

11. The compound of any one of claims 2-10, wherein the conjugate group comprises:
(a) one or more ligands, wherein the one or more ligands is a carbohydrate or multivalent carbohydrate cluster, optionally wherein the one or more ligands is N-acetylgalactosamine;
(b) a tether comprising the following structure: 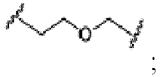 and/or
(c) a ligand having the following structure:

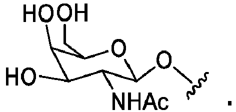

12. The compound of any one of the preceding claims, wherein the oligonucleotide is paired with a second oligonucleotide to form a double-stranded RNAi compound, optionally wherein the compound comprises a sense strand and an antisense strand, and wherein the antisense strand comprises the at least 10 contiguous nucleobases of the nucleobases sequence of SEQ ID N0:3.

13. The compound of claim 12, wherein the conjugate group is covalently attached to:
(a) the 5'-end of the sense strand; or
(b) the 3'-end of the sense strand.

14. A pharmaceutical composition comprising at least one compound of any of the preceding claims, and a pharmaceutically acceptable carrier or diluent.

15. The pharmaceutical composition of claim 14, for use in treating hypertriglyceridemia.


SEQUENCE LISTING

<110>  Ionis Pharmaceuticals, Inc.

<120>  COMPOSITIONS AND METHODS FOR MODULATION OF TARGET NUCLEIC ACIDS

<130>  CORE0116EP.D2

<150>  61/838,190
<151>  2013-06-21

<150>  61/871,683
<151>  2013-08-29

<160>  140

<170>  PatentIn version 3.5

<210>  1
<211>  3964
<212>  DNA
<213>  Homo sapiens

<400>  1
ctactccagg ctgtgttcag ggcttggggc tggtggaggg aggggcctga aattccagtg      60

tgaaaggctg agatgggccc gaggcccctg gcctatgtcc aagccatttc ccctctcacc     120

agcctctccc tggggagcca gtcagctagg aaggaatgag ggctccccag gcccaccccc     180

agttcctgag ctcatctggg ctgcagggct ggcgggacag cagcgtggac tcagtctcct     240

agggatttcc caactctccc gcccgcttgc tgcatctgga caccctgcct caggccctca     300

tctccactgg tcagcaggtg acctttgccc agcgccctgg gtcctcagtg cctgctgccc     360

tggagatgat ataaaacagg tcagaaccct cctgcctgtc tgctcagttc atccctagag     420

gcagctgctc caggtaatgc cctctgggga ggggaaagag gaggggagga ggatgaagag     480

gggcaagagg agctccctgc ccagcccagc cagcaagcct ggagaagcac ttgctagagc     540

taaggaagcc tcggagctgg acgggtgccc cccacccctc atcataacct gaagaacatg     600

gaggcccggg aggggtgtca cttgcccaaa gctacacagg gggtggggct ggaagtggct     660

ccaagtgcag gttcccccct cattcttcag gcttagggct ggaggaagcc ttagacagcc     720

cagtcctacc ccagacaggg aaactgaggc ctggagaggg ccagaaatca cccaaagaca     780

cacagcatgt tggctggact ggacggagat cagtccagac cgcaggtgcc ttgatgttca     840

gtctggtggg ttttctgctc catcccaccc acctcccttt gggcctcgat ccctcgcccc     900

tcaccagtcc cccttctgag agcccgtatt agcagggagc cggcccctac tccttctggc     960

agacccagct aaggttctac cttaggggcc acgccacctc cccagggagg ggtccagagg    1020

catggggacc tggggtgccc ctcacaggac acttccttgc aggaacagag gtgccatgca    1080

gccccgggta ctccttgttg ttgccctcct ggcgctcctg gcctctgccc gtaagcactt    1140

ggtgggactg ggctgggggc agggtggagg caacttgggg atcccagtcc caatgggtgg    1200

tcaagcagga gcccagggct cgtccagagg ccgatccacc ccactcagcc ctgctctttc 1260

ctcaggagct tcagaggccg aggatgcctc ccttctcagc ttcatgcagg gttacatgaa 1320

gcacgccacc aagaccgcca aggatgcact gagcagcgtg caggagtccc aggtggccca 1380

gcaggccagg tacacccgct ggcctccctc cccatccccc ctgccagctg cctccattcc 1440

cacccgcccc tgccctggtg agatcccaac aatggaatgg aggtgctcca gcctcccctg 1500

ggcctgtgcc tcttcagcct cctctttcct cacagggcct ttgtcaggct gctgcgggag 1560

agatgacaga gttgagactg cattcctccc aggtccctcc tttctccccg gagcagtcct 1620

agggcgtgcc gttttagccc tcatttccat tttcctttcc tttccctttc tttctctttc 1680

tatttctttc tttctttctt tctttctttc tttctttctt tctttctttc tttctttctt 1740

tctttctttc ctttctttct ttcctttctt tctttccttt ctttctttct ttcctttctt 1800

tctctttctt tctttctttc ctttttcttt ctttccctct cttcctttct ctctttcttt 1860

cttcttcttt tttttttaat ggagtctccc tctgtcacct aggctggagt gcagtggtgc 1920

catctcggct cactgcaacc tccgtctccc gggttcaacc cattctcctg cctcagcctc 1980

ccaagtagct gggattacag gcacgcgcca ccacacccag ctaatttttg tatttttagc 2040

agagatgggg tttcaccatg ttggccaggt tggtcttgaa ttcctgacct caggggatcc 2100

tcctgcctcg gcctcccaaa gtgctgggat tacaggcatg agccactgcg cctggcccca 2160

ttttcctttt ctgaaggtct ggctagagca gtggtcctca gcctttttgg caccagggac 2220

cagttttgtg gtggacaatt tttccatggg ccagcgggga tggttttggg atgaagctgt 2280

tccacctcag atcatcaggc attagattct cataaggagc cctccaccta gatccctggc 2340

atgtgcagtt cacaataggg ttcacactcc tatgagaatg taaggccact tgatctgaca 2400

ggaggcggag ctcaggcggt attgctcact cacccaccac tcacttcgtg ctgtgcagcc 2460

cggctcctaa cagtccatgg accagtacct atctatgact tgggggttgg ggacccctgg 2520

gctaggggtt tgccttggga ggccccacct gacccaattc aagcccgtga gtgcttctgc 2580

tttgttctaa gacctggggc cagtgtgagc agaagtgtgt ccttcctctc ccatcctgcc 2640

cctgcccatc agtactctcc tctcccctac tcccttctcc acctcaccct gactggcatt 2700

agctggcata gcagaggtgt tcataaacat tcttagtccc cagaaccggc tttggggtag 2760

gtgttatttt ctcactttgc agatgagaaa attgaggctc agagcgatta ggtgacctgc 2820

cccagatcac acaactaatc aatcctccaa tgactttcca aatgagaggc tgcctccctc 2880

tgtcctaccc tgctcagagc caccaggttg tgcaactcca ggcggtgctg tttgcacaga 2940

aaacaatgac agccttgacc tttcacatct ccccaccctg tcactttgtg cctcaggccc 3000

aggggcataa acatctgagg tgacctggag atggcagggt ttgacttgtg ctggggttcc 3060

tgcaaggata tctcttctcc cagggtggca gctgtggggg attcctgcct gaggtctcag 3120

ggctgtcgtc cagtgaagtt gagagggtgg tgtggtcctg actggtgtcg tccagtgggg 3180

acatgggtgt gggtcccatg gttgcctaca gaggagttct catgccctgc tctgttgctt 3240

cccctgactg atttaggggc tgggtgaccg atggcttcag ttccctgaaa gactactgga 3300

gcaccgttaa ggacaagttc tctgagttct gggatttgga ccctgaggtc agaccaactt 3360

cagccgtggc tgcctgagac ctcaataccc caagtccacc tgcctatcca tcctgcgagc 3420

tccttgggtc ctgcaatctc cagggctgcc cctgtaggtt gcttaaaagg gacagtattc 3480

tcagtgctct cctaccccac ctcatgcctg gcccccctcc aggcatgctg gcctcccaat 3540

aaagctggac aagaagctgc tatgagtggg ccgtcgcaag tgtgccatct gtgtctgggc 3600

atgggaaagg gccgaggctg ttctgtgggt gggcactgga cagactccag gtcaggcagg 3660

catggaggcc agcgctctat ccaccttctg gtagctgggc agtctctggg cctcagtttc 3720

ttcatctcta aggtaggaat caccctccgt accctgcctt ccttgacagc tttgtgcgga 3780

aggtcaaaca ggacaataag tttgctgata ctttgataaa ctgttaggtg ctgcacaaca 3840

tgacttgagt gtgtgcccca tgccagccac tatgcctggc acttaagttg tcatcagagt 3900

tgagactgtg tgtgtttact caaaactgtg gagctgacct cccctatcca ggccccctag 3960

ccct 3964

<210> 2
<211> 533
<212> DNA
<213> Homo sapiens

<400> 2
tgctcagttc atccctagag gcagctgctc caggaacaga ggtgccatgc agccccgggt 60

actccttgtt gttgccctcc tggcgctcct ggcctctgcc cgagcttcag aggccgagga 120

tgcctccctt ctcagcttca tgcagggtta catgaagcac gccaccaaga ccgccaagga 180

tgcactgagc agcgtgcagg agtcccaggt ggcccagcag gccaggggct gggtgaccga 240

tggcttcagt tccctgaaag actactggag caccgttaag gacaagttct ctgagttctg 300

ggatttggac cctgaggtca gaccaacttc agccgtggct gcctgagacc tcaatacccc 360

aagtccacct gcctatccat cctgcgagct ccttgggtcc tgcaatctcc agggctgccc 420

ctgtaggttg cttaaaaggg acagtattct cagtgctctc ctaccccacc tcatgcctgg 480

cccccctcca ggcatgctgg cctcccaata aagctggaca agaagctgct atg 533

<210> 3
<211> 21
<212> DNA
<213> Artificial sequence

<220>
<223>  Synthetic oligonucleotide

<400>  3
tcacugagaa uacuguccca a                                                21


<210>  4
<211>  16
<212>  DNA
<213>  Artificial sequence

<220>
<223>  Primer

<400>  4
gccgtggctg cctgag                                                      16


<210>  5
<211>  19
<212>  DNA
<213>  Artificial sequence

<220>
<223>  Primer

<400>  5
aggagctcgc aggatggat                                                   19


<210>  6
<211>  25
<212>  DNA
<213>  Artificial sequence

<220>
<223>  Probe

<400>  6
cctcaatacc ccaagtccac ctgcc                                            25


<210>  7
<211>  16
<212>  DNA
<213>  Artificial sequence

<220>
<223>  Synthetic oligonucleotide

<400>  7
gcactgagaa tactgt                                                      16


<210>  8
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  8
tgaauacugu cccuuuuaaa a                                                21


<210>  9
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  9
tagaauacug ucccuuuuaa a                                                21


<210>  10
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  10
tgagaauacu gucccuuuua a                                                21


<210>  11
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  11
tcugagaaua cugucccuua a                                                21

<210>  12
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  12
tugagaauac ugucccuuua a                                                21


<210>  13
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  13
tgcacugaga auacugucca a                                                21


<210>  14
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  14
tagcuucuug uccagcuuua a                                                21


<210>  15
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  15
tacugagaau acugucccua a                                                21


<210>  16
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  16
tguccagcuu uauugggaga a                                                21


<210>  17
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  17
tuguccuuaa cggugcucca a                                                21


<210>  18
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  18
taauacuguc ccuuuuaaga a                                                21

<210>  19
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  19
tucuugucca gcuuuauuga a                                                21


<210>  20
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  20
tagcagcuuc uuguccagca a                                                21


<210>  21
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  21
tuguccagcu uuauugggaa a                                                21


<210>  22
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  22
tcuuguccag cuuuauugga a                                                21


<210>  23
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  23
tuucuugucc agcuuuauua a                                                21


<210>  24
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  24
tgucuuucag ggaacugaaa a                                                21


<210>  25
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  25
tauagcagcu ucuuguccaa a                                                21

<210>  26
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  26
taacuugucc uuaacgguga a                                                21


<210>  27
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  27
tcuucuuguc cagcuuuaua a                                                21


<210>  28
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  28
tauacugucc cuuuuaagca a                                                21


<210>  29
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  29
tggccaccug ggacuccuga a                                                21


<210>  30
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  30
tccucuguuc cuggagcaga a                                                21


<210>  31
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  31
tgcagcuucu uguccagcua a                                                21


<210>  32
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  32
tgaacugaag ccaucgguca a                                                21

<210>  33
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  33
tcagcuucuu guccagcuua a                                                21


<210>  34
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  34
tuggagcagc ugccucuaga a                                                21


<210>  35
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  35
tuggccugcu gggccaccua a                                                21


<210>  36
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  36
tcuuucaggg aacugaagca a                                                21


<210>  37
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  37
tcagggaacu gaagccauca a                                                21


<210>  38
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  38
tacugaagcc aucggucaca a                                                21


<210>  39
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  39
tcauagcagc uucuugucca a                                                21

<210>  40
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  40
taguagucuu ucagggaaca a                                                21


<210>  41
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  41
tgccaucggu cacccagcca a                                                21


<210>  42
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  42
tuccagcuuu auugggagga a                                                21


<210>  43
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  43
tuuguccagc uuuauuggga a                                                21


<210>  44
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  44
tugaagccau cggucaccca a                                                21


<210>  45
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  45
tccaguaguc uuucagggaa a                                                21


<210>  46
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  46
tgcuucuugu ccagcuuuaa a                                                21

<210>  47
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  47
   tuacuguccc uuuuaagcaa a                                                21


<210>  48
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  48
tuagucuuuc agggaacuga a                                                21


<210>  49
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  49
tgguauugag gucucaggca a                                                21


<210>  50
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  50
taagccaucg gucacccaga a                                                21


<210>  51
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  51
tuguuccugg agcagcugca a                                                21


<210>  52
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  52
tuagcagcuu cuuguccaga a                                                21


<210>  53
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  53
tgggaacuga agccaucgga a                                                21

<210>  54
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  54
tuuuuaagca accuacagga a                                                21


<210>  55
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  55
tcuccaguag ucuuucagga a                                                21


<210>  56
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  56
tugcuccagu agucuuucaa a                                                21


<210>  57
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  57
tacggugcuc caguagucua a                                                21


<210>  58
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  58
   tacugucccu uuuaagcaaa a                                                21


<210>  59
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  59
tuccuuaacg gugcuccaga a                                                21


<210>  60
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  60
tggugcucca guagucuuua a                                                21

<210>  61
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  61
tuaacggugc uccaguagua a                                                21


<210>  62
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  62
tuuuaagcaa ccuacaggga a                                                21


<210>  63
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  63
tcugucccuu uuaagcaaca a                                                21


<210>  64
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  64
tuauugaggu cucaggcaga a                                                21


<210>  65
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  65
tcuuguccuu aacggugcua a                                                21


<210>  66
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  66
tcuuaacggu gcuccaguaa a                                                21


<210>  67
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  67
tucccuuuua agcaaccuaa a                                                21

<210>  68
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  68
tcccuuuuaa gcaaccuaca a                                                21


<210>  69
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  69
tuccucggcc ucugaagcua a                                                21


<210>  70
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  70
tuugaggucu caggcagcca a                                                21


<210>  71
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  71
tgucccuuuu aagcaaccua a                                                21


<210>  72
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  72
tccuuuuaag caaccuacaa a                                                21


<210>  73
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  73
tugcaggacc caaggagcua a                                                21


<210>  74
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  74
tcuuuuaagc aaccuacaga a                                                21

<210>  75
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  75
tgagcagcug ccucuaggga a                                                21


<210>  76
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  76
tagcugccuc uagggaugaa a                                                21


<210>  77
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  77
tcugccucua gggaugaaca a                                                21


<210>  78
<211>  21
<212>  DNA
<213>  Artificial sequence

<220>
<223>  Synthetic oligonucleotide

<220>
<221>  misc_feature
<222>  (2)..(21)
<223>  bases at these positions are RNA

<400>  78
tgcagcugcc ucuagggaua a                                                21


<210>  79
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  79
   tugucccuuu uaagcaacca a                                               21


<210>  80
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  80
tcauccuugg cggucuugga a                                                21


<210>  81
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  81
tuccuuggcg gucuugguga a                                                21

<210>  82
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  82
tucagugcau ccuuggcgga a                                                21


<210>  83
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  83
tagugcaucc uuggcgguca a                                                21


<210>  84
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  84
tcuuggcggu cuuggugcca a                                                21


<210>  85
<211>  20
<212>  DNA
<213>  Artificial sequence

<220>
<223>  Synthetic oligonucleotide

<400>  85
ctttccgttg gacccctggg                                                   20


<210>  86
<211>  20
<212>  DNA
<213>  Artificial sequence

<220>
<223>  Synthetic oligonucleotide

<400>  86
gtgcgcgcga gcccgaaatc                                                   20


<210>  87
<211>  19
<212>  RNA
<213>  Artificial sequence

<220>
<223>  Synthetic oligonucleotide

<400>  87
ucccugaaag acuacugga                                                    19


<210>  88
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  88
ugggugaccg auggcuucat t                                                 21


<210>  89
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  89
gauggcuuca guucccugat t                                                 21

<210>  90
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  90
ugcagccccg gguacuccut t                                                21


<210>  91
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  91
gcagccccgg guacuccuut t                                                21


<210>  92
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  92
ccgauggcuu caguucccut t                                                21


<210>  93
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  93
auggcuucag uucccugaat t                                                21


<210>  94
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  94
uggcuucagu ucccugaaat t                                                21


<210>  95
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  95
cugaaagacu acuggagcat t                                                21


<210>  96
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  96
agcaccguua aggacaagut t                                                21

<210>  97
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  97
gcaccguuaa ggacaaguut t                                                21


<210>  98
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  98
gcugccugag accucaauat t                                                21


<210>  99
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  99
cugagaccuc aauaccccat t                                                21


<210>  100
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  100
gcugccccug uagguugcut t                                                21


<210>  101
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  101
gcuuaaaagg gacaguauut t                                                21


<210>  102
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  102
cuggacaaga agcugcuaut t                                                21


<210>  103
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  103
cccuguaggu ugcuuaaaat t                                                21

<210>  104
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  104
caagaccgcc aaggaugcat t                                                21


<210>  105
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  105
ggugaccgau ggcuucagut t                                                21


<210>  106
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  106
gcuucaguuc ccugaaagat t                                                21


<210>  107
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  107
ccucaauacc ccaaguccat t                                                21


<210>  108
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  108
agguugcuua aaagggacat t                                                21


<210>  109
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  109
ugcuuaaaag ggacaguaut t                                                21


<210>  110
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  110
agacuacugg agcaccguut t                                                21

<210>  111
<211>  19
<212>  RNA
<213>  Artificial sequence

<220>
<223>  Synthetic oligonucleotide

<400>  111
uccaguaguc uuucaggga                                                    19


<210>  112
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  112
ugaagccauc ggucacccat t                                                 21


<210>  113
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  113
ucagggaacu gaagccauct t                                                 21


<210>  114
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  114
aggaguaccc ggggcugcat t                                                21

<210>  115
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  115
aaggaguacc cggggcugct t                                                21

<210>  116
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  116
agggaacuga agccaucggt t                                                21

<210>  117
<211>  21
<212>  DNA
<213>  Artificial sequence

<220>
<223>  Synthetic oligonucleotide

<220>
<221>  misc_feature
<222>  (1)..(19)
<223>  bases at these positions are RNA

<400>  117
uucagggaac ugaagccaut t                                                21

<210>  118
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  118
uuucagggaa cugaagccat t                                                21


<210>  119
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  119
ugcuccagua gucuuucagt t                                                21


<210>  120
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  120
acuuguccuu aacggugcut t                                                21


<210>  121
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  121
aacuuguccu uaacggugct t                                                 21


<210>  122
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  122
uauugagguc ucaggcagct t                                                 21


<210>  123
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  123
ugggguauug aggucucagt t                                                 21


<210>  124
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  124
agcaaccuac aggggcagct t                                                 21


<210>  125
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  125
aauacugucc cuuuuaagct t                                                21


<210>  126
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  126
auagcagcuu cuuguccagt t                                                21


<210>  127
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  127
uuuuaagcaa ccuacagggt t                                                21


<210>  128
<211>  21
<212>  DNA
<213>  Artificial sequence

<220>
<223>  Synthetic oligonucleotide


<220>
<221>  misc_feature
<222>  (2)..(21)
<223>  bases at these positions are RNA

<400>  128
ugcauccuug gcggucuugt t                                                21


<210>  129
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  129
acugaagcca ucggucacct t                                                21


<210>  130
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  130
ucuuucaggg aacugaagct t                                                21


<210>  131
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  131
uggacuuggg guauugaggt t                                                21


<210>  132
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  132
ugucccuuuu aagcaaccut t                                                21


<210>  133
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  133
auacuguccc uuuuaagcat t                                                21


<210>  134
<211>  21
<212>  DNA
<213>  Artificial sequence

<220>
<223>  Synthetic oligonucleotide


<220>
<221>  misc_feature
<222>  (1)..(19)
<223>  bases at these positions are RNA

<400>  134
aacggugcuc caguagucut t                                                21


<210>  135
<211>  20
<212>  DNA
<213>  Artificial sequence

<220>
<223>  Synthetic oligonucleotide

<400>  135
ctgctagcct ctggatttga                                                  20


<210>  136
<211>  21

<212>  DNA
<213>  Artificial sequence

<220>
<223>  Synthetic oligonucleotide


<220>
<221>  misc_feature
<222>  (2)..(21)
<223>  bases at these positions are RNA

<400>  136
tuaucuauaa ugaucaggua a                                                21


<210>  137
<211>  23
<212>  DNA
<213>  Artificial sequence

<220>
<223>  Synthetic oligonucleotide


<220>
<221>  misc_feature
<222>  (2)..(22)
<223>  bases at these positions are RNA

<400>  137
tuaucuauaa ugaucaggua aat                                              23


<210>  138
<211>  20
<212>  DNA
<213>  Artificial sequence

<220>
<223>  Synthetic oligonucleotide

<400>  138
agcttcttgt ccagctttat                                                  20


<210>  139
<211>  21
<212>  DNA
<213>  Artificial sequence

<220>
<223>  Synthetic oligonucleotide

<400>  139
tcactgagaa tactgtccca a                                                21


<210>  140
<211>  23
<212>  DNA
<213>  Artificial sequence

<220>
<223>  Synthetic oligonucleotide



<220>
<221>  misc_feature
<222>  (2)..(4)
<223>  bases at these positions are RNA

<220>
<221>  misc_feature
<222>  (6)..(10)
<223>  bases at these positions are RNA

<220>
<221>  misc_feature
<222>  (12)..(13)
<223>  bases at these positions are RNA

<220>
<221>  misc_feature
<222>  (15)..(15)
<223>  bases at these positions are RNA

<220>
<221>  misc_feature
<222>  (17)..(23)
<223>  bases at these positions are RNA

<400>  140
tcactgagaa tactgtccca aau                                              23